# EUROPEAN PATENT SPECIFICATION

(11) **EP 2 712 863 B1**
(45) Date of publication and mention of the grant of the patent: **22.10.2014**
(21) Application number: 12186521.6
(22) Date of filing: 28.09.2012
(51) Int. Cl.: C07D 231/54, C07D 401/04, C07D 403/04, C07D 403/12, C07D 405/04, C07D 409/04, C07D 401/06, C07D 401/12, C07D 401/14, C07D 405/06, C07D 405/14, C07D 413/04, C07D 417/04, C07D 417/12, A61K 31/416, A61P 31/00

(54) **Novel inhibitors of bacterial biofilms and related methods**
Neue Hemmer bakterieller Biofilme und zugehörige Verfahren
Nouveaux inhibiteurs de biofilms bactériens et procédés associés

(43) Date of publication of application: 02.04.2014
(73) Proprietor: Sequoia Sciences, Inc., St. Louis MO 63114 (US)
(72) Inventor: Eldridge, Gary R., Saint Louis, MO Missouri 63119 (US); Buckle, Ronald Neil, Delmar, NY New York 12054 (US); Ellis, Michael, Clifton Park, NY New York 12065 (US); Huang, Zhongping, Voorheesville, NY New York 12186 (US); Reilly, John Edward, Voorheesville, NY New York 12186 (US)
(74) Representative: De Groote, Christophe

(56) References cited:
- WO-A1-2006/031943
- WO-A2-2006/010147
- DATABASE CA [Online] CHEMICAL ABSTRACTS SERVICE, COLUMBUS, OHIO, US; 2010, TANG, JIE ET AL: "Maslinic acid derivatives, preparation method and medical application", XP002688759, retrieved from STN Database accession no. 2010:36239 & CN 101 619 088 A (SHANGHAI PUYI BIOTECHNOLOGY CO., LTD., PEOP. REP. CHINA; SHANGHAI INST) 6 January 2010 (2010-01-06)
- QIU W W ET AL: "Synthesis and biological evaluation of heterocyclic ring-substituted maslinic acid derivatives as novel inhibitors of protein tyrosine phosphatase 1B", BIOORGANIC & MEDICINAL CHEMISTRY LETTERS, PERGAMON, ELSEVIER SCIENCE, GB, vol. 19, no. 23, 1 December 2009 (2009-12-01), pages 6618-6622, XP026736069, ISSN: 0960-894X, DOI: 10.1016/J.BMCL.2009.10.017 [retrieved on 2009-10-08]
- LI J F ET AL: "Synthesis and evaluation of a novel series of heterocyclic oleanolic acid derivatives with anti-osteoclast formation activity", EUROPEAN JOURNAL OF MEDICINAL CHEMISTRY, EDITIONS SCIENTIFIQUE ELSEVIER, PARIS, FR, vol. 44, no. 7, 1 July 2009 (2009-07-01), pages 2796-2806, XP026087783, ISSN: 0223-5234, DOI: 10.1016/J.EJMECH.2008.12.024 [retrieved on 2008-12-31]
- ZHONGJIE LIANG ET AL: "Identification of pentacyclic triterpenes derivatives as potent inhibitors against glycogen phosphorylase based on 3D-QSAR studies", EUROPEAN JOURNAL OF MEDICINAL CHEMISTRY, EDITIONS SCIENTIFIQUE ELSEVIER, PARIS, FR, vol. 46, no. 6, 21 February 2011 (2011-02-21), pages 2011-2021, XP028199986, ISSN: 0223-5234, DOI: 10.1016/J.EJMECH.2011.02.053 [retrieved on 2011-02-27]
- WEN, XIAOAN ET AL: "Synthesis and biological activity of heterocycle-fused derivatives of pentacyclic triterpenes as glycogen phosphorylase inhibitors", JOURNAL OF CHINA PHARMACEUTICAL UNIVERSITY, vol. 40, no. 6, 27 September 2009 (2009-09-27), pages 491-496, XP009165436,

## Description

### TECHNICAL FIELD

The present invention generally relates to compounds and compositions useful for reducing or inhibiting the growth of a biofilm. The present invention also relates to compounds useful for reducing or inhibiting the formation of a biofilm and for controlling or treating a chronic bacterial infection involving biofilms.

### Description of Related Art

Bacterial biofilms exist in natural, medical, and engineering environments. The biofilms offer a selective advantage to a microorganism to ensure its survival, or allow it a certain amount of time to exist in a dormant state until growth conditions arise. Unfortunately, this selective advantage poses serious threats to animal health, especially human health.

Chronic infections involving biofilms are serious medical problems throughout the world. For example, biofilms are involved in 65% of human bacterial infections. Biofilms are involved in prostatitis, biliary tract infections, urinary tract infections, cystitis, lung infections, sinus infections, ear infections, acne, rosacea, dental caries, periodontitis, nosocomial infections, open wounds, and chronic wounds.

Compounds that modify biofilm formation would have a substantial medical impact by treating many chronic infections, reducing catheter- and medical device-related infections, and treating lung and ear infections. The potential market for biofilm inhibitors could be enormous given the sheer number of cases in which biofilms contribute to medical problems. The inhibitors may be used to cure, treat, or prevent a variety of conditions, such as, but are not limited to, arterial damage, gastritis, urinary tract infections, pyelonephritis, cystitis, otitis media, otitis externa, leprosy, tuberculosis, benign prostatic hyperplasia, chronic prostatitis, chronic lung infections of humans with cystic fibrosis, osteomyelitis, bloodstream infections, skin infections, open or chronic wound infections, cirrhosis, and any other acute or chronic infection that involves or possesses a biofilm.

In the United States, the market for antibiotics is greater than $10 billion. The antibiotic market is fueled by the continued increase in resistance to conventional antibiotics. Approximately 70% of bacteria found in hospitals resist at least one of the most commonly prescribed antibiotics. Because biofilms appear to reduce or prevent the efficacy of antibiotics, co-administration of biofilm inhibitors could significantly boost the antibiotic market.

Using the protection of biofilms, microbes can resist antibiotics at a concentration ranging from 1 to 1.5 thousand times higher than the amount used in conventional antibiotic therapy. During an infection, bacteria surrounded by biofilms are rarely resolved by the immune defense mechanisms of the host. It has been proposed that in a chronic infection, a biofilm gives bacteria a selective advantage by reducing the penetration of an antibiotic into the depths of the tissue needed to completely eradicate the bacteria's existence (Costerton, J. W. et al., Science. 1999 May 21; 284(5418):1318-22).

Traditionally, antibiotics are discovered using the susceptibility test methods established by the Clinical Laboratory and Standards Institute (CLSI). The methods identify compounds that specifically affect growth or death of bacteria. These methods involve inoculation of a bacterial species into a growth medium, followed by the addition of a test compound, and then plot of the bacterial growth over a time period post-incubation. Unfortunately these antibiotics derived from the CLSI methods would not be effective therapeutics against chronic infections involving biofilms because the methods do not test compounds against bacteria in a biofilm. Consistently, numerous publications have reported a difference in gene transcription in bacteria living in biofilms from bacteria in suspension, which further explains the failure of conventional antibiotics to eradicate biofilm infections (Sauer, K. et al. J. Bacteriol. 2001,183: 6579-6589).

Biofilm inhibitors can provide an alternative treatment approach for certain infections. Biofilm inhibitors, on the other hand, act on the biological mechanisms that provide bacteria protection from antibiotics and from a host's immune system. Biofilm inhibitors may be used to "clear the way" for the antibiotics to penetrate the affected cells and eradicate the infection. Traditionally, treatment of nosocomial infections requires an administration of a combination of products, such as amoxicillin/clavulanate and quinupristin/dalfopristin, or an administration of two antibiotics simultaneously. In one study of urinary catheters, rifampin was unable to eradicate methicillin-resistant Staphylococcus aureus in a biofilm but was effective against planktonic, or suspended cells (Jones, S. M., et. al., "Effect of vancomycin and rifampicin on methicillin-resistant Staphylococcus aureus biofilms", Lancet 357:40-41, 2001).

Bacteria have no known resistance to biofilm inhibitors. Biofilm inhibitors are not likely to trigger growth-resistance mechanisms or affect the growth of the normal human flora. Thus, biofilm inhibitors could potentially extend the product life of antibiotics. *Pseudomonas aeruginosa* in the lungs of cystic fibrosis (CF) patients is resistant to high doses of antibiotics because it forms biofilms. Biofilms are complex, heterogeneous communities of bacterial cells encased in extrapolymeric substances (EPS). These EPS are composed of polysaccharides, proteins, and extracellular DNA. EPS provide frameworks for communities of bacteria to exist and enhance attachment to themselves and surfaces. Bacteria within biofilms differentiate into stratified communities of phenotypically diverse cells that offer competitive advantages for different environmental conditions. One of these advantages is increased tolerance to antibiotics, which enables bacteria like *P. aeruginosa* to persist in chronic infections despite antibiotic therapy. While residing within these EPS, one hypothesis is that *P*. *aeruginosa* reduces its metabolism, which prevents its death from antibiotics. As antibiotic concentrations are reduced, specific populations of *P. aeruginosa* within the EPS increase their metabolism and spread. This cycle continuously repeats, enabling the spread of *P. aeruginosa* bacteria within the lungs of CF patients.

Chronic wound infection represents another illness that is difficult to eradicate. Examples of the most common types of chronic wounds are diabetic foot ulcers, venous leg ulcers, arterial leg ulcers, and pressure ulcers. Diabetic foot ulcers appear to be the most prevalent. These wounds are typically colonized by multiple species of bacteria including *Staphylococcus* spp., *Streptococcus* spp., *Pseudomonas* spp. and Gram-negative bacilli (Lipsky, B. Medical Treatment of Diabetic Foot Infections. Clin. Infect. Dis. 2004, 39, p.S104-14).

Based on clinical evidence, microorganisms cause or contribute to chronic wound infections. Only recently have biofilms been implicated in these infections (Harrison-Balestra, C. et al. A Wound-isolated Pseudomonas aeruginosa Grow a Biofilm In Vitro Within 10 Hours and Is Visualized by Light Microscopy, Dermatol Surg 2003, 29; 631-635; Edwards, R. et al. Bacteria and wound healing. Curr Opin Infect Dis, 2004, 17; 91-96). Approximately 140,000 amputations occur each year in the United States due to chronic wound infections that could not be treated with conventional antibiotics. Unfortunately, treating these infections with high doses of antibiotics over long periods of time contributes to the development of antibiotic resistance (Howell-Jones, R. S., et al. A review of the microbiology, antibiotic usage and resistance in chronic skin wounds. J. Antimicrob. Ther. Jan. 2005). Biofilm inhibitors in a combination therapy with antibiotics may provide an effective alternative to the treatment of chronic wounds. Recent publications describe the cycles of the pathogenesis of numerous species of bacteria involving biofilms. For example, *Escherichia coli,* which cause recurrent urinary tract infections, undergo a cycle of binding to and then invading a host's bladder epithelial cells. *E. coli* form a biofilm intracellularly, modify its morphology, and then burst out of the host cells to repeat the cycle of pathogenesis (Justice, S. et al. Differentiation and development pathways of uropathogenic Escherichia coli in urinary tract pathogenesis, PNAS 2004, 101 (5): 1333-1338). The authors suggest that this repetitive cycle of pathogenesis of *E*. *coli* may explain the recurrence of the infection.

In 1997, Finlay, B. *et al.* reported that numerous bacteria, including *Staphylococci, Streptococci, Bordetella pertussis, Neisseria* spp., *Helicobactor pylori,* and *Yersinia* spp., adhere to mammalian cells during their pathogenesis. The authors hypothesized that the adherence would lead to an invasion of the host cell. Later publications confirm this hypothesis (Cossart, P. Science, 2004, 304; 242-248; see additional references infra). Other publications presented similar hypotheses to Mulvey, M. *et al.* (Mulvey, M. et al. "Induction and Evasion of Host Defenses by Type 1-Piliated Uropathogenic E. coli" Science 1998, 282 p. 1494-1497). In particular, Mulvey, M. *et al.* stated invasion of *E. coli* into epithelial cells provide protection from the host's immune response to allow a build up of a large bacterial population.

Cellular invasion and biofilm formation appear to be integral to the pathogenesis of most, if not all bacteria. *P. aeruginosa* have been shown to invade epithelial cells during lung infections (Leroy-Dudal, J. et al. Microbes and Infection, 2004, 6, p. 875-881). *P. aeruginosa* are the principal infectious organisms found in the lungs of cystic fibrosis patients, and the bacteria exist within a biofilm. Antibiotics like tobramcyin, and other current antibacterial compounds, do not provide effective treatment against biofilms of chronic infections, perhaps because antibiotic therapy fails to eradicate the biofilm.

The pathogenesis of cellular invasion and biofilm formation gram-negative bacteria follow conserved mechanisms. For example, *Haemophilus influenzae* invade epithelial cells and form biofilms (Hardy, G. et al., Methods Mol. Med., 2003, 71; 1-18; Greiner, L. et al., Infection and Immunity, 2004, 72(7); 4249-4260). *Burkholderia* spp. invade epithelial cells and form biofilm (Utaisincharoen, P. et al., Microb Pathog. 2005, 38(2-3); 107-112; Schwab, U. et al. Infection and Immunity, 2003, 71 (11); 6607-6609). *Klebsiella pneumoniae* invade epithelial cells and form biofilm (Cortes, G et al. Infection and Immunity. 2002, 70(3); 1075-1080; Lavender, hours. et al., Infection and Immunity. 2004, 72(8); 4888-4890). Salmonella spp. invade epithelial cells and form biofilms (Cossart, P. Science, 2004, 304; 242-248; Boddicker, J. et al., Mol. Microbiol. 2002, 45(5); 1255-1265). *Yersinia pestis* invade epithelial cells and form biofilms (Cossart, P. Science, 2004, 304; 242-248; Jarrett, C. et al. J. Infect. Dis., 2004, 190; 783-792). Neisseria gonorrhea invade epithelial cells and form biofilms (Edwards, J. et al., Cellular Micro., 2002, 4(9); 585-598; Greiner, L. et al., Infection and Immunity. 2004, 73(4); 1964-1970). *Burkholderia* spp. are another important class of gram-negative bacterial pathogens. *Chlamydia* spp., including *Chlamydia pneumoniae* is an intracellular, gram-negative pathogen implicated in respiratory infections and chronic diseases such as atherosclerosis and Alzheimer's disease (Little, C. S. et al., Infection and Immunity. 2005, 73(3); 1723-34).

These Gram-negative bacteria cause lung, ear, and sinus infections, gonorrhoeae, plague, diarrhea, typhoid fever, and other infectious diseases. *E*. *coli* and *P*. *aeruginosa* are two of the most widely studied Gram-negative pathogens. Researchers believe that the pathogenesis of these bacteria involves invasion of host cells and formation of biofilms. These models have enabled those skilled in the art to understand the pathogenesis of other Gram-negative bacteria. Some biofilm inhibitors are already known, for example in document WO2006/031943. But these compounds do not always exhibit a satisfactory inhibition of biofilm formation.

Accordingly, for the reasons discussed above and others, there exists an unmet need for compounds that serve as biofilm inhibitors and/or that would be useful for reducing or inhibiting the formation or growth of bacterial biofilms and bacterial infections involving biofilms.

### SUMMARY OF THE INVENTION

The present invention provides compounds of the following general chemical structure 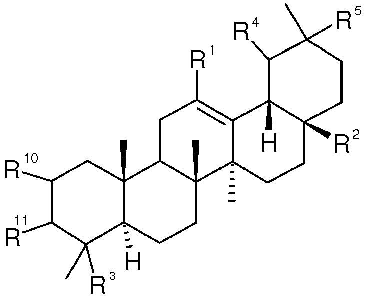
wherein R1 is selected from the group consisting of hydrogen, methyl, halide, lower haloalkyl, nitrile, lower alkyl nitrile, lower alkyl, substituted lower alkyl, lower alkenyl, substituted lower alkenyl, lower alkynyl, substituted lower alkynyl, lower cycloalkyl, lower cycloalkenyl, aryl, substituted aryl, heteroaryl, and substituted heteroaryl; wherein
R² is selected from the group consisting of carboxyl, amide, hydroxyamide, methylamide, -CH₂N(CH₃)₂, -CH₂NR⁶R⁷, 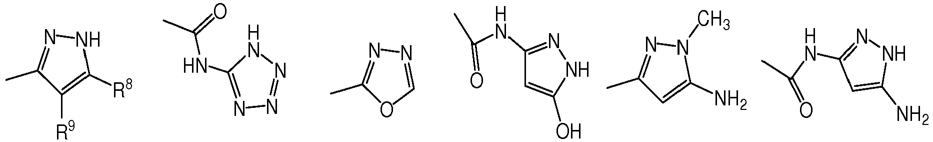 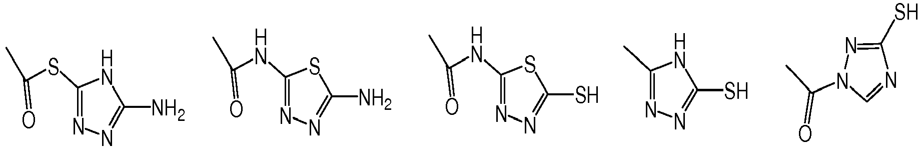 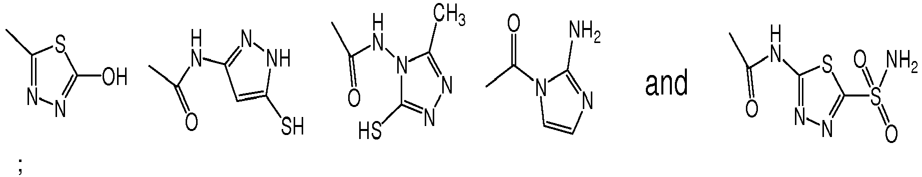
wherein R³ is selected from the group consisting of hydrogen and methyl; wherein one of R⁴ and R⁵ is hydrogen and the other is methyl; wherein R⁶ and R⁷ are independently selected from the group consisting of hydrogen, methyl, ethyl, propyl, isopropyl, and butyl; wherein R⁸ is selected from the group consisting of hydroxyl, amino, -N(CH₃)₂, and -NHCH₃; wherein R⁹ is selected from the group consisting of hydrogen, halide, lower alkyl, lower alkenyl, lower alkynyl, morpholinyl, piperazinyl, lower alkyl piperazinyl, heterocycloalkyl, lower cycloalkyl, lower cycloalkenyl; and lower alkyl, lower alkenyl, and lower alkynyl optionally substituted with moieties selected from the group consisting of hydroxyl, amino, lower aminoalkyl, halide, lower alkoxy, lower alkoxyalkyl, lower alkoxycarbonyl, lower alkylcarbonylamino, carboxyl, amide, hydroxyamide, -CONHCH₃, -NHCONH₂, -SO₂NH₂, -SO₂CH₃, -NHCOCH₃, -NHCSNH₂, and -NHSO₂CH₃; and wherein R¹⁰ and R¹¹ together with the carbon atoms to which they are attached form an aromatic N-heterocycle which is substituted or not selected from the group consisting of indole, imidazole, quinoline, isoquinoline, pyridine, triazine, pyrazine, pyrimidine, pyridazine, isoindole, pyrrole, benzimidazole, purine, pyrazole, benzopyrazole, indazole, quinoxaline, acridine, quinazoline, indolizine, carbazole and cinnoline; and salts thereof.

In a preferred embodiment, the present invention provides compounds of the following chemical Structure I 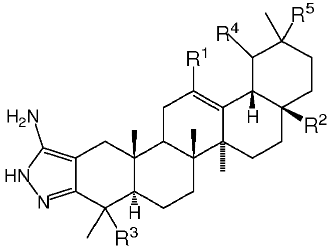
wherein R¹ is selected from the group consisting of hydrogen, methyl, halide, lower haloalkyl, nitrile, lower alkyl nitrile, lower alkyl, substituted lower alkyl, lower alkenyl, substituted lower alkenyl, lower alkynyl, lower cycloalkyl, lower cycloalkenyl, substituted lower alkynyl, aryl, substituted aryl, heteroaryl, and substituted heteroaryl;
R² is selected from the group consisting of carboxyl, amide, hydroxyamide, methylamide, -CH₂N(CH₃)₂, -CH₂NR⁶R⁷, 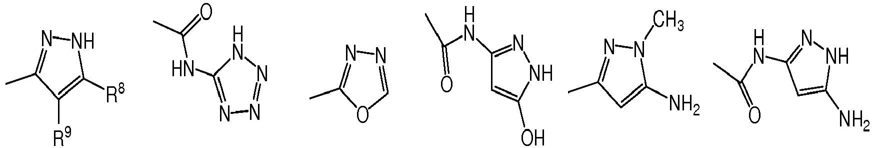 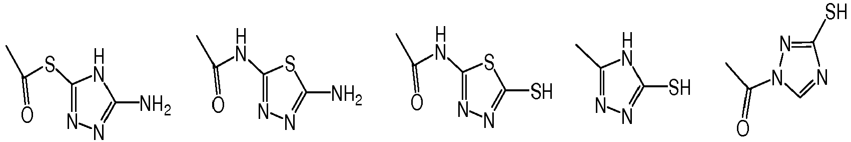 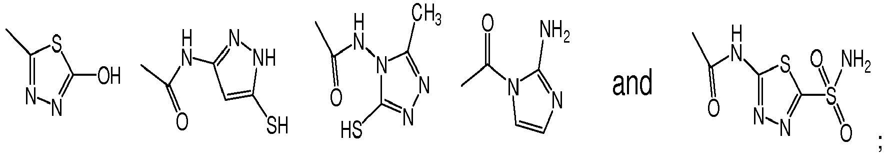
R³ is selected from the group consisting of hydrogen or methyl; one of R⁴ and R⁵ is hydrogen and the other is methyl; R⁶ and R⁷ are independently selected from the group consisting of hydrogen, methyl, ethyl, propyl, isopropyl, and butyl; R⁸ selected from the group consisting of hydroxyl, amino, -N(CH₃)₂, and -NHCH₃; and wherein R⁹ is selected from the group consisting of hydrogen, halide, lower alkyl, lower alkenyl, lower alkynyl, morpholinyl, piperazinyl, lower alkyl piperazinyl, heterocycloalkyl, lower cycloalkyl, lower cycloalkenyl; and lower alkyl, lower alkenyl, and lower alkynyl substituted with moieties selected from the group consisting of hydroxyl, amino, lower aminoalkyl, halide, lower alkoxy, lower alkoxyalkyl, lower alkoxycarbonyl, lower alkylcarbonylamino, carboxyl, amide, hydroxyamide, -CONHCH₃, -NHCONH₂, -SO₂NH₂, -SO₂CH₃, - NHCOCH₃, -NHCSNH₂, and -NHSO₂CH₃- Salts are also contemplated by the present invention as described in the examples.

A number of the compounds of the invention and intermediates may exist in different tautomeric forms. All such tautomeric forms are within the scope of the invention. The depiction of any tautomer herein is not intended to limit the scope of the invention to one specific tautomer. For example, the following is within the scope of the invention. Those skilled in the art will understand that Structure I may exist as a tautomer envisioned as the following. In any respect, 3-aminopyrazole of any structure of the invention may be as follows:

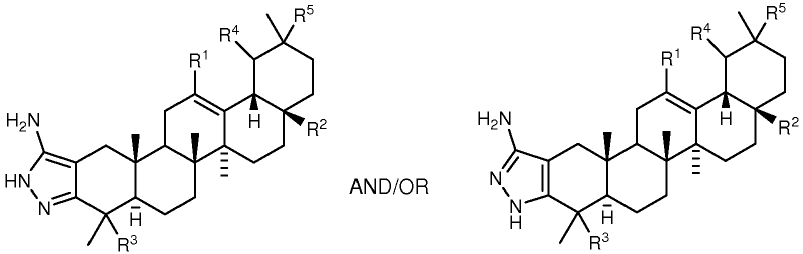

Or a 3-aminopyrazole of any structure of the invention may be as follows:

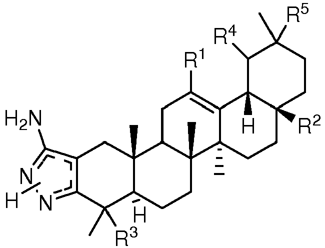

These tautomeric depictions are within the scope of the invention.

Furthermore, the hydroxypyrazole of compound 68 as described herein may exist as tautomers. These tautomers are within the scope of the invention.

Compositions containing the compounds described above and a pharmaceutically acceptable carrier are also contemplated by this invention. Such compositions containing the compounds described above optionally include an antimicrobial agent. As demonstrated herein such compositions are useful in reducing or inhibiting the formation or growth of biofilms.

These objects and other aspects of the invention are achieved with the compounds and/or formulation as claimed.

This invention also provides methods for reducing or inhibiting the formation or growth of biofilms comprising contacting the biofilm or cell capable of biofilm formation with an effective amount of a composition or a compound of the preceeding general chemical structure 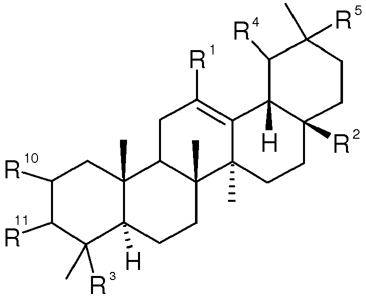
wherein R1 is selected from the group consisting of hydrogen, methyl, halide, lower haloalkyl, nitrile, lower alkyl nitrile, lower alkyl, substituted lower alkyl, lower alkenyl, substituted lower alkenyl, lower alkynyl, substituted lower alkynyl, lower cycloalkyl, lower cycloalkenyl, aryl, substituted aryl, heteroaryl, and substituted heteroaryl; wherein
R² is selected from the group consisting of carboxyl, amide, hydroxyamide, methylamide, -CH₂N(CH₃)₂, -CH₂NR⁶R⁷, 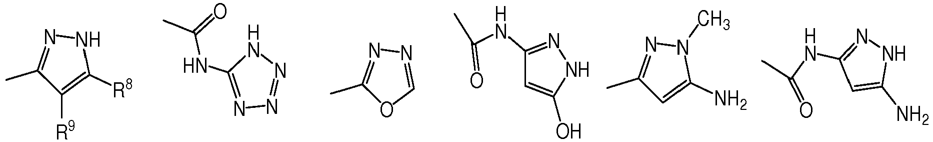 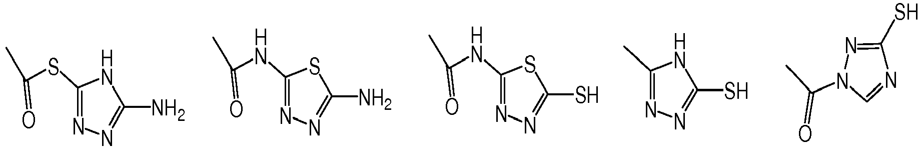 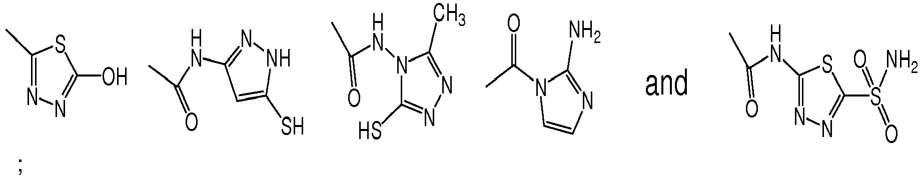
wherein R³ is selected from the group consisting of hydrogen and methyl; wherein one of R⁴ and R⁵ is hydrogen and the other is methyl; wherein R⁶ and R⁷ are independently selected from the group consisting of hydrogen, methyl, ethyl, propyl, isopropyl, and butyl; wherein R⁸ is selected from the group consisting of hydroxyl, amino, -N(CH₃)₂, and -NHCH₃; wherein R⁹ is selected from the group consisting of hydrogen, halide, lower alkyl, lower alkenyl, lower alkynyl, morpholinyl, piperazinyl, lower alkyl piperazinyl, heterocycloalkyl, lower cycloalkyl, lower cycloalkenyl; and lower alkyl, lower alkenyl, and lower alkynyl optionally substituted with moieties selected from the group consisting of hydroxyl, amino, lower aminoalkyl, halide, lower alkoxy, lower alkoxyalkyl, lower alkoxycarbonyl, lower alkylcarbonylamino, carboxyl, amide, hydroxyamide, -CONHCH₃, -NHCONH₂, -SO₂NH₂, -SO₂CH₃, -NHCOCH₃, -NHCSNH₂, and -NHSO₂CH₃; and wherein R¹⁰ and R¹¹ together with the carbon atoms to which they are attached form an aromatic N-heterocycle which is substituted or not selected from the group consisting of indole, imidazole, quinoline, isoquinoline, pyridine, triazine, pyrazine, pyrimidine, pyridazine, isoindole, pyrrole, benzimidazole, purine, pyrazole, benzopyrazole, indazole, quinoxaline, acridine, quinazoline, indolizine, carbazole and cinnoline; and salts thereof.

In a preferred embodiment, a method for reducing or inhibiting the formation or growth of biofilms comprises contacting the biofilm or cell capable of biofilm formation with an effective amount of a composition or a compound of the preceeding chemical Structure I wherein R¹ is selected from the group consisting of hydrogen, methyl, halide, lower haloalkyl, nitrile, lower alkyl nitrile, lower alkyl, substituted lower alkyl, lower alkenyl, substituted lower alkenyl, lower alkynyl, substituted lower alkynyl, lower cycloalkyl, lower cycloalkenyl, aryl, substituted aryl, heteroaryl, and substituted heteroaryl; R² is selected from the group consisting of carboxyl, amide, hydroxyamide, methylamide,-CH₂N(CH₃)₂, -CH₂NR⁶R⁷, 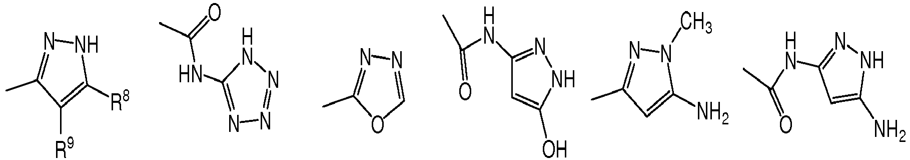 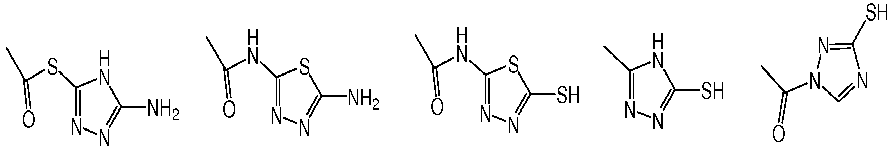 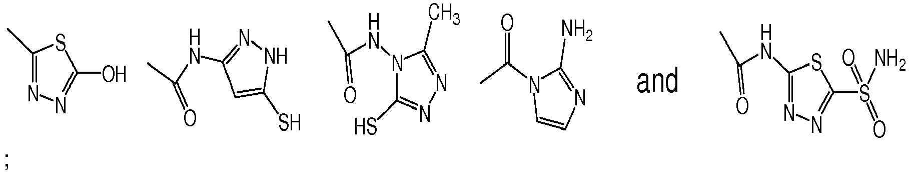 R³ is selected from the group consisting of hydrogen or methyl; one of R⁴ and R⁵ is hydrogen and the other is methyl; R⁶ and R⁷ are independently selected from the group consisting of hydrogen, methyl, ethyl, propyl, isopropyl, and butyl; R⁸ selected from the group consisting of hydroxyl, amino, -N(CH₃)₂, and -NHCH₃; and R⁹ is selected from the group consisting of hydrogen, halide, lower alkyl, lower alkenyl, lower alkynyl, morpholinyl, piperazinyl, lower alkyl piperazinyl, heterocycloalkyl, lower cycloalkyl, lower cycloalkenyl; and lower alkyl, lower alkenyl, and lower alkynyl substituted with moieties selected from the group consisting of hydroxyl, amino, lower aminoalkyl, halide, lower alkoxy, lower alkoxyalkyl, lower alkoxycarbonyl, lower alkylcarbonylamino, carboxyl, amide, hydroxyamide, -CONHCH₃, -NHCONH₂, -SO₂NH₂, -SO₂CH₃, - NHCOCH₃, -NHCSNH₂, and -NHSO₂CH₃. Salts are also contemplated by the present invention as described in the examples.

Inhibition or reduction of the formation or growth of biofilms may be effected either in vivo or in vitro. Compositions used to inhibit or reduce the formation or growth of biofilms may further include an antimicrobial agent, biocide, or antibiotic. The methods also provide for inhibiting or reducing the formation or growth of biofilms on a variety of substrates.

Inhibition or reduction of the formation or growth of biofilms reduces virulence of gram-negative bacteria. Adhesion, biofilm growth, invasion, and the secretion of enzymes or toxins contribute to virulence of gram-negative bacteria. It is well known to those skilled in the art that biofilms increase the virulence of gram-negative bacteria and an upregulation of virulence factors (i.e. enzymes, toxins) has been demonstrated in gram-negative bacterial biofilms. The compounds of the invention reduce virulence of gram-negative bacteria.

Without being bound by the theory, it is believed that compounds as described herein modulate cysB and genes under its control.

These objects and other aspects of the invention are achieved with the compounds and/or formulation and methods as claimed.

### BRIEF DESCRIPTION OF THE DRAWING

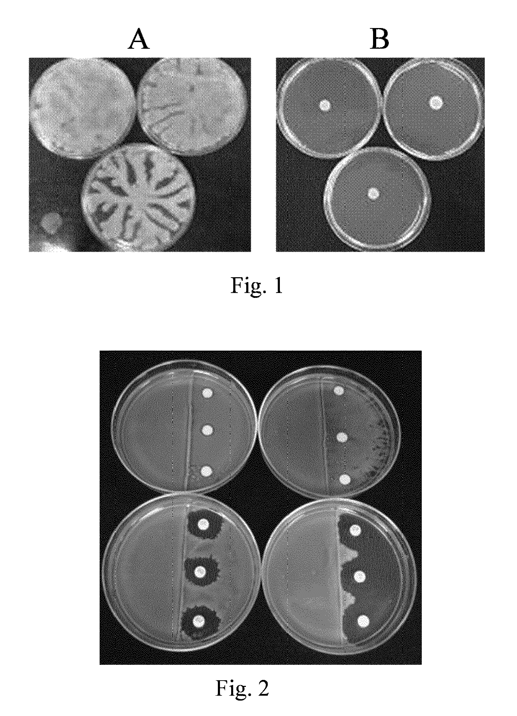

Figure 1 is a set of photographs of the Biofilm Growth Assay for Compound 1, as described in Example I, below, wherein the round plates on the left (A) are negative controls, in which the agar in the plates does not contain Compound 1, and wherein, as shown in the negative control plates, *P. aeruginosa* spread out from the inoculation point in the center of the plate during overnight incubation at 37°C and covered the entire plate, whereas the agar in the plates on the right (B) contained 0.5 µg/ml of Compound 1 of the invention, which prevented the growth of the spreading *P*. *aeruginosa* biofilm.
Figure 2 is a set of photographs of the Biofilm Growth Assay for Compound 1 in the presence of colistin (antibiotic) disks as described in Example VIII, below, wherein the top left plate was a negative control plate with paper disks without antibiotic and the top right plate had 0.125 µg/ml of Compound 1 in the agar on the right side of the plate with paper disks without antibiotic (and 0.125 µg/ml of Compound 1 was two dilutions below its optimal working concentration of 0.5 µg/ml), and the bottom left plate had colistin 10 µg disks, while the bottom right plate had 0.125 µg/ml of Compound 1 in the agar on the right side of the plate with colistin 10 µg disks, the inhibition of the growth of *P. aeruginosa* biofilm in the bottom right plate demonstrating that Compound 1 and colistin (antibiotic) are synergistic at preventing the growth of *P. aeruginosa* biofilms.

### DETAILED DESCRIPTION OF THE INVENTION

### Definitions

"Acceptable carrier" refers to a carrier that is not deleterious to the other ingredients of the composition and is not deleterious to material to which it is to be applied. "Pharmaceutically acceptable carrier" refers to a carrier that is not deleterious to the other ingredients of the composition and is not deleterious to the human or other animal recipient thereof. "Agriculturally acceptable carrier" refers to a carrier that is not deleterious to the other ingredients of the composition and is not deleterious to the plant recipient thereof. In the context of the other ingredients of the composition, "not deleterious" means that the carrier will not react with or degrade the other ingredients or otherwise interfere with their efficacy. Interference with the efficacy of an ingredient does not encompass mere dilution of the ingredient. In the context of the animal or plant host, "not deleterious" means that the carrier is not injurious or lethal to the plant or animal.

"Administration" refers to any means of providing a compound or composition to a subject. Non-limiting examples of administration means include oral, topical, rectal, percutaneous, parenteral injection, intranasal and inhalation delivery.

"Biofilm" refers to an extracellular matrix in which microorganisms are dispersed and/or form colonies. The biofilm typically is made of polysaccharides and other macromolecules.

"Biofilm Growth Assay" refers to an assay performed on semi-solid agar surface where the bacteria collectively move out from an inoculation point. To those skilled in the art, the act of bacteria collectively moving out from an inoculation point on a semi-solid surface may be referred to as swarming or a spreading biofilm. (Anderson, et al Microbiology, 2003, 149, 37-46; Daniels, et al. FEMS Microbiology Reviews, 2004, 28, 261-289). The procedure to perform this assay is described in detail in the Examples. This type of surface motility by bacteria has been reported in the literature by those skilled in the art. (Shrout, et al. Molecular Microbiology, 2006, 62(5), 1264-1277; Kim, et al. J. Bacteriology, 2003, 185(10), 3111-3117; Lai, et al. Environmental Microbiology, 2009, 11(1), 126-136; Overhage, et al. J. Bacteriology, 2008, 190(8), 2671-2679) These literature references also demonstrate a link to this type of surface motility and increased antibiotic tolerance.

"Commercial source" refers to a vendor that provides the desired compound.

"Direct synthesis" refers to production of the desired compound by reacting appropriate compound precursors under appropriate conditions to obtain the desired compound. "Effective amount" refers to the amount of compound or composition that, in the case of biofilm formation, will reduce the size or volume of existing biofilms; reduce the rate at which bacteria are capable of producing biofilm; or will inhibit or prevent the formation of biofilm by one or more microorganisms. In the context of treating a bacterial infection, an "effective amount" refers the amount of a compound or composition that will reduce the degree of an existing infection or will inhibit or prevent an infection from occurring.

"Essentially pure preparation" refers to a preparation in which the concentration of the desired ingredient is at least 95% or more of the preparation by weight. In the context of this processes used in this invention, the antimicrobial agents and compounds of the invention typically and preferably make up 99% or more by weight of the preparation and are referred to herein as "highly pure" preparations.

"In vivo", in the context of biofilm formation, refers to effects mediated in or upon living organisms or subjects. Effects mediated on biofilms associated with medical devices such as central venous catheters, urinary catheters, endotracheal tubes, mechanical heart valves, pacemakers, vascular grafts, stents, and prosthetic joints located within a living organism or subject are considered as "in vivo" uses of the compounds and compositions described herein.

"In vitro", in the context of biofilm formation, refers to effects mediated on substrates located outside of an organism that are potential sites of biofilm formation. Non-limiting examples of substrates include vessel hulls, cars, airplanes, industrial equipment, devices, membranes, filters, microtiter plates, continuous flow chambers, bioreactors, fermentors, chemostats and machinery.

"Is one that permits" as it relates to a pharmaceutically acceptable carrier that has characteristics that enable the preparation to be used for a given mode of administration of the composition. For example, pharmaceutically acceptable carriers that permit parenteral administration to an animal are liquids that are not injurious or lethal to the animals when so injected. Such carriers often comprise sterile water, which may be supplemented with various solutes to increase solubility. Sterile water or sterile water supplemented with solutes is thus a pharmaceutically acceptable carrier that permits parental administration.

As used herein, all agar percentages are expressed in terms of weight/volume; all formulation percentages are expressed in terms of weight/weight.

"Reducing or inhibiting" in reference to a biofilm refers to the inhibiting or reducing of biofilm formation or growth, a reduction in the rate of biofilm formation or growth,

reduction or removal of preformed or existing biofilm, as well as the partial or complete inhibition of biofilm formation or growth. This definition includes but is not limited to the biofilm growth that also occurs on semi-solid surfaces like 0.4% to 1.0% agar, but is not limited to these surfaces, as described in the Examples of the invention. This type of surface motility, which is also referred to as swarming or spreading biofilms and conducted on the same semi-solid agar plates independent of terminology, demonstrates mechanistic relationships between attached biofilms and gram-negative bacteria spreading across semi-solid agar plates as known to those skilled in the art. Michael Givskov and colleagues defined the movement of a *Pseudomonas* spp. across a 0.6% Bacto agar plate as surface motility with biofilm structures instead of swarming (Anderson, et al Microbiology, 2003, 149, 37-46). Jan Michiels and colleagues defined swarming as a biofilm spreading over a semi-solid surface (Daniels, et al. FEMS Microbiology Reviews, 2004, 28, 261-289). Even though terminology is evolving as more investigations are reported, all of these data have been generated examining the movement of gram-negative bacteria on 0.4% to 0.7% agar surfaces.

"Subject in need thereof" refers to living organism that would benefit from either prevention or reductions in the degree of a bacterial infection. Subjects may include animals or more specifically, mammals or humans. Subjects may also include plants. "Substrate" refers to any material to which the compound or a composition containing the compound may be applied.

The term "lower alkyl" as used herein refers to a saturated hydrocarbon chain having one, two, three, four, or five carbon atoms. Lower alkyl groups may be optionally substituted with one or more substituents as defined herein to form substituted lower alkyl groups. Lower alkyl groups may be straight or branched. Examples of lower alkyl groups include methyl, ethyl, propyl, isopropyl, butyl, isobutyl, t-butyl, pentyl, and isopentyl.

The phrase "substituted lower alkyl" as used herein, refers to a lower alkyl group, as previously defined, substituted by independent replacement of one, two, or three of the hydrogen atoms thereon with substituents including halide, nitrile, aryl, heteroaryl, substituted heteroaryl, lower cycloalkyl, lower cycloalkenyl, -SH, and lower thioalkyl. The term "lower alkenyl" as used herein refers to an unsaturated hydrocarbon chain having one, two, three, four, or five carbon atoms and having one or more carbon-carbon double bonds within the chain. The lower alkenyl groups may be straight or branched and may be optionally substituted with one or more substituents as defined herein to form substituted lower anlkenyl groups.

The phrase "substituted lower alkenyl" as used herein, refers to a lower alkenyl group, as previously defined, substituted by independent replacement of one, two, or three of the hydrogen atoms thereon with substituents including halide, nitrile, aryl, heteroaryl, substituted heteroaryl, lower cycloalkyl, lower cycloalkenyl, -SH, and lower thioalkyl. The term "lower alkynyl" as used herein refers to an unsaturated hydrocarbon chain having one, two, three, four, or five carbon atoms and having one or more carbon-carbon triple bonds within the chain. The lower alkynyl groups may be straight or branched and may be optionally substituted with one or more substituents as defined herein to form substituted lower alkynyl groups.

The phrase "N-heterocycle which is substituted or not" as used herein refers to a N-heterocycle group substituted or not by independent replacement of one, two, or three of the hydrogen atoms thereon with substituents including for example amino groups, Preferably, the aromatic N-heterocycle is selected from the group consisting of indole, imidazole, quinoline, isoquinoline, pyridine, triazine, pyrazine, pyrimidine, pyridazine, isoindole, pyrrole, benzimidazole, purine, pyrazole, benzopyrazole, indazole, quinoxaline, acridine, quinazoline, indolizine, carbazole and cinnoline. The aromatic N-heterocycle may be bicyclic moiety in which the N-heterocycle may be fused with an aryl ring. Preferably, the aromatic N-heterocycle is selected from the group consisting of indole, benzimidazole, quinoline, isoquinoline, isoindole, benzopyrazole, pyrrole, pyrazole.

The term "heterocycle", as used herein as a substituent is defined as including an aromatic or non aromatic cyclic alkyl, alkenyl, aryl or alkynyl moiety as defined above, having at least one O, S, P and/or N atom interrupting the carbocyclic ring structure and optionally, one of the carbon of the carbocyclic ring structure may be replaced by a carbonyl. Non-limiting examples of aromatic heterocycles are pyridyl, furyl, pyrrolyl, thienyl, isothiazolyl, imidazolyl, benzimidazolyl, tetrazolyl, quinazolinyl, quinolizinyl, naphthyridinyl, pyridazinyl, pyrimidinyl, pyrazinyl, quinolyl, isoquinolyl, isobenzofuranyl, benzothienyl, pyrazolyl, indolyl, indolizinyl, purinyl, isoindolyl, carbazolyl, thiazolyl, 1, 2, 4-thiadiazolyl, thieno (2,3-b) furanyl, furopyranyl, benzofuranyl, benzoxepinyl, isooxazolyl, oxazolyl, thianthrenyl, benzothiazolyl, or benzoxazolyl, cinnolinyl, phthalazinyl, quinoxalinyl, phenanthridinyl, acridinyl, perimidinyl, phenanthrolinyl, phenothiazinyl, furazanyl, isochromanyl, indolinyl, xanthenyl, hypoxanthinyl, pteridinyl, 5-azacytidinyl, 5-azauracilyl, triazolopyridinyl, imidazolopyridinyl, pyrrolopyrimidinyl, and pyrazolopyrimidinyl optionally substituted by alkyl or as described above for the alkyl groups. Non-limiting examples of non aromatic heterocycles are tetrahydrofuranyl, tetrahydropyranyl, piperidinyl, piperidyl, piperazinyl, imidazolidinyl, morpholino, morpholinyl, 1-oxaspiro(4,5)dec-2-yl, pyrrolidinyl, 2-oxo-pyrrolidinyl, sugar moieties (i.e. glucose, pentose, hexose, ribose, fructose, which may also be substituted) or the same which can optionally be substituted with any suitable group. The term "heterocycle" also includes bicyclic, tricyclic and tetracyclic, spiro groups in which any of the above heterocyclic rings is fused to one or two rings independently selected from an aryl ring, a cyclohexane ring, a cyclohexene ring, a cyclopentane ring, a cyclopentene ring or another monocyclic heterocyclic ring or where a monocyclic heterocyclic group is bridged by an alkylene group, such as quinuclidinyl, 7-azabicyclo (2,2,1) heptanyl, 7-oxabicyclo (2,2,1) heptanyl, 8-azabicyclo (3,2,1) octanyl. The term "heterocycle" also encompasses substituted heterocycle as defined above, bearing one or more substituent as defined above.

The phrase "substituted lower alkynyl" as used herein refers to a lower alkynyl group, as previously defined, substituted by independent replacement of one, two, or three of the hydrogen atoms thereon with substituents including halide, nitrile, aryl, heteroaryl, substituted heteroaryl, lower cycloalkyl, lower cycloalkenyl, -SH, and lower thioalkyl. The phrase "lower alkyl ethers" as used herein refers to ethers of the formula R'OR", wherein R' is a lower alkyl, lower alkenyl or lower alkynyl, and R" is a lower alkyl, lower alkenyl, lower alkynyl, or aryl, heteroaryl or heterocycloalkyl.

The term "lower alkoxy," as used herein by itself or as part of another substituent, means a radical of the formula -OR, wherein R is a lower alkyl, lower alkenyl, lower cycloalkyl, or lower cycloalkenyl group as defined herein. Representative examples of lower alkoxy groups include, but are not limited to, methoxy, ethoxy, propoxy, isopropoxy, butoxy, tert-butoxy, cyclopropyloxy, cyclopentyloxy, and the like.

The term "lower alkoxyalkyl" as used herein refers to a lower alkyl moiety as defined herein with one carbon atom replaced with an oxygen atom. Examples include -CH₂CH₂OCH₃ and -CH₂OCH₂CH₂CH₃.

The term "lower alkoxycarbonyl" as used herein by itself or as part of another substituent, refers to a radical of the formula -C(O)-(lower alkoxy), wherein lower alkoxy is as defined herein.

The phrase "lower alkyl nitrile" as used herein refers to lower alkyl or lower alkenyl with one nitrile group replacing one terminal carbon atom in the unbranched or branched chain. Lower alkyl nitrile includes, but is not limited to, -CH₂CN and -CH₂CH₂CN.

The term "lower aminoalkyl" as used herein refers to lower alkyl or lower alkenyl with one nitrogen atom replacing one carbon atom in the unbranched or branched chain. Lower aminoalkyl includes, but is not limited to, -NHCH₂CH₂CH₃, -CH₂CH₂-NHCH₃, -CH₂N(CH₃)₂, -N(CH₃)₂, and -CH₂CH₂NH₂.

The term "lower alkylcarbonylamino" as used herein refers to lower alkyl with a carbonylamino or aminocarbonyl replacing two carbon atoms in the unbranched or branched chain. Lower alkylcarbonylamino includes, but is not limited to, -NHCOCH₂CH₃, -CH₂CONHCH₃, -CON(CH₃)₂, and -CONHCH₃ (methylamide).

The terms "halo" and "halogen," as used herein, mean an atom selected from fluorine, chlorine, bromine and iodine and the term "halide" and used herein means the corresponding anion.

The term "lower haloalkyl" as used herein refers to a lower alkyl group wherein one or more hydrogen atoms attached to a member atom within the lower alkyl group is replaced with 1, 2, 3, or 4 halide atoms. Lower haloalkyl includes, but are not limited to, fluoromethyl, -CF₃, difluoroethyl, and trifluoromethyl.

The term "lower hydroxyalkyl" as used herein refers to lower alkyl or lower alkenyl wherein one or more hydrogen atoms attached to a member atom within the lower alkyl or lower alkenyl group is replaced with one or two hydroxyls. Lower hydroxyalkyl includes, but is not limited to, -CH₂CH₂-OH and -CH₂CH(-OH)CH₃, and the like.

The term "aryl" as used herein, means mono- or bicyclic carbocyclic ring systems comprising 6 to 12 carbon atoms, which consist of one or two aromatic rings, specifically including phenyl, naphthyl, tetrahydronaphthyl, indanyl, and idenyl; and specifically substitutions to these aryls by independent replacement of one two, or three of the hydrogen atoms thereon with substituents specifically selected from the group consisting of halide, lower haloalkyl, amino, hydroxyl, lower alkoxy, lower aminoalkyl, lower hydroxyalkyl, nitrile, lower alkyl nitrile, lower alkyl, lower alkenyl, lower alkynyl, nitro, carboxyl, amide, hydroxyamide, lower alkyl ethers, lower alkoxyalkyl, lower alkoxycarbonyl, lower alkylcarbonylamino, -SH, and lower thioalkyl.

The phrase "substituted aryl," as used herein, means a aryl group, as previously defined, substituted by independent replacement or one or two of the hydrogen atoms thereon with substituents specifically selected from the group consisting of -(CH₂)₀₋₃-lower cycloalkyl, -(CH₂)₀₋₃-aryl, -(CH₂)₀₋₃-lower cycloalkenyl, -(CH₂)₀₋₃-heteroaryl, -(CH₂)₀₋₃-heterocycloalkyl, -NH-lower cycloalkyl, -NH-aryl, -NH-lower cycloalkenyl, -NH-heteroaryl, -NH- heterocycloalkyl, -O-aryl, -O-heteroaryl, -O- heterocycloalkyl, -C(O)-lower alkyl, -C(O)-lower alkenyl, -C(O)-lower alkynyl, -C(O)-lower cycloalkyl, - C(O)-aryl, -C(O)-lower cycloalkenyl, -C(O)-heteroaryl, -C(O)- heterocycloalkyl, -CONH₂, -CONH-lower alkyl, -CONH-lower alkenyl, -CONH-lower alkynyl, -CONH-lower cycloalkyl, -CONH-aryl, -CONH-lower cycloalkenyl, -CONH-heteroaryl, -CONH-heterocycloalkyl, -OCO₂-lower alkyl, -OCO₂-lower alkenyl, -OCO₂-lower alkynyl, -OCO₂-lower cycloalkyl, -OCO₂-aryl, -OCO₂-lower cycloalkenyl, -OCO₂-heteroaryl, -OCO₂- heterocycloalkyl, -OCONH₂, -OCONH-lower alkyl, -OCONH-lower alkenyl, -OCONH-lower alkynyl, -OCONH-lower cycloalkyl, -OCONH-aryl, -OCONH-lower cycloalkenyl, -OCONH-heteroaryl, -OCONH- heterocycloalkyl, -NHC(O)-lower alkyl, -NHC(O)-lower alkenyl, -NHC(O)-lower alkynyl, -NHC(O)-lower cycloalkyl, - NHC(O)-aryl, -NHC(O)-lower cycloalkenyl, -NHC(O)-heteroaryl, -NHC(O)-heterocycloalkyl, -NHCO₂-lower alkyl, -NHCO₂-lower alkenyl, -NHCO₂-lower alkynyl,-NHCO₂-lower cycloalkyl, -NHCO₂-aryl, -NHCO₂-lower cycloalkenyl, -NHCO₂-heteroaryl, -NHCO₂- heterocycloalkyl, -NHC(O)NH₂, -NHC(O)NH-lower alkyl, -NHC(O)NH-lower alkenyl, -NHC(O)NH-lower alkynyl, -NHC(O)NH-lower cycloalkyl, -NHC(O)NH-aryl,-NHC(O)NH-lower cycloalkenyl, -NHC(O)NH-heteroaryl, -NHC(O)NH- heterocycloalkyl, -NHC(S)NH₂, -NHC(S)NH-lower alkyl, -NHC(S)NH-lower alkenyl, -NHC(S)NH-lower alkynyl, -NHC(S)NH-lower cycloalkyl, -NHC(S)NH-aryl, -NHC(S)NH-lower cycloalkenyl, -NHC(S)NH-heteroaryl, -NHC(S)NH- heterocycloalkyl, -S(O)-lower alkyl, -S(O)-lower alkenyl, -S(O)-lower alkynyl, -S(O)-lower cycloalkyl, -S(O)-aryl, -S(O)-lower cycloalkenyl, -S(O)-heteroaryl, -S(O)- heterocycloalkyl, -CH₂SO₂CH₃, -SO₂NH₂, -SO₂NH-lower alkyl, -SO₂NH-lower alkenyl, -SO₂NH-lower alkynyl, -SO₂NH-lower cycloalkyl, -SO₂NH-aryl, -SO₂NH-lower cycloalkenyl, -SO₂NH-heteroaryl, -SO₂NH-heterocycloalkyl, -NHSO₂-lower alkyl, -NHSO₂-lower alkenyl, -NHSO₂-lower alkynyl,-NHSO₂-lower cycloalkyl, -NHSO₂-aryl, -NHSO₂-lower cycloalkenyl, -NHSO₂-heteroaryl, -NHSO₂- heterocycloalkyl, -S-lower alkenyl, -S-lower alkynyl, -S-lower cycloalkyl, -S-aryl, -S-heteroaryl, -S- heterocycloalkyl, or methylthiomethyl.

The phrase "lower cycloalkyl," as used herein, means a saturated carbocyclic ring compound specifically including cyclopropyl, cyclobutyl, cyclopentyl, cyclohexyl, norbornane, and adamantine; and specifically substitutions to these lower cycloalkyls by independent replacement of one or two of the hydrogen atoms thereon with substituents specifically selected from the group consisting of halide, lower haloalkyl, amino, hydroxyl, lower alkoxy, lower aminoalkyl, lower hydroxyalkyl, nitrile, lower alkyl nitrile, lower alkyl, lower alkenyl, lower alkynyl, nitro, carboxyl, amide, hydroxyamide, lower alkyl ethers, lower alkoxyalkyl, lower alkoxycarbonyl, lower alkylcarbonylamino, - SH, and thioalkyl.

The term "lower cycloalkenyl" as used herein specifically refers to an unsaturated hydrocarbon ring with five or six carbons; and specifically substitutions to these lower cycloalkenyls by independent replacement of one or two of the hydrogen atoms thereon with substituents specifically selected from the group consisting of halide, lower haloalkyl, amino, hydroxyl, lower alkoxy, lower aminoalkyl, lower hydroxyalkyl, nitrile, lower alkyl nitrile, lower alkyl, lower alkenyl, lower alkynyl, nitro, carboxyl, amide, hydroxyamide, lower alkyl ethers, lower alkoxyalkyl, lower alkoxycarbonyl, lower alkylcarbonylamino, -SH, and thioalkyl.

The term "heteroaryl" as used herein specifically refers to pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; specifically their partially reduced forms as known to those skilled in the art like as tetrahydroisoquinolinyl is to isoquinolinyl; and specifically a heteroaryl (as defined herein) of 6 members or less fused with an aryl (as defined herein) of 6 members or less OR separately two heteroaryls (as defined herein) of 6 members or less fused together as known to those skilled in the art like as in pyrrolopyridinyl and its partially reduced form dihydropyrrolopyridinyl; and specifically substitutions to these heteroaryls by independent replacement of one two, or three of the hydrogen atoms thereon with substituents specifically selected from the group consisting of halide, lower haloalkyl, amino, hydroxyl, lower alkoxy, lower aminoalkyl, lower hydroxyalkyl, nitrile, lower alkyl nitrile, lower alkyl, lower alkenyl, lower alkynyl, nitro, carboxyl, amide, hydroxyamide, lower alkyl ethers, lower alkoxyalkyl, lower alkoxycarbonyl, lower alkylcarbonylamino, -SH, and thioalkyl; and heteroaryls as defined herein with substitutions by independent replacement of a hydrogen atom on a ring nitrogen specifically selected from the group consisting of lower alkyl, lower alkenyl, and lower haloalkyl.

The phrase "substituted heteroaryl," as used herein, means a heteroaryl group as previously defined, substituted by independent replacement or one or two of the hydrogen atoms thereon with substituents specifically selected from the group consisting of -(CH₂)₀₋₃-lower cycloalkyl, -(CH₂)₀₋₃-aryl, -(CH₂)₀₋₃-lower cycloalkenyl, -(CH₂)₀₋₃-heteroaryl, -(CH₂)₀₋₃- heterocycloalkyl, -NH-lower cycloalkyl, -NH-aryl, -NH-lower cycloalkenyl, -NH-heteroaryl, -NH- heterocycloalkyl, -O-aryl, -O-heteroaryl, -O-heterocycloalkyl, -C(O)-lower alkyl, -C(O)-lower alkenyl, -C(O)-lower alkynyl, -C(O)-lower cycloalkyl, -C(O)-aryl, -C(O)-lower cycloalkenyl, -C(O)-heteroaryl, -C(O)-heterocycloalkyl, -CONH₂, -CONH-lower alkyl, -CONH-lower alkenyl, -CONH-lower alkynyl, -CONH-lower cycloalkyl, -CONH-aryl, -CONH-lower cycloalkenyl, -CONH-heteroaryl, -CONH- heterocycloalkyl, -OCO₂-lower alkyl, -OCO₂-lower alkenyl, -OCO₂-lower alkynyl, -OCO₂-lower cycloalkyl, -OCO₂-aryl, -OCO₂-lower cycloalkenyl, -OCO₂-heteroaryl, -OCO₂- heterocycloalkyl, -OCONH₂, -OCONH-lower alkyl, -OCONH-lower alkenyl, -OCONH-lower alkynyl, -OCONH-lower cycloalkyl, -OCONH-aryl, -OCONH-lower cycloalkenyl, -OCONH-heteroaryl, -OCONH- heterocycloalkyl, -NHC(O)-lower alkyl, -NHC(O)-lower alkenyl, -NHC(O)-lower alkynyl, -NHC(O)-lower cycloalkyl, -NHC(O)-aryl, -NHC(O)-lower cycloalkenyl, -NHC(O)-heteroaryl, -NHC(O)-heterocycloalkyl, -NHCO₂-lower alkyl, -NHCO₂-lower alkenyl, -NHCO₂-lower alkynyl, -NHCO₂-lower cycloalkyl, -NHCO₂-aryl, -NHCO₂-lower cycloalkenyl, -NHCO₂-heteroaryl, -NHCO₂- heterocycloalkyl, -NHC(O)NH₂, -NHC(O)NH-lower alkyl,-NHC(O)NH-lower alkenyl, -NHC(O)NH-lower alkynyl, -NHC(O)NH-lower cycloalkyl, - NHC(O)NH-aryl, -NHC(O)NH-lower cycloalkenyl, -NHC(O)NH-heteroaryl, -NHC(O)NH-heterocycloalkyl, -NHC(S)NH₂, -NHC(S)NH-lower alkyl, -NHC(S)NH-lower alkenyl,-NHC(S)NH-lower alkynyl, -NHC(S)NH-lower cycloalkyl, -NHC(S)NH-aryl, -NHC(S)NH-lower cycloalkenyl, -NHC(S)NH-heteroaryl, -NHC(S)NH- heterocycloalkyl, -S(O)-lower alkyl, -S(O)-lower alkenyl, -S(O)-lower alkynyl, -S(O)-lower cycloalkyl, -S(O)-aryl,-S(O)-lower cycloalkenyl, -S(O)-heteroaryl, -S(O)- heterocycloalkyl, -CH₂SO₂CH₃,-SO₂NH₂, -SO₂NH-lower alkyl, -SO₂NH-lower alkenyl, -SO₂NH-lower alkynyl, -SO₂NH-lower cycloalkyl, -SO₂NH-aryl, -SO₂NH-lower cycloalkenyl, -SO₂NH-heteroaryl,-SO₂NH- heterocycloalkyl, -NHSO₂-lower alkyl, -NHSO₂-lower alkenyl, -NHSO₂-lower alkynyl, -NHSO₂-lower cycloalkyl, -NHSO₂-aryl, -NHSO₂-lower cycloalkenyl, -NHSO₂-heteroaryl, -NHSO₂- heterocycloalkyl, -S-lower alkenyl, -S-lower alkynyl, -S-lower cycloalkyl, -S-aryl, -S-heteroaryl, -S- heterocycloalkyl, or methylthiomethyl.

The term "heterocycloalkyl" as used herein specifically refers to azetidinyl, [1,3]dioxolane, pyrazolinyl, pyrrolidinyl, 2-pyrrolinyl, 3-pyrrolinyl, tetrahydrofuranyl, dihydrofuranyl, pyranyl, tetrahydropyranyl, dihydropyranyl, tetrahydrothienyl, imidazolinyl, imidazolidinyl, pyrazolidinyl, oxazolidinyl, isoxazolidinyl, thiazolidinyl, isothiazolidinyl, piperidinyl, homopiperidinyl, quinuclidinyl, piperazinyl, lower alkyl piperazinyl, morpholinyl, thiamorpholinyl, 1-pyrazolidinyl, azepinyl; and heterocycloalkyls as defined herein with substitutions by independent replacement of a hydrogen atom on a ring nitrogen specifically selected from the group consisting of lower alkyl, lower alkenyl, and lower haloalkyl; and specifically substitutions to these heterocycloalkyls by independent replacement of one or two of the hydrogen atoms thereon with substituents specifically selected from the group consisting of halide, lower haloalkyl, amino, hydroxyl, lower alkoxy, lower aminoalkyl, lower hydroxyalkyl, nitrile, lower alkyl nitrile, lower alkyl, lower alkenyl, lower alkynyl, carboxyl, amide, hydroxyamide, lower alkoxyalkyl, lower alkoxycarbonyl, lower alkylcarbonylamino, -SH, and thioalkyl. For example, substitutions to pyrrolidinyl may include hydroxyl-pyrrolidinyl, chloropyrrolidinyl, methoxy-pyrrolidinyl, nitrile-pyrrolidinyl, methyl-pyrrolidinyl, and amino-pyrrolidinyl.

The term "heteroatom" as used herein refers to a nitrogen, sulfur, or oxygen atom. The term "lower thioalkyl" as used herein by itself or as part of another substituent, means a radical of the formula -SR, wherein R is a lower alkyl or lower cycloalkyl group as defined herein. Examples of lower alkylthio groups include, but are not limited to, methylthio, ethylthio, propylthio, isopropylthio, butylthio, and tert-butylthio.

The term "carboxy," as used herein, means a group of formula -COOH.

The term "hydroxy," as used herein, means a group of formula -OH.

The phrase "hydroxy protecting group," as used herein, means a labile chemical moiety which is known in the art to protect a hydroxyl group against undesired reactions during synthetic procedures. Following such procedures, the hydroxy protecting group may be selectively removed. Examples of hydroxy protecting groups include, but are not limited to, methylthiomethyl, tert-butyldimethylsilyl, tertbutyldiphenylsilyl, acyl substituted with an aromatic group, and the like.

The phrase "protected hydroxy," as used herein, means a hydroxy group protected with a hydroxy protecting group, as defined above, including benzoyl, acetyl, trimethylsilyl, triethylsilyl, methoxymethyl groups, for example.

The phrase "amino protecting group," as used herein, means a labile chemical moiety which is known in the art to protect an amino group against undesired reactions during synthetic procedures. Following such procedures, the amino protecting group may be selectively removed. Examples of amino protecting groups include, but are not limited to, t-butoxycarbonyl, 9-fluorenylmethoxycarbonyl, benzyloxycarbonyl, and the like. The phrase "protected amino," as used herein, means an amino group protected with an amino protecting group as defined above.

In accordance with the present invention, compounds as disclosed herein are surprisingly effective at inhibiting or reducing the formation or growth of biofilms. Furthermore, it is also disclosed that the co-administration to a bacterial biofilm of compounds described herein with an antimicrobial agent, antibiotic, or biocide provides increased susceptibility of the bacteria within the biofilm to the antimicrobial agent, antibiotic, or biocide. The instant invention thus provides for novel compounds, compositions, compositions comprising biofilm inhibitors and antimicrobial agents or antibiotics or biocides, and various methods of using the compositions containing the biofilm inhibitors of the invention to reduce or inhibit the formation or growth of bacterial biofilms.

The compounds of the invention may be prepared by the techniques described in the examples below, starting from the ursane or oleanane triterpene scaffolds like ursolic acid and oleanolic acid. While a typical starting chemical compound in the semi-synthetic preparation of the compounds of the invention may be ursolic acid, oleanolic acid, corosolic acid, asiatic acid, or madecassic acid, oleanolic acid and ursolic acid are preferred staring compounds. In designing semi-synthetic strategies to prepare analogs of the starting chemical compound, modifications at certain positions of the scaffold of the basic chemical compound prove to be important for modulating biofilm inhibition, while other modifications at positions can improve the bioavailability of the compound. Many of these modifications or optimizations are taught in the literature known to those skilled in the art, including The Organic Chemistry of Drug Design and Drug Action, 2^{nd} Edition, by Richard B. Silverman. Improvement of the bioavailability of the compound expands the therapeutic range of the compounds by reducing certain cellular toxicities in the subject.

Compounds of this invention include:

| Compound | Chemical Structure | Compound | Chemical Structure |
|---|---|---|---|
| 1 | 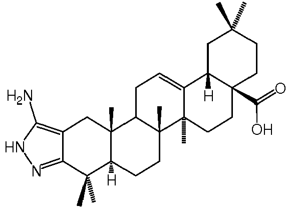 | 12 | 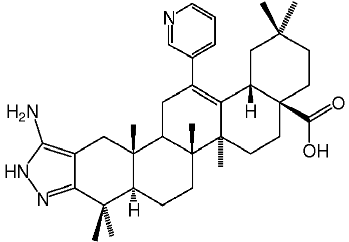 |
| 2 | 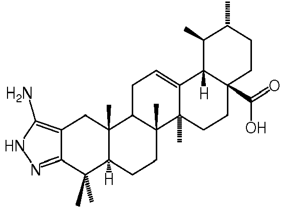 | 62 | 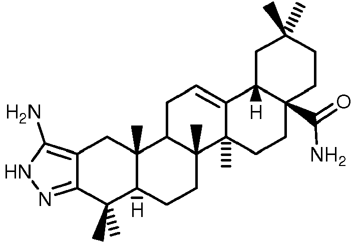 |
| 3 | 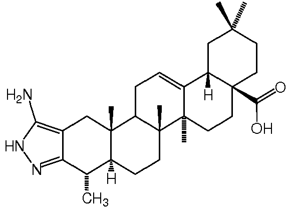 | 64 | 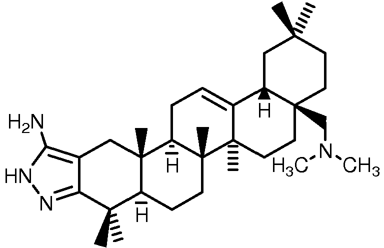 |
| 4 | 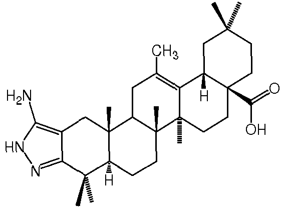 | 65 | 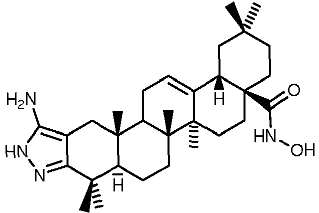 |
| 5 | 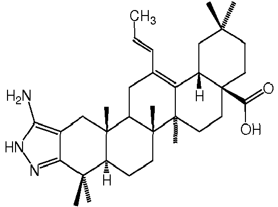 | 68 | 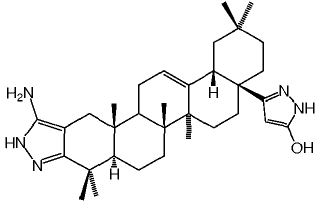 |
| 6 | 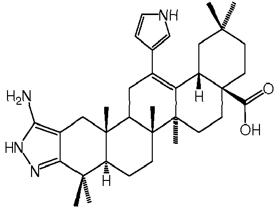 | 84 | 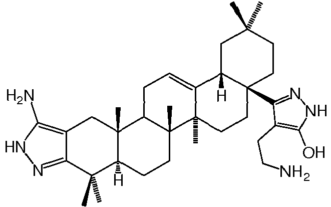 |
| 7 | 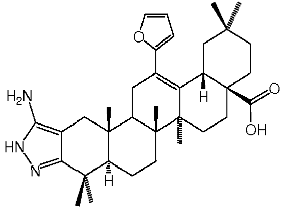 | 88 | 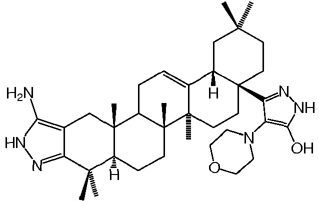 |
| 8 | 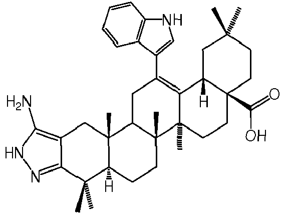 | 90 | 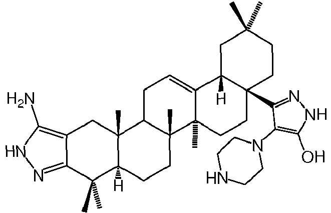 |
| 9 | 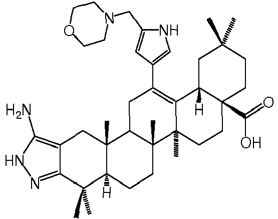 | 91 | 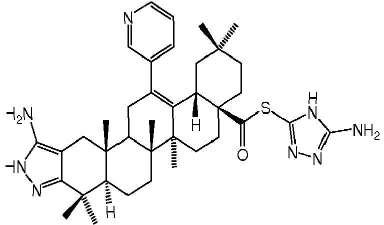 |
| 10 | 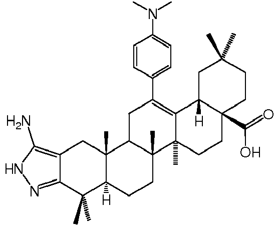 | 92 | 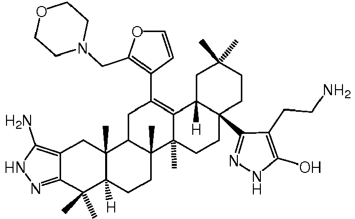 |
| 11 | 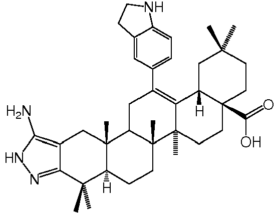 | | |

The invention especially teaches the remarkable discovery as demonstrated by the examples that the following Structure II 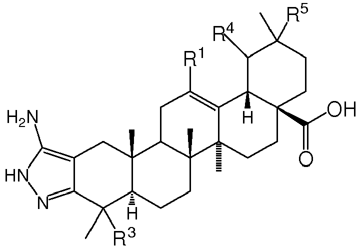 may contain a diverse variety of moieties and structures at R¹ and retain the ability to inhibit or reduce the formation or growth of biofilms. The examples demonstrate that a diverse group of aryl, substituted aryl, heteroaryl, and substituted heteroaryl moieties and structures may be added at R¹ and retain potent activity to reduce or inhibit the formation or growth of biofilms. A few representative chemical structures prepared semi-synthetically and described herein including the examples are as follows:

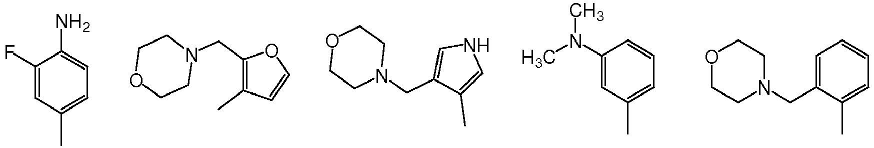

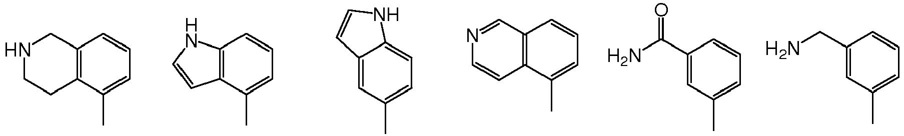

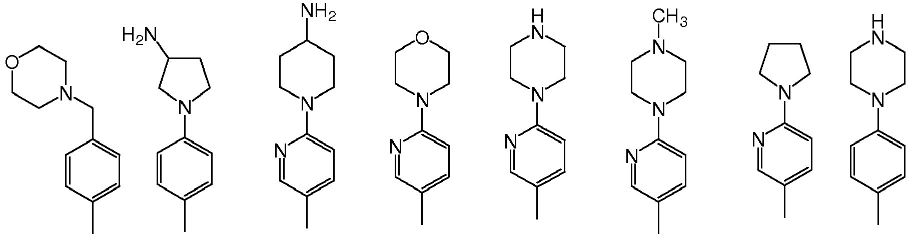

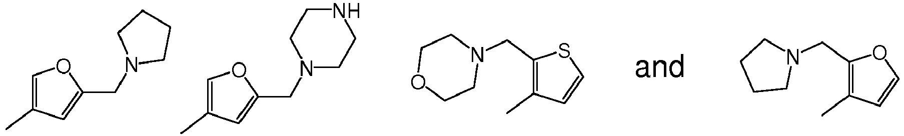

The present invention also includes compounds of the following chemical Structure III: 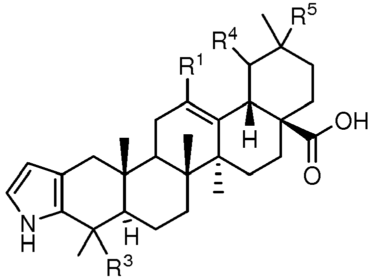 wherein R¹ is selected from the group consisting of hydrogen, methyl, halide, nitrile, lower alkyl, substituted lower alkyl, lower alkenyl, substituted lower alkenyl, lower alkynyl, substituted lower alkynyl, aryl, substituted aryl, heteroaryl, and substituted heteroaryl, R³ is selected from the group consisting of hydrogen or methyl, and one of R⁴ and R⁵ is hydrogen and the other is methyl. Salts are also contemplated by the present invention as described in the examples.

Based upon the teachings described herein, those skilled in the art appreciate that Structure III substituted with any of the following moieties at R¹ will exhibit potent activities at inhibiting the formation of biofilms.

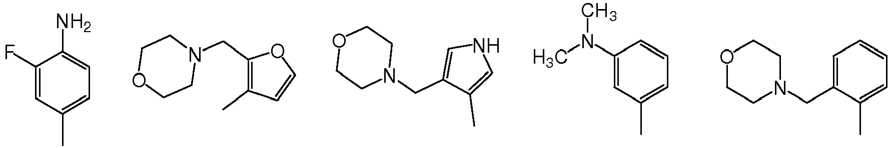

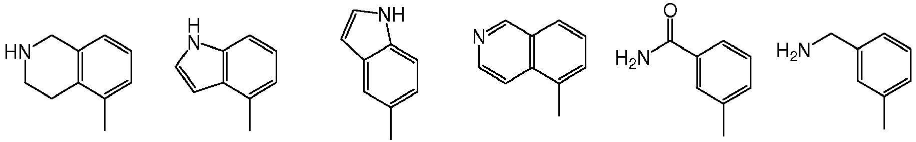

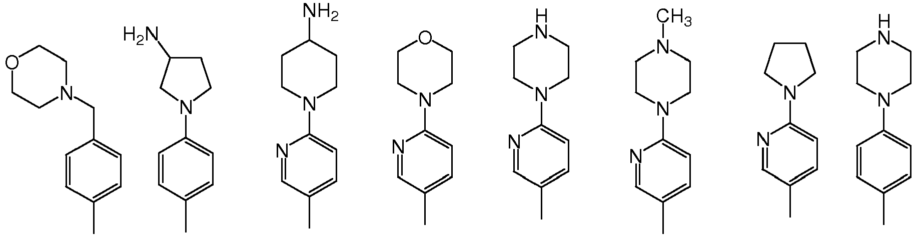

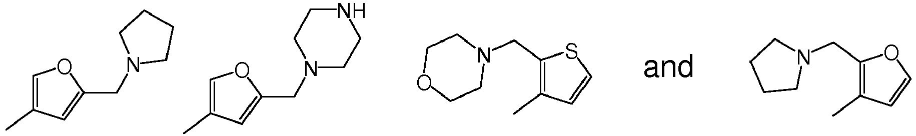

The present invention provides compounds of the following chemical Structure IV 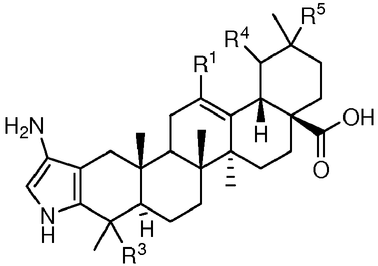 wherein R¹ is selected from the group consisting of hydrogen, methyl, halide, nitrile, lower alkyl, substituted lower alkyl, lower alkenyl, substituted lower alkenyl, lower alkynyl, substituted lower alkynyl, aryl, substituted aryl, heteroaryl, and substituted heteroaryl, wherein R³ is selected from the group consisting of hydrogen or methyl; wherein one of R⁴ and R⁵ is hydrogen and the other is methyl. Salts are also contemplated by the present invention as described in the examples.

Structure IV substituted with the following moieties at R¹ exhibit potent activities at inhibiting the formation of biofilms:

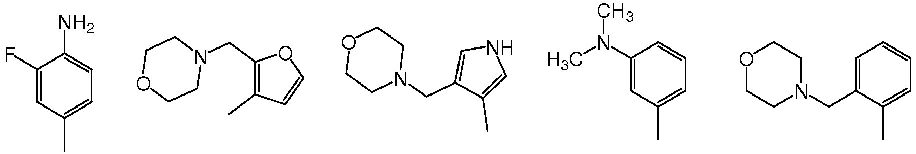

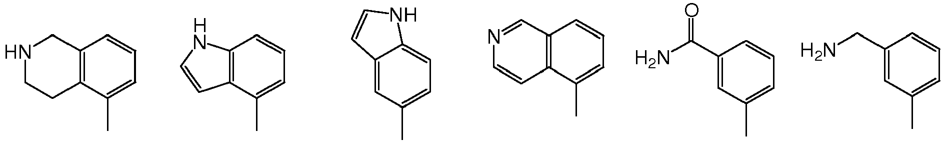

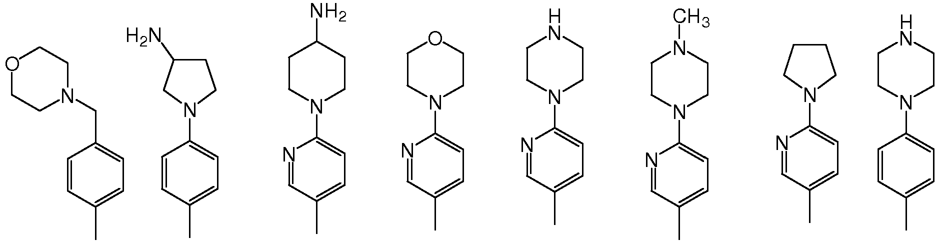

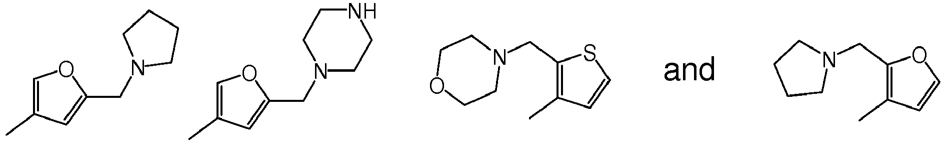

Various pharmaceutical compositions contemplated by the present invention, including the compounds of the invention and the specific examples described herein, further including pharmaceutically acceptable salt, ester, or salt of an ester thereof, which, upon administration to a patient, is capable of providing (directly or indirectly) a compound used in this invention. It will be recognized that the efficacy of the compounds of this invention is related to localized reaction sites on the compounds. Accordingly, as illustrated by the examples below, a wide variety of substitutions therefore may be made at various sites on the compounds spacially remote from the localized reaction sites that do not significantly interfere with the efficacy of the compounds. Likewise, substitutions to form pharmaceutically acceptable salts are contemplated herein as well. Thus, compounds with such innocuous substitutions do no depart from the scope of the invention.

On the other hand, however, certain moieties have been found to be so significant in size or reactivity as to interfere significantly with the efficacy of the compounds. Thus, highly reactive, polar, ionic or large substituents such as those shown in the examples below as having a deleterious affect on the activity of the compound are excluded from the most preferred embodiments of the invention.

Compounds useful in the present invention may, optionally, be converted to their therapeutically-active non-toxic acid salt forms by treatment with appropriate acids. Such acids include inorganic acids, e.g., hydrochloric and hydrobromic acids, sulfuric acid, nitric acid, phosphoric acid and like acids; or organic acids, such as acetic, propanoic, hydroxyacetic, 2-hydroxypropanoic, 2-oxo-propanoic, ethanedioic, propanedioic and like acids. Of course, the salt forms may be converted into the free base form by treatment with alkali. The pharmaceutically-acceptable acid salts of the present invention also comprise the solvates that the compositions of the present invention may form, which, of course, are included within the scope of the present invention. Non-limiting examples of such solvates are hydrates, alcoholates and the like.

Such pharmacologic compositions may be formulated in various ways known in the art for administration purposes. Pharmaceutical compositions of the present invention can be prepared by combining an effective amount of the particular compound of this invention, typically in base or acid salt form, as the active ingredient with one or more pharmaceutically-acceptable carriers and delivery vehicles. Numerous pharmaceutically acceptable carriers and delivery vehicles exist that are readily accessible and well-known in the art, which may be employed to generate the preparation desired (i.e. that permit administration of the pharmaceutical composition orally, topically, rectally, percutaneously, by parenteral injection, intranasally or by inhalation). Representative examples of pharmaceutically acceptable carriers and delivery vehicles include aluminum stearate, lecithin, serum proteins, such as human serum albumin; buffer substances such as the various phosphates, glycine, sorbic acid, potassium sorbate, partial glyceride mixtures of saturated vegetable fatty acids; water, salts or electrolytes, such as protamine sulfate, disodium hydrogen phosphate, potassium hydrogen phosphate, sodium chloride, and zinc salts; colloidal silica, magnesium trisilicate, polyvinyl pyrrolidone, cellulose-based substances, polyethylene glycol, sodium carboxymethylcellulose, polyarylates, waxes, polyethylene, polyoxypropylene-block polymers, polyethylene glycol and wool fat, and the like. Other constituents, such as aids for taste, color, tableting, and so forth, may be combined with the active ingredient and carrier for any of the many known purposes of such additives. Examples of such additives are discussed bleow.

The pharmacologic compositions described herein may further be prepared in unitary dosage form for administration orally, percutaneously, by parenteral injection (including subcutaneous, intramuscular, intravenous and intradermal), topically, intranasally, by inhalation, or for application to a medical device, such as an implant, catheter, or other device. In preparing the compositions that permit administration of an oral dosage, for example, any of the pharmaceutically acceptable carriers known in the art may be used, such as water, glycols, oils, alcohols and the like in the case of carriers that permit oral delivery of liquid preparations such as suspensions, syrups, elixirs and solutions. When solid pharmaceutically acceptable carriers are desired that permit oral or rectal administration, starches, sugars, kaolin, lubricants, binders, cellulose and its derivable prodrugs, and disintegrating agents and the like may be used to prepare, for example, powders, pills, capsules and tablets.

For pharmaceutically acceptable carriers that permit parenteral administration, the pharmaceutically acceptable carriers often comprise sterile water, which may be supplemented with various solutes to, for example, increase solubility. Injectable solutions may be prepared in which the pharmaceutically acceptable carrier comprises saline solution, glucose solution, or a mixture thereof, which may include certain well-known anti-oxidants, buffers, bacteriostats, and other solutes that render the formulation isotonic with the blood of the intended patient.

For pharmaceutically acceptable carriers that permit intranasal administration, the pharmaceutically acceptable carriers often comprise poly acrylic acids such as Carbopol.RTM. 940, a hydrogenated castor oil such as Cremophor.RTM. RH40, glycerol, vinylpyrrolidones such as PVP-K90.RTM. or PVP K30.RTM., polyethylene glycols such as PEG 1450.RTM., benzyl alcohol, Edetate sodium, hydroxycellulose, potassium chloride, potassium phosphate, and sodium phosphate. Compositions used for intranasal administration also commonly include benzalkonium chloride as an antimicrobial preservative.

For pharmaceutically acceptable carriers that permit administration by inhalation, the pharmaceutically acceptable carriers often comprise solvent/carrier/water mixtures that are easily dispersed and inhaled via a nebulizer or inhaler. For example, a mixture of ethanol/propylene glycol/water in the ratio of about 85:10:5 (parts ethanol: parts propylene glycol: parts water) can be used to administer the compounds and compositions of the invention via inhalation. Ratios as expressed herein are based on parts by weight.

For pharmaceutically acceptable carriers that permit percutaneous administration, the pharmaceutically acceptable carrier may, optionally, comprise a penetration enhancing agent and/or a wetting agent.

Dosage forms that permit topical or transdermal administration of a compound of this invention include ointments, pastes, creams, lotions, gels, powders, solutions, sprays, inhalants or patches. The active compound or compounds is/are mixed under sterile conditions with a pharmaceutically acceptable carrier and optionally one or more preservatives and/or buffers. In the context of certain embodiments of this invention, the active compound is a pentacyclic acid triterpene. In the context of other embodiments of this invention, the pentacyclic acid triterpene is combined in the composition with another active compound that is an antimicrobial agent or antibiotic. The ointments, pastes, creams and gels may contain, in addition to an active compound or compounds according to the present invention, pharmaceutically acceptable carriers that permit topical or transdermal administration such as animal and vegetable fats, oils, waxes, paraffins, starch, tragacanth, cellulose derivable prodrugs, polyethylene glycols, silicones, bentonites, silicic acid, talc and zinc oxide, or mixtures thereof.

In some cases, the pH of the pharmaceutical formulations contemplated herein may be adjusted with acceptable acids, bases or buffers to enhance the stability of one or more of the active compounds present or their delivery forms. In the context of certain embodiments of this invention, the active compound is a pentacyclic acid triterpene. In the context of other embodiments of this invention, the pentacyclic acid triterpene is combined in the composition with another active compound that is an antimicrobial agent or antibiotic.

Still further, in order to prolong the anti-bacterial effect of a compound disclosed herein, it may be desirable to slow the absorption of the compound from subcutaneous or intramuscular injection. This may be accomplished using a liquid suspension of crystalline or amorphous material with poor water solubility. The rate of absorption of the compound then depends upon its rate of dissolution, which, in turn, may depend upon crystal size and crystalline form. Alternatively, delayed absorption of a parenterally administered drug form may be accomplished by dissolving or suspending the compound in an oil vehicle.

Injectable depot forms are made, e.g., by forming microencapsule matrices of one or more compounds of the present invention in biodegradable polymers such as polylactide-polyglycolide. Depending upon the ratio of active(s) to polymer and the nature of the particular polymer employed, the rate at which such active(s) is released may be controlled. Examples of other biodegradable polymers include poly(orthoesters) and poly(anhydrides). Depot injectable formulations are also prepared by entrapping the compound in liposomes or microemulsions that are compatible with body tissues.

The pharmaceutical composition may also be a dentifrice. In the present invention, "dentifrice" is understood to broadly include compositions suitable for administering to the oral cavity, especially, for example, to the gingival/mucosal tissue or to the teeth. Thus, the dentifrice may include toothpastes, toothpowders, liquid dentifrices, mouth detergents, mouthwashes, troches, chewing gums, dental or gingival massage creams, dental strips, dental gels, and gargle tablets.

When the pharmaceutical composition of this invention is a dentifrice such as tooth paste, a tooth or gum adherence promoting substance selected from the group consisting of copolymers of methyl vinyl ether and maleic anhydride, copolymers of vinyl pyrrolidone and vinyl acetate, and cyclodextrins may also be included in the composition. Copolymers of methyl vinyl ether and maleic anhydride useful in this invention may have molecular weights ranging from 200,000 to 2,000,000 kD and may be free acids, mixed sodium and calcium salts, or half ester derivable prodrugs. Representative commercial sources of the copolymers of methyl vinyl ether and maleic anhydride include GANTREZ.RTM. AN(CAS # 9011-16-9) GANTREZ.RTM. S (CAS # 25153-40-69) GANTREZ.RTM. MS (CAS# 62386-95-2) GANTREZ.RTM. ES (CAS# 25087-06-3 or CAS# 25119-68-0) and can be obtained from International Specialty Products Wayne, N.J. Copolymers of vinyl pyrrolidone and vinyl acetate useful in the invention typically have a molecule weight of approximately 27,000 kD and are water soluble. Representative commercial sources of the copolymers of vinyl pyrrolidone and vinyl acetate PLASDONE.RTM. S-630 and can be obtained from International Specialty Products Wayne, N.J. Cyclodextrins useful in the invention are cyclic oligosaccharides composed of either 6, 7 or 8 glucose units (a-, b- and g-cyclodextrin, respectively). Representative commercial sources of the cyclodextrins useful in this invention include CAVAMAX.RTM. W6 Pharma, CAVAMAX.RTM.W7 Pharma and CAV AMAXW8 Pharma (a-, b- and g-cyclodextrin, respectively) and can be obtained from International Specialty Products Wayne, N.J.

When the composition of this invention is a dentifrice, an antimicrobial agent may be selected from the group consisting of triclosan, metronidazole, tetracyclines, quinolones, plant essential oils, camphor, thymol, carvacrol, menthol, eucalyptol, and methyl salicylate may also be included. Pharmaceutically acceptable carriers that permit administration of the pentacyclic acid triterpene compounds of this application as dentifrices include sorbitol, glycerin, silica, sodium lauryl sulfate and Xanthum gum. The dentifrices of this invention may also include sodium fluoride.

### Reducing or Inhibiting Bacterial Biofilms in Plants

Finally, bacterial biofilms also may be reduced or inhibited by the application or administration of compositions containing compounds disclosed herein when the subject is a plant. Thus, the compound or a composition containing an active compound described herein may be administered to a plant, such as a surface of a plant to reduce or inhibit the formation of a biofilm on the plant.

The methods described herein may be applicable to reducing or inhibiting a variety of bacterial biofilms in plants. *Pseudomonadaceae, Rhizobiaceae, Enterobacteriaceae, Corynebacteriaceae* and *Streptomycetaceae* bacteria are all economically significant plant pathogens that may be controlled by the present invention. Non-limiting examples of specific plant pathogens involving biofilms that may be effectively inhibited by the methods described herein include: *Xanthomonas* species, such as, for example, *Xanthomonas campestris* pv. oryzae; *Pseudomonas* species, such as, for example, *Pseudomonas syringae* pv. lachrymans; and *Erwinia* species, such as, for example, *Erwinia amylovora.* The compositions used in the methods of reducing or inhibiting bacterial biofilms of plants described herein may further comprise antimicrobial agents such as bronopol, dichlorophen, nitrapyrin, nickel dimethyldithiocarbamate, kasugamycin, octhilinone, furancarboxyl, oxytetracyclin, probenazole, streptomycin, tecloftalam, copper sulphate and other copper preparations.

In an alternative manner, methods of reducing or inhibiting bacterial biofilms described herein can be used to treat all plants and parts of plants. By reference to "plants," what is meant here is all plants and plant populations such as desired and undesired wild plants or crop plants (including naturally occurring crop plants). Crop plants can be plants obtainable by conventional breeding and optimization methods or by biotechnological and genetic engineering methods or combinations of these methods, and include the transgenic plants and the plant varieties that can or cannot be protected by varietal property rights. The phrase "parts of plants" as used herein is to be understood as meaning all above-ground and below-ground parts and organs of plants, such as shoot, leaf, flower and root, examples which may be mentioned being leaves, needles, stems, trunks, flowers, fruit-bodies, fruits and seeds and also roots, tubers and rhizomes. Parts of plants also include harvested plants and vegetative and generative propagation material, for example, seedlings, tubers, rhizomes, cuttings and seeds.

The treatment of the plants and the parts of plants with the active compounds according to the invention is carried out directly or by action on their surroundings, habitat or storage space, according to customary treatment methods, for example by dipping, spraying, evaporating, atomizing, broadcasting, spreading-on and, in the case of propagation material, in particular in the case of seeds, furthermore by one- or multilayer coating.

### Agriculturally Acceptable Carriers and Compositions

Depending on their particular physical and/or chemical properties, the compounds and compositions described herein can be converted to the customary formulations, such as solutions, emulsions, suspensions, powders, foams, pastes, granules, aerosols and microencapsulations in polymeric substances and in coating compositions for seeds, and ULV cool and warm fogging formulations.

The following examples illustrate various aspects of the present invention and are not intended to limit the scope of the present invention. Those of skill in the art should, in light of the present disclosure, appreciate that many changes can be made in the specific embodiments which are disclosed and still obtain a like or similar result without departing from the spirit and scope of the invention.

### EXAMPLE I.

### SPECIFICITY OF THE BIOFILM GROWTH ASSAY

Table 1, below, shows experimental results illustrating remarkable properties of Compound 1 to inhibit the spreading of clinical isolates of *P. aeruginosa* and *E. coli* bioflms as determined in a biofilm growth assay. The related scaffolds and analogs shown in Table 1 have been found to exhibit significantly less inhibitory activities than does Compound 1 in the biofilm growth assay even though certain of the compounds consist of chemical structures similar to that of Compound 1. These data demonstrate the novel and unique aspects of Compound 1 and its discovery and the specificity of the biofilm growth assay to identify Compound 1 as a potent inhibitor of spreading gram-negative bacterial biofilms.

The biofilm growth assay was carried out to measure swarming or biofilm spreading on semi-solid agar media in round plates (for example, 100 × 15 mm), also referred to as Petri dishes. For *E*. *coli,* the plates contained LB medium with 0.6% agar and 0.5% glucose. *P. aeruginosa* plates were made with M8 agar supplemented with 0.2% glucose, 100 µM CaCl₂, 1 mM MgSO₄, and 0.5% Casamino acids. The agar plates of this assay consisted of 0.4% to 0.7% agar (Bacto or Noble agar), although other media may also be used as described in the literature and known to those skilled in the art. Test compounds at the desired concentration were added to the cooled, autoclaved media. Portions of the media were then poured into the plates (20 mL/plate), and the plates allowed to dry at room temperature for approximately 3 to 4 hours. Alternatively, however, plates may be dried in a laminar flow hood for approximately fifteen to twenty minutes. Overnight cultures were grown in a 37°C shaker in LB (*E*. *coli*) or TSB (*P. aeruginosa*)*.* Plates were inoculated by placing 5 µL of the appropriate overnight culture in the centers of the plates. The plates then were incubated overnight at 37°C. The area of the zone of spreading bacteria was then measured and replicates are averaged. Percent inhibition was calculated as 100 × (area without compound - area with compound)/(area without compound). An active compound was considered that which reduces the area of the spreading biofilm compared to negative controls by greater than or equal to 85%. The values shown in Table 1 are the concentrations of compounds (µg/ml) tested in the biofilm growth assay that reduce the area of the spreading biofilm compared to negative controls by greater than or equal to 85% unless noted differently in Table 1. A > ("greater than" symbol) as used in Table 1 is well understood to those skilled in the art as an indication that the listed concentration was not effective at this concentration.

This example demonstrates the reduction of virulence of gram-negative bacteria. Inhibition or reduction of the growth of biofilms reduces virulence of gram-negative bacteria. Adhesion, biofilm growth, invasion, and the secretion of enzymes or toxins contribute to virulence of gram-negative bacteria. It is well known to those skilled in the art that biofilms increase the virulence of gram-negative bacteria and an upregulation of virulence factors (i.e. enzymes, toxins) has been demonstrated in gram-negative bacterial biofilms. The compounds of the invention reduce virulence of gram-negative bacteria.

**Table 1. Related Scaffolds and Analogs of Compound 1**

| COMPOUND | STRUCTURE | *P. aeruginosa* (µg/ml) | *E. coli* (µg/ml) |
|---|---|---|---|
| Compound 1 | 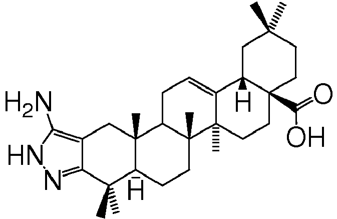 | 0.5 | 1 |
| Uvaol | 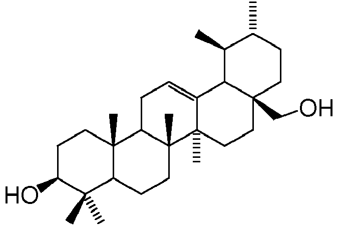 | > 128 | >8 |
| alpha-Amyrin | 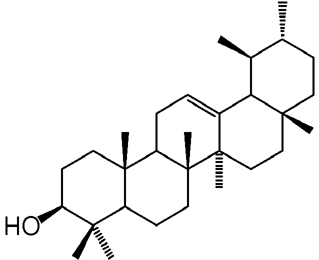 | > 32 | Not tested |
| Lupeol | 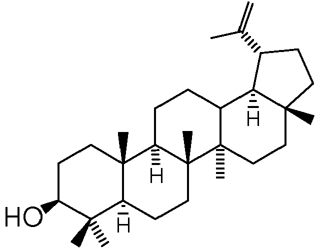 | >16 | >8 |
| Betulin | 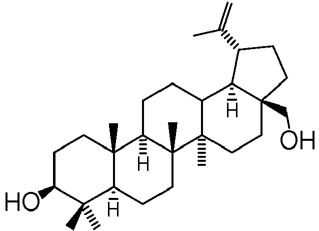 | 16 | > 16 |
| Betulinic acid | 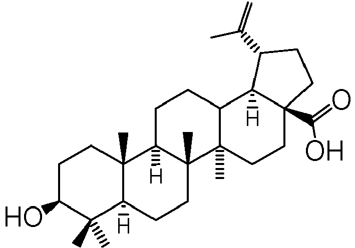 | > 16 | > 16 |
| Oleanolic acid | 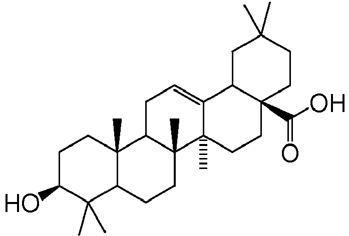 | 4 | >8 |
| Ursolic acid | 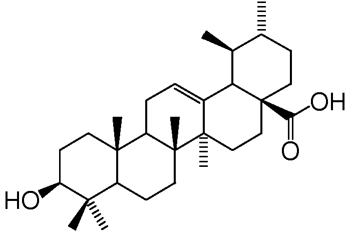 | 8 | 8 |
| Oleanolic acid methyl ester | 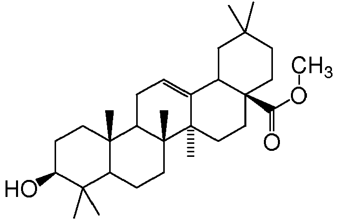 | >16 | > 16 |
| Corosolic acid | 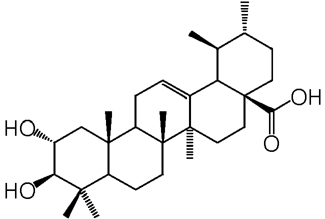 | 4 | Not tested |
| Maslinic acid | 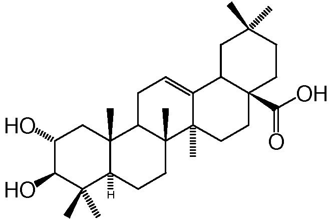 | 4 | Not tested |
| A | 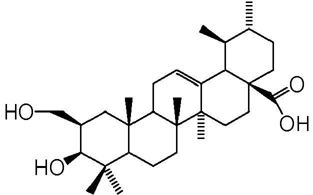 | 8 | >8 |
| B | 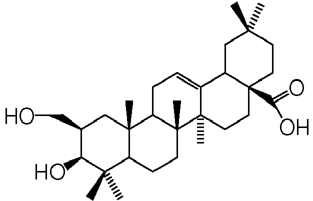 | 4 | 8 |
| C | 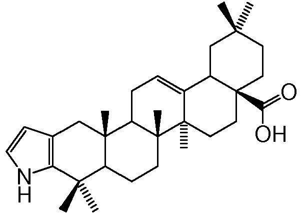 | 0.5 | 0.5 |
| D | 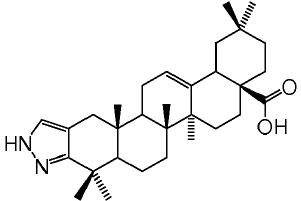 | 1 | 4 |
| E | 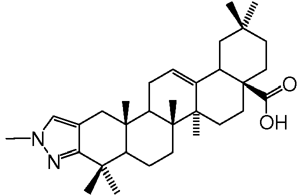 | 2 | >16 |
| F | 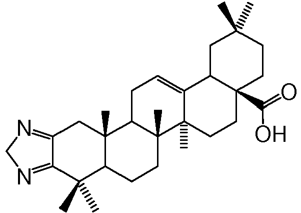 | 4 | >8 |
| G | 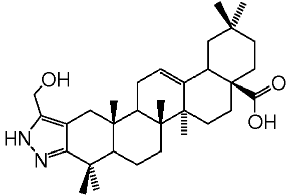 | >8 | >8 |
| H | 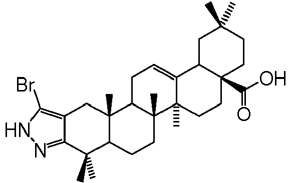 | 8 | >8 |
| I | 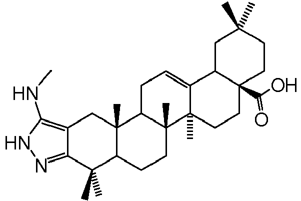 | > 16 | 8 |
| J | 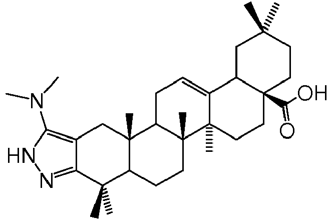 | > 16 | > 16 |
| K | 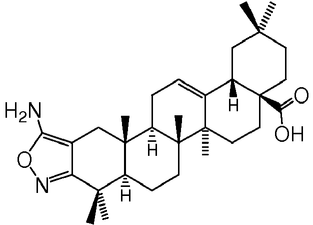 | >8 | >8 |
| L | 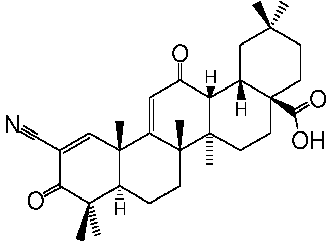 | > 16 | > 16 |
| M | 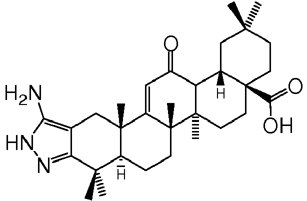 | 8 | 8 |
| N | 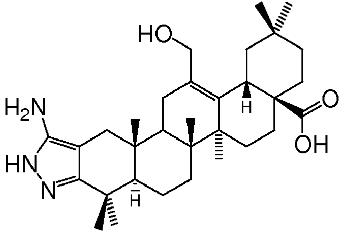 | 8 | 8 |

### EXAMPLE II

All of the compounds shown in Tables 2, 3, 4, and 5, below, were prepared semi-synthetically from oleanolic acid, except for Compounds 2 and 3. Compound 2 was prepared from ursolic acid. Compound 3 was prepared from hederagenin, but could also be prepared from an oleanolic acid analog with a hydroxyl at C₂₃ or C₂₄. These compounds were tested in the spreading biofilm assay according to the methods detailed in Example I, above. The values shown in Tables 2, 3, 4, and 5 are the concentrations of compounds tested in the biofilm growth assay that reduce the area of the spreading biofilm compared to negative controls by greater than or equal to 85%.

**Table 2. Compound 1 and its Analogs and their inhibitory concentrations in the biofilm growth assay as detailed in Example I, above**

| COMPOUND | Chemical Structure | *P. aeruginosa* (µg/ml) | *E. coli* (µg/ml) |
|---|---|---|---|
| 1 | 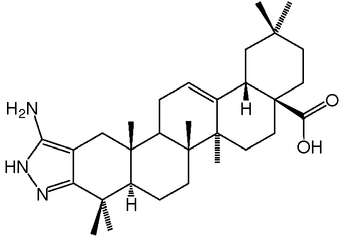 | 0.5 | 1 |
| 2 | 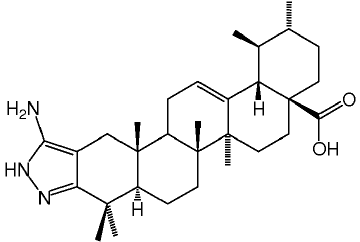 | 0.5 | 1 |
| 3 | 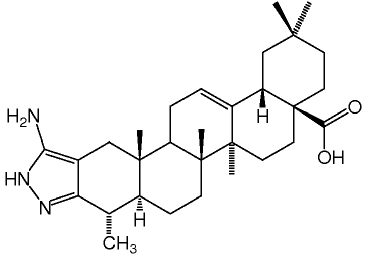 | 0.5 | 1 |
| 4 | 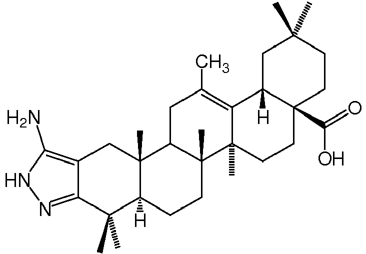 | 0.25 | 1 |
| 5 | 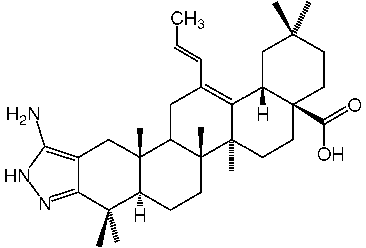 | 0.5 | 1 |
| 6 | 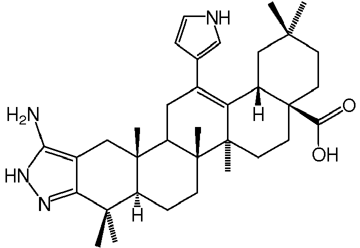 | 0.125 | 0.25 |
| 7 | 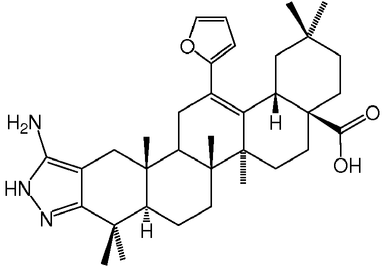 | 0.25 | 1 |
| 8 | 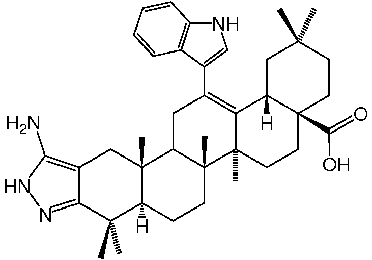 | 0.25 | 1 |
| 9 | 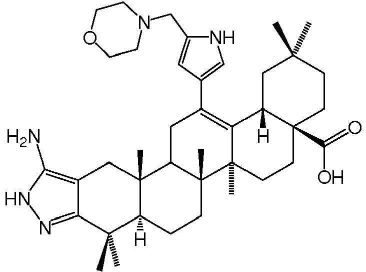 | 0.5 | 1 |
| 10 | 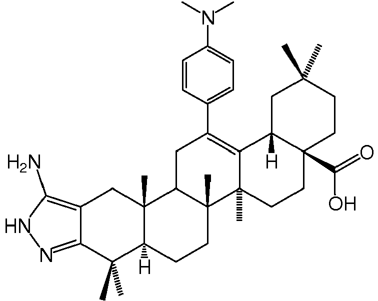 | 0.5 | 1 |
| 11 | 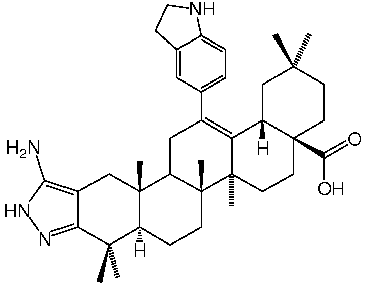 | 0.5 | 0.5 |
| 12 | 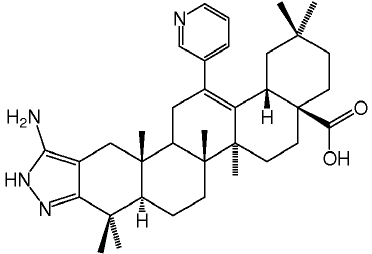 | 0.5 | 1 |

**Table 3. Analogs of Compound 1**

| COMPOUND | Chemical Structure | *P. aeruginosa* (µg/ml) | *E. coli* (µg/ml) |
|---|---|---|---|
| | 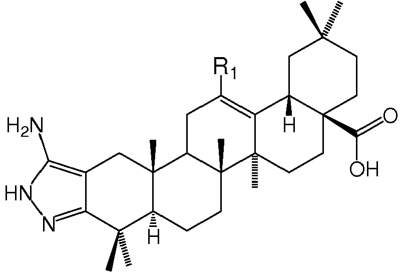 | | |

| | R₁ | | |
|---|---|---|---|
| 13 | -CH=CH₂ | 0.5 | 1 |
| 14 | -CH₂CH₃ | 0.5 | 2 |
| 15 | 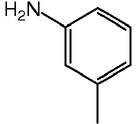 | 0.25 | 1 |
| 16 | 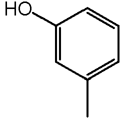 | 1 | 2 |
| 17 | 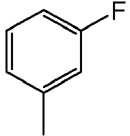 | 0.5 | > 2 |
| 18 | 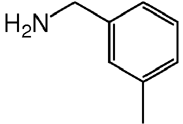 | 0.5 | 1 |
| 19 | 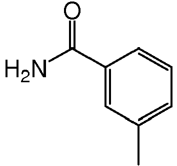 | 1 | 1 |
| 20 | 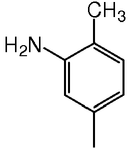 | 0.5 | 2 |
| 21 | 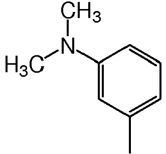 | 0.5 | > 2 |
| 22 | 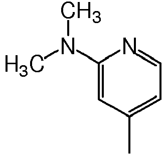 | 1 | >2 |
| 23 | 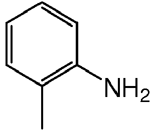 | 1 | 2 |
| 24 | -Cl | 0.5 | 1 |
| 25 | -Br | 0.5 | > 2 |
| 26 | 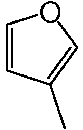 | 0.25 | 1 |
| 27 | 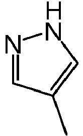 | 1 | > 2 |
| 28 | 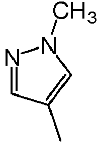 | 1 | 2 |
| 29 | 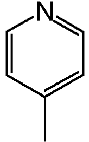 | 1 | 1 |
| 30 | 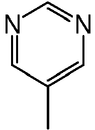 | 2 | 1 |
| 31 | 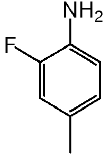 | 0.5 | 1 |
| 32 | 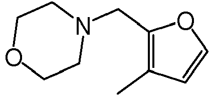 | 0.5 | 1 |
| 33 | 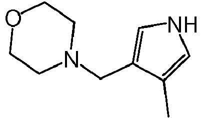 | 2 | 4 |
| 34 | 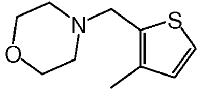 | 1 | 1 |
| 35 | 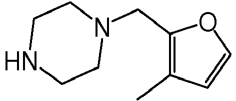 | > 1 | 1 |
| 36 | 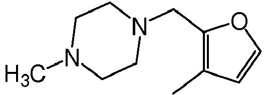 | > 1 | 2 |
| 37 | 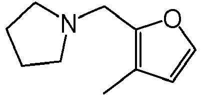 | > 1 | 1 |
| 38 | 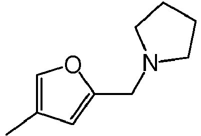 | 1 | 2 |
| 39 | 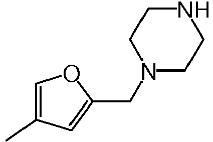 | 1 | 2 |
| 40 | 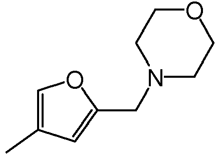 | 0.5 | 2 |
| 41 | 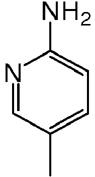 | 1 | 2 |
| 42 | 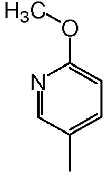 | 0.25 | 1 |
| 43 | 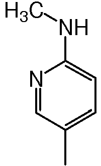 | 1 | 1 |
| 44 | 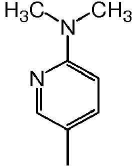 | 0.5 | 2 |
| 45 | 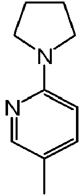 | 0.5 | 1 |
| 46 | 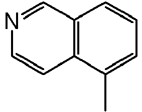 | 0.5 | 1 |
| 47 | 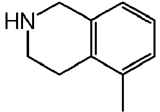 | 0.5 | 1 |
| 48 | 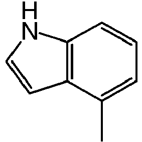 | 0.5 | 1 |
| 49 | 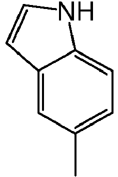 | 0.5 | > 1 |
| 50 | 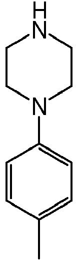 | 2 | 1 |
| 51 | 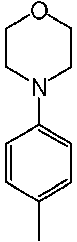 | 0.5 | 2 |
| 52 | 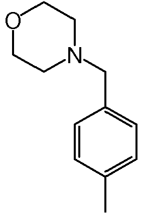 | 1 | 2 |
| 53 | 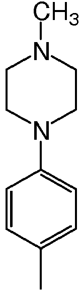 | 0.5 | 2 |
| 54 | 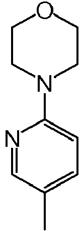 | 1 | 2 |
| 55 | 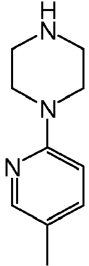 | > 1 | 2 |
| 56 | 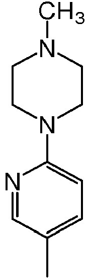 | 1 | 2 |
| 57 | 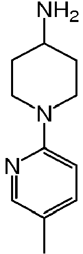 | > 2 | 1 |
| 58 | 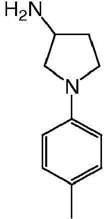 | 1 | 2 |
| 59 | 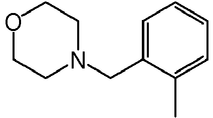 | 0.5 | > 1 |

**Table 4. Analogs of Compound 1**

| COMPOUND | CHEMICAL STRUCTURE | *P. aeruginosa* (µg/ml) | *E. coli* (µg/ml) |
|---|---|---|---|
| | 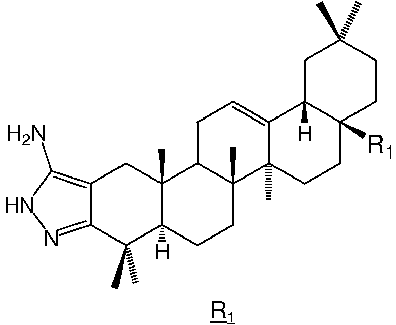 | | |
| 60 | 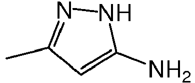 | 0.5 | 1 |
| 61 | 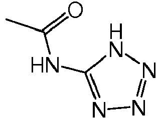 | 0.5 | 0.5 |
| 62 | 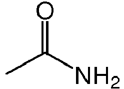 | 1 | 1 |
| 63 | 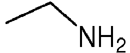 | 4 | 1 |
| 64 | 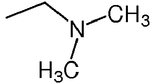 | 0.5 | 1 |
| 65 | 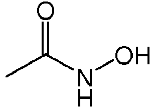 | 0.25 | 0.25 |
| 66 | 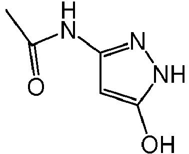 | 0.25 | 1 |
| 67 | 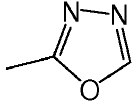 | 1 | 2 |
| 68 | 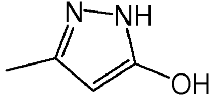 | 0.5 | 0.5 |
| 69 | 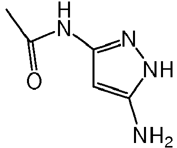 | 1 | 1 |
| 70 | 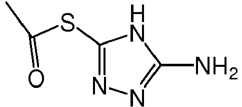 | 0.5 | 2 |
| 71 | 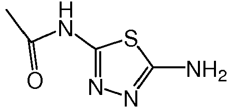 | 0.5 | 2 |
| 72 | 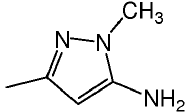 | 1 | >2 |
| 73 | 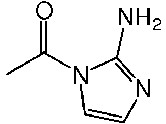 | 1 | 1 |
| 74 | 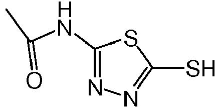 | 1 | >2 |
| 75 | 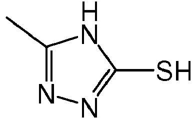 | 0.5 | 1 |
| 76 | 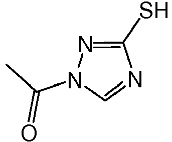 | 0.5 | 2 |
| 77 | 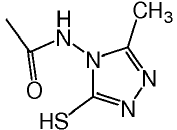 | 1 | 2 |
| 78 | 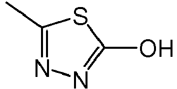 | >1 | 1 |
| 79 | 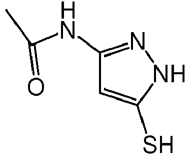 | 0.25 | 0.5 |
| 80 | 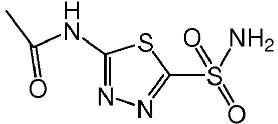 | 1 | 1 |
| 81 | 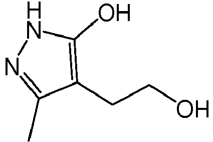 | 0.5 | 1 |
| 82 | 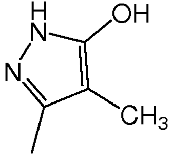 | 0.5 | 1 |
| 83 | 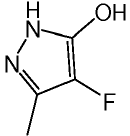 | 0.25 | 0.5 |
| 84 | 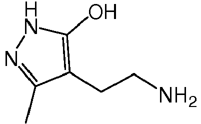 | 0.125 | 0.5 |
| 85 | 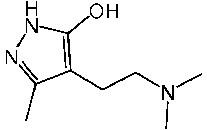 | 0.5 | 1 |
| 86 | 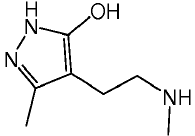 | 0.5 | 2 |
| 87 | 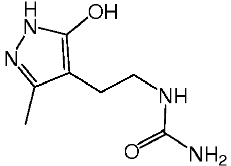 | 0.5 | 1 |
| 88 | 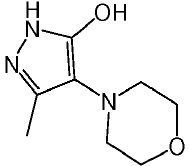 | 0.25 | 0.5 |
| 89 | 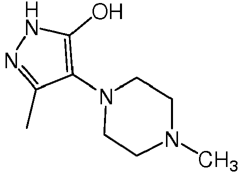 | 1 | 1 |
| 90 | 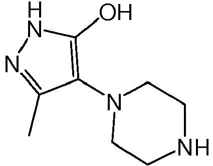 | 0.5 | 1 |

**Table 5. Analogs of Compound 1. Compounds 91 and 92 demonstrate that combined modifications at the R¹ and R² positions can be prepared and retain potent inhibitory activities against biofilms.**

| COMPOUND | CHEMICAL STRUCTURE | *P*. *aeruginosa* (µg/ml) | *E. coli* (µg/ml) |
|---|---|---|---|
| 91 | 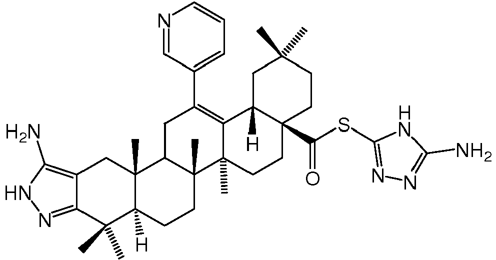 | 1 | 1 |
| 92 | 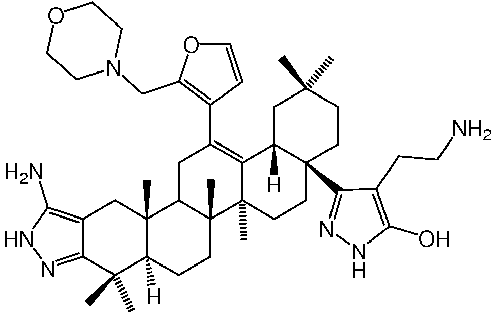 | 0.5 | 2 |

### EXAMPLE III

### SEMI-SYNTHESIS PROCEDURES FOR THE COMPOUNDS SHOWN IN EXAMPLE II

Preparative HPLC: Preparative HPLC was conducted using a SunFire Prep C18 OBD Column, 5 µm, 19 × 100 mm eluting with a gradient from 90:10 (water:acetonitrile, both with 0.05% trifluoroacetic acid) to 10:90 (water:acetonitrile, both with 0.05% trifluoroacetic acid) over 14 minutes followed by a 2 minute hold at 10:90 (water:acetonitrile, both with 0.05% trifluoroacetic acid) with a flow rate at 25 mL/min. CMA: CMA = 80:18:2 Methylene Chloride/Methanol/concentrated Ammonium Hydroxide

### Preparation of Common Intermediate I

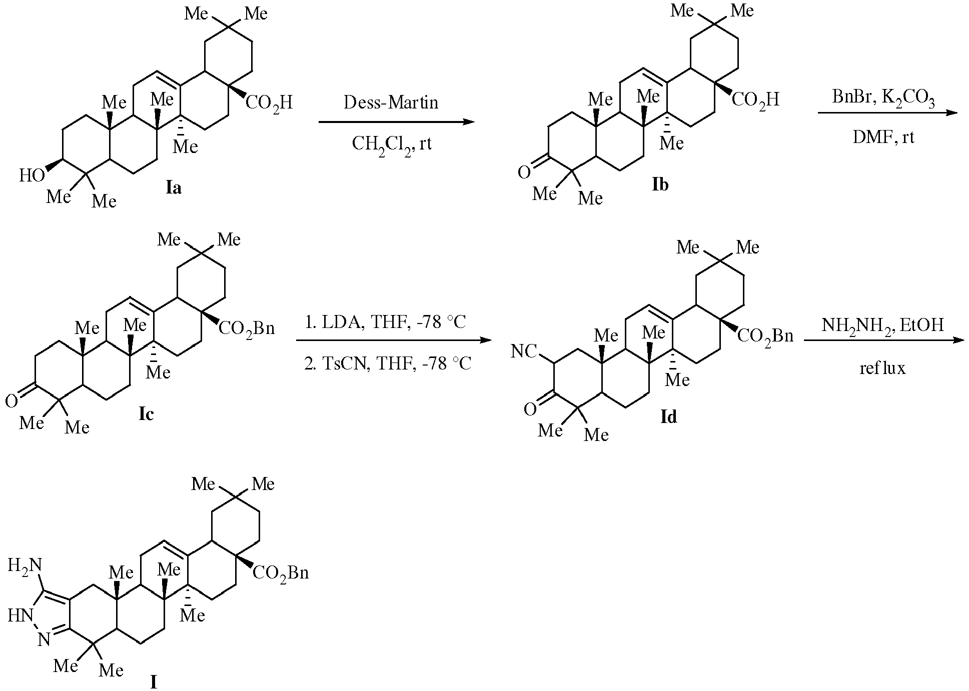

### (i) Preparation of Ib: (4aS,6aS,6bR,12aR)-2,2,6a,6b,9,9,12a-Heptamethyl-10-oxo-1,2,3,4,4a,5,6,6a,6b,7,8,8a,9,10,11,12,12a,12b,13,14b-icosahydropicene-4a-carboxylic acid

To a mixture of oleanolic acid (**Ia**, 5.0 g, 10.9 mmol) and CH₂Cl₂ (200 mL) was added the Dess-Martin reagent (6.0 g, 14.2 mmo l) under nitrogen at room temperature. After stirring at room temperature for 1 hour, the starting material was consumed, as indicated by TLC (1:1 hexanes:diethyl ether). The reaction mixture was quenched with the addition of a solution of sodium thiosulfate and NaHCO₃ (50 g sodium thiosulfate in 200 mL saturated NaHCO₃ solution). The mixture was stirred at room temperature for 10 minutes. The layers were separated and the aqueous layer was extracted with EtOAc (3 × 250 mL). The combined organics were dried (Na₂SO₄), filtered and concentrated under reduced pressure. The residue was purified by column chromatography (silica, 3:1 hexanes/diethyl ether) to provide the sub-title compound (4.9 g, 99%) as a white foam.
¹H NMR (300 MHz, CDCl₃) 0.81 (s, 3H), 0.90-2.05 (m, 39H), 2.42-2.44 (m, 1 H), 2.59-2.61 (m, 1 H), 2.83-2.85 (m, 1 H), 5.29-5.31 (m, 1 H). ESI MS *m*/*z* 455 [C₃₀H₄₆O₃ + H]⁺.

### (ii) Preparation of Ic: (4aS,6aS,6bR,12aR)-Benzyl 2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

A mixture of **Ib** (4.9 g, 10.8 mmol), benzyl bromide (1.9 mL, 16.2 mmol), K₂CO₃ (2.2 g, 16.2 mmol) and DMF (185 mL) was stirred at room temperature for 2.5 hours. The solvent then was removed under reduced pressure and the residue was partitioned between H₂O (250 mL) and EtOAc (250 mL). The layers were separated and the aqueous layer was extracted with EtOAc (200 mL). The combined organics were dried (Na₂SO₄) and filtered. The solvent was removed under reduced pressure and the residue was purified by column chromatography (silica, 3:1 hexanes/diethyl ether) to provide the sub-title compound (4.7 g, 81%) as a white solid.
¹H NMR (300 MHz, CDCl₃) 0.65 (s, 3H), 0.89 (s, 3H), 0.92 (s, 3H), 1.09-2.05 (m, 32H), 2.36-2.38 (m, 1H), 2.49-2.51 (m, 1H), 2.87-2.89 (m, 1H), 5.07-5.09 (m, 2H), 5.29-5.31 (m, 1H), 7.29-7.38 (m, 5H). ESI MS *m*/*z* 545 [C₃₇H₅₂O₃ + H]⁺.

### (iii) Preparation of Id: (4aS,6aS,6bR,12aR)-Benzyl11-cyano-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

A solution of diisopropylamine (31.8 mL, 227 mmol) and THF (250 mL) was cooled to -78°C under nitrogen. A solution of *n*-butyllithium (2.5 M in hexanes, 100 mL, 251 mmol) was slowly added, maintaining the internal temperature below -70 °C. The solution was allowed to stir for 30 min and was then slowly added to a solution of **Ic** (65.0 g, 120 mmol) and THF (1.4 L) at -78°C under nitrogen. This solution was stirred for 30 min after which time a suspension of *p*-toluenesulfonyl cyanide (43.3 g, 239 mmol) and THF (200 mL) was added over 45 min. The solution was stirred for 10 min and then quenched by the addition of saturated ammonium chloride solution (250 mL) at -78°C. The mixture was allowed to warm to room temperature overnight. The organic layer was separated and the aqueous layer was extracted with EtOAc. The combined organic layers were dried (Na₂SO₄) and filtered. The solvent was removed under reduced pressure and the residue was purified by column chromatography (silica, 1:1 hexanes/EtOAc) to provide the sub-title compound (58 g, 85%) as a white solid.
¹H NMR(300MHz,CDCl₃) 0.65 (s, 3H), 2.14-2.16 (m, 39H), 2.93-2.95 (m, 1H), 5.06-5.08 (m, 2H), 5.29-5.31 (m, 1H), 7.29-7.37 (m, 5H).

### (iv) Preparation of I: (4aS,6aS,6bR,13aR)-Benzyl12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A solution of **Id** (2.0 g, 3.5 mmol) and hydrazine (0.33 mL, 10.6 mmol) in EtOH (18 mL) was heated at reflux for 16 hours. The solvent and excess hydrazine were removed under reduced pressure. The residue was purified by column chromatography (silica, 0-7% MeOH in CH₂Cl₂) to provide the title compound (1.8 g, 88%) as an off-white solid.
¹H NMR (300 MHz, CDCl₃) 0.69 (s, 3H), 0.85-2.03 (m, 37H), 2.24-2.35 (m, 1 H), 2.92-2.98 (m, 1 H), 5.06-5.08 (m, 2H), 5.22-5.24 (m, 1 H), 7.28-7.37 (m, 5H). APCI MS *m*/*z* 584 [C₃₈H₅₃N₃O₂ + H]⁺.

### Preparation of Common Intermediate II

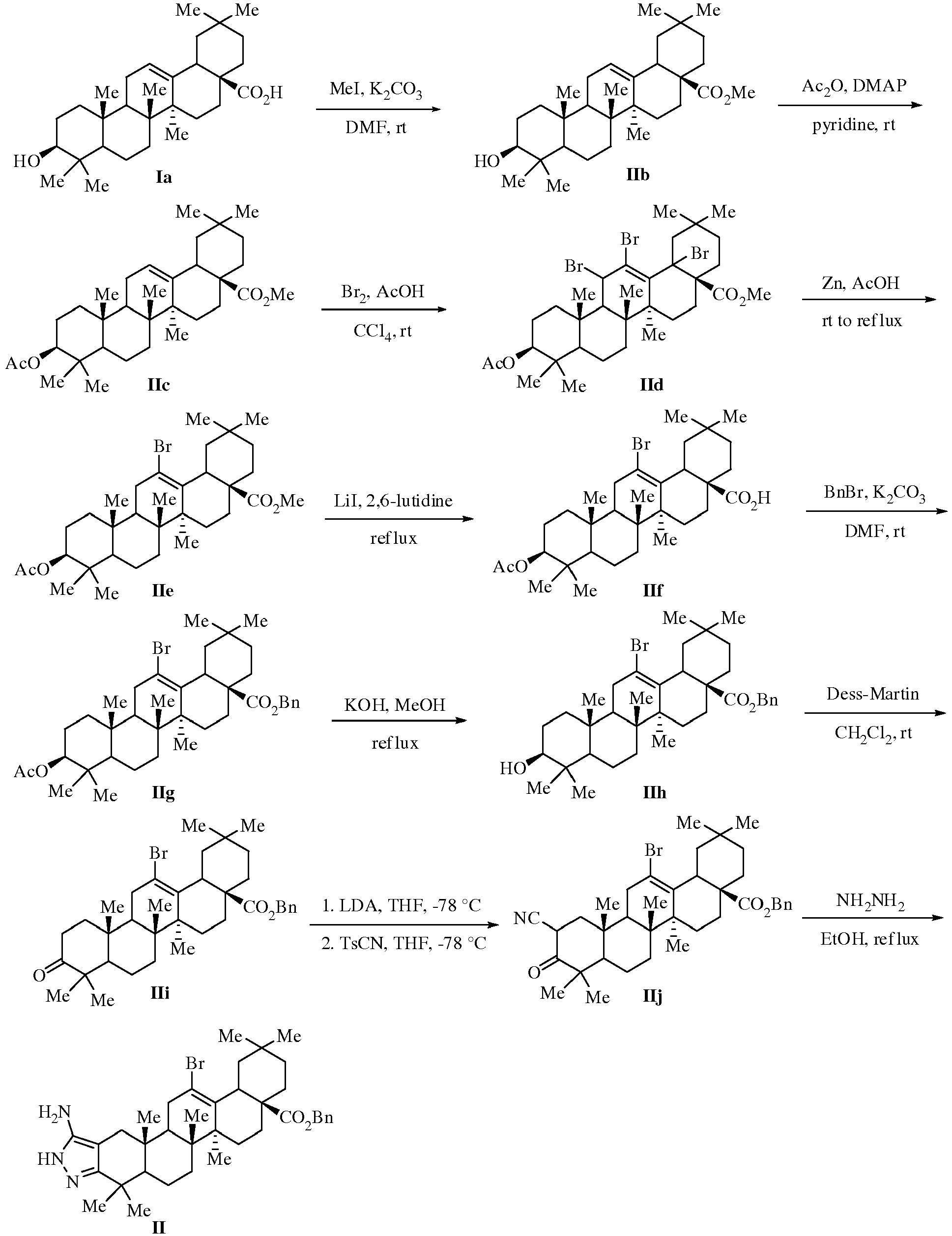

### (i) Preparation of IIb: (4aS,6aS,6bR,10S,12aR)-Methyl 10-hydroxy-2,2,6a,6b,9,9,12a-heptamethyl-1,2,3,4,4a,5,6,6a,6b,7,8,8a,9,10,11,12,12a,12b,13,14b-icosahydropicene-4a-carboxylate

Oleanolic acid (20 g, 43.79 mmol) was dissolved in DMF (200 mL). Potassium carbonate (9.06 g, 65.68 mmol) and methyl iodide (3.3 mL, 52.55 mmol) were added. The mixture was stirred at room temperature overnight and then poured into H₂O (500 mL). The precipitate was collected by filtration. The precipitate was dissolved in EtOAc (500 mL). The solution was washed with brine then dried (Na₂SO₄), filtered and concentrated to give the sub-title compound (20.9 g, 100%) which was used without further purification.
¹H NMR (300 MHz, CDCl₃) δ 0.70-1.97 (m, 42H), 2.83-2.84 (m, 1H), 3.19-3.22 (m, 1 H), 3.62 (s, 3H), 5.28 (t, *J* = 3.6 Hz, 1H).

### (ii) Preparation of IIc: (4aS,6aS,6bR,10S,12aR)-Methyl 10-acetoxy-2,2,6a,6b,9,9,12a-heptamethyl-1,2,3,4,4a,5,6,6a,6b,7,8,8a,9,10,11,12,12a,12b,13,14b-icosahydropicene-4a-carboxylate

To a solution of **IIb** (20.9 g, 44.47 mmol) in pyridine (120 mL) was added DMAP (100 mg, 0.81 mmol) and acetic anhydride (5.04 mL, 53.36 mmol). The mixture was stirred at room temperature for 20 hours and poured into H₂O (300 mL). The precipitate was collected by filtration. The precipitate was dried in a vacuum oven overnight to obtain the sub-title compound (21.33 g, 94%) which was used without further purification.
¹H NMR (300 MHz, CDCl₃) δ 0.72-1.97 (m, 43H), 2.04 (s, 3H), 2.82-2.88 (m, 1H), 3.62 (s, 3H), 4.46-4.51 (m, 1H), 5.27 (t, *J* = 3.6 Hz, 1H).

### (iii) Preparation of IId: (4aR,6aS,6bR,10S,12aS)-Methyl 10-acetoxy-13,14,14b-tribromo-2,2,6a,6b,9,9,12a-heptamethyl-1,2,3,4,4a,5,6,6a,6b,7,8,8a,9,10,11,12,12a,12b,13,14b-icosahydropicene-4a-carboxylate

To a solution of **IIc** (35 g, 68.35 mmol) in AcOH (330 mL) and CCl₄ (160 mL) was added bromine (14.05 mL, 273.4 mmol) slowly. The mixture was stirred at room temperature for 17 hours. The CCl₄ was removed under reduced pressure and the reaction mixture was poured into H₂O (1 L). The precipitate was collected by filtration and then dissolved in EtOAc (1 L). The solution was washed with brine then dried (Na₂SO₄), filtered and concentrated to give the sub-title compound (50.5 g, 98%) which was used without further purification.
¹H NMR (300 MHz, CDCl₃) δ 0.75-1.75 (m, 38H), 2.04 (s, 3H), 2.40-2.43 (m, 2H), 2.57 (t, *J* = 12.6 Hz, 1 H), 3.63 (s, 3H), 4.44-4.50 (m, 1 H), 4.58-4.64 (m, 1H).

### (iv) Preparation of IIe: (4aS,6aS,6bR,10S,12aR)-Methyl 10-acetoxy-14-bromo-2,2,6a,6b,9,9,12a-heptamethyl-1,2,3,4,4a,5,6,6a,6b,7,8,8a,9,10,11,12,12a,12b,13,14b-icosahydropicene-4a-carboxylate

Zinc dust (21.8 g, 335.7 mmol) was added to a solution of **IId** (50.5 g, 67.15 mmol) and AcOH (450 mL). The mixture was stirred at room temperature overnight and then heated at reflux for 2 hours. The reaction mixture was cooled to room temperature and then poured into H₂O (600 mL). The precipitate was collected by filtration and then dissolved in EtOAc (1 L). The solution was washed with brine then dried (Na₂SO₄), filtered and concentrated. The residue was purified by column chromatography (silica, 0-10% EtOAc in hexanes) to obtain the sub-title compound (21.5 g, 54%).
¹H NMR (300 MHz, CDCl₃) δ 0.75-1.78 (m, 40H), 1.93-1.95 (m, 1 H), 2.04 (s, 3H), 2.41 (m, 2H), 3.54 (m, 1 H), 3.63 (s, 3H), 4.48 (m, 1H).

### (v) Preparation of IIf: (4aS,6aS,6bR,10S,12aR)-10-Acetoxy-14-bromo-2,2,6a,6b,9,9,12a-heptamethyl-1,2,3,4,4a,5,6,6a,6b,7,8,8a,9,10,11,12,12a,12b,13,14b-icosahydropicene-4a-carboxylic acid

To a solution of **IIe** (21.5 g, 36.31 mmol) in 2,6-lutidine (300 mL) was added lithium iodide (72.9 g, 544.7 mmol). The mixture was heated at 143 °C overnight and then cooled to room temperature. The mixture was acidified (pH ≈ 4) with aqueous HCl and extracted with EtOAc (300 mL × 3). The combined extracts were washed with H₂O and brine then dried (Na₂SO₄), filtered and concentrated to afford the sub-title compound (22.8 g, 109%) which was used without further purification.
¹H NMR (300 MHz, CDCl₃) δ 0.80-1.82 (m, 42H), 2.04 (s, 3H), 2.41 (m, 2H), 3.51 (m, 1 H), 4.48 (m, 1 H).

### (vi) Preparation of IIg: (4aS,6aS,6bR,10S,12aR)-Benzyl 10-acetoxy-14-bromo-2,2,6a,6b,9,9,12a-heptamethyl-1,2,3,4,4a,5,6,6a,6b,7,8,8a,9,10,11,12,12a,12b,13,14b-icosahydropicene-4a-carboxylate

To a solution of **IIf** (21.2 g, 36.67 mmol) and DMF (350 mL) was added K₂CO₃ and BnBr (as used herein, "Bn" represents benzyl). The reaction mixture was stirred overnight at room temperature and then poured into H₂O (1 L). The precipitate was collected by filtration and then dissolved in EtOAc (1 L). The solution was washed with H₂O and brine then dried (Na₂SO₄), filtered and concentrated to afford the sub-title compound (24.5 g, 100%) which was used without further purification.
¹H NMR (300 MHz, CDCl₃) δ 0.57-2.00 (m, 41 H), 2.04 (s, 3H), 2.34 (d, *J* = 8.7 Hz, 2H), 3.59 (dd, *J* = 4.8, 4.2 Hz, 1 H), 4.49 (m, 1 H), 5.08 (s, 2H), 7.29-7.38 (m, 5H).

### (vii) Preparation of IIh: (4aS,6aS,6bR,10S,12aR)-Benzyl 14-bromo-10-hydroxy-2,2,6a,6b,9,9,12a-heptamethyl-1,2,3,4,4a,5,6,6a,6b,7,8,8a,9,10,11,12,12a,12b,13,14b-icosahydropicene-4a-carboxylate

To a solution of **IIg** (24.5 g, 36.67 mmol) in MeOH (500 mL) was added potassium hydroxide (8.2 g, 146.7 mmol) and the mixture was heated at reflux for 2.5 hours. The resulting mixture was concentrated and the residue was dissolved in EtOAc (1 L). The solution was washed with H₂O and brine then dried (Na₂SO₄), filtered and concentrated. The residue was purified by column chromatography (silica, 0-30% EtOAc in hexanes) to afford the sub-title compound (21.3 g, 93%).
¹H NMR (300 MHz, CDCl₃) δ 0.58-1.81 (m, 41 H), 2.01 (m, 1 H), 2.37 (d, *J* = 9.3 Hz, 2H), 3.21 (m, 1 H), 3.58 (dd, *J* = 4.8, 4.1 Hz, 1 H), 5.08 (s, 2H), 7.29-7.38 (m, 5H).

### (viii) Preparation of IIi: (4aS,6aS,6bR,12aR)-Benzyl14-bromo-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylatelate

To a solution of **IIh** (21.3 g, 34.02 mmol) in CH₂Cl₂ (400 mL) was added the Dess-Martin reagent (18.76 g, 44.23 mmol). The mixture was stirred at room temperature for 5 hours, quenched with aqueous sodium thiosulfate and NaHCO₃ and then extracted with EtOAc (3 x 300 mL). The organic solution was washed with H₂O and brine then dried (Na₂SO₄), filtered and concentrated. The residue was purified by column chromatography (silica, 0-15% EtOAc in hexanes) to afford the sub-title compound (19.3g, 90%).
¹H NMR (300 MHz, CDCl₃) δ 0.24-2.27 (m, 40H), 2.94 (d, *J* = 8.7 Hz, 1 H), 3.44 (dd, *J* = 4.8, 4.2 Hz, 1 H), 5.13-5.15 (m, 2H), 7.33-7.35 (m, 5H).

### (ix) Preparation of IIj: (4aS,6aS,6bR,12aR)-Benzyl 14-bromo-11-cyano-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

To a solution of diisopropylamine (0.64 mL, 4.5 mmol) in THF (15 mL) was added *n*-butyl lithium (2.5 M; 1.9 mL, 4.8 mmol) at -78°C. The mixture was stirred at -78°C for 10 min. The LDA solution was added to a solution of **IIi** (1.5 g, 2.4 mmol) in THF (40 mL) pre-cooled to -78°C. The mixture was stirred at-78°C for 1 hour. A solution of 4-methylbenzenesulfonyl cyanide (815 mg, 4.5 mmol) in THF (5 mL) was added and stirred for 30 min at -78°C. The reaction mixture was warmed to -20 °C over 1 hour. The reaction mixture was quenched with saturated NH₄Cl solution and extracted with EtOAc (3 × 50 mL). The organic layer was washed with H₂O and brine then dried (Na₂SO₄), filtered and concentrated. The residue was purified by column chromatography (silica, 0-20% EtOAc in hexanes) to afford the sub-title compound (1.2g, 77%).
¹H NMR (300 MHz, CDCl₃) δ 0.56-2.42 (m, 41 H), 3.61-3.89 (m, 2H), 5.08 (s, 2H), 7.35 (m, 5H).

### (x) Preparation of II: (4aS,6aS,6bR,13aR)-Benzyl 12-amino-15-bromo-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a solution of **IIj** (6.4 g, 9.89 mmol) and EtOH (30 mL) was added hydrazine (0.62 mL, 19.78 mmol). The solution was heated at reflux overnight. The reaction mixture was concentrated and then the residue was purified by column chromatography (silica, 0-10% MeOH in CH₂Cl₂) to afford the title compound (5.0 g, 76%).
¹H NMR (300 MHz, CDCl₃) δ 0.63-2.06 (m, 38H), 2.29 (d, *J* = 14.7 Hz, 1 H), 2.45 (d, *J* = 9.0 Hz, 2H), 3.65 (dd, *J* = 4.4, 4.2 Hz, 1 H), 5.08 (s, 2H), 7.29-7.39 (m, 5H). APCI MS (Positive Mode) *m*/*z* 662 [C₃₈H₅₂BrN₃O₂ + H]⁺.

### Preparation of Common Intermediate III

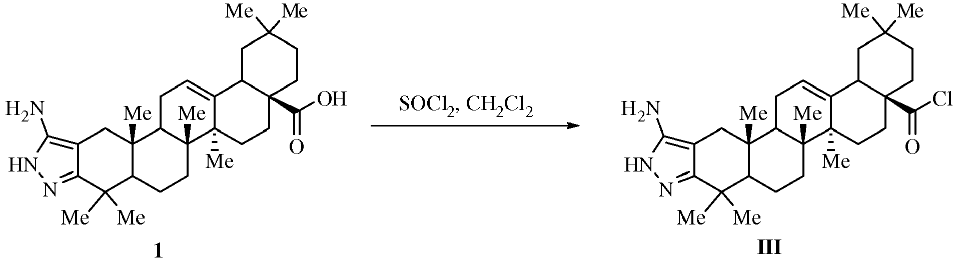

### (i) Preparation of III: (4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carbonyl chloride

To a suspension of 1 (as defined above) (3.0 g, 6.08 mmol) in CH₂Cl₂ was added thionyl chloride (4.4 mL, 60.85 mmol) at room temperature. The mixture was stirred for 2.5 hours, after which time the solvent was removed under reduced pressure. The residue was dried in a high vacuum to provide the title compound (3.2 g, >100%). The material was used without further purification. APCI MS (Positive Mode) *m*/*z* 512 [C₃₁H₄₆ClN₃O + H]⁺.

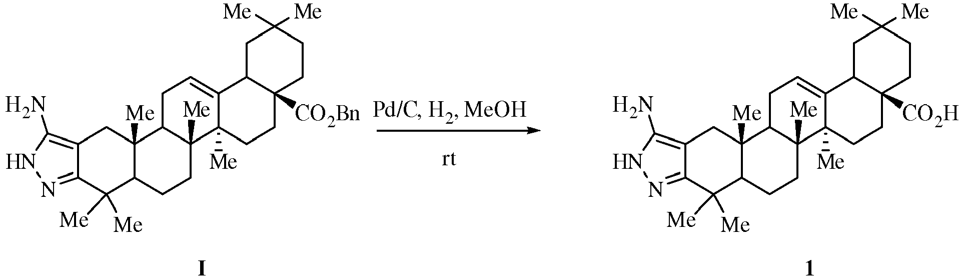

### (i) Preparation of 1: (4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A suspension of **I** (as defined above)(1.8g, 3.1 mmol), 10% Pd/C (1.0 g) and MeOH (100 mL) was stirred under hydrogen at atmospheric pressure for 2 hours. The catalyst was removed by filtration through diatomaceous earth and the filtrate was concentrated under reduced pressure to provide the title compound (1.4 g, 88%) as an off-white solid. *R_{f}* 0.37 (9:1 Methylene Chloride/Methanol) m.p. 263-266 °C.
H NMR (500 MHz, CD₃OD) δ 0.82-0.98 (m, 9H), 1.12-1.31 (m, 15H), 1.39-1.87 (m, 13H), 2.01-2.10 (m, 3H), 2.35-2.42 (m, 1 H), 2.87-2.91 (m, 1 H), 5.28-5.33 (m, 1H). ESI MS *m*/*z* 494 [C₃₁H₄₇N₃O₂ + H]⁺. HPLC 98.8% (area %), *t*_{R} = 16.2 min.

### Example 2

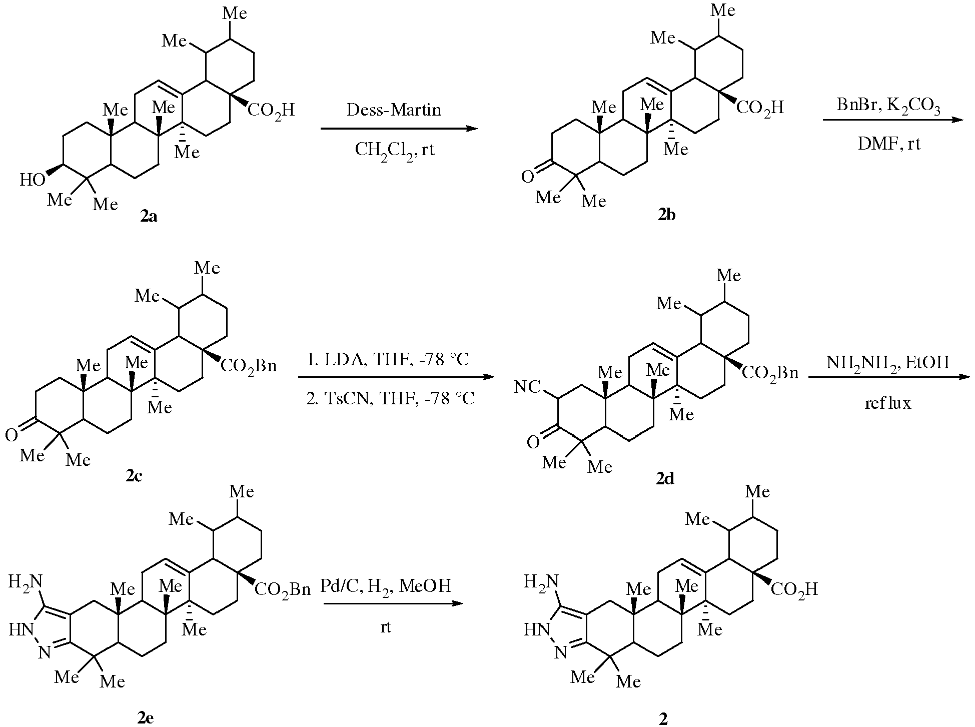

### (i) Preparation of 2b: (4aS,6aS,6bR,12aR)-1,2,6a,6b,9,9,12a-Heptamethyl-10-oxo-1,2,3,4,4a,5,6,6a,6b,7,8,8a,9,10,11,12,12a,12b,13,14b-icosahydropicene-4a-carboxylic acid

To a mixture of ursolic acid (**2a**, 1.0 g, 2.18 mmol) and CH₂Cl₂ (55 mL) was added the Dess-Martin reagent (1.2 g, 2.83 mmol) under nitrogen at room temperature. After stirring at room temperature for 2.5 hours, the starting material was consumed as indicated by TLC (1:1 hexanes:diethyl ether). The reaction mixture was quenched with the addition of a solution of sodium thiosulfate and NaHCO₃ (6.3 g sodium thiosulfate in 25 mL saturated NaHCO₃ solution). The mixture was stirred at room temperature for 10 minutes. The layers were separated and the aqueous layer was extracted with EtOAc (3 x 250 mL). The combined organics were dried (Na₂SO₄), filtered and concentrated under reduced pressure. The residue was purified by column chromatography (silica, 3:1 hexanes/diethyl ether) to provide the sub-title compound (924 mg, 92%) as a white foam solid.

### (ii) Preparation of 2c: (4aS,6aS,6bR,12aR)-Benzyl 1,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

A mixture of **2b** (924 mg, 2.03 mmol), benzyl bromide (0.31 mL, 0.64 mmol), K₂CO₃ (393 mg, 2.84 mmol) and DMF (50 mL) was stirred at room temperature for 12 hours. The solvent was then removed under reduced pressure and the residue was partitioned between H₂O (150 mL) and EtOAc (150 mL). The layers were separated and the aqueous layer was extracted with EtOAc (50 mL). The combined organics were dried (Na₂SO₄) and filtered. The solvent was removed under reduced pressure and the residue was purified by column chromatography (silica, 1:1 hexanes/diethyl ether) to provide the sub-title compound (1.0 g, 92%) as a white solid.
¹H NMR (300 MHz, CDCl₃) 0.68 (s, 3H), 0.84 (d, *J* = 6.3 Hz, 3H), 0.92 (d, *J* = 6.0 Hz, 3H), 1.02 (s, 3H), 1.03 (s, 3H), 1.08 (s, 6H), 1.20-2.05 (m, 20H), 2.26 (d, *J* = 15.0 Hz, 1 H), 2.30 (m, 1 H), 2.50 (m, 1 H), 4.96 (m, 2H), 5.24 (m, 1 H), 7.30-7.35 (m, 5H).

### (iii) Preparation of 2d: (4aS,6aS,6bR,12aR)-Benzyl 11-cyano-1,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

A solution of diisopropylamine (0.45 mL, 3.24 mmol) and THF (5 mL) was cooled to -78°C under nitrogen. A solution of *n*-butyllithium (2.5 M in hexanes, 1.43 mL, 3.56 mmol) was slowly added, maintaining the internal temperature below -70 °C. The solution was allowed to stir for 30 min and was then slowly added to a solution of **2c** (880 mg, 1.62 mmol) and THF (10 mL) at -78°C under nitrogen. This solution was stirred for 30 min after which time a suspension of *p*-toluenesulfonyl cyanide (587 mg, 3.24 mmol) and THF (2 mL) was added over 45 min. The solution was stirred for 10 min and then quenched by the addition of saturated ammonium chloride solution (3 mL) at-78°C. The mixture was allowed to warm to room temperature overnight. The organic layer was separated and the aqueous layer was extracted with EtOAc. The combined organic layers were dried (Na₂SO₄) and filtered. The solvent was removed under reduced pressure and the residue was purified by column chromatography (silica, 3:1 hexanes/Et₂O) to provide the sub-title compound (600 mg, 65%) as a white foam solid.

### (iv) Preparation of 2e: (4aS,6aS,6bR,13aR)-Benzyl 12-amino-1,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A solution of **2d** (303 mg, 0.53 mmol) and hydrazine (0.050 mL, 1.59 mmol) in EtOH (5 mL) was heated at reflux for 12 hours. The solvent and excess hydrazine were removed under reduced pressure. The residue was purified by column chromatography (silica, 0-10% MeOH in CH₂Cl₂ to provide the sub-title compound (234 mg, 75%) as a white foam solid.
¹H NMR (300 MHz, CDCl₃) δ 0.69 (s,3H), 0.86 (d, *J* = 5.7 Hz, 3H), 0.88 (s, 3H), 0.93 (d, *J*= 6.0 Hz, 3H), 1.09 (s, 3H), 1.13 (s, 3H), 1.22 (s, 3H), 1.38-2.05 (m, 19H), 2.28 (m, 1 H), 2.30 (d, *J* = 14.7 Hz, 1 H), 4.96 (q, *J* = 12.6 Hz, 2H), 5.29 (s, 1 H), 7.32 (m, 5H).

### (v) Preparation of 2: (4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A suspension of **2e** (234 mg, 0.40 mmol), 10% Pd/C (60 mg) and MeOH (6 mL) was stirred under hydrogen at atmospheric pressure for 9 hours. The catalyst was removed by filtration and the filtrate was concentrated under reduced pressure. The residue was purified by column chromatography (silica, 0-15% MeOH in CH₂Cl₂) to provide the title compound (103 mg, 52%) as an off-white solid.
*R_{f}* 0.16 (89:18:2 Methylene Chloride/Methanol/concentrated Ammonium Hydroxide).
m.p. 258-278°C. ¹H NMR (300 MHz, CD₃OD) δ 0.89 (s, 9H), 0.91 (s, 3H), 1.15 (s, 6H), 1.19 (s, 3H), 2.05-2.10 (m, 19H), 2.22 (m, 1 H), 2.40 (m, 1 H), 5.30 (s, 1H). ESI MS *m*/*z* 494 [C₃₁H₄₇N₃O₂ + H]⁺.

### Example 3

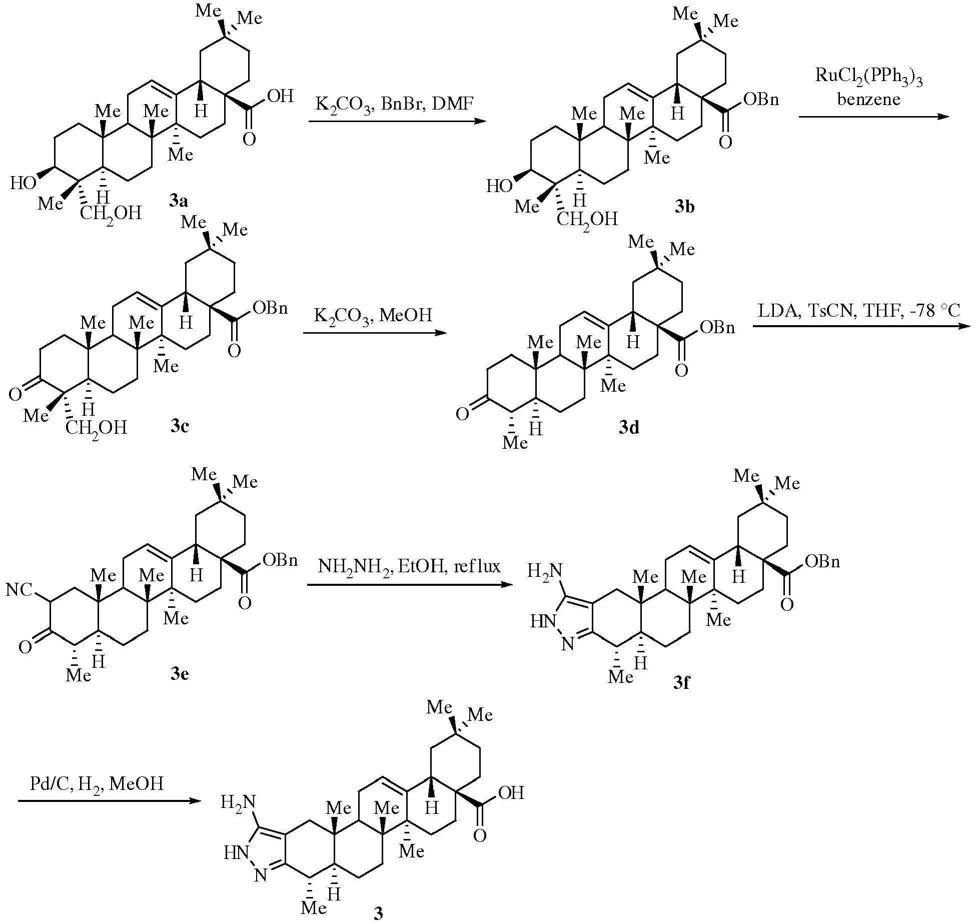

### (i) Preparation of 3b: (4aS,6aS,6bR,8aR,9R,10S,12aR,14bS)-Benzyl 10-hydroxy-9-(hydroxymethyl)-2,2,6a,6b,9,12a-hexamethyl-1,2,3,4,4a,5,6,6a,6b,7,8,8a,9,10,11,12,12a,12b,13,14b-icosahydropicene-4a-carboxylate

A mixture of **3a** (2.0 g, 4.2 mmol), benzyl bromide (0.6 mL, 5.0 mmol) and K₂CO₃ (580 mg, 6.3 mmol) in DMF (20 mL) was stirred at room temperature for 12 hours. The reaction mixture was diluted with EtOAc (300 mL). The organic phase was washed with brine then dried (MgSO₄), filtered and concentrated to dryness. The residue was purified by column chromatography (silica, 0-50% EtOAc in hexanes) to afford the sub-title compound (2.3 g, 95%).

### (ii) Preparation of 3c: (4aS,6aS,6bR,8aR,9R,12aR,14bS)-Benzyl 9-(hydroxymethyl)-2,2,6a,6b,9,12a-hexamethyl-10-oxo-1,2,3,4,4a,5,6,6a,6b,7,8,8a,9,10,11,12,12a,12b,13,14b-icosahydropicene-4a-carboxylate

To a solution of **3b** (400 mg, 0.71 mmol) in benzene (20 mL) was added RuCl₂(PPh₃)₃ (682 mg, 0.71 mmol). The mixture was stirred at room temperature for 12 hours. The reaction was not complete. An additional amount of RuCl₂(PPh₃)₃ (341 mg, 0.36 mmol) was added and the reaction mixture was continued to stir for 8 hours. The reaction mixture was diluted with EtOAc (150 mL). The organic phase was washed with brine then dried (MgSO₄), filtered and concentrated to dryness. The residue was purified by column chromatography (silica, 0-50% EtOAc in hexanes) to afford the sub-title compound (150 mg, 38%).

### (iii) Preparation of 3d: (4aS,6aS,6bR,8aS,9S,12aS,14bS)-Benzyl

### 2,2,6a,6b,9,12a-hexamethyl-10-oxo-1,2,3,4,4a,5,6,6a,6b,7,8,8a,9,10,11,12,12a,12b,13,14b-icosahydropicene-4a-carboxylate

A mixture of **3c** (150 mg, 0.26 mmol) and K₂CO₃ (44 mg, 0.32 mmol) in MeOH (10 mL) was stirred at room temperature for 24 hours. The reaction mixture was diluted with EtOAc (150 mL) and washed with brine then dried (MgSO₄), filtered and concentrated to dryness. The residue was purified by column chromatography (silica, 0-30% EtOAc in hexanes) to afford the sub-title compound (70 mg, 49%).

### (iv) Preparation of 3e: (4aS,6aS,6bR,8aS,9S,12aS,14bS)-Benzyl 11-cyano-2,2,6a,6b,9,12a-hexamethyl-10-oxo-1,2,3,4,4a,5,6,6a,6b,7,8,8a,9,10,11,12,12a,12b,13,14b-icosahydropicene-4a-carboxylate

To a solution of diisopropylamine (0.073 mL, 0.52 mmol) in THF (4 mL) was added *n*-butyllithium (0.22 mL, 2.5 M in hexanes, 0.54 mmol) at-78°C. The solution was stirred for 30 min. The LDA solution was added to **3d** (150 mg, 0.27 mmol) in THF (5 mL). The mixture was allowed to warm to -40°C for 5 min and cooled to -78°C. A suspension of *p*-toluene sulfonyl cyanide (98 mg, 0.54 mmol) in THF (2 mL) was added. The reaction mixture was allowed to warm to -40°C over 1.5 hours. The reaction was quenched by saturated NH₄Cl (3 mL) and extracted with EtOAc (100 mL). The organic phase was washed with brine then dried (MgSO₄), filtered and concentrated to dryness. The residue was purified by column chromatography (silica, 0-30% EtOAc in hexanes) to afford the sub-title compound (90 mg, 58%).
¹H NMR (300 MHz, CDCl₃) δ 0.65 (s, 3H), 0.86 (s, 3H), 0.88 (s, 3H), 1.0-2.0 (m, 28H), 2.35 (m, 2H), 2.92 (m, 1 H), 3.61 (m, 1 H), 5.15 (s, 2 H), 5.30 (m, 1 H), 7.30 (s, 5H).

### (v) Preparation of 3f: (4aS,6aS,6bR,8aS,9S,13aS,15bS)-Benzyl 12-amino-2,2,6a,6b,9,13a-hexamethyl-2,3,4,4a,5,6,6a,6b,7,8,8a,9,11,13,13a,13b,14,15b-octadecahydro-1H-chryseno[1,2-f]indazole-4a-carboxylate

A mixture of **3e** (90 mg, 0.15 mmol) and hydrazine (0.025 mL) in EtOH (3 mL) was heated at reflux overnight. The reaction mixture was concentrated to dryness under reduced pressure. The residue was purified by column chromatography (silica, 0-10% MeOH in CH₂Cl₂) to afford the sub-title compound (60 mg, 65%).

### (v) Preparation of 3: (4aS,6aS,6bR,8aS,9S,13aS,15bS)-12-Amino-2,2,6a,6b,9,13a-hexamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **3f** (60 mg, 0.10 mmol) and 10% Pd(OH)₂/C (60 mg) in MeOH (5 mL) and EtOAc (5 mL) was stirred under hydrogen balloon for 5 hours. The reaction mixture was filtered through a pad of diatomaceous earth and washed with CMA (25 mL). The filtrate was concentrated to dryness under reduced pressure. The residue was purified by column chromatography (silica, 0-60% CMA in CH₂Cl₂) to afford the title compound (32 mg, 68%) as a brown solid.
*R_{f}* 0.40 (80:18:2 Methylene Chloride/Methanol/concentrated Ammonium Hydroxide).
¹H NMR (300 MHz, DMSO-*d*₆) δ 0.78 (s, 3H), 0.80 (s, 3H), 0.91 (s, 6H), 1.05 (s, 3H), 1.10-2.0 (m, 25H), 2.15 (m, 1 H), 2.25 (d, *J* = 15.0 Hz, 1 H), 2.80 (m, 1 H), 5.24 (s, 1 H), 11.4 (bs, 1H). mp >300 °C. ESI MS (Positive Mode) *m*/z 478 [C₃₀H₄₅N₃O₂ + H]⁺_{.}

### Example 4

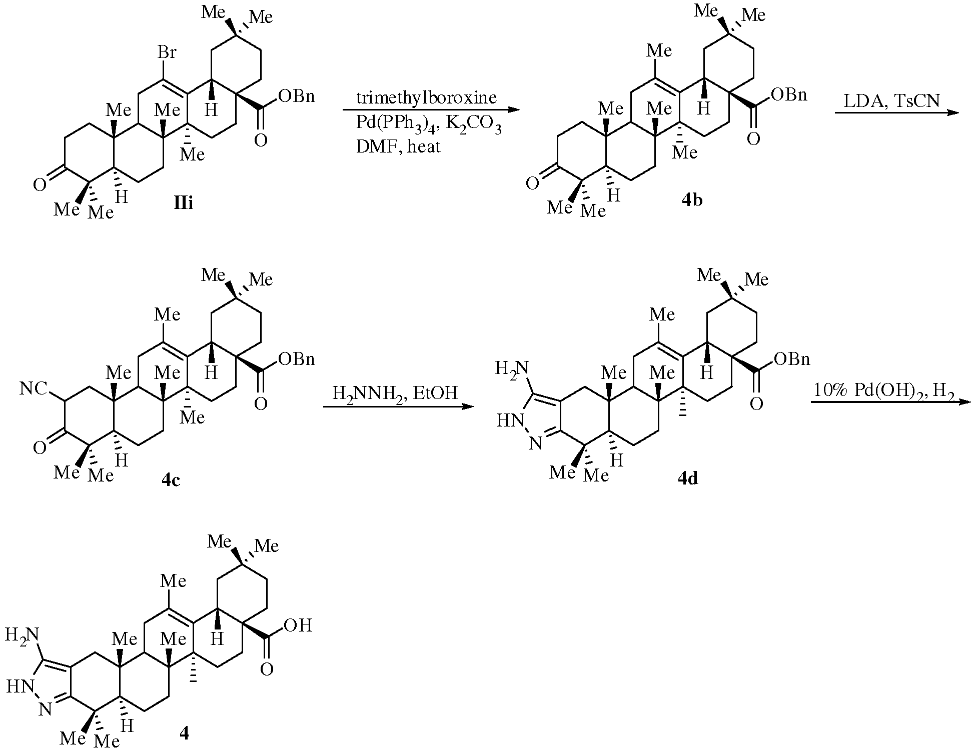

### (i) Preparation of 4b: (4aS,6aS,6bR,8aR,12aR,14bS)-Benzyl 2,2,6a,6b,9,9,12a,14-octamethyl-10-oxo-1,2,3,4,4a,5,6,6a,6b,7,8,8a,9,10,11,12,12a,12b,13,14b-icosahydropicene-4a-carboxylate

A mixture of **IIi** (300 mg, 0.48 mmol), trimethylboroxine (0.3 mL, 2.15 mmol), Pd(PPh₃)₄ (60 mg, 0.048 mmol) and K₂CO₃ (300 mg, 2.17 mmol) in DMF (9 mL) was heated at 100 °C for 12 hours. The reaction mixture was diluted with EtOAc (100 mL). The organic phase was washed with brine then dried (MgSO₄), filtered and concentrated to dryness. The residue was purified by column chromatography (silica, 0-10% EtOAc in hexanes) to afford the sub-title compound (220 mg, 82%).

### (ii) Preparation of 4c: (4aS,6aS,6bR,8aR,12aR,14bS)-Benzyl 11-cyano-2,2,6a,6b,9,9,12a,14-octamethyl-10-oxo-1,2,3,4,4a,5,6,6a,6b,7,8,8a,9,10,11,12,12a,12b,13,14b-icosahydropicene-4a-carboxylate

To a solution of diisopropylamine (0.15 mL, 1.07 mmol) in THF (5 mL) was added *n*-butyllithium (0.45 mL, 2.5 M in hexanes, 1.1 mmol) at -78°C. The solution was stirred for 30 min. The LDA solution was added to **4b** (315 mg, 0.56 mmol) in THF (5 mL). The mixture was allowed to warm to -40°C for 5 min and cooled to -78°C. A suspension of *p*-toluene sulfonyl cyanide (203 mg, 1.1 mmol) in THF (2 mL) was added. The reaction mixture was allowed to warm to -40°C over 1.5 hours. The reaction was quenched by saturated NH₄Cl (3 mL) and extracted with EtOAc (100 mL). The organic phase was washed with brine then dried (MgSO₄), filtered and concentrated to dryness. The residue was purified by column chromatography (silica, 0-20% EtOAc in hexanes) to afford the sub-title compound (215 mg, 66%).

### (iii) Preparation of 4d: (4aS,6aS,6bR,8aR,13aR,15bS)-Benzyl 12-amino-2,2,6a,6b,9,9,13a,15-octamethyl-2,3,4,4a,5,6,6a,6b,7,8,8a,9,11,13,13a,13b,14,15b-octadecahydro-1H-chryseno[1,2-f]indazole-4a-carboxylate

A mixture of **4c** (215 mg, 0.36 mmol) and hydrazine (0.060 mL, 1.85 mmol) in EtOH (3 mL) was heated at reflux overnight. The reaction mixture was concentrated to dryness under reduced pressure. The residue was purified by column chromatography (silica, 0-10% MeOH in CH₂Cl₂) to afford the sub-title compound (180 mg, 84%).
ESI MS (Positive Mode) *m*/*z* 598 [C₃₉H₅₅N₃O₂ + H]⁺.

### (iv) Preparation of 4: (4aS,6aS,6bR,8aR,13aR,15bS)-12-Amino-2,2,6a,6b,9,9,13a,15-octamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **4d** (180 mg, 0.30 mmol) and 10% Pd(OH)₂/C (100 mg) in MeOH (15 mL) and EtOAc (2 mL) was stirred under hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-50% CMA in CH₂Cl₂) followed by preparative HPLC to provide the title compound (28 mg, 18%) as an off-white solid. *R_{f}*0.62 (80:18:2 Methylene Chloride/Methanol/concentrated Ammonium Hydroxide). ¹H NMR (300 MHz, CD₃OD) δ 0.85 (s, 3H), 0.90 (s, 3H), 0.93 (s, 3H), 0.99 (s, 3H), 1.20 (s, 3H), 1.23 (s, 3H), 1.38 (s, 3H), 1.40-2.05 (m, 23H), 2.39 (d, *J* = 14.7 Hz, 1H). mp >300 °C. ESI MS (Positive Mode) *m*/*z* 508 [C₃₂H₄₉N₃O₂ + H]⁺.

### Example 5

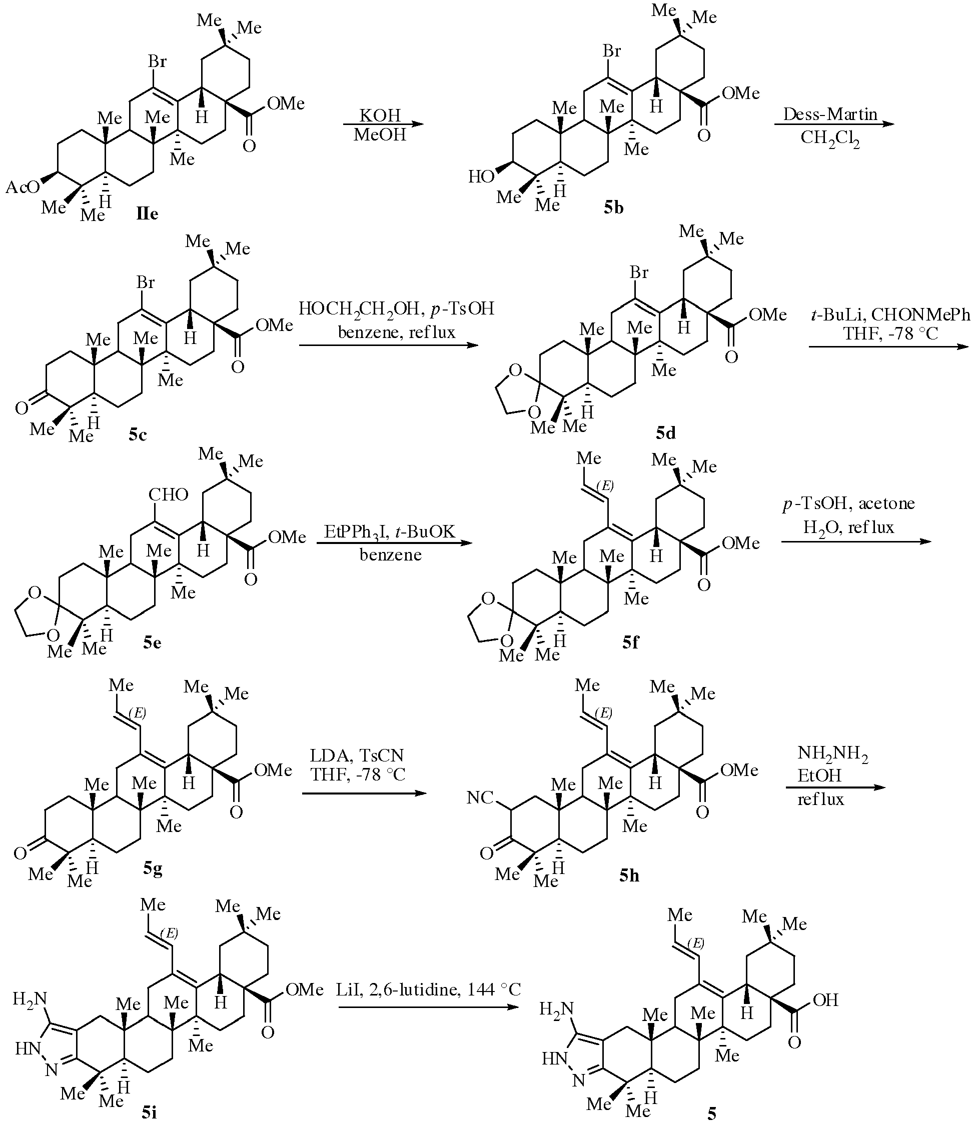

### (i) Preparation of 5b: (4aS,6aS,6bR,8aR,10S,12aR,14bR)-Methyl 14-bromo-10-hydroxy-2,2,6a,6b,9,9,12a-heptamethyl-1,2,3,4,4a,5,6,6a,6b,7,8,8a,9,10,11,12,12a,12b,13,14b-icosahydropicene-4a-carboxylate

**A** mixture of **IIe** (36.0 g, 61.0 mmol) and KOH (13.6 g, 243 mmol) in MeOH (500 mL) was heated at reflux for 3 hours. The mixture was cooled to room temperature and concentrated to remove MeOH. The residue was acidified with HCl (2 M) to pH 5 and extracted with EtOAc (500 mL). The organic phase was washed with brine then dried (MgSO₄), filtered and concentrated to afford the sub-title compound (34.0 g, 94%) which was used for the next step without further purification.
¹H NMR (300 MHz, CDCl₃) δ 0.78 (s, 3H), 0.80 (s, 3H), 0.95 (s, 3H), 0.98 (s, 3H), 1.01 -2.35 (m, 33H), 3.20 (m, 1 H), 3.62 (s, 3H).

### (ii) Preparation of 5c: (4aS,6aS,6bR,8aR,12aR,14bR)-Methyl 14-bromo-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

A mixture of **5b** (32.0 g, 61.0 mmol) and Dess-Martin reagent (31.0 g, 73.0 mmol) in CH₂Cl₂ (500 mL) was stirred at room temperature for 4 hours. The mixture was quenched with saturated sodium thiosulfate (50 mL) and NaHCO₃ (50 mL) and extracted with EtOAc (500 mL). The organic phase was washed with brine then dried (MgSO₄), filtered and concentrated to dryness under reduced pressure. The residue was purified by column chromatography (silica, 5-10% EtOAc in hexanes) to afford the sub-title compound (26.0 g, 81 %).
¹H NMR (300 MHz, CDCl₃) δ 0.80 (s, 3H), 0.90 (s, 3H), 0.98 (s, 3H), 1.02 (s, 3H), 1.05 (s, 6H), 1.06-2.55 (m, 26H), 3.62 (s, 3H).

### (iii) Preparation of 5d: (4a'R,6a'R,6b'S,8a'S,12a'R,14b'R)-Methyl 13'-bromo-4',4',6a',6b',11',11',14b'-heptamethyl-2',4',4a',5',6',6a',6b',7',8',8a',9',10',11',12',12a',14',14a',14b'-octadecahydro-1'H-spiro[[1,3]dioxolane-2,3'-picene]-8a'-carboxylate

A flask equipped with a Dean-Stark trap was charged **5c** (26.0 g, 47.4 mmol), ethylene glycol (7.9 mL, 142.3 mmol) and *p*-toluenesulfoinc acid monohydrate (894 mg, 4.7 mmol) in benzene (500 mL). The mixture was heated at reflux overnight. The reaction mixture was cooled to room temperature and diluted with EtOAc (500 mL). The organic phase was washed with saturated NaHCO₃ (50 mL) and brine (200 mL) then dried (MgSO₄), filtered and concentrated to dryness. The residue was purified by column chromatography (silica, 0-10% EtOAc in hexanes) to afford the sub-title compound (18.0g, 64%).
¹H NMR (300 MHz, CDCl₃) δ 0.74 (s, 3H), 0.83 (s, 3H), 0.92 (s, 3H), 0.94 (s, 3H), 0.97 (s, 3H), 0.99 (s,3H), 1.14 (s, 3H), 1.15-2.05 (m, 21 H), 2.39 (d, *J* = 9.0 Hz, 1 H), 3.57 (m, 1 H), 3.63 (s, 3H), 3.93 (m, 4H).

### (iv) Preparation of 5e: (4a'R,6a'R,6b'S,8a'S,12a'S,14b'R)-Methyl 13'-formyl-4',4',6a',6b',11',11',14b'-heptamethyl-2',4',4a',5',6',6a',6b',7',8',8a',9',10',11',12',12a',14',14a',14b'-octadecahydro-1'H spiro[[1,3]dioxolane-2,3'-picene]-8a'-carboxylate

To a solution of **5d** (3.0 g, 5.1 mmol) in THF (60 mL) was added *tert-*butyllithium (10 mL, 1.5 M in heptane, 15.0 mmol) at -78°C. The mixture was stirred for 20 min. *n*-Methylformanilide (1.8 mL, 15.2 mmol) was added. The reaction mixture was allowed to warm to -10 °C over 1 hour. The reaction mixture was quenched by saturated NH₄Cl (10 mL) and extracted with EtOAc (200 mL). The organic phase was washed with brine (200 mL) then dried (MgSO₄), filtered and concentrated to dryness. The residue was purified by column chromatography (silica, 0-10% EtOAc in hexanes) to afford the sub-title compund (2.0 g, 73%).
¹H NMR (300 MHz, CDCl₃) δ 0.65 (s, 3H), 0.81 (s, 3H), 0.90 (s, 6H), 0.92 (s, 3H), 0.99 (s, 3H), 1.12 (s, 3H), 1.25-2.18 (m, 19H), 2.65 (s, 3H), 3.85 (m, 4H), 10.3 (s, 1H).

### (v) Preparation of 5f: (4a'R,6a'R,6b'S,8a'S,12a'S,14b'R)-Methyl

### 4',4',6a',6b',11',11',14b'-heptamethyl-13'(prop-1-enyl)-2',4',4a',5',6',6a',6b',7',8',8a',9',10',11',12',12a',14',14a',14b'-octadecahydro-1'H-spiro[[1,3]dioxolane-2,3'-picene]-8a'-carboxylate

To a suspension of ethyltriphenylphosphonium iodide (1.2 g, 2.96 mmol) in benzene (20 mL) was added potassium *tert*-butoxide (332 mg, 2.96 mmol). The mixture was stirred at room temperature for 1 hour. A solution of **5e** (400 mg, 0.74 mmol) in THF (5 mL) was added. The mixture was stirred at room temperature for 1.5 hours. The reaction mixture was diluted with EtOAc (100 mL). The organic phase was washed with brine then dried (MgSO₄), filtered and concentrated to dryness. The residue was purified by column chromatography (silica, 0-10% EtOAc in hexanes) to afford the sub-title compound (380 mg, 93%) as a mixture of *cis-* and *trans*-isomers.

### (vi) Preparation of 5g: (4aS,6aS,6bR,8aR,12aR,14bS)-Methyl 2,2,6a,6b,9,9,1 2a. heptamethyl-10-oxo-14-(prop-1-enyl)-1,2,3,4,4a,5,6,6a,6b,7,8,8a,9,10,11,12,12a,12b,13,14b-icosahydropicene-4a-carboxylate

A mixture of **5f** (571 mg, 0.92 mmol) and *p*-toluenesulfonic acid monohydrate (176 mg, 0.92 mmol) in acetone (20 mL) and H₂O (2 mL) was stirred at room temperature for 1.5 hours. The reaction mixture was concentrated to remove acetone under reduced pressure and extracted with EtOAc (100 mL). The organic phase was washed with brine then dried (MgSO₄), filtered and concentrated to dryness. The residue was purified by column chromatography (silica, 0-10% EtOAc in hexanes) to afford the sub-title compound (500 mg, 95%).
¹H NMR (300 MHz, CDCl₃) δ 0.72 (s, 1.8H), 0.81 (s, 1.2H), 0.89 (s, 3H), 0.91 (s, 3H), 1.0-2.50 (m, 38H), 3.65 (s, 3H), 5.40-5.70 (m, 1 H), 6.05 (d, *J* = 12.0 Hz, 0.4H), 6.53 (d, *J* = 15.0 Hz, 0.6H).

### (vii) Preparation of 5h: (4aS,6aS,6bR,8aR,12aR,14bS)-Methyl 11-cyano-2,2,6a,6b,9,9,12a-heptamethyl-10-oxo-14-(prop-1-enyl)-1,2,3,4,4a,5,6,6a,6b,7,8,8a,9,10,11,12,12a,12b,13,14b-icosahydropicene-4a-carboxylate

To a solution of diisopropylamine (0.26 mL, 1.87 mmol) in THF (5 mL) was added *n*-butyllithium (0.78 mL, 2.5 M in hexanes, 1.96 mmol) at-78°C. The solution was stirred for 30 min. The LDA solution was added to **5g** (500 mg, 0.98 mmol) in THF (5 mL). The mixture was allowed to warm to -40°C for 5 min and cooled to -78°C. A suspension of *p*-toluene sulfonyl cyanide (339 mg, 1.87 mmol) in THF (2 mL) was added. The reaction mixture was allowed to warm to -40°C over 1.5 hours. The reaction was quenched by saturated NH₄Cl (3 mL) and extracted with EtOAc (100 mL). The organic phase was washed with brine then dried (MgSO₄), filtered and concentrated to dryness. The residue was purified by column chromatography (silica, 0-20% EtOAc in hexanes) to afford the sub-title compound (210 mg, 40%).
¹H NMR (300 MHz, CDCl₃) δ 0.65-2.01 (m, 44H), 2.42-2.80 (m, 1 H), 3.60 (s, 3H), 4.21 (m, 1 H), 5.41-5.70 (m, 1 H), 6.01 (m, 1H).

### (viii) Preparation of 5i: (4aS,6aS,6bR,8aR,13aR,15bS)-Methyl 12-amino-2,2,6a,6b,9,9,13a-heptamethyl-15-((E)-prop-1-enyl)-2,3,4,4a,5,6,6a,6b,7,8,8a,9,11,13,13a,13b,14,15b-octadecahydro-1H-chryseno[1,2-f]indazole-4a-carboxylate

A mixture of **5h** (200 mg, 0.37 mmol) and hydrazine (0.014 mL, 0.45 mmol) in EtOH (3 mL) was heated at reflux overnight. The reaction mixture was concentrated to dryness under reduced pressure. The residue was purified by column chromatography (silica, 0-10% MeOH in CH₂Cl₂) to afford the sub-title compound (130 mg, 64%).
¹H NMR (300 MHz, CDCl₃) δ 0.71 (s, 1.8H), 0.81 (s, 1.2H), 0.88 (s, 3H), 0.89 (s, 6H), 0.98 (s, 1.8H), 1.02 (s, 1.2H), 1.05 (s, 3H), 1.12 (s, 3H), 1.16-2.35 (m, 27H), 3.65 (s, 3H), 5.50 (m, 0.4H), 5.70 (m, 0.6H), 6.05 (d, *J*= 12.0 Hz, 0.4H), 6.55 (d, *J*= 15.0 Hz, 0.6H).

### (ix) Preparation of 5: (4aS,6aS,6bR,8aR,13aR,15bS)-12-Amino-2,2,6a,6b,9,9,13a-heptamethyl-15-((E)-prop-1-enyl)-2,3,4,4a,5,6,6a,6b,7,8,8a,9,11,13,13a,13b,14,15b-octadecahydro-1H-chryseno[1,2-f]indazole-4a-carboxylic acid

A mixture of **5h** (90 mg, 0.16 mmol) and lithium iodide (330 mg, 2.47 mmol) in 2,6-lutidine (3 mL) was heated to 144 °C for 6 hours. The reaction mixture was cooled to room temperature and neutralized with HCl (2 M) and extracted with CH₂Cl₂/*i*-PrOH (3:1). The organic phase was dried (MgSO₄), filtered and concentrated to dryness under reduced pressure. The residue was purified by column chromatography (silica, 0-40% CMA in CH₂Cl₂) followed by preparative HPLC to provide the title compound (20 mg, 23%) as an off-white solid.
*R_{f}* 0.85 (80:18:2 Methylene Chloride/Methanol/concentrated Ammonium Hydroxide).
¹H NMR (300 MHz, CD₃OD) δ 0.80 (s, 3H), 0.90 (s, 6H), 1.02 (s, 3H), 1.18 (s, 3H), 1.20 (s, 3H), 1.28 (s, 3H), 1.30-2.30 (m, 22H), 2.52 (d, *J*= 15.0 Hz, 1 H), 3.63 (m, 1 H), 5.68 (dd, *J*= 6.0, 15.0 Hz, 1 H), 6.59 (d, *J*= 15.0 Hz, 1H). mp>300 °C. ESI MS (Positive Mode) *m*/*z* 534 [C₃₄H₅₁N₃O₂ + H]⁺.

### Example 6

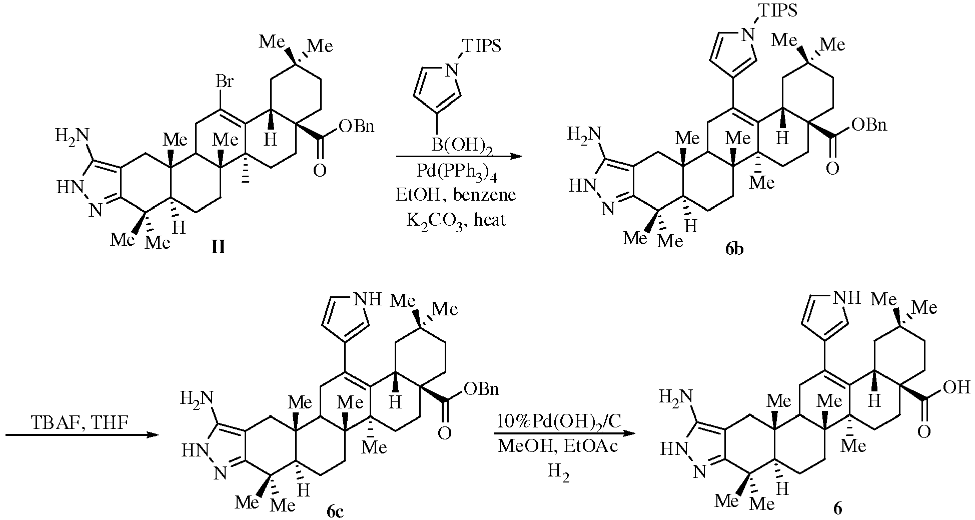

wherein TIPS refers to triisopropylsilyl.

### (i) Preparation of 6b: (4aS,6aS,6bR,8aR,13aR,15bS)-Benzyl 12-amino-2,2,6a,6b,9,9,13a-heptamethyl-15-(1-(triisopropylsilyl)-1H-pyrrol-3-yl)-2,3,4,4a,5,6,6a,6b,7,8,8a,9,11,13,13a,13b,14,15b-octadecahydro-1H-chryseno[1,2-indazole-4a-carboxylate

A mixture of **II** (250 mg, 0.37 mmol), 1-(triisopropylsilyl)-1*H*-pyrrol-3-ylboronic acid (303 mg, 1.13 mmol), Pd(PPh₃)₄ (43 mg, 0.037 mmol) and K₂CO₃ (203 mg, 1.47 mmol) in benzene (3.5 mL) and EtOH (1.5 mL) was sealed and heated to 120 °C by microwave for 1 hour. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (130 mg, 43%). APCI MS (Positive Mode) *m*/*z* 805 [C₅₁H₇₆N₄O₂Si + H]⁺.

### (ii) Preparation of 6c: (4aS,6aS,6bR,8aR,13aR,15bS)-Benzyl 12-amino-2,2,6a,6b,9,9,13a-heptamethyl-15-(1H-pyrrol-3-yl)-2,3,4,4a,5,6,6a,6b,7,8,8a,9,11,13,13a,13b,14,15b-octadecahydro-1H-chryseno[1,2-f]indazole-4a-carboxylate

To a solution of **6b** (130 mg, 0.16 mmol) in THF (2 mL) was added tetrabutylammonium fluoride (0.49 mL, 1 M in THF, 0.49 mmol). The mixture was stirred at room temperature for 2 hours. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (90 mg, 87%). APCI MS (Positive Mode) *m*/*z* 649 [C₄₂H₅₆N₄O₂ + H]⁺.

### (iii) Preparation of 6: (4aS,6aS,6bR,8aR,13aR,15bS)-12-Amino-2,2,6a,6b,9,9,13a-heptamethyl-15-(1H-pyrrol-3-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **6c** (90 mg, 0.13 mmol) and 10% Pd(OH)₂/C (50 mg) in MeOH (12 mL) and EtOAc (3 mL) was stirred under a hydrogen balloon for 5 hours. The reaction mixture was filtered through a pad of diatomaceous earth and washed with CMA (25 mL). The filtrate was concentrated to dryness under reduced pressure. The residue was purified by column chromatography (silica, 0-50% CMA in CH₂Cl₂) to afford the title compound (18 mg, 25%) as a brown solid.
*R_{f}*0.26 (9:1 Methylene Chloride/Methanol).
¹H NMR (400 MHz, CD₃OD) δ 0.50 (s, 3H), 0.80 (s, 3H), 0.86 (s, 3H), 0.95 (s, 3H), 1.14 (s, 3H), 1.21 (s, 3H), 1.24 (s, 3H), 1.25-2.18 (m, 19H), 2.26 (s, 1 H), 2.36 (d, *J*= 14.8 Hz, 1 H), 3.62 (m, 1 H), 6.08 (s, 1 H), 6.65 (s, 1 H), 9.91 (s, 1 H). mp >300 °C. APCI MS (Positive Mode) *m*/*z* 559 [C₃₅H₅₀N₄O₂ + H]⁺.

### Example 7

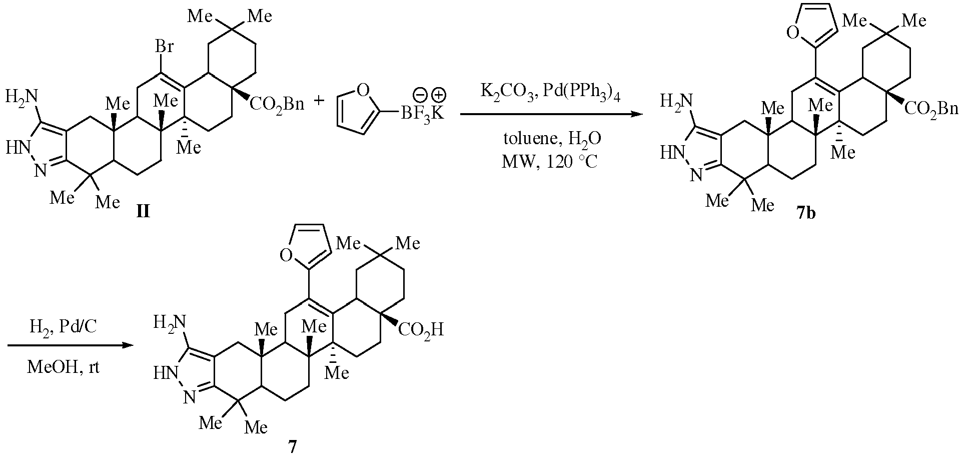

### (i) Preparation of 7b: (4aS,6aS,6bR,13aR)-Benzyl 12-amino-15-(furan-2-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a mixture of **II** (250 mg, 0.377 mmol) and potassium 2-furantrifluoroborate (196 mg, 1.12 mmol) in toluene (4.5 mL) and H₂O (0.5 mL) was added K₂CO₃ (207 mg, 1.5 mmol). The mixture was sparged with nitrogen and then Pd(PPh₃)₄ (86 mg, 0.074 mmol) was added. The reaction mixture was heated at 120 °C for 1 hour using microwave irradiation. The solvent was concentrated under reduced pressure. The residue was taken up in EtOAc (40 mL) and washed with brine (3 x 15 mL). The organic layer was dried (Na₂SO₄), filtered and concentrated. The residue was purified by column chromatography (silica, 0-10% MeOH in CH₂Cl₂) to afford the sub-title compound (200 mg, 81%). APCI MS *m*/*z* 650 [C₄₂H₅₅N₃O₃ + H]⁺.

### (ii) Preparation of 7: (4aS,6aS,6bR,13aR)-12-Amino-15-(furan-2-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **7b** (200 mg, 0.308 mmol) and MeOH (15 mL) was flushed with nitrogen and then 10% Pd/C (200 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-10% MeOH in CH₂Cl₂) followed by preparative HPLC to afford the title compound (19 mg, 11%).
*R_{f}*0.20 (90:9:1 Methylene Chloride/Methanol/concentrated Ammonium Hydroxide).
¹H NMR (400 MHz, CD₃OD) δ 0.78 (s, 3H), 0.86-2.50 (m, 42H), 3.74 (d, *J*= 12.1 Hz, 1 H), 6.38(s, 2H), 7.41 (s, 1H). APCI MS *m*/*z* 560 [C₃₉H₄₉N₃O₃ + H]⁺. m.p. 250-270 °C dec. HPLC (Method A) 98.3% (214 nm) *t*_{R} = 16.6 min.

### Example 8

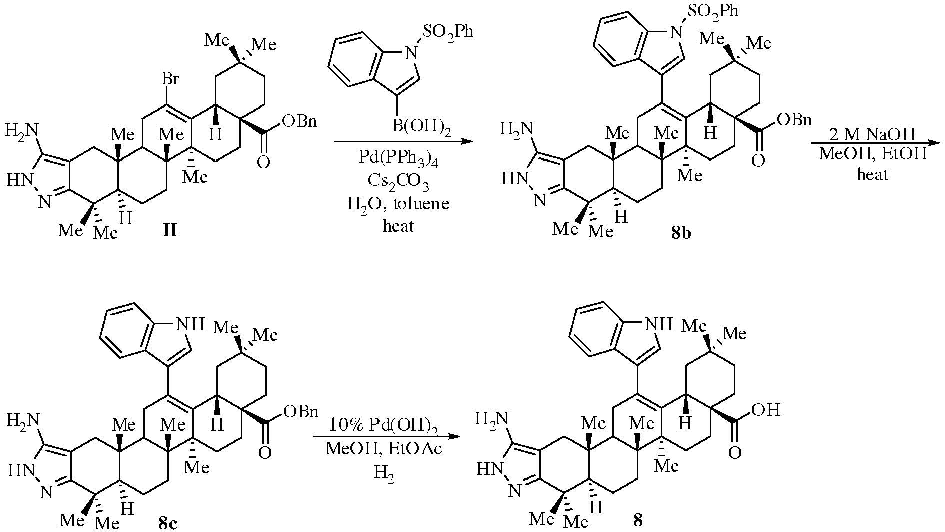

### (i) Preparation of 8b: (4aS,6aS,6bR,8aR,13aR,15bS)-Benzyl 12-amino-2,2,6a,6b,9,9,13a-heptamethyl-15-(1-(phenylsulfonyl)-1 H-indol-3-yl)-2,3,4,4a,5,6,6a,6b,7,8,8a,9,11,13,13a,13b,14,15b-octadecahydro-1H-chryseno[1,2-f]indazole-4a-carboxylate

A mixture of **II** (250 mg, 0.37 mmol), 1-(phenylsulfonyl)-1*H*-indol-3-ylboronic acid (341 mg, 1.13 mmol), Pd(PPh₃)₄ (43 mg, 0.037 mmol) and cesium carbonate (491 mg, 1.50 mmol) in toluene (4.0 mL) and H₂O (1.0 mL) was sealed and heated to 120°C by microwave for 1 hour. The reaction mixture was diluted with EtOAc (100 mL). The organic phase was washed with brine then dried (MgSO₄), filtered and concentrated to dryness. The residue was purified by column chromatography (silica, 0-5% MeOH in CH₂Cl₂) to afford the sub-title compound (264 mg, 83%). APCI MS (Positive Mode) *m*/*z* 839 [C₅₂H₆₂N₄O₄S + H]⁺.

### (ii) Preparation of 8c: (4aS,6aS,6bR,8aR,13aR,15bS)-Benzyl12-amino-15-(1H-indol-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **8b** (260 mg, 0.30 mmol) and NaOH (15 mL, 2.0 M) in MeOH (10 mL) and EtOH (30 mL) was heated at reflux for 24 hours. The reaction mixture was extracted with EtOAc (100 mL). The organic phase was washed with brine then dried (MgSO₄), filtered and concentrated to dryness. The residue was purified by column chromatography (silica, 0-5% MeOH in CH₂Cl₂) to afford the sub-title compound (115 mg, 53%).

### (iii) Preparation of 8: (4aS,6aS,6bR,8aR,13aR,15bS)-12-Amino-15-(1H-indol-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A mixture **8c** (115 mg, 0.16 mmol) and 10% Pd(OH)₂/C (60 mg) in MeOH (15 mL) and EtOAc (5 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-40% CMA in CMA) to afford the title compound (56 mg, 76%) as a brown solid.
*R_{f}* 0.75 (80:18:2 Methylene Chloride/Methanol/concentrated Ammonium Hydroxide).
¹H NMR (400 MHz, CD₃OD) δ 0.14 (s, 3H), 0.65 (s, 3H), 0.88 (s, 3H), 1.09 (s, 3H), 1.16 (s, 3H), 1.26 (s, 3H), 1.34 (s, 3H), 1.35-2.20 (m, 19H), 2.30 (d, *J*= 14.4 Hz, 1 H), 2.40 (m, 1 H), 6.96 (t, *J* = 8.0 Hz, 1 H), 7.05 (t, *J*= 8.0 Hz, 1 H), 7.07 (s, 1 H), 7.31 (d, *J =* 8.0 Hz, 1 H), 7.42 (d, *J* = 8.0 Hz, 1 H). mp >300 °C dec. APCI MS (Positive Mode) *m*/*z* 609 [C₃₉H₅₂N₄O₂ + H]⁺.

### Example 9

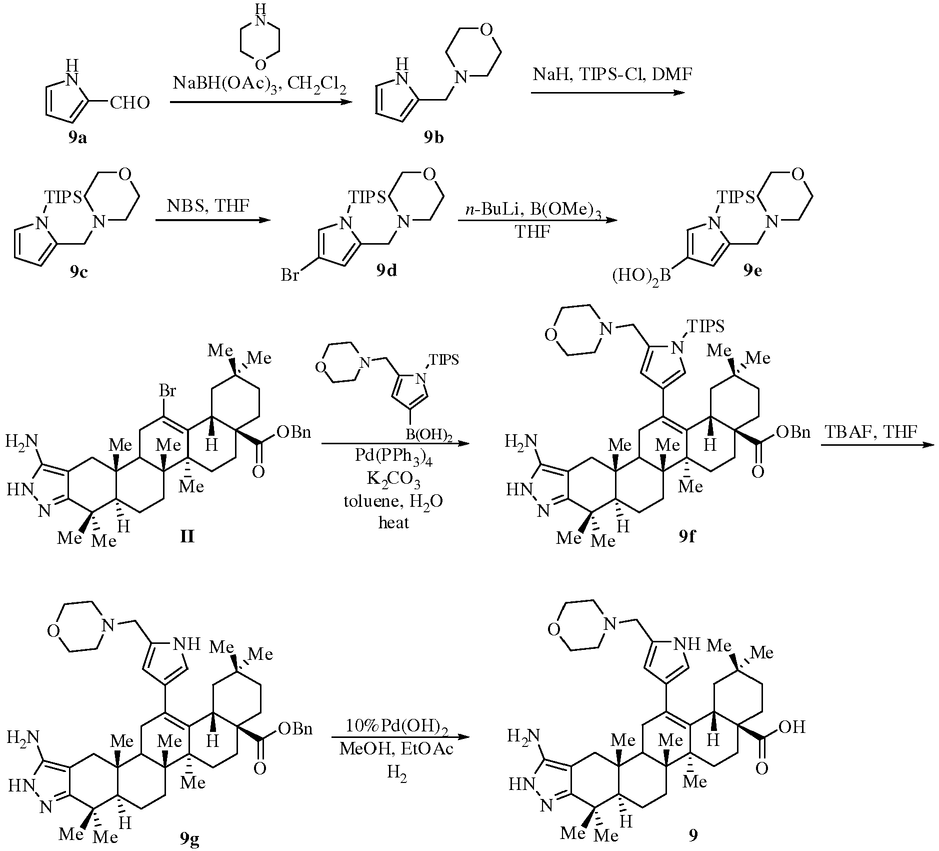

### (i) Preparation of 9b: 4-((1H-Pyrrol-2-yl)methyl)morpholine

To a solution of 1*H*-pyrrole-2-carbaldehyde (5.0 g, 52.5 mmol) and morpholine (5.0 mL, 57.8 mmol) in CH₂Cl₂ (160 mL) was added sodium triacetoxyborohydride (12.2 g, 57.8 mmol). The mixture was stirred at room temperature for 4 hours. The reaction mixture was diluted with EtOAc (200 mL). The organic phase was washed with aqueous NaHCO₃ and brine then dried (MgSO₄), filtered and concentrated to dryness. The residue was purified by column chromatography (silica, 0-10% MeOH in CH₂Cl₂) to afford the sub-title compound (5.3 g, 61%).
¹H NMR (400 MHz, CDCl₃) δ 2.40 (s, 4H), 3.42 (s, 2H), 3.70 (m, 4H), 6.03 (s, 1 H), 6.09 (s, 1 H), 6.74 (s, 1 H), 8.30 (bs, 1H).

### (ii) Preparation of 9c: 4-((1-(Triisopropylsilyl)-1H-pyrrol-2-yl)methyl)morpholine

To a solution of **9b** (5.3 g, 31.9 mmol) in DMF (60 mL) was added sodium hydride (60% in mineral oil, 1.4 g, 35.1 mmol) in an ice bath. The mixture was stirred for 5 min. Chlorotriisopropylsilane (7.4 mL, 35.1 mmol) was added. The reaction mixture was stirred at room temperature for 12 hours. The reaction mixture was diluted with EtOAc (200 mL). The organic phase was washed with brine then dried (MgSO₄), filtered and concentrated to dryness. The residue was purified by column chromatography (silica, 0-20% EtOAc in hexanes) to afford the sub-title compound (10.0 g, 98%).
¹H NMR (400 MHz, CDCl₃) δ 1.10 (d, *J* = 2.5 Hz, 18H), 1.62 (m, 3H), 2.40 (s, 4H), 3.42 (s, 2H), 3.70 (m, 4H), 6.20 (s, 2H), 6.80 (s, 1H).

### (iii) Preparation of 9d: 4-((4-Bromo-1-(triisopropylsilyl)-1H-pyrrol-2-yl)methyl)morpholine

To a solution of **9c** (1.0 g, 3.1 mmol) in THF (10 mL) was added *N*-bromosuccinimide (552 mg, 3.1 mmol) at-78°C. The mixture was stirred at-78°C for 2 hours and warmed to room temperature for 1 hour. The reaction was quenched with H₂O (5 mL) and extracted with EtOAc (100 mL). The organic phase was washed with brine then dried (MgSO₄), filtered and concentrated to dryness. The residue was purified by column chromatography (silica, 0-10% EtOAc in hexanes) to afford the sub-title compound (1.0 g, 83%).
¹H NMR (400 MHz, CDCl₃) δ 1.15 (d, *J* = 2.5 Hz, 18H), 1.58 (m, 3H), 2.40 (m, 4H), 3.32 (s, 2H), 3.68 (m, 4H), 6.20 (s, 1 H), 6.72 (s, 1H).

### (iv) Preparation of 9e: 5-(Morpholinomethyl)-1-(triisopropylsilyl)-1H-pyrrol-3-ylboronic acid

To a solution of **9d** (200 mg, 0.49 mmol) in THF (5 mL) was added *n*-butyllithium (2.5 M in hexanes, 0.30 mL, 0.74 mmol) at -78°C. The mixture was stirred at -78°C for 20 min. Trimethyl borate (0.28 mL, 2.45 mmol) was added. The reaction mixture was stirred at -78°C for 1 hour and quenched with MeOH (4 mL). The reaction mixture was warmed to room temperature for 1 hour and extracted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (70 mg, 39%).
¹H NMR (400 MHz, CDCl₃) δ 1.15 (d, *J* = 2.5 Hz, 18H), 1.58 (m, 3H), 2.40 (m, 4H), 3.32 (s, 2H), 3.68 (m, 4H), 6.35 (s, 1 H), 7.15 (s, 1H).

### (v) Preparation of 9f: (4aS,6aS,6bR,8aR,13aR,15bS)-Benzyl 12-amino-2,2,6a,6b,9,9,13a-heptamethyl-15-(5-(morpholinomethyl)-1-(triisopropylsilyl)-1H-pyrrol-3-yl)-2,3,4,4a,5,6,6a,6b,7,8,8a,9,11,13,13a,13b,14,15b-octadecahydro-1H-chryseno[1,2-f]indazole-4a-carboxylate

A mixture of **II** (220 mg, 0.33 mmol), **9e** (310 mg, 0.84 mmol), Pd(PPh₃)₄ (60 mg, 0.052 mmol) and K₂CO₃ (184 mg, 1.32 mmol) in toluene (4.0 mL) and H₂O (0.5 mL) was sealed and heated to 120 °C by microwave for 1 hour. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (250 mg, 83%).
¹H NMR (300 MHz, CDCl₃) δ 0.42 (s, 3H), 0.73 (s, 3H), 0.78 (s, 3H), 0.85 (s, 3H), 1.11-2.40 (m, 55H), 3.43 (s, 3H), 3.60 (m, 6H), 5.02 (m, 2H), 6.08 (s, 1 H), 6.68 (s, 1 H), 7.33 (m, 5H). APCI MS (Positive Mode) *m*/*z* 904 [C₅₆H₈₅N₅O₃Si + H]⁺.

### (vi) Preparation of 9g: (4aS,6aS,6bR,8aR,13aR,15bS)-Benzyl 12-amino-2,2,6a,6b,9,9,13a-heptamethyl-15-(5-(morpholinomethyl)-1 H-pyrrol-3-yl)-2,3,4,4a,5,6,6a,6b,7,8,8a,9,11,13,13a,13b,14,15b-octadecahydro-1H-chryseno[1,2-f]indazole-4a-carboxylate

To a solution of **9f** (250 mg, 0.27 mmol) in THF (5 mL) was add tetrabutylammonium fluoride (0.83 mL, 1 M in THF, 0.83 mmol). The mixture was stirred at room temperature for 2 hours. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (176 mg, 87%). APCI MS (Positive Mode) *m*/*z* 748 [C₄₇H₆₅N₅O₃ + H]⁺.

### (vii) Preparation of 9: (4aS,6aS,6bR,8aR,13aR,15bS)-12-Amino-2,2,6a,6b,9,9,13a-heptamethyl-15-(5-(morpholinomethyl)-1H-pyrrol-3-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **9g** (176 mg, 0.23 mmol) and 10% Pd(OH)₂/C (100 mg) in MeOH (15 mL) and EtOAc (5 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-60% CMA in CH₂Cl₂) to afford the title compound (25 mg, 17%) as an off-white solid.
*R_{f}*0.50 (80:18:2 Methylene Chloride/Methanol/concentrated Ammonium Hydroxide).
¹H NMR (400 MHz, CD₃OD) δ 0.55 (s, 3H), 0.78 (s, 3H), 0.86 (s, 3H), 0.94 (s, 3H), 1.12 (s, 3H), 1.21 (s, 3H), 1.24 (s, 3H), 1.25-2.18 (m, 19H), 2.26 (m, 1 H), 2.36 (d, *J* = 14.8 Hz, 1 H), 3.49 (m, 4H), 3.52 (m, 3H), 3.67 (m, 6H), 6.03 (s, 1 H), 6.63 (s, 1H). mp 270-280 °C dec. ESI MS (Positive Mode) *m*/*z* 659 [C₄₀H₅₉N₅O₃ + H]⁺.

### Example 10

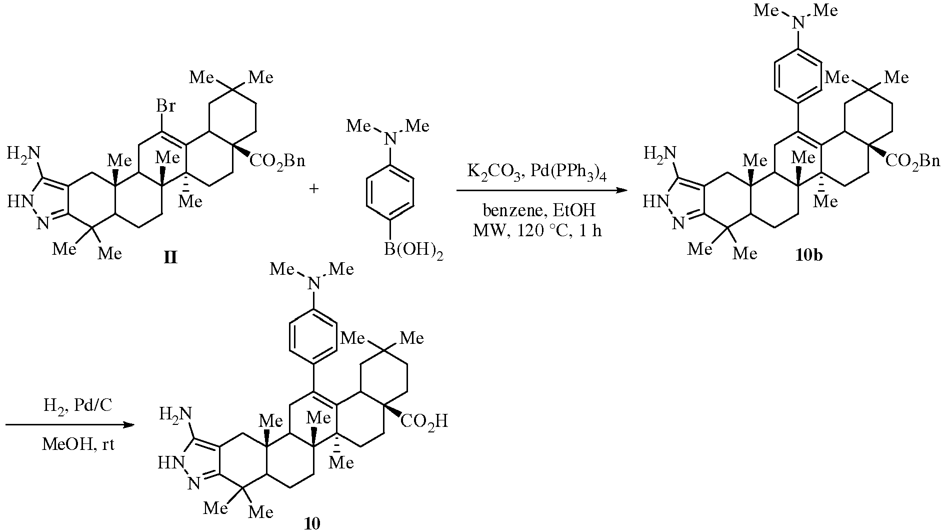

### (i) Preparation of 10b: (4aS,6aS,6bR,13aR)-Benzyl 12-amino-15-(4-(dimethylamino)phenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a solution of **II** (300 mg, 0.45 mmol), 4-(dimethylamino)phenylboronic acid (223 mg, 1.35 mmol), benzene (4 mL) and EtOH (1 mL) was added K₂CO₃ (249 mg, 1.80 mmol). The mixture was sparged with nitrogen and then Pd(PPh₃)₄ (104 mg, 0.08 mmol) was added. The reaction mixture was heated at 120 °C for 1 hour using microwave irradiation and then concentrated under reduced pressure. The residue was dissolved in EtOAc (20 mL) and the solution was washed with brine then dried (Na₂SO₄), filtered and concentrated. The residue was purified (silica, 0-5% MeOH in CH₂Cl₂) to afford the sub-title compound (254 mg, 79%).
¹H NMR (300 MHz, CDCl₃) δ 0.32-2.31 (m, 41 H), 2.89 (s, 6H), 3.15 (m, 1 H), 5.08 (m, 2H), 6.63 (d, *J*= 8.7 Hz, 2H), 7.03(d, *J*= 8.7 Hz, 2H), 7.34-7.37 (m, 5H).
APCI MS *m*/*z* 703 [C₄₆H₆₂N₄O₂ + H]⁺.

### (ii) Preparation of 10: (4aS,6aS,6bR,13aR)-12-Amino-15-(4-(dimethylamino)phenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **10b** (248 mg, 0.35 mmol) and MeOH (20 mL) was flushed with nitrogen and then 10% Pd/C (150 mg) was added. The mixture was flushed with nitrogen followed by hydrogen. The mixture was stirred under hydrogen at atmospheric pressure overnight. The catalyst was removed by filtration and the filtrate was concentrated. Purification by column chromatography (silica, 0-50% CMA in CH₂Cl₂) afforded the title compound (40 mg, 19%).
*R_{f}* 0.32 (90:9:1 Methylene Chloride/Methanol/concentrated Ammonium Hydroxide).
¹H NMR (300 MHz, CD₃OD) δ 0.31-2.37 (m, 41 H), 2.88 (s, 6H), 3.18 (m, 1 H), 6.81 (d, *J* = 8.7 Hz, 2H), 7.13 (d, *J*= 8.4 Hz, 2H). APCI MS *m*/*z* 613 [C₃₉H₅₆N₄O₂ + H]⁺. m.p. >300 °C. HPLC (Method A) 97.0% (214 nm) *t*_{R} = 13.2 min.

### Example 11

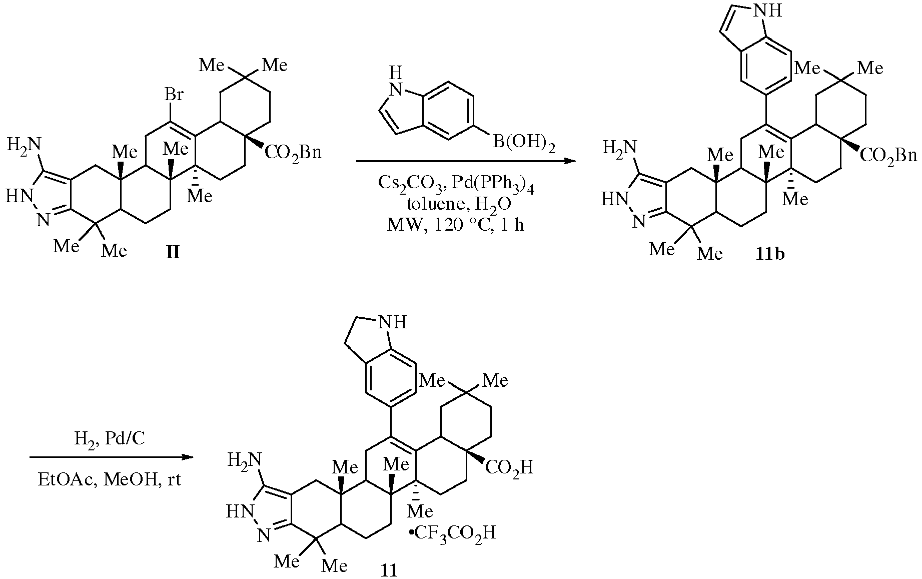

### (i) Preparation of 11 b: (4aS,6aS,6bR,13aR)-Benzyl 12-amino-15-(1H-indol-5-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a solution of **II** (200 mg, 0.302 mmol), 1*H*-indol-5-ylboronic acid (145 mg, 0.90 mmol), toluene (4.5 mL) and H₂O (0.5 mL) was added cesium carbonate (391 mg, 1.20 mmol). The mixture was sparged with nitrogen and then Pd(PPh₃)₄ (69 mg, 0.06 mmol) was added. The reaction mixture was heated at 120 °C for 1 hour using microwave irradiation and then concentrated under reduced pressure. The residue was dissolved in EtOAc (30 mL) and the solution was washed with brine then dried (Na₂SO₄), filtered and concentrated. The residue was purified by column chromatography (silica, 0-5% MeOH in CH₂Cl₂) to afford the title compound (195 mg, 92%). APCI MS *m*/*z* 699 [C₄₆H₅₈N₄O₂ + H]⁺.

### (ii) Preparation of 11: (4aS,6aS,6bR,13aR)-12-Amino-15-(indolin-5-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **11b** (192 mg, 0.274 mmol), MeOH (12 mL) and EtOAc (3 mL) was flushed with nitrogen and then 10% Pd/C (228 mg) was added. The mixture was flushed with nitrogen followed by hydrogen. The mixture was stirred under hydrogen at atmospheric pressure overnight. The catalyst was removed by filtration and the filtrate was concentrated. The residue was purified by column chromatography (silica, 0-50% CMA in CH₂Cl₂) followed by preparative HPLC to afford the title compound (7.5 mg, 5%) as a solid.
*R_{f}* 0.15 (90:9:1 Methylene Chloride/Methanol/concentrated Ammonium Hydroxide).
¹H NMR (400 MHz, CD₃OD) δ 0.30 (s, 3H), 0.77-2.39 (m, 43H), 2.99-3.03 (m, 1 H), 3.34 (t, *J*= 7.75 Hz, 2H), 3.85 (t, *J* = 7.75 Hz, 2H), 7.35-7.44 (m, 3H).
APCI MS *m*/*z* 611 [C₃₉H₅₄N₄O₂ + H]⁺. m.p. 170-190 °C dec. HPLC (Method A) 97.7% (214 nm) *t*_{R} = 12.6 min.

### Example 12

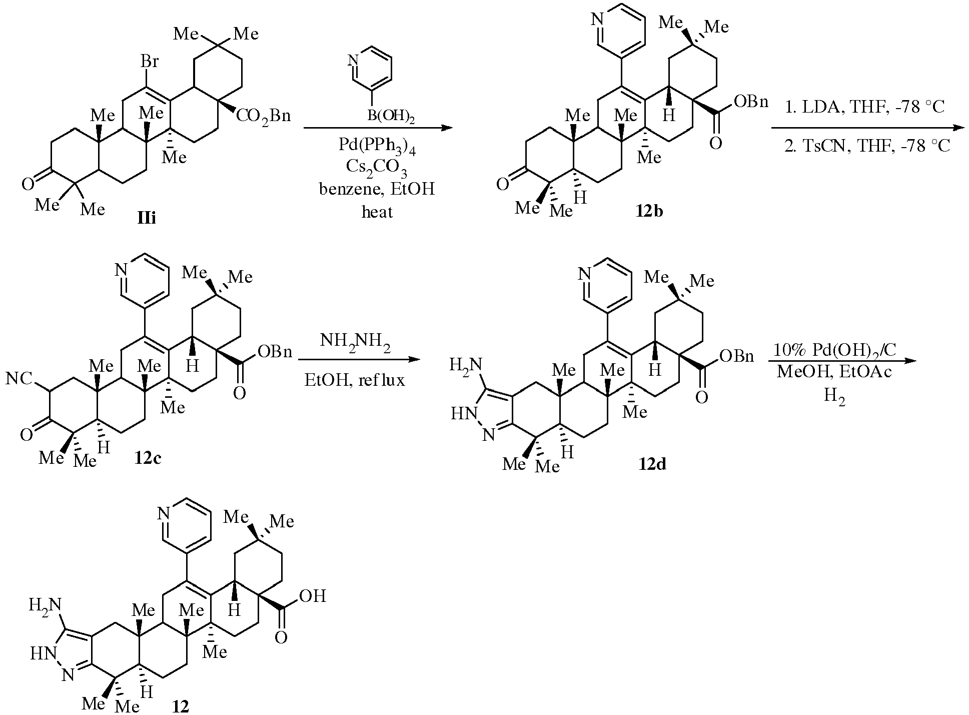

### (i) Preparation of 12b: (4aS,6aS,6bR,8aR,12aR,14bS)-Benzyl

### 2,2,6a,6b,9,9,12a-heptamethyl-10-oxo-14-(pyridin-3-yl)-1,2,3,4,4a,5,6,6a,6b,7,8,8a,9,10,11,12,12a,12b,13,14b-icosahydropicene-4a-carboxylate

A mixture of **IIi** (400 mg, 0.64 mmol), pyridin-3-ylboronic acid (237 mg, 1.93 mmol), Pd(PPh₃)₄ (74 mg, 0.064 mmol) and cesium carbonate (629 mg, 1.93 mmol) in benzene (15 mL) and EtOH (5 mL) was heated at reflux for 12 hours. The reaction mixture was diluted with EtOAc (100 mL). The organic phase was washed with brine then dried (MgSO₄), filtered and concentrated to dryness. The residue was purified by column chromatography (silica, 0-50% EtOAc in hexanes) to afford the sub-title compound (200 mg, 48%).
¹H NMR (300 MHz, CDCl₃) δ 0.25 (s, 3H), 0.75 (s, 3H), 0.83 (s, 3H), 1.02 (s, 3H), 1.04 (s, 3H), 1.09 (s, 3H), 1.22 (s, 3H), 1.23-2.02 (m, 20H), 2.30-2.50 (m, 2H), 2.90 (m, 1 H), 5.05 (m, 2H), 7.14 (m, 1 H), 7.37 (m, 5H), 7.48 (m, 1H), 8.42(m, 2H).

### (ii) Preparation of 12c: (4aS,6aS,6bR,8aR,12aR,14bS)-Benzyl 11-cyano-2,2,6a,6b,9,9,12a-heptamethyl-10-oxo-14-(pyridin-3-yl)-1,2,3,4,4a,5,6,6a,6b,7,8,8a,9,10,11,12,12a,12b,13,14b-icosahydropicene-4a-carboxylate

To a solution of diisopropylamine (0.085 mL, 0.61 mmol) in THF (3 mL) was added n-butyllithium (0.26 mL, 2.5 M in hexanes, 0.64 mmol) at-78°C. The solution was stirred for 30 min. The LDA solution was added to **12b** (200 mg, 0.32 mmol) in THF (5 mL). The mixture was allowed to warm to -40°C for 5 min and cooled to -78°C. A suspension of *p*-toluene sulfonyl cyanide (116 mg, 0.64 mmol) in THF (2 mL) was added. The reaction mixture was allowed to warm to -40 °C over 1.5 hours. The reaction was quenched by saturated NH₄Cl (3 mL) and extracted with EtOAc (100 mL). The organic phase was washed with brine then dried (MgSO₄), filtered and concentrated to dryness. The residue was purified by column chromatography (silica, 0-50% EtOAc in hexanes) to afford the sub-title compound (80 mg, 39%).
¹H NMR (300 MHz, CDCl₃) δ 0.25 (s, 3H), 0.75 (s, 3H), 0.83 (s, 3H), 1.08 (s, 3H), 1.13 (s, 3H), 1.15 (s, 3H), 1.21 (s, 3H), 1.23-2.25 (m, 20H), 2.90 (m, 1H), 3.82 (m, 1H), 5.04 (m, 2H), 7.14 (m, 1 H), 7.37 (m, 5H), 7.48 (m, 1 H), 8.43 (m, 2H).

### (iii) Preparation of 12d: (4aS,6aS,6bR,8aR,13aR,15bS)-Benzyl 12-amino-2,2,6a,6b,9,9,13a-heptamethyl-15-(pyridin-3-yl)-2,3,4,4a,5,6,6a,6b,7,8,8a,9,11,13,13a,13b,14,15b-octadecahydro-1H-chryseno[1,2-f]indazole-4a-carboxylate

A mixture of **12c** (80 mg, 0.12 mmol) and hydrazine (0.020 mL) in EtOH (2 mL) was heated at reflux overnight. The reaction mixture was concentrated to dryness under reduced pressure. The residue was purified by column chromatography (silica, 0-50% CMA in CH₂Cl₂) to afford the sub-title compound (74 mg, 93%). ESI MS (Positive Mode) m/z 661 [C₄₃H₅₆N₄O₂ + H]⁺.

### (iv) Preparation of 12: (4aS,6aS,6bR,8aR,13aR,15bS)-12-Amino-2,2,6a,6b,9,9,13a-heptamethyl-15-(pyridin-3-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **12d** (74 mg, 0.11 mmol) and 10% Pd(OH)₂/C (60 mg) in MeOH (10 mL) and EtOAc (2 mL) was stirred under a hydrogen balloon for 5 hours. The reaction mixture was filtered through a pad of diatomaceous earth and washed with CMA (25 mL). The filtrate was concentrated to dryness under reduced pressure. The residue was purified by column chromatography (silica, 0-70% CMA in CH₂Cl₂) to afford the title compound (25 mg, 40%) as a brown solid.
*R_{f}* 0.30 (80:18:2 Methylene Chloride/Methanol/concentrated Ammonium Hydroxide).
¹H NMR (300 MHz, CD₃OD) δ 0.55 (s, 3H), 0.81 (s, 3H), 0.87 (s, 3H), 0.97 (s, 3H), 1.03 (s, 3H), 1.08 (s, 6H), 1.25-2.10 (m, 19H), 2.10 (d, *J* = 14.8 Hz, 1 H), 2.75 (m, 1 H), 7.18 (t, *J* = 7.8 Hz, 1 H), 7.56 (d, *J* = 7.2 Hz, 1 H), 8.16 (d, *J* = 4.8 Hz, 1 H), 8.26 (s, 1 H). mp >300 °C. APCI MS (Positive Mode) *m*/*z* 571 [C₃₆H₅₀N₄O₂ + H]⁺.

### Example 13

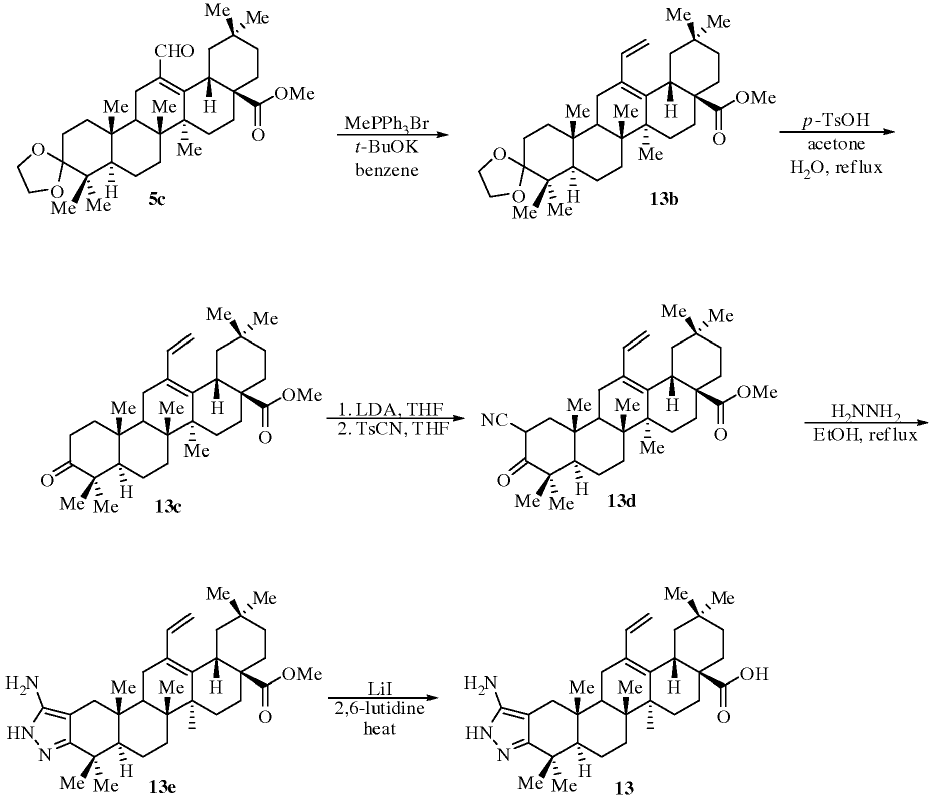

### (i) Preparation of 13b: (4a'R,6a'R,6b'S,8a'S,12a'S,14b'R)-Methyl 4',4',6a',6b',11',11',14b'-heptamethyl-13'-vinyl-2',4',4a',5',6',6a',6b',7',8',8a',9',10',11',12',12a',14',14a',14b'-octadecahydro-1'H-spiro[[1,3]dioxolane-2,3'-picene]-8a'-carboxylate

To a suspension of methyltriphenylphosphonium iodide (808 mg, 2.0 mmol) in benzene (30 mL) was added potassium *tert*-butoxide (224 mg, 2.0 mmol). The mixture was stirred at room temperature for 1 hour. A solution of **5c** (600 mg, 1.11 mmol) in THF (5 mL) was added. The mixture was stirred at room temperature for 1.5 hours. The reaction mixture was diluted with EtOAc (100 mL). The organic phase was washed with brine then dried (MgSO₄), filtered and concentrated to dryness. The residue was purified by column chromatography (silica, 0-15% EtOAc in hexanes) to afford the sub-title compound (490 mg, 82%).
¹H NMR (300 MHz, CDCl₃) δ 0.63 (s, 3H), 0.85 (s, 3H), 0.89 (s, 3H), 0.91 (s, 3H), 0.94 (s, 3H), 0.97 (s, 3H), 0.99 (s, 3H), 1.11 (s, 3H), 1.15-2.05 (m, 22H), 3.60 (s, 3H), 3.95 (m, 4H), 4.95 (m, 1H), 4.95(d, *J*= 9.3 Hz, 2H), 5.09 (d, *J* = 16.2 Hz, 1 H).

### (ii) Preparation of 13c: (4aS,6aS,6bR,8aR,12aR,14bS)-Methyl 2,2,6a,6b,9,9,12a-heptamethyl-10-oxo-14-vinyl-1,2,3,4,4a,5,6,6a,6b,7,8,8a,9,10,11,12,12a,12b,13,14b-icosahydropicene-4a-carboxylate

A mixture of **13b** (600 mg, 1.11 mmol) and *p*-toluenesulfonic acid monohydrate (211 mg, 1.11 mmol) in acetone (20 mL) and H₂O (2 mL) was heated at reflux for 4 hours. The reaction mixture was concentrated to remove acetone under reduced pressure and extracted with EtOAc (100 mL). The organic phase was washed with brine then dried (MgSO₄), filtered and concentrated to dryness. The residue was purified by column chromatography (silica, 0-15% EtOAc in hexanes) to afford the sub-title compound (500 mg, 91%).
¹H NMR (300 MHz, CDCl₃) δ 0.72 (s, 1.8H), 0.81 (s, 1.2H), 0.89 (s, 3H), 0.91 (s, 3H), 1.0-2.50 (m, 38H), 3.65 (s, 3H), 5.40-5.70 (m, 1 H), 6.05 (d, *J* = 12.0 Hz, 0.4H), 6.53 (d, *J*= 15.0 Hz, 0.6H).

### (iii) Preparation of 13d: (4aS,6aS,6bR,8aR,12aR,14bS)-Methyl 11-cyano-2,2,6a,6b,9,9,12a-heptamethyl-10-oxo-14-vinyl-1,2,3,4,4a,5,6,6a,6b,7,8,8a,9,10,11,12,12a,12b,13,14b-icosahydropicene-4a-carboxylate

To a solution of diisopropylamine (0.27 mL, 1.92 mmol) in THF (6 mL) was added n-butyllithium (0.81 mL, 2.5 M in hexanes, 2.02 mmol) at -78°C. The solution was stirred for 30 min. The LDA solution was added to **13c** (500 mg, 1.01 mmol) in THF (10 mL). The mixture was allowed to warm to -40°C for 5 min and cooled to -78°C. A suspension of *p*-toluene sulfonyl cyanide (329 mg, 1.82 mmol) in THF (3 mL) was added. The reaction mixture was allowed to warm to -40°C over 1.5 hours. The reaction was quenched by saturated NH₄Cl (3 mL) and extracted with EtOAc (100 mL). The organic phase was washed with brine then dried (MgSO₄), filtered and concentrated to dryness. The residue was purified by column chromatography (silica, 0-15% EtOAc in hexanes) to afford the sub-title compound (200 mg, 38%).

### (iv) Preparation of 13e: (4aS,6aS,6bR,8aR,13aR,15bS)-Methyl 12-amino-2,2,6a,6b,9,9,13a-heptamethyl-15-vinyl-2,3,4,4a,5,6,6a,6b,7,8,8a,9,11,13,13a,13b,14,15b-octadecahydro-1H-chryseno[1,2-f]indazole-4a-carboxylate

A mixture of **13d** (200 mg, 0.38 mmol) and hydrazine (0.015 mL, 0.46 mmol) in EtOH (3 mL) was heated at reflux overnight. The reaction mixture was concentrated to dryness under reduced pressure. The residue was purified by column chromatography (silica, 0-10% MeOH in CH₂Cl₂) to afford the sub-title compound (180 mg, 89%). ESI MS (Positive Mode) *m*/*z* 534 [C₃₄H₅₁N₃O₂ + H]⁺.

### (v) Preparation of 13: (4aS,6aS,6bR,8aR,13aR,15bS)-12-Amino-2,2,6a,6b,9,9,13a-heptamethyl-15-vinyl-2,3,4,4a,5,6,6a,6b,7,8,8a,9,11,13,13a,13b,14,15b-octadecahydro-1H-chryseno[1,2-f]indazole-4a-carboxylic acid

A mixture of **13e** (80 mg, 0.15 mmol) and lithium iodide (300 mg, 2.25 mmol) in 2,6-lutidine (3 mL) was heated to 144 °C for 5 hours. The reaction mixture was cooled to room temperature and neutralized with HCl (2 M) and extracted with CH₂Cl₂/*i*-PrOH (3:1). The organic phase was dried (MgSO₄), filtered and concentrated to dryness under reduced pressure. The residue was purified by column chromatography (silica, 0-50% CMA in CH₂Cl₂) followed by preparative HPLC to provide the title compound (6 mg, 8%) as an off-white solid.
*R_{f}* 0.35 (80:18:2 Methylene Chloride/Methanol/concentrated Ammonium Hydroxide).
¹H NMR (300 MHz, CD₃OD) δ 0.81 (s, 3H), 0.91 (s, 3H), 0.94 (s, 3H), 1.01 (s, 3H), 1.17 (s, 3H), 1.22 (s, 3H), 1.25 (m, 3H), 1.30-2.30 (m, 19H), 2.53 (d, *J* = 15.0 Hz, 1 H), 3.53 (m, 1 H), 4.98 (d, *J* = 12.3 Hz, 1 H), 5.18 (d, *J* = 19.2 Hz, 1 H), 6.95 (dd, *J* = 12.3, 19.2 Hz, 1H). mp >300 °C. ESI MS (Positive Mode) *m*/*z* 520 [C₃₃H₄₉N₃O₂ + H]⁺.

### Example 14

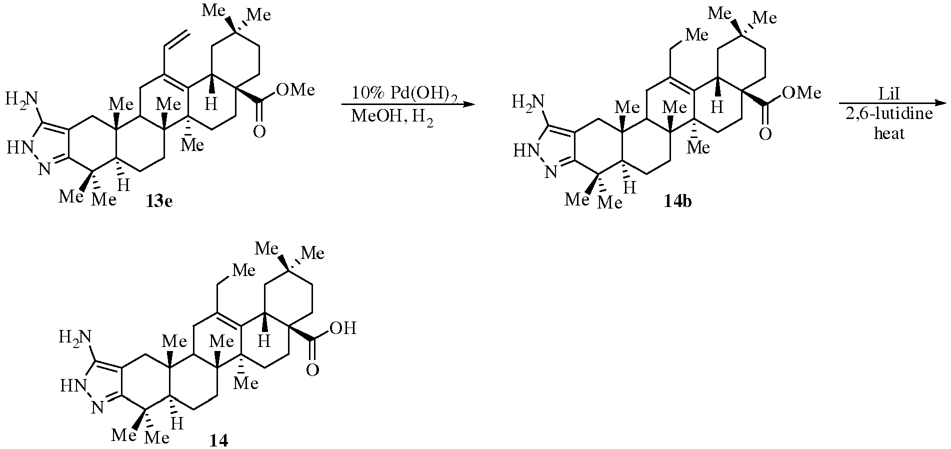

### (i) Preparation of 14b: (4aS,6aS,6bR,8aR,13aR,15bS)-Methyl 12-amino-15-ethyl-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **13e** (80 mg, 0.15 mmol) and 10% Pd(OH)₂/C (30 mg) in MeOH (10 mL) was stirred under a hydrogen balloon for 12 hours. The reaction mixture was filtered through a pad of diatomaceous earth and washed with CMA (20 mL). The filtrate was concentrated to dryness and the residue purified by column chromatography (silica, 0-10% MeOH in CH₂Cl₂) to afford the sub-title compound (70 mg, 87%). ESI MS (Positive Mode) *m*/*z* 536 [C₃₄H₅₃N₃O₂ + H]⁺.

### (ii) Preparation of 14: (4aS,6aS,6bR,8aR,13aR,15bS)-12-Amino-15-ethyl-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **14b** (70 mg, 0.13 mmol) and lithium iodide (263 mg, 1.96 mmol) in 2,6-lutidine (3 mL) was heated to 144 °C for 5 hours. The reaction mixture was cooled to room temperature and neutralized with HCl (2 M) and extracted with CH₂Cl₂/*i*-PrOH (3:1). The organic phase was dried (MgSO₄), filtered and concentrated to dryness under reduced pressure. The residue was purified by column chromatography (silica, 0-50% CMA in CH₂Cl₂) followed by preparative HPLC to provide the title compound (13 mg, 19%) as an off-white solid.
R_{f}0.80 (80:18:2 Methylene Chloride/Methanol/concentrated Ammonium Hydroxide).
¹H NMR (300 MHz, CD₃OD) δ 0.83 (s, 3H), 0.92 (s, 6H), 0.99 (s, 3H), 1.11 (s, 3H), 1.21 (s, 3H), 1.29 (s, 3H), 1.30-2.30 (m, 25H), 2.44 (d, *J*= 15.0 Hz, 1H), mp >300 °C. ESI MS (Positive Mode) *m*/*z* 522 [C₃₃H₅₁N₃O₂ + H]⁺.

### Example 15

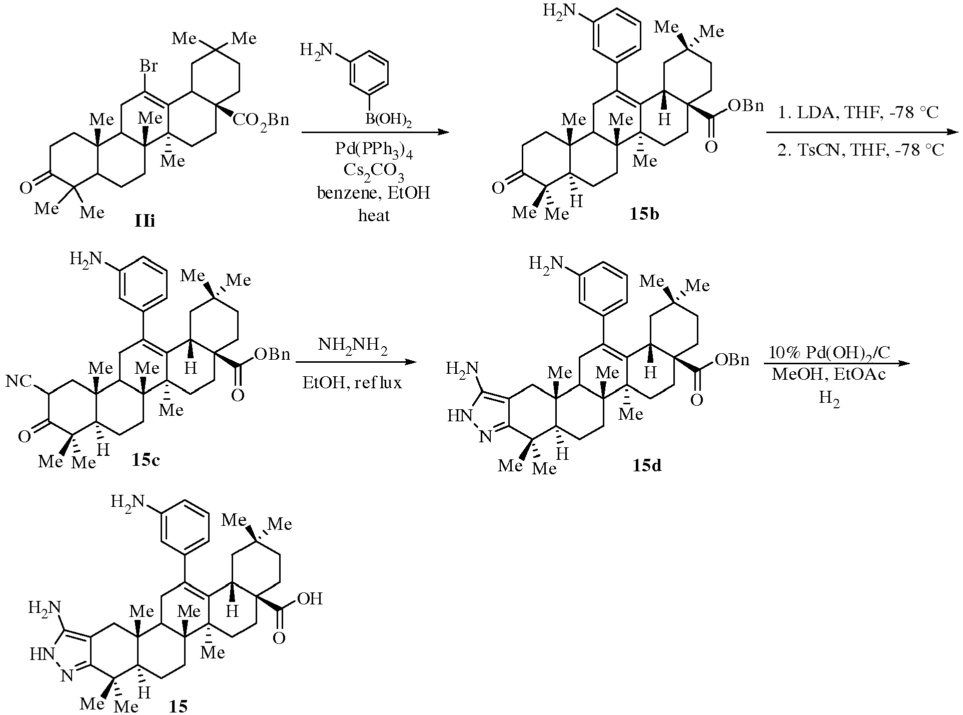

### (i) Preparation of 15b: (4aS,6aS,6bR,8aR,12aR,14bS)-Benzyl 14-(3-aminophenyl)-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

A mixture of **IIi** (600 mg, 0.96 mmol), 3-aminophenylboronic acid (362 mg, 2.66 mmol), Pd(PPh₃)₄ (104 mg, 0.09 mmol) and K₂CO₃ (353 mg, 2.56 mmol) in benzene (16 mL) and EtOH (5 mL) was heated at reflux for 12 hours. The reaction mixture was diluted with EtOAc (100 mL). The organic phase was washed with brine then dried (MgSO₄), filtered and concentrated to dryness. The residue was purified by column chromatography (silica, 0-40% EtOAc in hexanes) to afford the sub-title compound (400 mg, 65%).
¹H NMR (300 MHz, CDCl₃) δ 0.30 (s, 3H), 0.76 (s, 3H), 0.86 (s, 3H), 1.04 (s, 3H), 1.06 (s, 3H), 1.08 (s, 3H), 1.19 (s, 3H), 1.20-2.50 (m, 24H), 3.12 (m, 1 H), 5.02 (m, 2H), 6.48 (m, 3H), 7.01 (m, 1H), 7.33 (m, 5H).

### (ii) Preparation of 15c: (4aS,6aS,6bR,8aR,12aR,14bS)-Benzyl 14-(3-aminophenyl)-11-cyano-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

To a solution of diisopropylamine (0.22 mL, 1.57 mmol) in THF (5 mL) was added n-butyllithium (0.64 mL, 2.5 M in hexanes, 1.61 mmol) at-78°C. The solution was stirred for 30 min. The LDA solution was added to **15b** (400 mg, 0.62 mmol) in THF (5 mL). The mixture was allowed to warm to -40°C for 5 min and cooled to -78°C. A suspension of *p*-toluene sulfonyl cyanide (285 mg, 1.57 mmol) in THF (3 mL) was added. The reaction mixture was allowed to warm to -40°C over 1.5 hours. The reaction was quenched by saturated NH₄Cl (3 mL) and extracted with EtOAc (100 mL). The organic phase was washed with brine then dried (MgSO₄), filtered and concentrated to dryness. The residue was purified by column chromatography (silica, 0-20% EtOAc in hexanes) to afford the sub-title compound (100 mg, 25%).
¹H NMR (300 MHz, CDCl₃) δ 0.32 (s, 3H), 0.76 (s, 3H), 0.86 (s, 3H), 0.87-2.30 (m, 32H), 3.12 (m, 1 H), 3.30 (s, 2H), 3.82 (m, 1 H), 5.02 (m, 2H), 6.48 (m, 3H), 7.01 (m, 1H), 7.33 (m, 5H).

### (iii) Preparation of 15d: (4aS,6aS,6bR,8aR,13aR,15bS)-Benzyl 12-amino-15-(3. aminophenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **15c** (100 mg, 0.15 mmol) and hydrazine (0.020 mL) in EtOH (2 mL) was heated at reflux overnight. The reaction mixture was concentrated to dryness under reduced pressure. The residue was purified by column chromatography (silica, 0-30% CMA in CH₂Cl₂) to afford the sub-title compound (70 mg, 69%). APCI MS (Positive Mode) *m*/*z* 675 [C₄₄H₅₈N₄O₂ + H]⁺.

### (iv) Preparation of 15: (4aS,6aS,6bR,8aR,13aR,15bS)-12-Amino-15-(3-aminophenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **15d** (70 mg, 0.10 mmol) and 10% Pd(OH)₂/C (35 mg) in MeOH (12 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-80% CMA in CH₂Cl₂) followed by preparative HPLC to provide the title compound (32 mg, 55%) as a brown solid.
*R_{f}* 0.70 (80:18:2 Methylene Chloride/Methanol/concentrated Ammonium Hydroxide).
¹H NMR (300 MHz, DMSO-*d*₆) δ 0.30 (s, 3H), 0.78 (s, 3H), 0.85 (s, 3H), 0.96 (s, 3H), 1.18 (s, 3H), 1.31 (s, 6H), 1.32-2.28 (m, 19H), 2.26 (d, *J*= 14.7 Hz, 1H), 3.0 (m, 1 H), 7.16 (m, 4H). mp >300 °C. APCI MS (Positive Mode) *m*/*z* 585 [C₃₇H₅₂N₄O₂ + H]⁺.

### Example 16

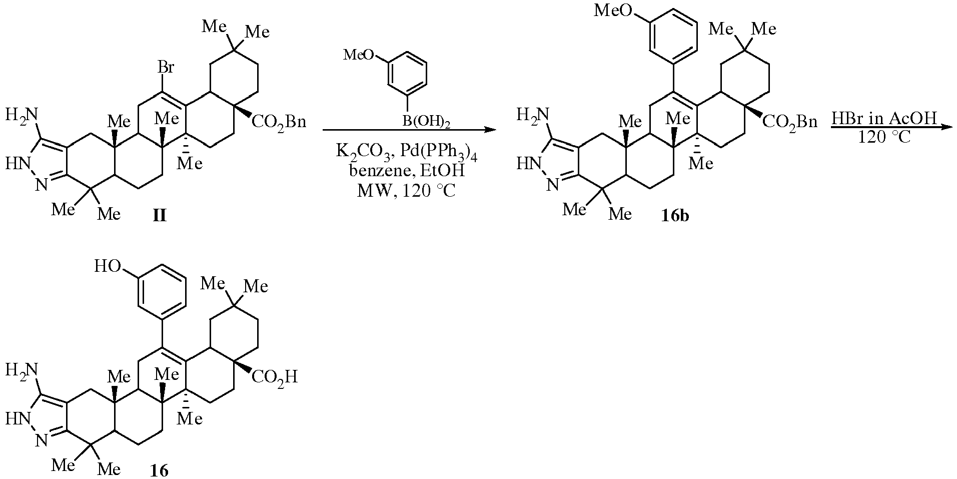

### (i) Preparation of 16b: (4aS,6aS,6bR,13aR)-Benzyl 12-amino-15-(3-methoxyphenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a mixture of **II** (200 mg, 0.30 mmol) and 3-methoxyphenylboronic acid (92 mg, 0.60 mmol) in benzene (4 mL) and EtOH (1 mL) was added K₂CO₃ (125 mg, 0.90 mmol). The mixture was sparged with nitrogen and then Pd(PPh₃)₄ (70 mg, 0.06 mmol) was added. The reaction mixture was heated at 120 °C for 1 hour using microwave irradiation and then concentrated. The residue was dissolved in EtOAc (20 mL) and the solution was washed with brine then dried (Na₂SO₄), filtered and concentrated under reduced pressure. The residue was purified by column chromatography (silica, 0-30% CMA in CH₂Cl₂ to afford the sub-title compound (179 mg, 86%).
¹H NMR (300 MHz, CDCl₃) δ 0.30-2.25 (m, 39H), 3.11 (m, 1 H), 3.40 (s, 2H), 3.82 (s, 3H), 5.08 (d, *J* = 12.6 Hz, 1 H), 5.20 (d, *J* = 12.3 Hz, 1H),6.75(m, 1H), 6.78 (s, 1 H), 7.15 (t, *J* = 7.8 Hz, 1 H), 7.31-7.36 (m, 5H). APCI MS *m*/*z* 690 [C₄₅H₅₉N₃O₃ + H]⁺

### (ii) Preparation of 16: (4aS,6aS,6bR,13aR)-12-Amino-15-(3-hydroxyphenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **16b** (179 mg, 0.25 mmol) and HBr (2 mL, 33% in AcOH) was heated at 120 °C for 1 hour. The reaction mixture was concentrated under reduced pressure and the residue was purified by column chromatography (silica, 0-80% CMA in CH₂Cl₂) to afford the title compound (20 mg, 13%).
*R_{f}* 0.30 (40:9:1 Methylene Chloride/Methanol/concentrated Ammonium Hydroxide).
¹H NMR (300 MHz, CD₃OD) δ 0.34-2.37 (m, 41 H), 3.15 (d, *J* = 10.8 Hz, 1 H), 6.61-6.70 (m, 3H), 7.11 (t, *J*= 7.8 Hz, 1 H). APCI MS *m*/*z* 586 [C₃₇H₅₁N₃O₃ + H]⁺. m.p. 260-280 °C dec. HPLC (Method A) 92.4% (214 nm) *t*_{R} = 15.6 min.

### Example 17

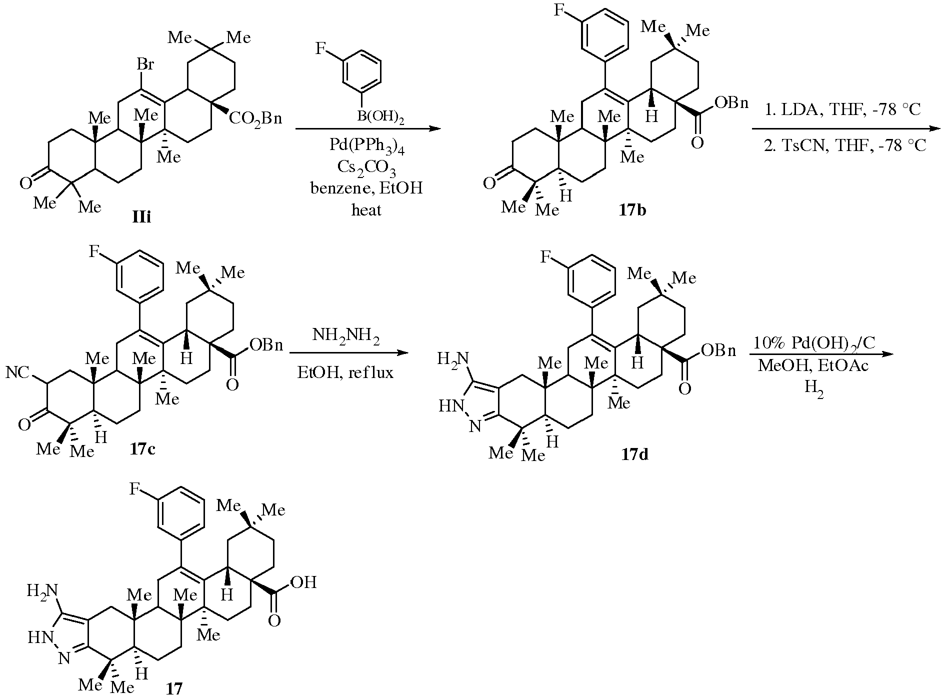

### (i) Preparation of 17b: (4aS,6aS,6bR,8aR,12aR,14bS)-Benzyl 14-(3-fluorophenyl)-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

A mixture of **IIi** (300 mg, 0.48 mmol), 3-fluorophenylboronic acid (100 mg, 0.72 mmol), Pd(PPh₃)₄ (55 mg, 0.048 mmol) and cesium carbonate (469 mg, 1.44 mmol) in benzene (15 mL) and EtOH (5 mL) was heated at reflux for 12 hours. The reaction mixture was diluted with EtOAc (100 mL). The organic phase was washed with brine then dried (MgSO₄), filtered and concentrated to dryness. The residue was purified by column chromatography (silica, 0-10% EtOAc in hexanes) to afford the sub-title compound (250 mg, 82%).
¹H NMR (300 MHz, CDCl₃) δ 0.25 (s, 3H), 0.78 (s, 3H), 0.83 (s, 3H), 1.05 (s, 3H), 1.07 (s, 3H), 1.12 (s, 3H), 1.21 (s, 3H), 1.23-2.55 (m, 22H), 3.01 (m, 1 H), 5.04 (m, 2H), 6.87 (m, 3H), 7.15 (m, 1 H), 7.34 (m, 5H).

### (ii) Preparation of 17c: (4aS,6aS,6bR,8aR,12aR,14bS)-Benzyl 11-cyano-14-(3-fluorophenyl)-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

To a solution of diisopropylamine (0.17 mL, 1.22 mmol) in THF (6 mL) was added *n*-butyllithium (0.51 mL, 2.5 M in hexanes, 1.28 mmol) at-78°C. The solution was stirred for 30 min. The LDA solution was added to **17b** (408 mg, 0.64 mmol) in THF (4 mL). The mixture was allowed to warm to -40°C for 5 min and cooled to -78°C. A suspension of *p*-toluene sulfonyl cyanide (232 mg, 1.28 mmol) in THF (3 mL) was added at -78°C. The reaction mixture was allowed to warm to -40°C over 1.5 hours. The reaction was quenched by saturated NH₄Cl (3 mL) and extracted with EtOAc (100 mL). The organic phase was washed with brine then dried (MgSO₄), filtered and concentrated to dryness. The residue was purified by column chromatography (silica, 0-20% EtOAc in hexanes) to afford the sub-title compound (280 mg, 66%).
¹H NMR (300 MHz, CDCl₃) δ 0.25 (s, 3H), 0.78 (s, 3H), 0.83 (s, 3H), 1.05 (s, 3H), 1.07 (s, 3H), 1.12 (s, 3H), 1.22 (s, 3H), 1.23-2.30 (m, 20H), 3.01 (m, 1 H), 3.83 (m, 1 H), 5.04 (m, 2H), 6.87 (m, 3H), 7.15 (m, 1 H), 7.34 (m, 5H).

### (iii) Preparation of 17d: (4aS,6aS,6bR,8aR,13aR,15bS)-Benzyl 12-amino-15-(3-fluorophenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **17c** (280 mg, 0.42 mmol) and hydrazine (0.053 mL) in EtOH (5 mL) was heated at reflux overnight. The reaction mixture was concentrated to dryness under reduced pressure. The residue was purified by column chromatography (silica, 0-10% MeOH in CH₂Cl₂) to afford the sub-title compound (230 mg, 81%). ESI MS (Positive Mode) *m*/*z* 678 [C₄₄H₅₆FN₃O₂ + H]⁺.

### (iv) Preparation of 17: (4aS,6aS,6bR,8aR,13aR,15bS)-12-Amino-15-(3-fluorophenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **17d** (230 mg, 0.34 mmol) and 10% Pd(OH)₂/C (100 mg) in MeOH (6 mL) and EtOAc (6 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-50% CMA in CH₂Cl₂) to afford the title compound (110 mg, 55%) as a white solid.
*R_{f}*0.75 (4:1 Methylene Chloride/Methanol).
¹H NMR (300 MHz, CD₃OD) δ 0.30 (s, 3H), 0.75 (s, 3H), 0.87 (s, 3H), 0.99 (s, 3H), 1.16 (s, 3H), 1.19 (s, 3H), 1.25 (s, 3H), 1.26-2.25 (m, 19H), 2.30 (d, *J*= 14.7 Hz, 1 H), 3.15 (m, 1H), 6.91 (t, *J*= 8.4 Hz, 1 H), 7.01 (m, 2H), 7.28 (q, *J*= 8.1 Hz, 1 H). mp >300 °C.
APCI MS (Positive Mode) *m*/*z* 588 [C₃₇H₅₀FN₃O₂ + H]⁺.

### Example 18

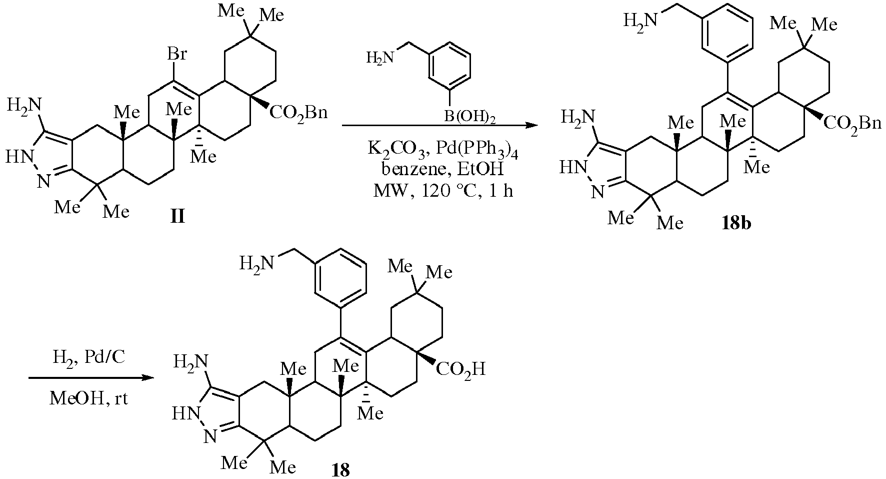

### (i) Preparation of 18b: (4aS,6aS,6bR,13aR)-Benzyl 12-amino-15-(3-(aminomethyl)phenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a mixture of II (200 mg, 0.30 mmol) and 3-(aminomethyl)phenylboronic acid (169 mg, 0.90 mmol) in benzene (4 mL) and EtOH (1 mL) was added K₂CO₃ (166 mg, 1.20 mmol). The mixture was sparged with nitrogen and then Pd(PPh₃)₄ (70 mg, 0.06 mmol) was added. The reaction mixture was heated at 120 °C for 1 hour using microwave irradiation and then concentrated. The residue was dissolved in EtOAc (20 mL) and the organic layer was washed with brine then dried (Na₂SO₄), filtered and concentrated under reduced pressure. The residue was purified by column chromatography (silica, 0-40% CMA in CH₂Cl₂) to afford the sub-title compound (170 mg, 82%).
¹H NMR (300 MHz, CDCl₃) δ 0.23-2.25 (m, 45H), 3.05 (m, 1H), 3.40 (s, 1H), 3.82 (s, 2H), 5.06 (d, *J*= 12.6 Hz, 1 H), 5.23 (d, *J* = 12.3 Hz, 1 H), 7.06-7.20 (m, 4H), 7.33-7.38 (m, 5H). APCI MS *m*/*z* 689 [C₄₅H₆₀N₄O₂ + H]⁺.

### (ii) Preparation of 18: (4aS,6aS,6bR,13aR)-12-Amino-15-(3-(aminomethyl)phenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **18b** (160 mg, 0.23 mmol) and MeOH (20 mL) was flushed with nitrogen and then 10% Pd/C (100 mg) was added. The mixture was flushed with nitrogen followed by hydrogen. The mixture was stirred under hydrogen at atmospheric pressure overnight. The catalyst was removed by filtration and the filtrate was concentrated under reduced pressure. The residue was purified by column chromatography (silica, 0-70% CMA in CH₂Cl₂) followed by preparative HPLC to afford the title compound (48 mg, 35%) as a solid.
R*_{f}*0.13 (40:9:1 Methylene Chloride/Methanol/concentrated Ammonium Hydroxide).
¹H NMR (300 MHz, CD₃OD) δ 0.27-2.34 (m, 41 H), 2.99 (d, *J*= 11.1 Hz, 1H), 4.13 (s, 2H), 7.30-7.44 (m, 4H). APCI MS *m*/*z* 599 [C₃₈H₅₄N₄O₂ + H]⁺m.p. 270-290 °C dec. HPLC (Method A) >99% (214 nm) *t*_{R} = 12.4 min.

### Example 19

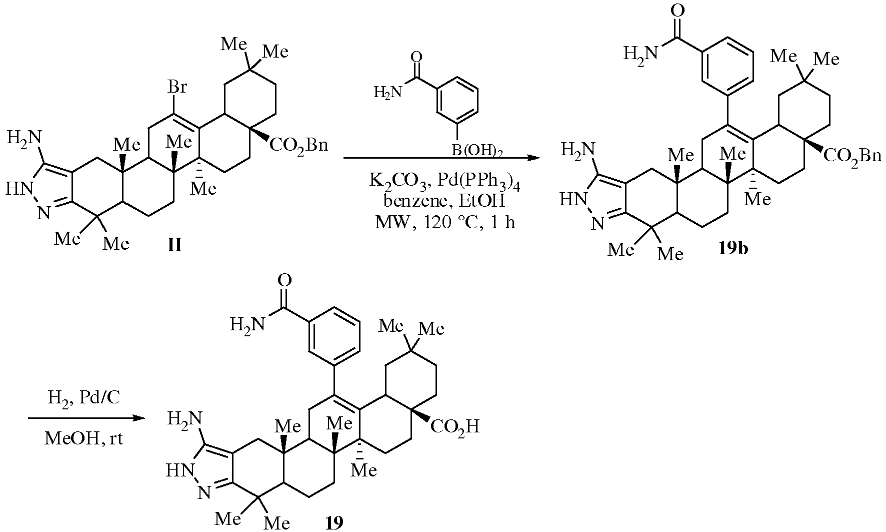

### (i) Preparation of 19b: (4aS,6aS,6bR,13aR)-Benzyl 12-amino-15-(3-carbamoylphenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a mixture of II (200 mg, 0.30 mmol) and 3-carbamoylphenylboronic acid (99 mg, 0.64 mmol) in benzene (4 mL) and EtOH (1 mL) was added K₂CO₃ (125 mg, 0.90 mmol). The mixture was sparged with nitrogen and then Pd(PPh₃)₄ (69 mg, 0.06 mmol) was added. The reaction mixture was heated at 120 °C for 1 hour using microwave irradiation and then concentrated. The residue was dissolved in EtOAc (20 mL) and the solution was washed with brine then dried (Na₂SO₄), filtered and concentrated under reduced pressure. The residue was purified by column chromatography (silica, 0-40% CMA in CH₂Cl₂) to afford the sub-title compound (170 mg, 80%). APCI MS *m*/*z* 703 [C₄₅H₅₈N₄O₃ + H]⁺

### (ii) Preparation of 19: (4aS,6aS,6bR,13aR)-12-Amino-15-(3-carbamoylphenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **19b** (170 mg, 0.24 mmol) and MeOH (20 mL) was flushed with nitrogen and then 10% Pd/C (100 mg) was added. The mixture was flushed with nitrogen followed by hydrogen. The mixture was stirred under hydrogen at atmospheric pressure overnight. The catalyst was removed by filtration through diatomaceous earth and the filtrate was concentrated under reduced pressure. The residue was purified by column chromatography (silica, 0-60% CMA in CH₂Cl₂) to afford the title compound (60 mg, 40%).
R_{f}0.22 (40:9:1 Methylene Chloride/Methanol/concentrated Ammonium Hydroxide).
¹H NMR (300 MHz, CD₃OD) δ 0.24-2.38 (m, 41 H), 3.02 (d, J = 11.4 Hz, 1 H), 7.42-7.45 (m, 2H), 7.73-7.76 (m, 2 H). APCI MS m/z 613 [C₃₈H₅₂N₄O₃ + H]⁺. m.p. >300 °C. HPLC (Method A) >99% (214 nm) t_{R} = 14.1 min.

### Example 20

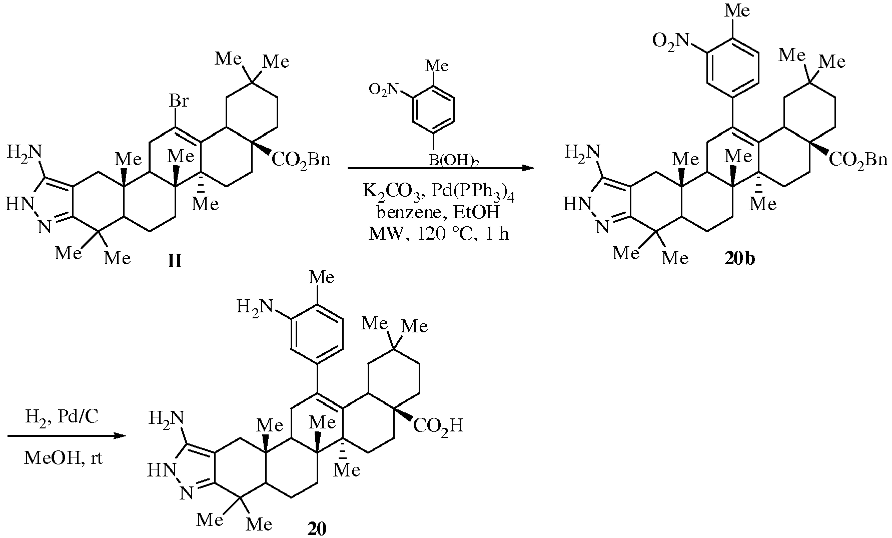

### (i) Preparation of 20b: (4aS,6aS,6bR,13aR)-Benzyl 12-amino-2,2,6a,6b,9,9,13a-heptamethyl-15-(4-methyl-3-nitrophenyl)-2,3,4,4a,5,6,6a,6b,7,8,8a,9,11,13,13a,13b,14,15b-octadecahydro-1H-chryseno[1,2-f]indazole-4a-carboxylate

To a mixture of **II** (250 mg, 0.37 mmol) and 4-methyl-3-nitrophenylboronic acid (136 mg, 0.75 mmol) in benzene (4 mL) and EtOH (1 mL) was added K₂CO₃ (156 mg, 1.13 mmol). The mixture was sparged with nitrogen and then Pd(PPh₃)₄ (87 mg, 0.07 mmol) was added. The reaction mixture was heated at 120 °C for 1 hour using microwave irradiation. The solvent was removed under reduced pressure and then the residue was dissolved in EtOAc (20 mL). The solution was washed with brine then dried (Na₂SO₄), filtered and concentrated under reduced pressure. The residue was purified by column chromatography (silica, 0-40% CMA in CH₂Cl₂) to afford the sub-title compound (186 mg, 69%).
¹H NMR (300 MHz, CD₃OD) δ 0.26-2.34 (m, 41 H), 2.51 (s, 3H), 3.01 (m, 1H), 5.05 (d, *J* = 12.0 Hz, 1 H), 5.23 (d, *J* = 12.0 Hz, 1 H), 7.22-7.37 (m, 7H), 7.80 (s, 1 H). APCI MS *m*/*z* 719 [C₄₅H₅₈N₄O₄ + H]⁺.

### (ii) Perparation of 20: (4aS,6aS,6bR,13aR)-12-Amino-15-(3-amino-4-methylphenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **20b** (180 mg, 0.25 mmol) and MeOH (20 mL) was flushed with nitrogen and then 10% Pd/C (180 mg) was added. The mixture was flushed with nitrogen followed by hydrogen. The mixture was stirred under hydrogen at atmospheric pressure overnight. The catalyst was removed by filtration through diatomaceous earth and the filtrate was concentrated under reduced pressure. The residue was purified by column chromatography (silica, 0-40% CMA in CH₂Cl₂) followed by preparative HPLC to afford the title compound (16 mg, 10%) as a solid.
*R_{f}*0.23 (90:9:1 Methylene Chloride/Methanol/concentrated Ammonium Hydroxide).
¹H NMR (300 MHz, CD₃OD) δ 0.31-2.27 (m, 41 H), 2.36 (s, 3H), 2.99 (d, *J* = 10.5 Hz, 1 H), 7.14-7.32 (m, 3H). APCI MS *m*/*z* 599 [C₃₈H₅₄N₄O₂ + H]⁺m.p. 220-240 °C dec. HPLC (Method A) >99% (214 nm) *t*_{R} = 12.4 min.

### Example 21

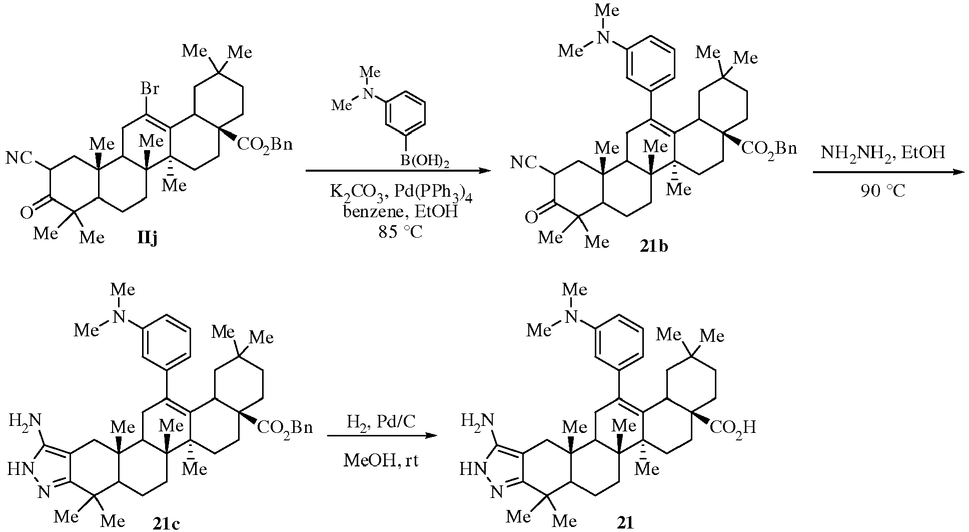

### (i) Preparation of 21b: (4aS,6aS,6bR,12aR)-Benzyl 11-cyano-14-(3-(dimethylamino)phenyl)-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

To a mixture of **IIj** (300 mg, 0.46 mmol) and 3-(dimethylamino)phenylboronic acid (458 mg, 2.78 mmol) in benzene (10 mL) and EtOH (5 mL) was added K₂CO₃ (511 mg, 3.70 mmol). The mixture was sparged with nitrogen and then Pd(PPh₃)₄ (107 mg, 0.09 mmol) was added. The mixture was heated at 85 °C overnight and then concentrated under reduced pressure. The residue was dissolved in EtOAc (50 mL) and the solution was washed with brine then dried (Na₂SO₄), filtered and concentrated under reduced pressure. The residue was purified by column chromatography (silica, 0-10% EtOAc in hexanes) to afford the sub-title compound (270 mg, 84%).

¹H NMR (300 MHz, CDCl₃) δ 0.29-2.30 (m, 41 H), 2.95 (s, 6H), 3.13 (d, *J* = 10.5 Hz, 1 H), 3.81-3.91 (m, 1 H), 5.07 (d, *J*= 12.3 Hz, 1 H), 5.17 (d, *J*= 15.3 Hz, 1H), 6.51 (d, *J* = 6.9 Hz, 1 H), 6.51 (d, *J*= 6.9 Hz, 1 H), 7.03-7.09 (m, 1 H), 7.34-7.36 (m, 5H).

### (ii) Preparation of 21c: (4aS,6aS,6bR,13aR)-Benzyl 12-amino-15-(3-(dimethylamino)phenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a solution of **21b** (266 mg, 0.38 mmol) and EtOH (4 mL) was added hydrazine (60 µL, 1.93 mmol). The mixture was heated at 90 °C overnight and then concentrated under reduced pressure. The residue was purified by column chromatography (0-30% EtOAc in hexanes and 50% CMA in CH₂Cl₂) to afford the sub-title compound (191 mg, 70%).
¹H NMR (300 MHz, CD₃OD) δ 0.27-2.36 (m, 42H), 2.89 (s, 6H), 5.05 (m, 1 H), 5.22 (m, 1 H), 6.49-7.06 (m, 4H), 7.35-7.7.36 (m, 5H). APCI MS *m*/*z* 703 [C₄₆H₆₂N₄O₂ + H]⁺.

### (iii) Preparation of 21: (4aS,6aS,6bR,13aR)-12-Amino-15-(3-(dimethylamino)phenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **21c** (180 mg, 0.25 mmol) and MeOH (20 mL) was flushed with nitrogen and then 10% Pd/C (90 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-40% CMA in CH₂Cl₂) followed by preparative HPLC to afford the title compound (36 mg, 23%) as a solid.
*R_{f}* 0.44 (90:9:1 Methylene Chloride/Methanol/concentrated Ammonium Hydroxide).
¹H NMR (300 MHz, CD₃OD) δ 0.27-2.43 (m, 41 H), 3.04 (d, *J* = 9.6 Hz, 1 H), 3.18 (s, 6H), 7.16-7.47 (m, 4H). APCI MS *m*/*z* 613 [C₃₉H₅₆N₄O₂ + H]⁺. m.p. >300 °C. HPLC (Method A) 98.1 % (214 nm) *t*_{R} = 12.0 min.

### Example 22

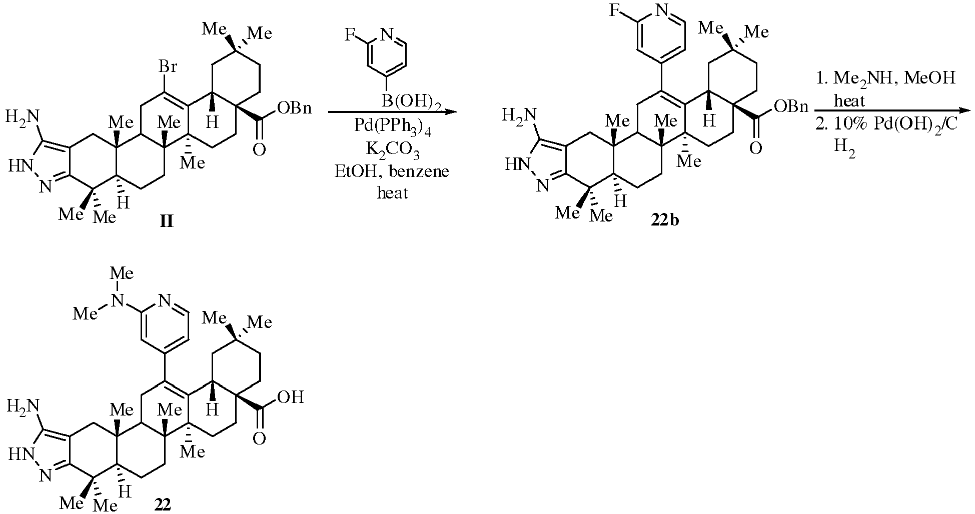

### (i) Preparation of 22b: (4aS,6aS,6bR,8aR,13aR,15bS)-Benzyl 12-amino-15-(2-fluoropyridin-4-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **II** (500 mg, 0.75 mmol), 2-fluoropyridin-4-ylboronic acid (210 mg, 1.50 mmol), Pd(PPh₃)₄ (80 mg, 0.075 mmol) and K₂CO₃ (310 mg, 2.25 mmol) in benzene (4.0 mL) and EtOH (1.0 mL) was sealed and heated to 120 °C by microwave for 1 hour. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (448 mg, 88%) as a brown solid. APCI MS (Positive Mode) *m*/*z* 679 [C₄₃H₅₅FN₄O₂ + H]⁺.

### (ii) Preparation of 22: (4aS,6aS,6bR,8aR,13aR,15bS)-12-Amino-15-(2-(dimethylamino)pyridin-4-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **22b** (150 mg, 0.22 mmol) and dimethylamine (4.0 mL, 2 M in MeOH, 8.0 mmol) was sealed and heated to 140°C by microwave for 5 hours. The mixture was concentrated to dryness. The residue and 10% Pd(OH)₂/C (50 mg) in MeOH (12 mL) was stirred under a hydrogen balloon for 5 hours. The reaction mixture was filtered through a pad of diatomaceous earth and washed with CMA (25 mL). The filtrate was concentrated to dryness under reduced pressure. The residue was purified by column chromatography (silica, 0-10% MeOH in CH₂Cl₂) followed by preparative HPLC to provide the title compound (25 mg, 19%) as an off-white solid.
*R_{f}* 0.65 (80:18:2 Methylene Chloride/Methanol/concentrated Ammonium Hydroxide).
¹H NMR (300 MHz, CD₃OD) δ 0.50 (s, 3H), 0.82 (s, 3H), 0.90 (s, 3H), 0.98 (s, 3H), 1.22 (s, 3H), 1.31 (s, 6H), 1.35-2.20 (m, 24H), 2.28 (m, 1H), 2.31 (d, *J* = 14.8 Hz, 1H), 3.05 (m, 1H), 6.90 (s, 1H), 7.31 (s, 1H), 7.90 (s, 1H). mp >300 °C. APCI MS (Positive Mode) *m*/*z* 614 [C₃₈H₅₅N₅O₂ + H]⁺.

### Example 23

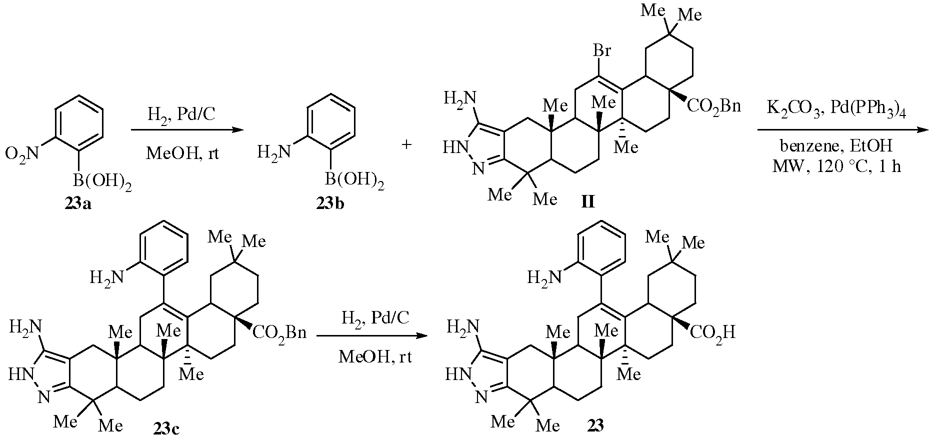

### (i) Preparation of 23b: 2-Aminophenylboronic acid

To a solution of 2-nitrophenylboronic acid (500 mg, 2.99 mmol) and MeOH (10 mL) was added 10% Pd/C (250 mg). The mixture was stirred under hydrogen at atmospheric pressure for 2 hours. The catalyst was removed by filtration through diatomaceous earth and the filtrate was concentrated under reduced pressure. The residue was purified by column chromatography (silica, 0-50% CMA in CH₂Cl₂) to afford the sub-title compound (182 mg, 44%).
¹H NMR (300 MHz, CD₃OD) δ 6.69-7.75 (m, 4H).

### (ii) Preparation of 23c: (4aS,6aS,6bR,13aR)-Benzyl 12-amino-15-(2-aminophenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a mixture of **II** (150 mg, 0.22 mmol) and **23b** (77 mg, 0.56 mmol) in benzene (4 mL) and EtOH (1 mL) was added K₂CO₃ (124 mg, 0.90 mmol). The mixture was sparged with nitrogen and then Pd(PPh₃)₄ (52 mg, 0.04 mmol) was added. The reaction mixture was heated at 120 °C for 1 hour using microwave irradiation and then concentrated under reduced pressure. The residue was dissolved in EtOAc (20 mL). The organic solution was washed with brine then dried (Na₂SO₄), filtered and concentrated. The residue was purified by column chromatography (silica, 0-40% CMA in CH₂Cl₂) to afford the sub-title compound (190 mg, 75%).
APCI MS *m*/*z* 675 [C₄₄H₅₈N₄O₂ + H]⁺.

### (iii) Preparation of 23: (4aS,6aS,6bR,13aR)-12-Amino-15-(2-aminophenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **23c** (180 mg, 0.26 mmol) and MeOH (20 mL) was flushed with nitrogen and then 10% Pd/C (150 mg) was added. The mixture was flushed with nitrogen followed by hydrogen. The mixture was stirred under hydrogen at atmospheric pressure overnight. The catalyst was removed by filtration through diatomaceous earth and the filtrate was concentrated under reduced pressure. The residue was purified by column chromatography (silica, 0-100% CMA in CH₂Cl₂) followed by preparative HPLC to afford the title compound (17 mg, 10%) as a solid.
*R_{f}* 0.28 (40:9:1 Methylene Chloride/Methanol/concentrated Ammonium Hydroxide).
¹H NMR (300 MHz, CD₃OD) δ 0.20-2.47 (m, 48H), δ 7.29 (d, *J* = 7.2 Hz, 1 H), δ 7.41- 7.42 (m, 3H). APCI MS *m*/*z* 585 [C₃₇H₅₂N₄O₂ + H]⁺ m.p. 210-230 °C dec. HPLC (Method A) >99% (214 nm) *t*_{R} = 12.7 min.

### Example 24

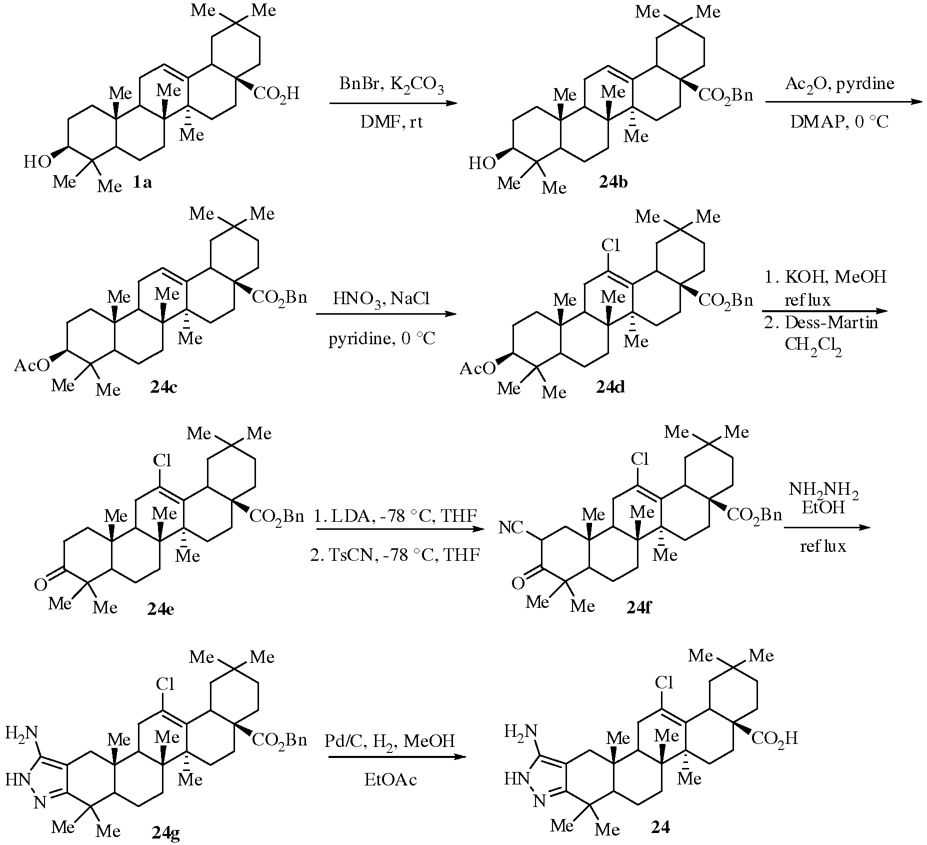

### (i) Preparation of 24b: (4aS,6aS,6bR,10S,12aR)-Benzyl 10-hydroxy-2,2,6a,6b,9,9,12a-heptamethyl-1,2,3,4,4a,5,6,6a,6b,7,8,8a,9,10,11,12,12a,12b,13,14b-icosahydropicene-4a-carboxylate

A mixture of oleanolic acid (**1a,** 6.0 g, 13.1 mmol), benzyl bromide (2.8 g, 16.4 mmol), K₂CO₃ (2.7 g, 19.7 mmol) and DMF (120 mL) was stirred at room temperature for 15 hours. The solvent was removed under reduced pressure and the residue was purified by column chromatography (silica, 30% EtOAc in hexanes) to provide the sub-title compound (6.6 g, 92%) as a white solid.
¹H NMR (300 MHz, CDCl₃) 0.61 (s, 3H), 0.65-0.72 (m, 1 H), 0.78 (s, 3H), 0.87-1.00 (m, 10H), 1.05-1.46 (m, 12H), 1.49-1.72 (m, 12H), 1.81-2.05 (m, 3H), 2.86-2.91 (m, 1 H), 3.15-3.27 (m, 1 H), 5.02-5.11 (m, 2H), 5.43-5.45 (m, 1 H), 7.31-7.49 (m, 5H).

### (ii) Preparation of 24c: (4aS,6aS,6bR,10S,12aR)-Benzyl 10-acetoxy-2,2,6a,6b,9,9,12a-heptamethyl-1,2,3,4,4a,5,6,6a,6b,7,8,8a,9,10,11,12,12a,12b,13,14b-icosahydropicene-4a-carboxylate

To a solution of **24b** (6.6 g, 12.1 mmol), DMAP (200 mg) and pyridine (65 mL) was slowly added acetic anhydride (1.5 g, 14.5 mmol) at 0 °C under nitrogen. The mixture was allowed to slowly warm to room temperature overnight and was quenched by pouring into one liter of H₂O. The precipitate was collected by filtration and dried in a vacuum oven at 40°C to provide the sub-title compound (6.6 g, 93%) as a white solid.
¹H NMR (300 MHz, CDCl₃) 0.61 (s, 3H), 0.77-0.94 (m, 16H), 0.98-1.75 (m, 21 H), 1.84-2.02 (m, 3H), 2.04 (s, 3H), 2.85-2.95 (m, 1 H), 4.46-4.51 (m, 1 H), 5.01-5.08 (m, 2H), 5.27-5.29 (m, 1 H), 7.29-7.35 (m, 5H).

### (iii) Preparation of 24d: (4aS,6aS,6bR,10S,12aR)-Benzyl 10-acetoxy-14-chloro-2,2,6a,6b,9,9,12a-heptamethyl-1,2,3,4,4a,5,6,6a,6b,7,8,8a,9,10,11,12,12a,12b,13,14b-icosahydropicene-4a-carboxylate

To a suspension of **24c** (3.0 g, 5.13 mmol) and pyridine (65 mL) at 0 °C was added chlorine gas which was generated by addition of nitric acid to sodium chloride. After 20 min at 0 °C, the mixture was poured into H₂O and the solid was collected by filtration. The crude material was purified by column chromatography (silica, 20% diethyl ether in hexanes) to provide the sub-title compound (1.3 g, 40%).
¹H NMR (500 MHz, CDCl₃) 0.82-0.91 (m, 12H), 1.00 (s, 3H), 1.12-1.79 (m, 22H), 1.88-2.33 (m, 9H), 3.63-3.74 (m, 1 H), 4.45-4.52 (m, 1 H), 5.11-5.21 (m, 2H), 7.31-7.44 (m, 5H).

### (iv) Preparation of 24e: (4aS,6aS,6bR,12aR)-Benzyl 14-chloro-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

A mixture of **24d** (1.0 g, 1.62 mmol), KOH (360 mg, 4.86 mmol) and MeOH (70 mL) was heated at reflux for 96 hours. The solvent was removed and the residue was partitioned between H₂O (50 mL) and CH₂Cl₂ (50 mL). The organic layer was separated then dried (Na₂SO₄), filtered and concentrated under reduced pressure. The crude product was dissolved in CH₂Cl₂ (40 mL) and the Dess-Martin reagent (900 mg, 2.1 mmol) was added at room temperature. The mixture was stirred for 14 hours before quenching with a solution of sodium thiosulfate (12.5 g) and saturated NaHCO₃ (50 mL). After stirring 30 min, the layers were separated and the aqueous layer was extracted with CH₂Cl₂ (50 mL). The combined organic layers were dried (Na₂SO₄), filtered and concentrated under reduced pressure. The residue was purified by column chromatography (silica, 0-25% EtOAc in hexanes) to provide the sub-title compound (785 mg, 100 %) as a white solid.
¹H NMR (300 MHz, CDCl₃) 0.64 (s, 3H), 0.89-1.18 (m, 21 H), 1.22-2.07 (m, 15H), 2.23-2.41 (m, 3H), 2.47-2.61 (m, 1 H), 3.62-3.75 (m, 1 H), 5.05-5.16 (m, 2H), 7.29-7.41 (m, 5H).

### (v) Preparation of 24f: (4aS,6aS,6bR,12aR)-Benzyl 14-chloro-11-cyano-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

A solution of diisopropylamine (0.2 mL, 1.46 mmol) and THF (7 mL) was cooled to -78°C under nitrogen. A solution of *n*-butyllithium (2.5 M in hexanes, 0.65 mL, 1.63 mmol) was slowly added, maintaining the internal temperature below -70 °C. The solution was allowed to stir for 45 min and was then slowly added to a solution of **24e** (500 mg, 0.86 mmol) and THF (10 mL) at -78°C under nitrogen. This solution was stirred for 30 min after which time a suspension of *p*-toluenesulfonyl cyanide (310 mg, 1.72 mmol) and THF (3 mL) was added over 15 min. The solution was stirred for 30 min and then quenched by addition of saturated ammonium chloride solution (10 mL) at -78 OC. The mixture was allowed to warm to room temperature overnight. The organic layer was separated and the aqueous layer was extracted with EtOAc. The combined organic layers were dried (Na₂SO₄) and filtered. The solvent was removed under reduced pressure and the residue was purified by column chromatography (silica, 0-25% EtOAc in hexanes) to provide the sub-title compound (350 mg, 67%).
¹H NMR (500 MHz, CD₃OD) 0.59 (s, 3H), 0.82-2.29 (m, 39H), 3.63-3.74 (m, 1 H), 5.03-5.15 (m, 2H), 7.31-2.7.45 (m, 5H).

### (vi) Preparation of 24g: (4aS,6aS,6bR,13aR)-Benzyl 12-amino-15-chloro-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A solution of **24f** (350 mg, 0.58 mmol) and hydrazine (74 mg, 2.32 mmol) in EtOH (10 mL) was heated at reflux for 16 hours. The solvent and excess hydrazine were removed under reduced pressure. The residue was purified by column chromatography (silica, 0-50% CMA in CH₂Cl₂) to provide the sub-title compound (175 mg, 50%).
¹H NMR (300 MHz, CDCl₃) 0.61 (s, 3H), 0.88 (s, 3H), 0.91 (s, 3H), 0.98 (s, 3H), 1.09-2.06 (m, 27H), 2.23-2.35 (m, 3H), 3.66-3.78 (m, 1 H), 5.02-5.14 (m, 2H), 7.29-7.41 (m, 5H).

### (vii) Preparation of 24: (4aS,6aS,6bR,13aR)-12-Amino-15-chloro-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A suspension of **24g** (150 mg, 0.24 mmol), 10% Pd/C (100 mg) and EtOAc/ MeOH (20% MeOH, 25 mL) was stirred under hydrogen at atmospheric pressure for 23 hours. The catalyst was removed by filtration through diatomaceous earth and the filtrate was concentrated under reduced pressure. The residue was purified by column chromatography (silica, 50-100% CMA in CH₂Cl₂) to provide the title compound (48 mg, 38%) as an off-white solid.
¹H NMR (300 MHz, CD₃OD) 0.89-1.01 (m, 12H), 1.12-1.30 (m, 12H), 1.34-2.10 (m, 15H), 2.33-2.41 (m, 3H), 3.62-3.70 (m, 1H). APCI MS *m*/*z* 528 [C₃₁H₄₆ClN₃O₂ + H]⁺.
HPLC >99% (area %), *t*_{R} = 15.9 min.

### Example 25

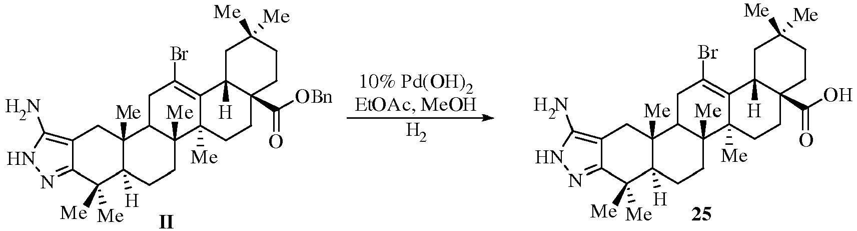

### (i) Preparation of 25: (4aS,6aS,6bR,8aR,13aR,15bR)-12-Amino-15-bromo-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **II** (75 mg, 0.11 mmol) and 10% Pd(OH)₂/C (75 mg) in EtOAc (10 mL) and MeOH (2 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-60% CMA in CH₂Cl₂) to afford the title compound (20 mg, 32%) as a brown solid.
*R_{f}* 0.40 (80:18:2 Methylene Chloride/Methanol/concentrated Ammonium Hydroxide). ¹H NMR (300 MHz, CD₃OD) δ 0.91 (s, 6H), 0.93 (s, 3H), 1.0 (s, 3H), 1.12 (s, 3H), 1.15 (s, 3H), 1.21 (s, 3H), 1.32-2.28 (m, 20H), 2.35 (d, *J* = 14.4 Hz, 1 H), 2.53 (d, *J* = 8.7 Hz, 2H), 3.53 (m, 1H). mp 268-270 °C. ESI MS (Positive Mode) *m*/*z* 572 [C₃₁H₄₆BrN₃O₂ + H]⁺.

### Example 26

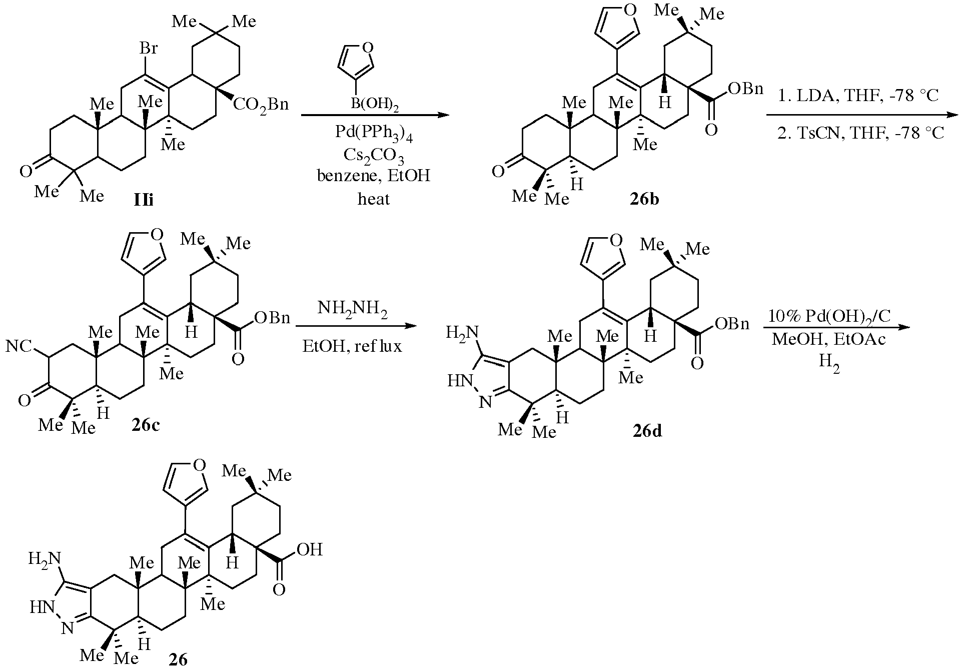

### (i) Preparation of 26b: (4aS,6aS,6bR,8aR,12aR,14bS)-benzyl 14-(furan-3-yl)-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

A mixture of **IIi** (360 mg, 0.57 mmol), furan-3-ylboronic acid (194 mg, 1.73 mmol), Pd(PPh₃)₄ (66 mg, 0.057 mmol) and cesium carbonate (564 mg, 1.73 mmol) in benzene (15 mL) and EtOH (4 mL) was heated at reflux for 12 hours. The reaction mixture was diluted with EtOAc (100 mL). The organic phase was washed with brine then dried (MgSO₄), filtered and concentrated to dryness. The residue was purified by column chromatography (silica, 0-20% EtOAc in hexanes) to afford the sub-title compound (340 mg, 97%).
¹H NMR (300 MHz, CDCl₃) δ 0.57 (s, 3H), 0.77 (s, 3H), 0.81 (s, 3H), 1.05 (s, 3H), 1.07 (s, 3H), 1.12 (s, 3H), 1.22 (s, 3H), 1.23-2.55 (m, 22H), 3.31 (m, 1 H), 5.04 (m, 2H), 6.32 (s, 1 H), 7.20 (s, 1 H), 7.31 (m, 6H).

### (ii) Preparation of 26c: (4aS,6aS,6bR,8aR,12aR,14bS)-benzyl 11-cyano-14-(furan-3-yl)-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

To a solution of diisopropylamine (0.12 mL, 0.84 mmol) in THF (5 mL) was added n-butyllithium (0.35 mL, 2.5 M in hexanes, 0.88 mmol) at-78°C. The solution was stirred for 30 min. The LDA solution was added to **26b** (270 mg, 0.44 mmol) in THF (5 mL). The mixture was allowed to warm to -40°C for 5 min and cooled to -78°C. A suspension of *p*-toluene sulfonyl cyanide (203 mg, 1.12 mmol) in THF (2 mL) was added. The reaction mixture was allowed to warm to -40°C over 1.5 hours. The reaction was quenched by saturated NH₄Cl (3 mL) and extracted with EtOAc (100 mL). The organic phase was washed with brine then dried (MgSO₄), filtered and concentrated to dryness. The residue was purified by column chromatography (silica, 0-20% EtOAc in hexanes) to afford the sub-title compound (150 mg, 54%).
¹H NMR (300 MHz, CDCl₃) δ 0.51 (s, 3H), 0.62 (s, 3H), 0.65 (s, 3H), 1.03 (s, 3H), 1.04 (s, 3H), 1.07 (s, 3H), 1.16 (s, 3H), 1.23-2.55 (m, 21 H), 3.32 (m, 1 H), 5.02 (m, 2H), 6.32 (s, 1 H), 7.20 (s, 1 H), 7.31 (m, 6H).

### (iii) Preparation of 26d: (4aS,6aS,6bR,8aR,13aR,15bS)-Benzyl 12-amino-15-(furan-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **26c** (150 mg, 0.23 mmol) and hydrazine (0.030 mL) in EtOH (3 mL) was heated at reflux overnight. The reaction mixture was concentrated to dryness under reduced pressure. The residue was purified by column chromatography (silica, 0-10% MeOH in CH₂Cl₂) to afford the sub-title compound (110 mg, 71 %).
ESI MS (Positive Mode) *m*/*z* 650 [C₄₂H₅₅N₃O₃ + H]⁺.

### (iv) Preparation of 26: (4aS,6aS,6bR,8aR,13aR,15bS)-12-Amino-15-(furan-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **26d** (110 mg, 0.17 mmol) and 10% Pd(OH)₂/C (50 mg) in MeOH (6 mL) and EtOAc (6 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-50% CMA in CH₂Cl₂) to afford the title compound (20 mg, 21%) as a brown solid.
R_{f}0.45 (80:18:2 Methylene Chloride/Methanol/concentrated Ammonium Hydroxide). ¹H NMR (300 MHz, DMSO-*d*₆) δ 0.53 (s, 3H), 0.78 (s, 3H), 0.80 (s, 3H), 0.84 (s, 3H), 1.05 (s, 3H), 1.16 (s, 6H), 1.26-2.25 (m, 19H), 2.25 (d, *J*= 14.7 Hz, 1 H), 6.48 (s, 1 H), 7.48 (s, 1 H), 7.59 (s, 1 H). mp >300 °C. APCI MS (Positive Mode) *m*/*z* 560 [C₃₅H₄₉N₃O₃ + H]⁺.

### Example 27

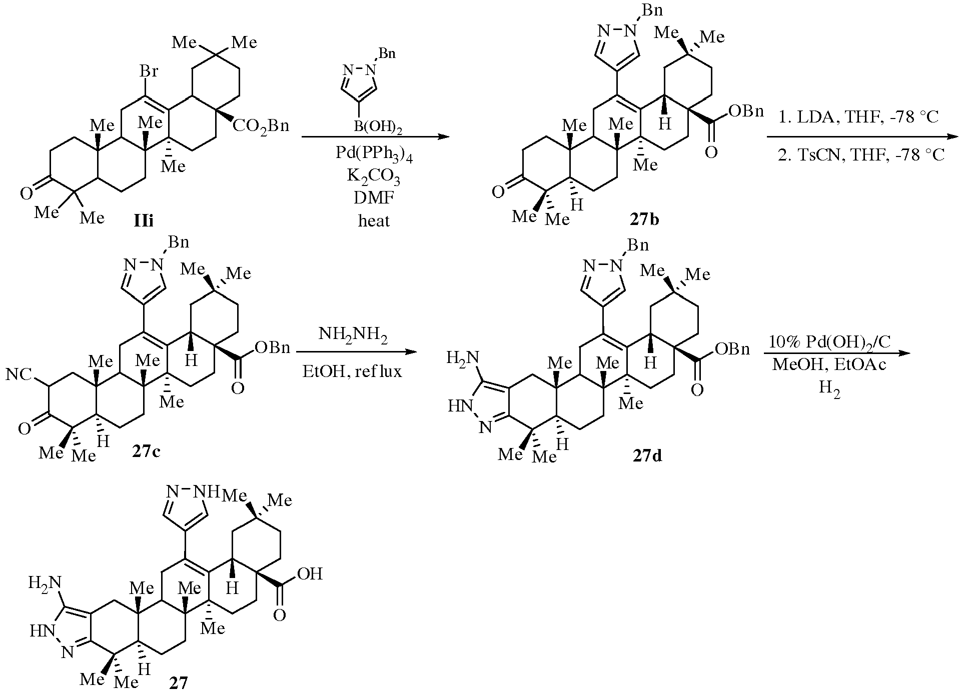

### (i) Preparation of 27b: (4aS,6aS,6bR,8aR,2aR,4bS)-Benzyl 14-(1-benzyl-1H-pyrazol-4-yl)-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate carboxylate

A mixture of **IIi (**300 mg, 0.48 mmol), 1-benzyl-1*H*-pyrazol-4-ylboronic acid (291 mg, 1.44 mmol), Pd(PPh₃)₄ (60 mg, 0.048 mmol) and K₂CO₃ (198 mg, 1.44 mmol) in DMF (9 mL) was heated at 100 °C for 24 hours. The reaction mixture was diluted with EtOAc (100 mL). The organic phase was washed with brine then dried (MgSO₄), filtered and concentrated to dryness. The residue was purified by column chromatography (silica, 0-20% EtOAc in hexanes) to afford the sub-title compound (280 mg, 83%).
¹H NMR (300 MHz, CDCl₃)δ 0.43 (s, 3H), 0.76 (s, 3H), 0.77 (s, 3H), 0.78-2.53 (m, 37H), 3.32 (m, 1 H), 5.02 (m, 4H), 7.15 (m, 4H), 7.31 (m, 8H).

### (ii) Preparation of 27c: (4aS,6aS,6bR,8aR,2aR,4bS)-Benzyl 14-(1-benzyl-1H-pyrazol-4-yl)-11-cyano-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylate

To a solution of diisopropylamine (0.13 mL, 0.95 mmol) in THF (5 mL) was added *n*-butyllithium (0.4 mL, 2.5 M in hexanes, 1.0 mmol) at-78°C. The solution was stirred for 30 min. The LDA solution was added to **27b** (350 mg, 0.50 mmol) in THF (5 mL). The mixture was allowed to warm to -40°C for 5 min and cooled to -78°C. A suspension of *p*-toluene sulfonyl cyanide (181 mg, 1.0 mmol) in THF (2 mL) was added. The reaction mixture was allowed to warm to -40°C over 1.5 hours. The reaction was quenched by saturated NH₄Cl (3 mL) and extracted with EtOAc (100 mL). The organic phase was washed with brine then dried (MgSO₄), filtered and concentrated to dryness. The residue was purified by column chromatography (silica, 0-20% EtOAc in hexanes) to afford the sub-title compound (160 mg, 44%).

### (iii) Preparation of 27d: (4aS,6aS,6bR,8aR,13aR,15bS)-Benzyl 12-amino-15-(1-benzyl-1H-pyrazol-4-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **27c** (160 mg, 0.22 mmol) and hydrazine (0.10 mL, 0.69 mmol) in EtOH (3 mL) was heated at reflux overnight. The reaction mixture was concentrated to dryness under reduced pressure. The residue was purified by column chromatography (silica, 0-40% CMA in CH₂Cl₂) to afford the sub-title compound (60 mg, 62%). APCI MS (Positive Mode) *m*/*z* 740 [C₄₁H₅₅N₅O₂ + H]⁺.

### (iv) Preparation of 27: (4aS,6aS,6bR,8aR,13aR,15bS)-12-Amino-2,2,6a,6b,9,9,13a-heptamethyl-15-(1H-pyrazol-4-yl)-2,3,4,4a,5,6,6a,6b,7,8,8a,9,11,13,13a,13b,14,15b-octadecahydro-1 H-chryseno[1,2-f]indazole-4a-carboxylic acid

A mixture of **27d** (60 mg, 0.082 mmol) and 10% Pd(OH)₂/C (60 mg) in MeOH (12 mL) and EtOAc (2 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-80% CMA in CH₂Cl₂) to afford the title compound (8 mg, 18%) as a brown solid.
*R_{f}* 0.20 (80:18:2 Methylene Chloride/Methanol/concentrated Ammonium Hydroxide).
¹H NMR (300 MHz, CD₃OD + CDCl₃) δ 0.52 (s, 3H), 0.77 (s, 3H), 0.88 (s, 3H), 0.95 (s, 3H), 1.16 (s, 3H), 1.24 (s, 6H), 1.32-2.28 (m, 20H), 2.32 (d, *J* = 14.7 Hz, 1 H), 7.57 (s, 1 H), 7.82 (s, 1H). mp >300 °C. APCI MS (Positive Mode) *m*/*z* 560 [C₃₄H₄₉N₅O₂ + H]⁺.

### Example 28

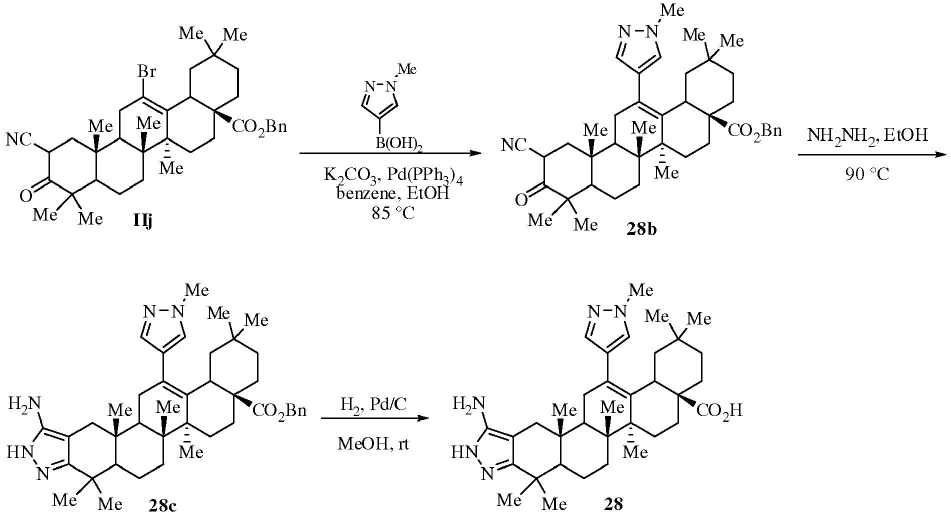

### (i) Preparation of 28b: (4aS,6aS,6bR,8aR,12aR,14bS)-Benzyl 11-cyano-2,2,6a,6b,9,9,12a-heptamethyl-14-(1-methyl-1H-pyrazol-4-yl)-10-oxo-1,2,3,4,4a,5,6,6a,6b,7,8,8a,9,10,11,12,12a,12b,13,14b-icosahydropicene-4a-carboxylate

A mixture of **IIj** (100 mg, 0.15 mmol), 1-methyl-1*H*-pyrazol-4-ylboronic acid (75.0 mg, 0.60 mmol), Pd(PPh₃)₄ (34 mg, 0.030 mmol) and K₂CO₃ (184 mg, 1.33 mmol) in benzene (3.5 mL) and EtOH (1.5 mL) was sealed and heated to 120 °C by microwave for 1 hour. The reaction mixture was diluted with EtOAc (100 mL). The organic phase was washed with brine then dried (MgSO₄), filtered and concentrated to dryness. The residue was purified by column chromatography (silica, 0-30% EtOAc in hexanes) to afford the sub-title compound (60 mg, 61%).
¹H NMR (300 MHz, CDCl₃) δ 0.54 (s, 3H), 0.76 (s, 3H), 0.80 (s, 3H), 0.81-2.05 (m, 32H), 3.32 (m, 1 H), 3.77 (s, 3H), 3.85 (m, 1 H), 5.02 (m, 2H), 7.10 (s, 1 H), 7.33 (m, 6H).

### (ii) Preparation of 28c: (4aS,6aS,6bR,8aR,13aR,15bS)-Benzyl 12-amino-2,2,6a,6b,9,9,13a-heptamethyl-15-(1H-pyrrol-3-yl)-2,3,4,4a,5,6,6a,6b,7,8,8a,9,11,13,13a,13b,14,15b-octadecahydro-1H-chryseno[1,2-f]indazole-4a-carboxyl ate

A mixture of **28b** (60 mg, 0.09 mmol) and hydrazine (0.05 mL) in EtOH (1.5 mL) was heated at reflux overnight. The reaction mixture was concentrated to dryness under reduced pressure. The residue was purified by column chromatography (silica, 0-30% CMA in CH₂Cl₂) to afford the sub-title compound (33 mg, 54%). APCI MS (Positive Mode) *m*/*z* 664 [C₄₂H₅₇N₅O₂ + H]⁺.

### (iii) Preparation of 28: (4aS,6aS,6bR,8aR,13aR,15bS)-12-Amino-2,2,6a,6b,9,9,13a-heptamethyl-15-(1-methyl-1H-pyrazol-4-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **28c** (33 mg, 0.05 mmol) and 10% Pd(OH)₂/C (16 mg) in MeOH (12 mL) was stirred under a hydrogen balloon for 5 hours. The reaction mixture was filtered through a pad of diatomaceous earth and washed with CMA (25 mL). The filtrate was concentrated to dryness under reduced pressure. The residue was purified by column chromatography (silica, 0-50% CMA in CH₂Cl₂) to afford the title compound (20 mg, 71 %) as a brown solid.
*R_{f}* 0.64 (9:1 Methylene Chloride/Methanol).
¹H NMR (300 MHz, CD₃OD) δ 0.55 (s, 3H), 0.81 (s, 3H), 0.87 (s, 3H), 0.93 (s, 3H), 1.15 (s, 3H), 1.23 (s, 3H), 1.24 (s, 3H), 1.25-2.18 (m, 19H), 2.36 (d, *J*= 14.8 Hz, 1H), 3.34 (m, 1 H), 7.45 (s, 1 H), 7.52 (s, 1 H). mp >300 °C. APCI MS (Positive Mode) *m*/*z* 574 **[C₃₅H₅₁N₅O₂ + H]⁺.**

### Example 29

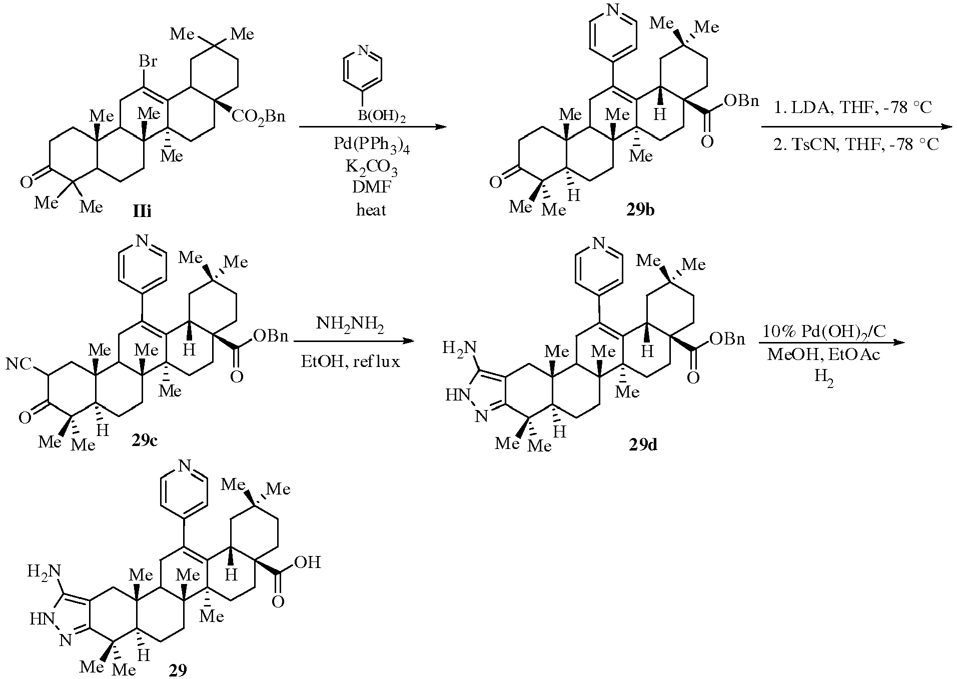

### (i) Preparation of 29b: (4aS,6aS,6bR,8aR,12aR,14bS)-Benzyl 2,2,6a,6b,9,9,12a-heptamethyl-10-oxo-14-(pyridin-4-yl)-1,2,3,4,4a,5,6,6a,6b,7,8,8a,9,10,11,12,12a,12b,13,14b-icosahydropicene-4a-carboxylate

A mixture of **IIi** (400 mg, 0.64 mmol), pyridine-4ylboronic acid (395 mg, 3.21 mmol), Pd(PPh₃)₄ (222 mg, 0.19 mmol) and K₂CO₃ (443 mg, 3.21 mmol) in DMF (10 mL) was heated at 100 °C for 24 hours. The reaction mixture was diluted with EtOAc (100 mL). The organic phase was washed with brine then dried (MgSO₄), filtered and concentrated to dryness. The residue was purified by column chromatography (silica, 0-30% EtOAc in hexanes) to afford the sub-title compound (185 mg, 46%).
¹H NMR (300 MHz, CDCl₃) δ 0.32 (s, 3H), 0.75 (s, 3H), 0.89 (s, 3H), 1.02 (s, 3H), 1.04 (s, 3H), 1.10 (s, 3H), 1.23 (s, 3H), 1.32-2.05 (m, 20H), 2.30 (m, 1H), 2.45 (m,1H), 2.98 (m, 1H), 5.10 (m, 2H), 7.10 (d, *J* = 5.7 Hz, 2H), 7.30 (m, 5H), 8.35 (d, *J*= 5.7 Hz, 2H).

### (ii) Preparation of 29c: (4aS,6aS,6bR,8aR,12aR,14bS)-Benzyl 11-cyano-2,2,6a,6b,9,9,12a-heptamethyl-10-oxo-14-(pyridin-4-yl)-1,2,3,4,4a,5,6,6a,6b,7,8,8a,9,10,11,12,12a,12b,13,14b-icosahydropicene-4a-carboxylate

To a solution of diisopropylamine (0.16 mL, 1.16 mmol) in THF (5 mL) was added *n*-butyllithium (0.48 mL, 2.5 M in hexanes, 1.2 mmol) at -78°C. The solution was stirred for 30 min. The LDA solution was added to **29b** (326 mg, 0.52 mmol) in THF (5 mL). The mixture was allowed to warm to -40°C for 5 min and cooled to -78°C. A suspension of p-toluene sulfonyl cyanide (188 mg, 1.0 mmol) in THF (2 mL) was added. The reaction mixture was allowed to warm to -40 °C over 1.5 hours. The reaction was quenched by saturated NH₄Cl (3 mL) and extracted with EtOAc (100 mL). The organic phase was washed with brine then dried (MgSO₄), filtered and concentrated to dryness. The residue was purified by column chromatography (silica, 0-30% EtOAc in hexanes) to afford the sub-title compound (171 mg, 51 %).
¹H NMR (300 MHz, CDCl₃) δ 0.25 (s, 3H), 0.75 (s, 3H), 0.82 (s, 3H), 1.09 (s, 3H), 1.13 (s, 3H), 1.15 (s, 3H), 1.17 (s, 3H), 1.18-2.25 (m, 20H), 2.95 (m, 1H), 3.85 (m, 1H), 5.04 (m, 2H), 7.06 (m, 2H), 7.37 (m, 5H), 8.44 (m, 2H).

### (iii) Preparation of 29d: (4aS,6aS,6bR,8aR,13aR,15bS)-Benzyl 12-amino-2,2,6a,6b,9,9,13a-heptamethyl-15-(pyridin-4-yl)-2,3,4,4a,5,6,6a,6b,7,8,8a,9,11,13,13a,13b,14,15b-octadecahydro-1H-chryseno[1,2-f]indazole-4a-carboxylate

A mixture of **29c** (171 mg, 0.26 mmol) and hydrazine (0.025 mL, 0.80 mmol) in EtOH (3 mL) was heated at reflux overnight. The reaction mixture was concentrated to dryness under reduced pressure. The residue was purified by column chromatography (silica, 0-10% MeOH in CH₂Cl₂) to afford the sub-title compound (143 mg, 83%).
ESI MS (Positive Mode) *m*/*z* 661 [C₄₃H₅₆N₄O₂ + H]⁺.

### (iv) Preparation of 29: (4aS,6aS,6bR,8aR,13aR,15bS)-12-Amino-2,2,6a,6b,9,9,13a-heptamethyl-15-(pyridin-4-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **29d** (143 mg, 0.21 mmol) and 10% Pd(OH)₂/C (50 mg) in MeOH (15 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-50% CMA in CH₂Cl₂) to afford the title compound (85 mg, 72%) as a brown solid.
R*_{f}*0.24 (80:18:2 Methylene Chloride/Methanol/concentrated Ammonium Hydroxide).
¹H NMR (300 MHz, CD₃OD) δ 0.32 (s, 3H), 0.75 (s, 3H), 0.89 (s, 3H), 0.98 (s, 3H), 1.10 (s, 3H), 1.19 (s, 3H), 1.25 (s, 3H), 1.32-2.28 (m, 20H), 2.32 (d, *J*= 14.7 Hz, 1 H), 3.0 (m, 1 H), 7.40 (d, *J* = 5.7 Hz, 2H), 8.47 (d, *J* = 5.7 Hz, 1 H). mp >300 °C. ESI MS (Positive Mode) *m*/*z* 571 [C₃₆H₅₀N₄O₂ + H]⁺.

### Example 30

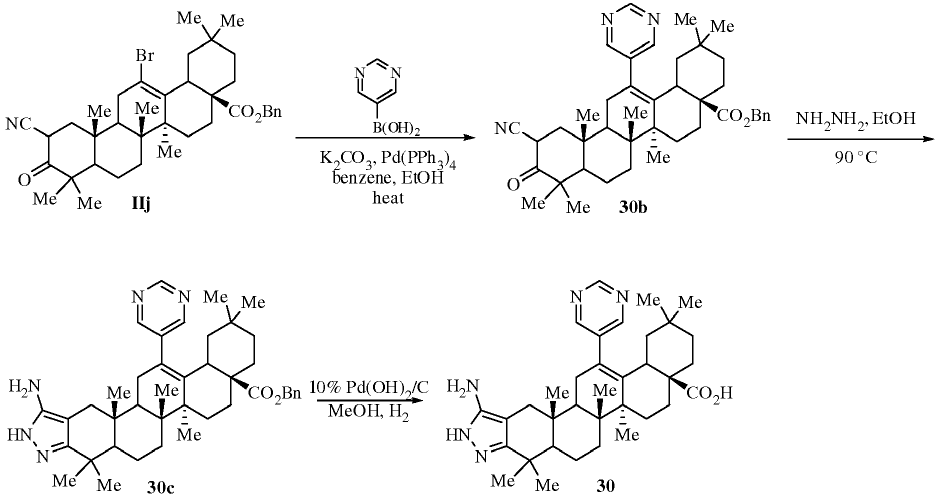

### (i) Preparation of 30b: (4aS,6aS,6bR,8aR,12aR,14bS)-Benzyl 11-cyano-2,2,6a,6b,9,9,12a-heptamethyl-10-oxo-14-(pyrimidin-5-yl)-1,2,3,4,4a,5,6,6a,6b,7,8,8a,9,10,11,12,12a,12b,13,14b-icosahydropicene-4a-carboxylate

A mixture of **IIj** (200 mg, 0.30 mmol), pyrimidin-5-ylboronic acid (115 mg, 0.91 mmol), Pd(PPh₃)₄ (34 mg, 0.030 mmol) and K₂CO₃ (184 mg, 1.33 mmol) in benzene (3.5 mL) and EtOH (1.5 mL) was sealed and heated to 120 °C by microwave for 1 hour. The reaction mixture was diluted with EtOAc (100 mL). The organic phase was washed with brine then dried (MgSO₄), filtered and concentrated to dryness. The residue was purified by column chromatography (silica, 0-40% EtOAc in hexanes) to afford the sub-title compound (90 mg, 40%).

### (ii) Preparation of 30c: (4aS,6aS,6bR,8aR,13aR,15bS)-Benzyl 12-amino-2,2,6a,6b,9,9,13a-heptamethyl-15-(pyrimidin-5-yl)-2,3,4,4a,5,6,6a,6b,7,8,8a,9,11,13,13a,13b,14,15b-octadecahydro-1H-chryseno[1,2-f]indazole-4a-carboxylate

A mixture of **30b** (90 mg, 0.13 mmol) and hydrazine (0.04 mL) in EtOH (1.5 mL) was heated at reflux overnight. The reaction mixture was concentrated to dryness under reduced pressure. The residue was purified by column chromatography (silica, 0-30% CMA in CH₂Cl₂) to afford the sub-title compound (65 mg, 71%).
APCI MS (Positive Mode) *m*/*z* 664 [C₄₂H₅₅N₅O₂ + H]⁺.

### (iii) Preparation of 30: (4aS,6aS,6bR,8aR,13aR,15bS)-12-Amino-2,2,6a,6b,9,9,13a-heptamethyl-15-(pyrimidin-5-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **30c** (65 mg, 0.098 mmol) and 10% Pd(OH)₂/C (30 mg) in MeOH (10 mL) was stirred under a hydrogen balloon for 5 hours. The reaction mixture was filtered through a pad of diatomaceous earth and washed with CMA (25 mL). The filtrate was concentrated to dryness under reduced pressure. The residue was purified by column chromatography (silica, 0-60% CMA in CH₂Cl₂) to afford the title compound (32 mg, 57%) as a brown solid.
*R_{f}*0.50 (80:18:2 Methylene Chloride/Methanol/concentrated Ammonium Hydroxide).
¹H NMR (300 MHz, CD₃OD) δ 0.31 (s, 3H), 0.80 (s, 3H), 0.89 (s, 3H), 1.02 (s, 3H), 1.20 (s, 3H), 1.25 (s, 3H), 1.31 (s, 3H), 1.32-2.30 (m, 21 H), 2.33 (d, *J* = 14.8 Hz, 1 H), 2.88 (m, 1 H), 8.78 (s, 1 H), 9.04 (s, 1 H). mp >300 °C. APCI MS (Positive Mode) *m*/*z* 572 [C₃₅H₄₉N₅O₂ + H]⁺.

### Example 31

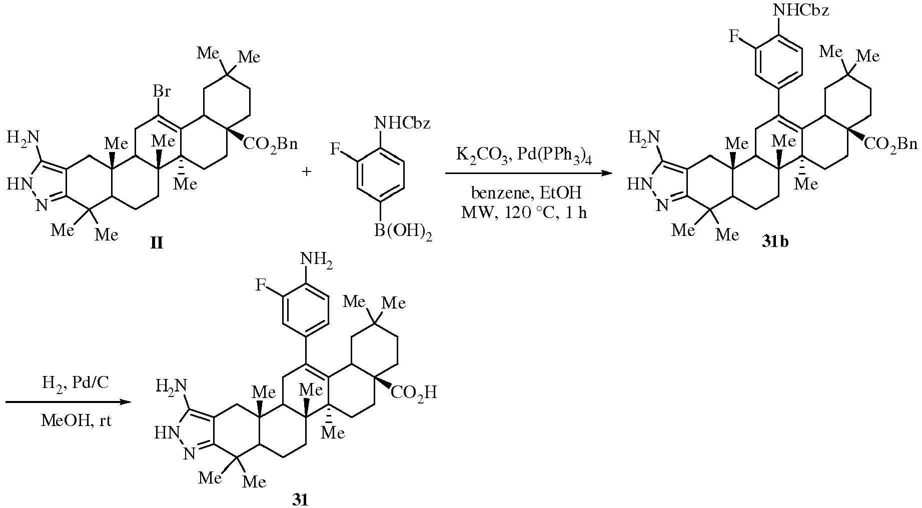

### (i) Preparation of 31b: (4aS,6aS,6bR,13aR)-Benzyl 12-amino-15-(4-(benzyloxycarbonylamino)-3-fluorophenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a mixture of **II** (200 mg, 0.30 mmol) and 4-(benzyloxycarbonylamino)-3-fluorophenylboronic acid (262 mg, 0.90 mmol) in benzene (4 mL) and EtOH (1 mL) was added K₂CO₃ (166 mg, 1.20 mmol). The mixture was sparged with nitrogen and then Pd(PPh₃)₄ (69 mg, 0.06 mmol) was added. The reaction mixture was heated at 120 °C for 1 hour using microwave irradiation and then solvent was removed under reduced pressure. The residue was dissolved in EtOAc (20 mL) and washed with brine. The organic layer was dried (Na₂SO₄), filtered and concentrated. The residue was purified by column chromatography (silica, 0-10% MeOH in CH₂Cl₂) to afford the sub-title compound (190 mg, 76%).
APCI MS *m*/*z* 827 [C₅₂H₆₃N₄O₄ + H]⁺.

### (ii) Preparation of 31: (4aS,6aS,6bR,13aR)-12-Amino-15-(4-amino-3-fluorophenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **31 b** (190 mg, 0.22 mmol) and MeOH (15 mL) was flushed with nitrogen and then 10% Pd/C (300 mg) was added. The mixture was flushed with nitrogen followed by hydrogen. The mixture was stirred under hydrogen at atmospheric pressure overnight. The catalyst was removed by filtration through diatomaceous earth and the filtrate was concentrated under reduced pressure. The residue was purified by column chromatography (silica, 0-30% CMA in CH₂Cl₂) followed by preparative HPLC to afford the title compound (35 mg, 21%) as as solid.
*R_{f}* 0.74 (40:9:1 Methylene Chloride/Methanol/concentrated Ammonium Hydroxide).
¹H NMR (300 MHz, CD₃OD) δ 0.37-2.43 (m, 41 H), 3.07(dd, *J* = 3.4, 6.5 Hz, 1 H), 6.99-7.10 (m, 3H). APCI MS *m*/*z* 603 [C₃₇H₅₁FN₄O₂ + H]⁺. m.p. 180-200 °C dec. HPLC (Method A) >99% (214 nm) *t_{R}* = 13.4 min.

### Example 32

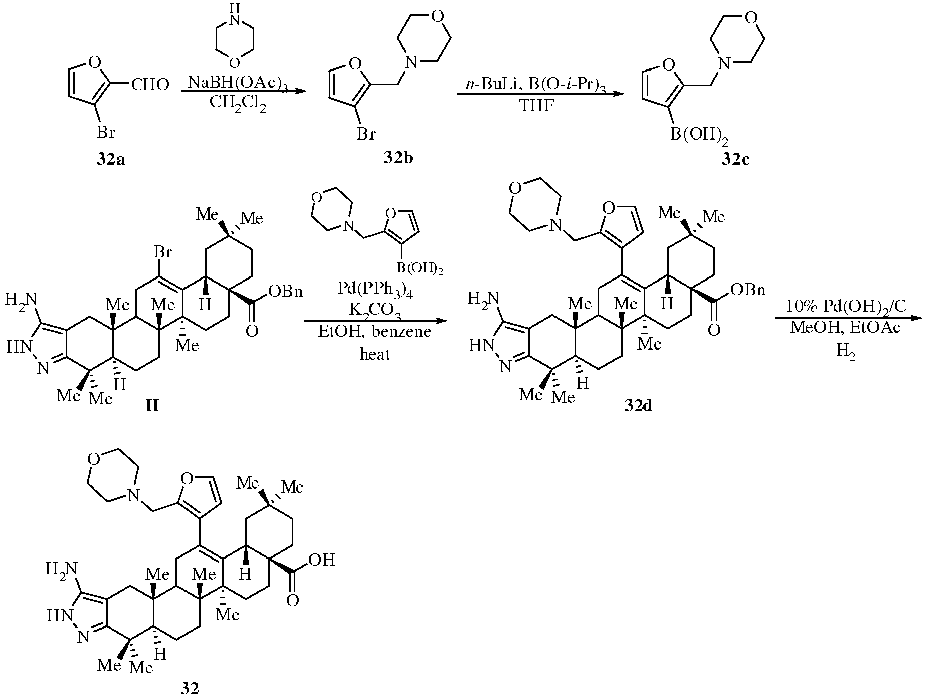

### (i) Preparation of 32b: 4-((3-Bromofuran-2-yl)methyl)morpholine

To a solution of 3-bromofuran-2-carbaldehyde (500 mg, 2.85 mmol) and morpholine (0.5 mL, 5.71 mmol) in CH₂Cl₂ (10 mL) was added sodium triacetoxyborohydride (1.2 g, 5.71 mmol). The mixture was stirred at room temperature for 12 hours. The reaction mixture was diluted with EtOAc (100 mL). The organic phase was washed with aqueous NaHCO₃ and brine then dried (MgSO₄), filtered and concentrated to dryness. The residue was purified by column chromatography (silica, 0-10% MeOH in CH₂Cl₂) to afford the sub-title compound (670 mg, 89%).
¹H NMR (300 MHz, CDCl₃) δ 2.50 (s, 4H), 3.62 (s, 2H), 3.70 (m, 4H), 6.43 (s, 1 H), 7.35 (s, 1H).

### (ii) Preparation of 32c: 2-(Morpholinomethyl)furan-3-ylboronic acid

To a solution of **32b** (670 mg, 2.54 mmol) in THF (15 mL) was added *n*-butyllithium (2.5 M in hexanes, 1.30 mL, 3.31 mmol) at -78°C. The mixture was stirred at -78°C for 20 min. Triisopropyl borate (2.9 mL, 12.7 mmol) was added. The reaction mixture was stirred at -78°C for 1 hour and quenched with HCl (2 M, 4 mL). The reaction mixture was stirred for 5 min and neutralized by NaOH (2 M) and extracted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (450 mg, 83%).
¹H NMR (300 MHz, CDCl₃) δ 2.40 (s, 4H), 3.65 (s, 2H), 3.68 (m, 4H), 6.52 (s, 1 H), 7.30 (s, 1 H).

### (iii) Preparation of 32d: (4aS,6aS,6bR,8aR,13aR,15bS)-Benzyl 12-amino-2,2,6a,6b,9,9,13a-heptamethyl-15-(2-(morpholinomethyl)furan-3-yl)-2,3,4,4a,5,6,6a,6b,7,8,8a,9,11,13,13a,13b,14,15b-octadecahydro-1H-chryseno[1,2-f]indazole-4a-carboxylate

A mixture of **II (200** mg, 0.30 mmol), **32c** (250 mg, 1.18 mmol), Pd(PPh₃)₄ (35 mg, 0.030 mmol) and K₂CO₃ (207 mg, 1.50 mmol) in benzene (3.5 mL) and EtOH (1.5 mL) was sealed and heated to 120 °C by microwave for 1 hour. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (135 mg, 60%).
APCI MS (Positive Mode) *m*/*z* 749 [C₄₇H₆₄N₄O₄ + H]⁺.

### (iv) Preparation of 32: (4aS,6aS,6bR,8aR,13aR,15bS)-12-Amino-2,2,6a,6b,9,9,13a-heptamethyl-15-(2-(morpholinomethyl)furan-3-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **32d** (135 mg, 0.18 mmol) and 10% Pd(OH)₂/C (100 mg) in MeOH (12 mL) and EtOAc (3 mL) was stirred under a hydrogen balloon for 6 hours. The reaction mixture was filtered through a pad of diatomaceous earth and washed with CMA (25 mL). The filtrate was concentrated to dryness under reduced pressure. The residue was purified by column chromatography (silica, 0-50% CMA in CMA) to afford the title compound (35 mg, 30%) as a brown solid.
*R_{f}* 0.63 (80:18:2 Methylene Chloride/Methanol/concentrated Ammonium Hydroxide).
¹H NMR (300 MHz, CD₃OD) δ 0.55 (s, 3H), 0.81 (s, 3H), 0.89 (s, 3H), 0.99 (s, 3H), 1.14 (s, 3H), 1.28 (s, 6H), 1.35-2.28 (m, 20H), 2.61 (m, 4H), 2.90 (m, 1 H), 3.53 (m, 6H), 6.33 (s, 1 H), 7.45 (s, 1H). mp >300 °C dec. APCI MS (Positive Mode) *m*/*z* 659 [C₄₀H₅₈N₄O₄ + H]⁺.

### Example 33

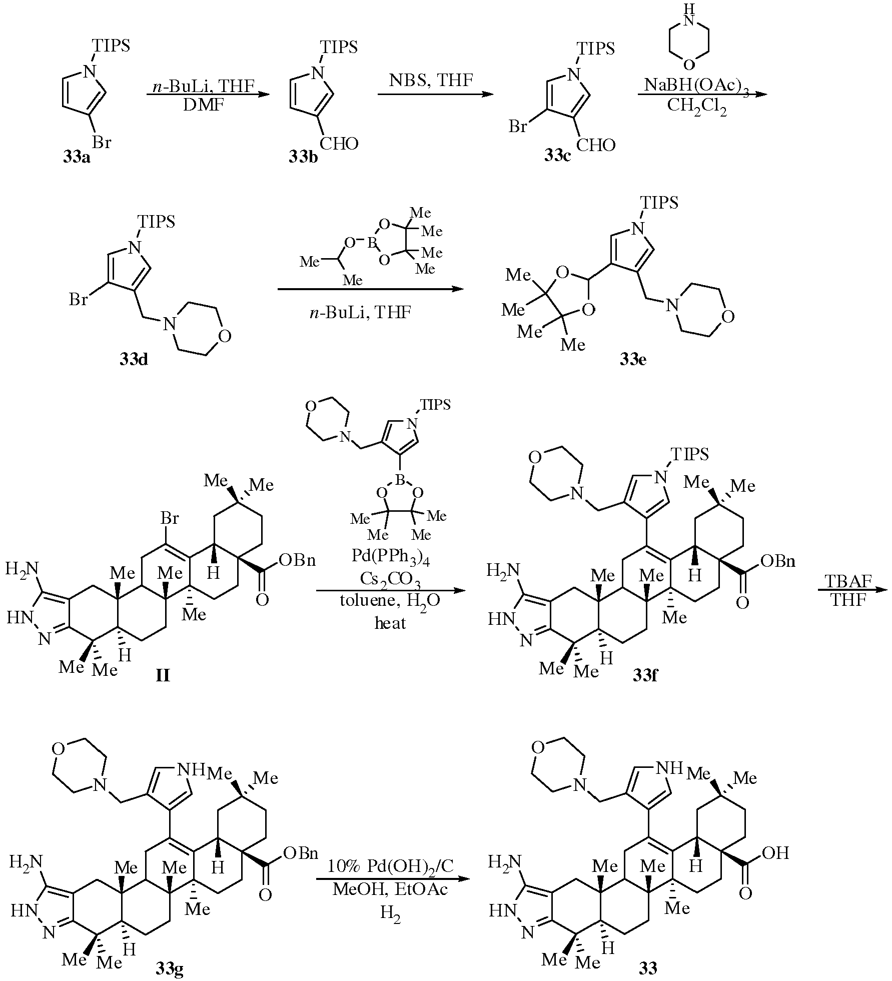

### (i) Preparation of 33b: 1-(Triisopropylsilyl)-1H-pyrrole-3-carbaldehyde

To a solution of 3-bromo-1-(triisopropylsilyl)-1*H*-pyrrole (2.0 g, 6.61 mmol) in THF (30 mL) was added *n*-butyllithium (2.5 M in hexanes, 3.4 mL, 8.60 mmol) at-78°C. The mixture was stirred at -78°C for 10 min then DMF (0.76 mL, 9.92 mmol) was added and the mixture was warmed to 0°C over 1 hour. The reaction mixture was quenched by saturated NH₄Cl and extracted with EtOAc (200 mL). The organic phase was washed with brine then dried (MgSO₄), filtered and concentrated to dryness. The residue was purified by column chromatography (silica, 0-10% EtOAc in hexanes) to afford the sub-title compound (1.65 g, 100%).
¹H NMR (400 MHz, CDCl₃) δ 2.40 (m, 4H), 3.42 (s, 2H), 3.70 (m, 4H), 6.03 (s, 1H), 6.09 (s, 1 H), 6.74 (s, 1 H), 8.30 (bs, 1 H).

### (ii) Preparation of 33c: 4-Bromo-1-(triisopropylsilyl)-1H-pyrrole-3-carbaldehyde

To a solution of **33b** (1.45 g, 5.8 mmol) in THF (40 mL) was added *N-*bromosuccinimide (1.03 g, 5.8 mmol) at room temperature. The mixture was stirred for 2 hours and quenched with H₂O (5 mL) and extracted with EtOAc (100 mL). The organic phase was washed with brine then dried (MgSO₄), filtered and concentrated to dryness. The residue was purified by column chromatography (silica, 0-10% EtOAc in hexanes) to afford the sub-title compound (1.4 g, 74%).
¹H NMR (400 MHz, CDCl₃) δ 1.15 (d, *J*= 2.5 Hz, 18H), 1.58 (m, 3H), 6.70 (s, 1H), 7.32 (s, 1 H), 9.82 (s, 1 H).

### (iii) Preparation of 33d: 4-((4-Bromo-1-(triisopropylsilyl)-1H-pyrrol-3-yl)methyl)morpholine

To a solution of **33c** (1.5 g, 4.5 mmol) and morpholine (0.79 mL, 9.0 mmol) in CH₂Cl₂ (30 mL) was added sodium triacetoxyborohydride (1.9 g, 9.0 mmol). The mixture was stirred at room temperature for 12 hours. The reaction mixture was diluted with EtOAc (200 mL). The organic phase was washed with aqueous NaHCO₃ and brine then dried (MgSO₄), filtered and concentrated to dryness. The residue was purified by column chromatography (silica, 0-10% EtOAc in hexanes) to afford the sub-title compound (1.8 g, 91%).
¹H NMR (400 MHz, CDCl₃) δ 1.15 (d, *J* = 2.5 Hz, 18H), 1.58 (m, 3H), 2.40 (m, 4H), 3.40 (s, 2H), 3.72 (m, 4H), 6.61 (s, 1H), 6.65 (s, 1H).

### (iv) Preparation of 33e: 4-((4-(4,4,5,5-Tetramethyl-1,3-dioxolan-2-yl)-1-(triisopropylsilyl)-1H-pyrrol-3-yl)methyl)morpholine

To a solution of **33d** (400 mg, 1.0 mmol) was added *n*-butyllithium (2.5 M in hexanes, 0.52 mmol, 1.3 mmol) at-78°C. The mixture was stirred for 20 min and 2-isopropoxy-4,4,5,5-tetramethyl-1,3,2-dioxaborolane (0.24 mL, 1.2 mmol) was added. The mixture was stirred at -78°C for 30 min and warmed to room temperature over 1 hour. The reaction was quenched with H₂O and extracted with EtOAc (100 mL). The organic phase was washed with brine then dried (MgSO₄), filtered and concentrated to dryness. The residue was subjected to column chromatography (silica, 0-50% EtOAc in hexanes) to afford the still impure sub-title compound (448 mg, 100%) which was used without further purification.

### (v) Preparation of 33f: (4aS,6aS,6bR,8aR,13aR,15bS)-Benzyl 12-amino-2,2,6a,6b,9,9,13a-heptamethyl-15-(4-(morpholinomethyl)-1-(triisopropylsilyl)-1H-pyrrol-3-yl)-2,3,4,4a,5,6,6a,6b,7,8,8a,9,11,13,13a,13b,14,15b-octadecahydro-1H-chryseno[1,2-f]indazole-4a-carboxylate

A mixture of **II** (200 mg, 0.30 mmol), **33e** (500 mg, 1.06 mmol), Pd(PPh₃)₄ (35 mg, 0.030 mmol) and cesium carbonate (390 mg, 1.20 mmol) in toluene (4.5 mL) and H₂O (0.3 mL) was sealed and heated to 140°C by microwave for 1 hour. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (176 mg, 65%).

### (vi) Preparation of 33g: (4aS,6aS,6bR,8aR,13aR,15bS)-Benzyl 12-amino-2,2,6a,6b,9,9,13a-heptamethyl-15-(4-(morpholinomethyl)-1H-pyrrol-3-yl)-2,3,4,4a,5,6,6a,6b,7,8,8a,9,11,13,13a,13b,14,15b-octadecahydro-1H-chryseno[1,2-f]indazole-4a-carboxylate

To a solution of **33f** (176 mg, 0.19 mmol) in THF (3 mL) was added tetrabutylammonium fluoride (0.38 mL, 1 M in THF, 0.38 mmol). The mixture was stirred at room temperature for 2 hours. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (60 mg, 42%).
APCI MS (Positive Mode) *m*/*z* 748 [C₄₇H₆₅N₅O₃ + H]⁺.

### (vii) Preparation of 33: (4aS,6aS,6bR,8aR,13aR,15bS)-12-Amino-2,2,6a,6b,9,9,13a-heptamethyl-15-(4-(morpholinomethyl)-1H-pyrrol-3-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **33g** (60 mg, 0.08 mmol) and 10% Pd(OH)₂/C (30 mg) in MeOH (12 mL) and EtOAc (3 mL) was stirred under a hydrogen balloon for 6 hours. The reaction mixture was filtered through a pad of diatomaceous earth and washed with CMA (25 mL). The filtrate was concentrated to dryness under reduced pressure. The residue was purified by column chromatography (silica, 0-70% CMA in CMA) to afford the title compound (35 mg, 67%) as a brown solid.
*R_{f}*0.54 (80:18:2 Methylene Chloride/Methanol/concentrated Ammonium Hydroxide).
¹H NMR (400 MHz, CD₃OD) δ 0.55 (s, 3H), 0.85 (s, 3H), 0.92 (s, 3H), 1.12 (s, 3H), 1.15 (s, 3H), 1.38 (m, 6H), 1.39-2.20 (m, 21 H), 2.26 (d, *J* = 14.4 Hz, 1 H), 2.90 (m, 1 H), 3.12 (m, 3H), 3.75 (m, 6H), 6.55 (s, 1H), 7.92 (s, 1H). mp >300 °C dec. APCI MS (Positive Mode) *m*/*z* 658 [C₄₀H₅₉N₅O₃ + H]⁺.

### Example 34

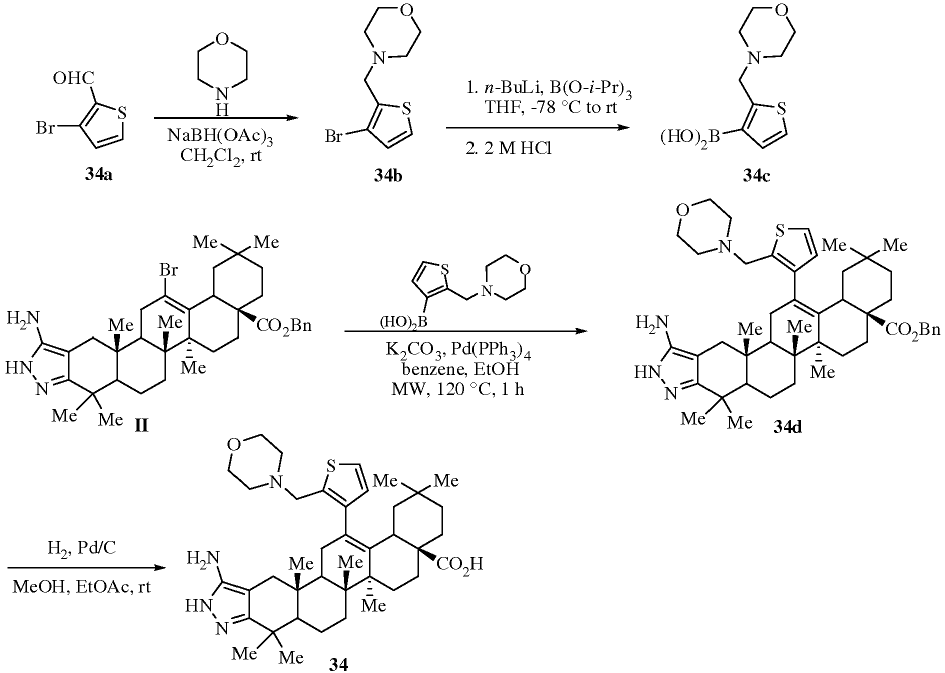

### (i) Preparation of 34b: 4-((3-Bromothiophen-2-yl)methyl)morpholine

To a solution of 3-bromothiophene-2-carbaldehyde (500 mg, 2.62 mmol) and morpholine (0.68 mL, 7.85 mmol) and CH₂Cl₂ (10 mL) was added sodium triacetoxyborohydride (1.11 g, 5.24 mmol). The mixture was stirred at room temperature overnight. The resulting mixture was diluted with EtOAc (100 mL) and the organic layer was washed with brine then dried (Na₂SO₄), filtered and concentrated. The residue was purified by column chromatography (silica, 0-5% MeOH in CH₂Cl₂) to afford the sub-title compound (580 mg, 84%).
¹H NMR (300 MHz, CDCl₃) δ 2.47-2.54 (m, 4H), 3.68-3.71 (m, 4H), 6.90 (d, *J*= 5.2 Hz, 1 H), 7.23 (d, *J* = 5.2 Hz, 1 H).

### (ii) Preparation of 34c: 2-(Morpholinomethyl)thiophen-3-ylboronic acid

To a solution of **34b** (500 mg, 1.90 mmol) in THF (10 mL) was added n-butyllithium (1.14 mL, 2.86 mmol) at -78°C. After stirring for 20 min, triisopropyl borate (1.99 mL, 8.69 mmol) was added at -78°C. The mixture was slowly warmed to room temperature and then quenched with aqueous HCl (2.0 M, 2 mL). The reaction mixture was neutralized with 2 M NaOH solution and extracted with EtOAc (3 × 10 mL) followed by *i*-PrOH/CH₂Cl₂ (1:2, 10 mL × 2). The combined extracts were dried (Na₂SO₄), filtered and concentrated under reduced pressure. The residue was purified by column chromatography (silica, 0-10% MeOH in CH₂Cl₂) to afford the sub-title compound (220 mg, 51 %).
¹H NMR (300 MHz, CDCl₃) δ 2.47-2.49 (m, 4H), 3.52-3.76 (m, 6H), 6.91-6.98 (m, 1 H), 7.22-7.24 (m, 1H).

### (iii) Preparation of 34d: (4aS,6aS,6bR,13aR)-Benzyl 12-amino-2,2,6a,6b,9,9,13a-heptamethyl-15-(2-(morpholinomethyl)thiophen-3-yl)-2,3,4,4a,5,6,6a,6b,7,8,8a,9,11,13,13a,13b,14,15b-octadecahydro-1H-chryseno[1,2-f]indazole-4a-carboxylate

To a mixture of **II** (250 mg, 0.38 mmol) and **34c** (220 mg, 0.96 mmol) in benzene (4 mL) and EtOH (1 mL) was added K₂CO₃ (182 mg, 1.32 mmol). The mixture was sparged with nitrogen and then Pd(PPh₃)₄ (87 mg, 0.075 mmol) was added. The reaction mixture was heated at 120 °C for 1 hour using microwave irradiation. The solvent was concentrated under reduced pressure. The residue was taken up in EtOAc (20 mL) and washed with brine. The organic layer was dried (Na₂SO₄), filtered and concentrated. The residue was purified by column chromatography (silica, 0-10% MeOH in CH₂Cl₂) to afford the sub-title compound (190 mg, 66%).
APCI MS *m*/*z* 765 [C₄₇H₆₄N₄O₃S + H]⁺.

### (iv) Preparation of 34: (4aS,6aS,6bR,13aR)-12-Amino-2,2,6a,6b,9,9,13a-heptamethyl-15-(2-(morpholinomethyl)thiophen-3-yl)-2,3,4,4a,5,6,6a,6b,7,8,8a,9,11,13,13a,13b,14,15b-octadecahydro-1H-chryseno[1,2-f]indazole-4a-carboxylic acid

A solution of **34d** (190 mg, 0.24 mmol), EtOH (2 mL) and MeOH (8 mL) was flushed with nitrogen and then 10% Pd/C (400 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-50% CMA in CH₂Cl₂) followed by preparative HPLC to afford the title compound (18 mg, 11%) as a solid.
*R_{f}* 0.13 (90:9:1 Methylene Chloride/Methanol/concentrated Ammonium Hydroxide).
¹H NMR (300 MHz, CD₃OD) δ 0.40 (s, 3H), 0.80 (s, 3H), 0.92 (s, 3H), 1.05 (s, 3H), 1.10-2.67 (m, 32H), 3.30 (s, 3H), 3.97 (s, 4H), 4.57-4.62 (m, 1H), 6.95 (d, *J* = 4.95 Hz, 1 H), 7.67 (d, *J* = 4.95 Hz, 1H). APCI MS *m*/*z* 675 [C₄₀H₅₈N₄O₃S + H]⁺. m.p. 280-300 °C dec. HPLC (Method A) 98.7% (214 nm) *t*_{R} = 12.9 min.

### Example 35

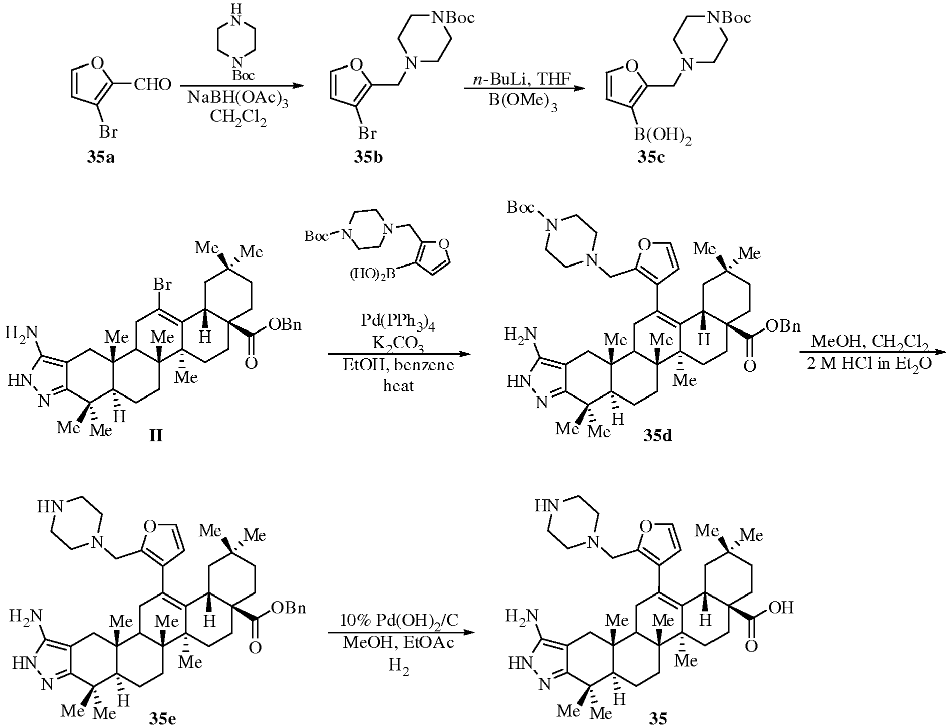

### (i) Preparation of 35b: tert-Butyl 4-((3-bromofuran-2-yl)methyl)piperazine-1-carboxylate

To a solution of 3-bromofuran-2-carbaldehyde (500 mg, 2.85 mmol) and *tert-*butyl piperazine-1-carboxylate (1.1 g, 5.71 mmol) in CH₂Cl₂ (10 mL) was added sodium triacetoxyborohydride (1.2 g, 5.71 mmol). The mixture was stirred at room temperature for 12 hours. The reaction mixture was diluted with EtOAc (100 mL). The organic phase was washed with aqueous NaHCO₃ and brine then dried (MgSO₄), filtered and concentrated to dryness. The residue was purified by column chromatography (silica, 0-10% MeOH in CH₂Cl₂) to afford the sub-title compound (910 mg, 92%).
¹H NMR (300 MHz, CDCl₃) δ 1.42 (s, 9H), 2.40 (m, 4H), 3.42 (m, 4H), 3.60 (s, 2H), 6.35 (s, 1H), 7.35 (s,1H).

### (ii) Preparation of 35c: 2-((4-(tert-Butoxycarbonyl)piperazin-1-yl)methyl)furan-3-ylboronic acid

To a solution of **35b** (910 mg, 2.63 mmol) in THF (20 mL) was added *n*-butyllithium (2.5 M in hexanes, 1.4 mL, 3.43 mmol) at-78°C. The mixture was stirred at-78°C for 20 minutes. Triisopropyl borate (3.0 mL, 13.2 mmol) was added. The reaction mixture was stirred at -78°C for 1 hour and quenched with HCl (2 M, 4 mL). The reaction mixture was stirred for 5 min then neutralized by NaOH (2 M) and extracted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (560 mg, 68%).
¹H NMR (300 MHz, CDCl₃) δ 1.42 (s, 9H), 2.40 (m, 4H), 3.65 (m, 4H), 3.68 (s, 2H), 6.52 (s, 1H), 7.31 (s, 1H).

### (iii) Preparation of 35d: (4aS,6aS,6bR,8aR,13aR,15bS)-Benzyl 12-amino-15-(2-((4-(tert-butoxycarbonyl)piperazin-1-yl)methyl)furan-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **II** (300 mg, 0.45 mmol), **35c** (418 mg, 1.35 mmol), Pd(PPh₃)₄ (52 mg, 0.045 mmol) and K₂CO₃ (248 mg, 1.80 mmol) in benzene (3.5 mL) and EtOH (1.5 mL) was sealed and heated to 120 °C by microwave for 1 hour. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (400 mg, 100%).
APCI MS (Positive Mode) *m*/*z* 848 [C₅₂H₇₃N₅O₅ + H]⁺.

### (iv) Preparation of 35e: (4aS,6aS,6bR,8aR,13aR,15bS)-Benzyl 12-amino-2,2,6a,6b,9,9,13a-heptamethyl-15-(2-(piperazin-1-ylmethyl)furan-3-yl)-2,3,4,4a,5,6,6a,6b,7,8,8a,9,11,13,13a,13b,14,15b-octadecahydro-1H-chryseno[1,2-f]indazole-4a-carboxylate

To a solution of **35d** (400 mg, 0.45 mmol) in MeOH (3 mL) and CH₂Cl₂ (2 mL) was added HCl (2.3 mL, 2 M in Et₂O, 4.6 mmol). The mixture was stirred at room temperature for 12 hours. The reaction mixture was concentrated to dryness. The residue was purified by column chromatography (silica, 0-10% MeOH in CH₂Cl₂) to afford the sub-title compound (250 mg, 74%).
APCI MS (Positive Mode) *m*/*z* 748 [C₄₇H₆₅N₅O₃ + H]⁺.

### (v) Preparation of 35: (4aS,6aS,6bR,8aR,13aR,15bS)-12-Amino-2,2,6a,6b,9,9,13a-heptamethyl-15-(2-(piperazin-1-ylmethyl)furan-3-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **35e** (250 mg, 0.33 mmol) and 10% Pd(OH)₂/C (110 mg) in MeOH (15 mL) and EtOAc (5 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-10% MeOH in CMA) to afford the title compound (160 mg, 75%) as a brown sold.
*R_{f}* 0.20 (80:28:2 Methylene Chloride/Methanol/concentrated Ammonium Hydroxide).
¹H NMR (300 MHz, CD₃OD) δ 0.55 (s, 3H), 0.78 (s, 3H), 0.86 (s, 3H), 0.94 (s, 3H), 1.12 (s, 3H), 1.21 (s, 3H), 1.24 (s, 3H), 1.35-2.18 (m, 19H), 2.36 (d, *J*= 14.8 Hz, 1H), 2.73 (m, 4H), 3.0 (m, 1 H), 3.18 (m, 4H), 3.52 (m, 1H), 3.90 (m, 1H), 6.33 (s, 1H), 7.44 (s, 1H).mp >300 °C. APCI MS (Positive Mode) *m*/*z* 658 [C₄₀H₅₉N₅O₃ + H]⁺.

### Example 36

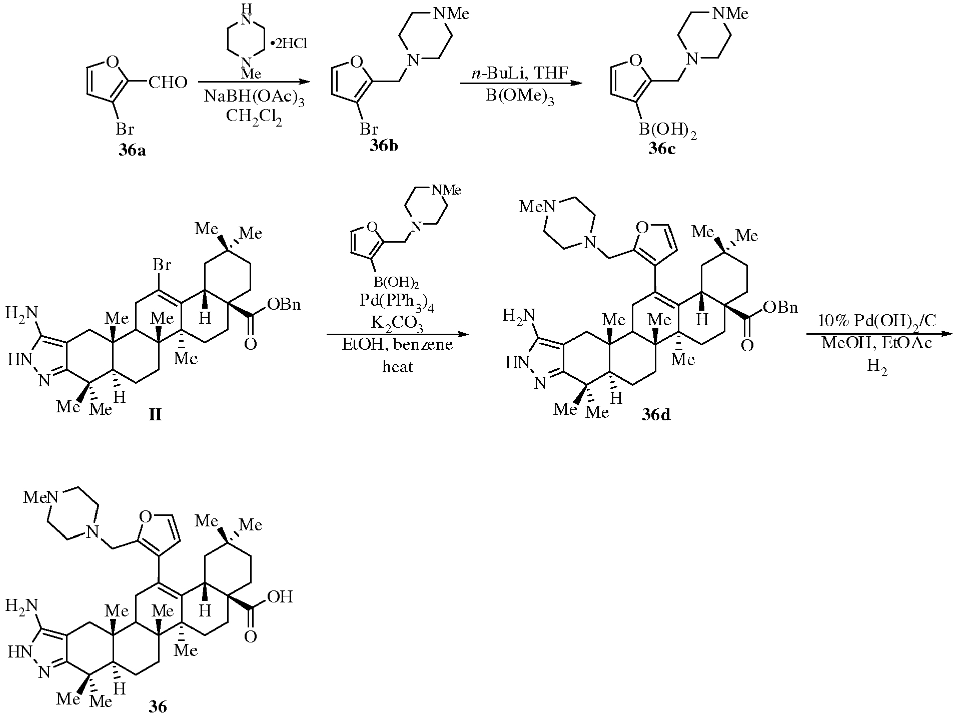

### (i) Preparation of 36b: 1-((3-Bromofuran-2-yl)methyl)-4-methylpiperazine

To a solution of 3-bromofuran-2-carbaldehyde (500 mg, 2.85 mmol), 1-methylpiperazine dihydrochloride (986 mg, 5.71 mmol) and triethylamine (1.5 mL, 11.4 mmol) in CH₂Cl₂ (10 mL) was added sodium triacetoxyborohydride (1.2 g, 5.71 mmol). The mixture was stirred at room temperature for 12 hours. The reaction mixture was diluted with EtOAc (100 mL). The organic phase was washed with aqueous NaHCO₃ and brine then dried (MgSO₄), filtered and concentrated to dryness. The residue was purified by column chromatography (silica, 0-10% MeOH in CH₂Cl₂) to afford the sub-title compound (570 mg, 78%).
¹H NMR (300 MHz, CDCl₃) δ 2.25 (s, 3H), 2.40 (m, 4H), 2.60 (m, 4H), 3.60 (s, 2H), 6.35 (s, 1 H), 7.35 (s, 1 H).

### (ii) Preparation of 36c: 2-((4-Methylpiperazin-1-yl)methyl)furan-3-ylboronic acid

To a solution of **36b** (570 mg, 2.20 mmol) in THF (15 mL) was added *n*-butyllithium (2.5 M in hexanes, 1.1 mL, 2.80 mmol) at-78°C. The mixture was stirred at-78°C for 20 minutes. Triisopropyl borate (2.5 mL, 11.0 mmol) was added. The reaction mixture was stirred at -78°C for 1 hour and quenched with HCl (2 M, 4 mL). The reaction mixture was stirred for 5 min then neutralized by NaOH (2 M) and extracted with EtOAc (100 mL). The organic phase was washed with brine then dried (MgSO₄), filtered and concentrated to dryness. The residue was purified by column chromatography (silica, 0-15% MeOH in CH₂Cl₂) to afford the sub-title compound (200 mg, 41 %).
¹H NMR (300 MHz, CD₃OD) δ 2.32 (s, 3H), 2.40 (m, 4H), 2.80 (m, 4H), 3.95 (s, 2H), 6.46 (s, 1H), 7.38 (s,1H).

### (iii) Preparation of 36d: (4aS,6aS,6bR,8aR,13aR,15bS)-Benzyl 12-amino-2,2,6a,6b,9,9,13a-heptamethyl-15-(2-((4-methylpiperazin-1-yl)methyl)furan-3-yl)-2,3,4,4a,5,6,6a,6b,7,8,8a,9,11,13,13a,13b,14,15b-octadecahydro-1H-chryseno[1,2-f]indazole-4a-carboxylate

A mixture of **II** (200 mg, 0.30 mmol), **36c** (200 mg, 0.89 mmol), Pd(PPh₃)₄ (35 mg, 0.030 mmol) and K₂CO₃ (166 mg, 1.20 mmol) in benzene (3.5 mL) and EtOH (1.5 mL) was sealed and heated to 120°C by microwave for 1 hour. The reaction mixture was diluted with EtOAc (100 mL). The organic phase was washed with brine then dried (MgSO₄), filtered and concentrated to dryness. The residue was purified by column chromatography (silica, 0-50% CMA in CH₂Cl₂) to afford the sub-title compound (200 mg, 87%).
APCI MS (Positive Mode) *m*/*z* 762 [C₄₈H₆₇N₅O₃ + H]⁺.

### (iv) Preparation of 36: (4aS,6aS,6bR,8aR,13aR,15bS)-12-Amino-2,2,6a,6b,9,9,13a-heptamethyl-15-(2-((4-methylpiperazin-1-yl)methyl)furan-3-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **36d** (200 mg, 0.26 mmol) and 10% Pd(OH)₂/C (100 mg) in MeOH (15 mL) and EtOAc (5 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-70% CMA in CH₂Cl₂) to afford the title compound (22 mg, 13%) as a brown solid.
*R_{f}* 0.30 (80:28:2 Methylene Chloride/Methanol/concentrated Ammonium Hydroxide).
¹H NMR (300 MHz, CD₃OD) δ 0.55 (s, 3H), 0.78 (s, 3H), 0.86 (s, 3H), 0.99 (s, 3H), 1.12 (s, 3H), 1.28 (s, 6H), 1.35-2.18 (m, 16H), 2.28 (d, *J* = 14.8 Hz, 1H), 2.29 (m, 4H), 2.40 (m, 9H), 2.90 (m, 1 H), 3.52 (m, 2H), 3.80 (m, 1 H), 6.33 (s, 1 H), 7.47 (s, 1 H). mp >300°C. APCI MS (Positive Mode) *m*/*z* 672 [C₄₁H₆₁N₅O₃ + H]⁺.

### Example 37

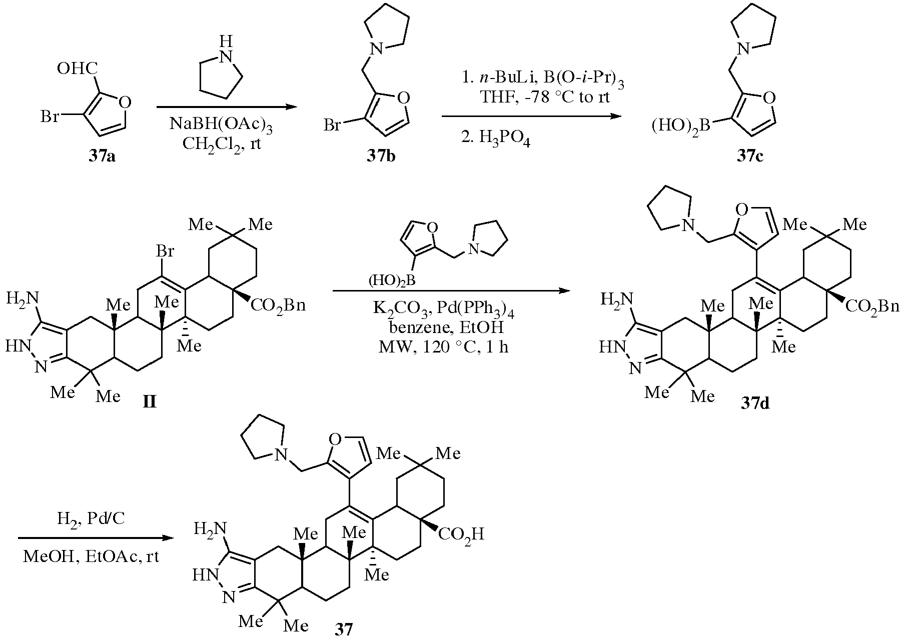

### (i) Preparation of 37b: 1-((3-Bromofuran-2-yl)methyl)pyrrolidine

To a solution of 3-bromofuran-2-carbaldehyde (1.5 g, 8.57 mmol) and pyrrolidine (1.42 mL, 17.14 mmol) and CH₂Cl₂ (30 mL) was added sodium triacetoxyborohydride (3.63 g, 17.14 mmol). The mixture was stirred at room temperature for 5 hours. The resulting mixture was diluted with EtOAc (300 mL) and the organic layer was washed with saturated NaHCO₃ and brine then dried (Na₂SO₄), filtered and concentrated. The residue was purified by column chromatography (silica, 0-10% MeOH in CH₂Cl₂) to afford the sub-title compound (1.26 g, 65%).
¹H NMR (400 MHz, CDCl₃) δ 1.74-1.80 (m, 4H), 2.56-2.59 (m, 4H), 3.67 (s, 2H), 6.37 (d, *J*= 2.0 Hz, 1 H), 7.34 (d, *J*= 2.0 Hz, 1 H).

### (ii) Preparation of 37c: 2-(Pyrrolidin-1-ylmethyl)furan-3-ylboronic acid

To a solution of **37b** (1.29 g, 5.60 mmol) in THF (20 mL) was added *n*-butyllithium (4.26 mL, 10.65 mmol) at-78°C. After stirring for 20 min, triisopropylborate (1.99 mL, 8.69 mmol) was added at -78°C. The mixture was slowly warmed to room temperature and then quenched with H₃PO₄ (85% in H₂O, 3 mL). The reaction mixture was neutralized with 2 N NaOH solution and extracted with EtOAc (3 × 10 mL) followed by *i-*PrOH/CH₂Cl₂ (2:1, 3 × 10 mL). The combined extracts were dried (Na₂SO₄), filtered and concentrated under reduced pressure. The residue was purified by column chromatography (silica, 0-50% CMA in CH₂Cl₂) to afford the sub-title compound (515 mg, 47%).
¹H NMR (400 MHz, CD₃OD) δ 2.04-2.07 (m, 4H), 3.15-3.18 (m, 4H), 3.29-3.31 (m, 2H), 4.19 (s, 2H), 6.41 (d, *J*= 1.6 Hz, 1H), 7.32 (d, *J*= 1.6 Hz, 1H).

### (iii) Preparation of 37d: (4aS,6aS,6bR,13aR)-Benzyl 12-amino-2,2,6a,6b,9,9,13a-heptamethyl-15-(2-(pyrrolidin-1-ylmethyl)furan-3-yl)-2,3,4,4a,5,6,6a,6b,7,8,8a,9,11,13,13a,13b,14,15b-octadecahydro-1H-chryseno[1 ,2-f]indazole-4a-carboxylate

To a mixture of **II** (500 mg, 0.75 mmol) and **37c** (441 mg, 2.26 mmol) in benzene (4 mL) and EtOH (1 mL) was added K₂CO₃ (416 mg, 3.02 mmol). The mixture was sparged with nitrogen and then Pd(PPh₃)₄ (174 mg, 0.15 mmol) was added. The reaction mixture was heated at 130°C for 1 hour using microwave irradiation. The solvent was concentrated under reduced pressure. The residue was dissolved in EtOAc (40 mL) and washed with brine (3 × 15 mL). The organic layer was dried (Na₂SO₄), filtered and concentrated. The residue was purified by column chromatography (silica, 0-10% MeOH in CH₂Cl₂ followed by 30% CMA in CH₂Cl₂) to afford the sub-title compound (260 mg, 47%).
¹H NMR (400 MHz, CD₃OD) δ 0.51-2.66 (m, 50H), 3.05-3.08 (m, 1H), 3.61-3.87 (m, 3H), 5.0 (d, *J* = 12.20 Hz, 1H), 5.21 (d, *J* = 12.20 Hz, 1H), 6.23 (d, *J*= 1.56 Hz, 1H), 7.31-7.52 (m, 7H). APCI MS *m*/*z* 733 [C₄₇H₆₄N₄O₃ + H]⁺.

### (iv) Preparation of 37: (4aS,6aS,6bR,13aR)-12-Amino-2,2,6a,6b,9,9,13a-heptamethyl-15-(2-(pyrrolidin-1-ylmethyl)furan-3-yl)-2,3,4,4a,5,6,6a,6b,7,8,8a,9,11,13,13a,13b,14,15b-octadecahydro-1H-chryseno[1,2-f]indazole-4a-carboxylic acid

A solution of **37d** (260 mg, 0.35 mmol), EtOAc (5 mL) and MeOH (10 mL) was flushed with nitrogen and then 10% Pd/C (130 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-50% CMA in CH₂Cl₂) to afford the title compound (85 mg, 37%).
*R_{f}*0.24 (90:9:1 Methylene Chloride/Methanol/concentrated Ammonium Hydroxide).
¹H NMR (300 MHz, CD₃OD) δ 0.59 (s, 3H), 0.85 (s, 3H), 0.89 (s, 3H), 1.04-2.32 (m, 36H), 2.75-2.79 (m, 1 H), 3.11-3.15 (m, 2H), 3.48 (s, 2H), 4.08-4.19 (m, 2H), 6.38 (d, *J* = 1.5 Hz, 1 H), 7.60 (d, *J* = 1.5 Hz, 1H). APCI MS *m*/*z* 643 [C₄₀H₅₈N₄O₃ + H]⁺. m.p. 260-280°C dec. HPLC (Method A) 98.4 % (214 nm) *t*_{R} = 13.1 min

### Example 38

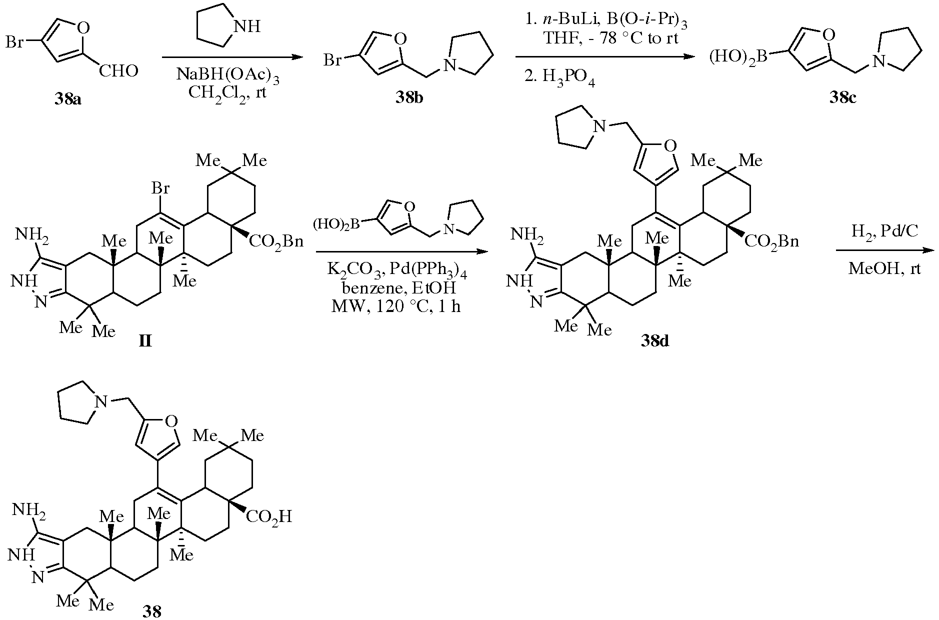

### (i) Preparation of 38b: 1-((4-Bromofuran-2-yl)methyl)pyrrolidine

To a solution of 4-bromofuran-2-carbaldehyde (500 mg, 2.85 mmol) and pyrrolidine (0.47 mL, 5.74 mmol) and CH₂Cl₂ (10 mL) was added sodium triacetoxyborohydride (1.2 g, 5.71 mmol). The mixture was stirred at room temperature overnight. The resulting mixture was diluted with EtOAc (100 mL) and the organic layer was washed with brine then dried (Na₂SO₄), filtered and concentrated. The residue was purified by column chromatography (silica, 0-5% MeOH in CH₂Cl₂) to afford the sub-title compound (400 mg, 61%).
¹H NMR (300 MHz, CDCl₃) δ 1.77-1.82 (m, 4H), 2.53-2.58 (m, 4H), 3.62 (s, 2H), 6.25 (s, 1 H), 7.35 (s, 1 H).

### (ii) Preparation of 38c: 5-(Pyrrolidin-1-ylmethyl)furan-3-ylboronic acid

To a solution of **38b** (400 mg, 1.73 mmol) in THF (10 mL) was added n-butyllithium (0.90 mL, 2.26 mmol) at -78°C. After stirring for 20 min, triisopropylborate (1.99 mL, 8.69 mmol) was added at -78°C. The mixture was slowly warmed to room temperature and then quenched with phosphoric acid. The reaction mixture was neutralized with 2 M NaOH solution and extracted with EtOAc (3 × 10 mL) followed by *i*-PrOH/CH₂Cl₂ (1:2, 2 × 10 mL). The combined extracts were dried (Na₂SO₄), filtered and concentrated under reduced pressure. The residue was purified by column chromatography (silica, 0-100% CMA in CH₂Cl₂) to afford the sub-title compound (197 mg, 58%).
¹H NMR (300 MHz, CD₃OD) δ 1.84 (d, *J* **=** 3.9 Hz, 4H), 2.74 (m, 4H), 3.81 (d, *J* = 4.2 Hz, 2H), 6.46 (d, *J* = 4.2 Hz, 1 H), 7.62 (d, *J* = 3.9 Hz, 1 H).

### (iii) Preparation of 38d: (4aS,6aS,6bR,13aR)-Benzyl 12-amino-2,2,6a,6b,9,9,13a-heptamethyl-15-(5-(pyrrolidin-1-ylmethyl)furan-3-yl)-2,3,4,4a,5,6,6a,6b,7,8,8a,9,11,13,13a,13b,14,15b-octadecahydro-1H-chryseno[1,2-f]indazole-4a-carboxylate

To a mixture of **II** (200 mg, 0.30 mmol) and **38c** (176 mg, 0.96 mmol) in benzene (4 mL) and EtOH (1 mL) was added K₂CO₃ (166 mg, 1.20 mmol). The mixture was sparged with nitrogen and then Pd(PPh₃)₄ (69 mg, 0.06 mmol) was added. The reaction mixture was heated at 120°C for 1 hour using microwave irradiation. The solvent was concentrated under reduced pressure. The residue was taken up in EtOAc (20 mL) and washed with brine. The organic layer was dried (Na₂SO₄), filtered and concentrated. The residue was purified by column chromatography (silica, 0-10% MeOH in CH₂Cl₂) to afford the sub-title compound (200 mg, 90%).
¹H NMR (300 MHz, CD₃OD) δ 0.59-3.69 (m, 52H), 5.03 (d, *J* = 12.0 Hz, 1 H), 5.21 (d, *J* = 12.3 Hz, 1 H), 6.22 (s, 1 H), 7.21 (s, 1 H), 7.58-7.67 (m, 5H). APCI MS *m*/*z* 733 [C₄₇H₆₄N₄O₃ + H]⁺.

### (iv) Preparation of 38: (4aS,6aS,6bR,13aR)-12-Amino-2,2,6a,6b,9,9,13a-heptamethyl-15-(5-(pyrrolidin-1-ylmethyl)furan-3-yl)-2,3,4,4a,5,6,6a,6b,7,8,8a,9,11,13,13a,13b,14,15b-octadecahydro-1H-chryseno[1,2-f]indazole-4a-carboxylic acid

A solution of **38d** (200 mg, 0.27 mmol) and MeOH (15 mL) was flushed with nitrogen and then 10% Pd/C (300 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-80% CMA in CH₂Cl₂) followed by preparative HPLC to afford the title compound (16 mg, 9%) as a solid.
*R_{f}*0.30 (40:9:1 Methylene Chloride/Methanol/concentrated Ammonium Hydroxide).
¹H NMR (300 MHz, CD₃OD) δ 0.62-2.19 (m, 46H), 2.41 (d, *J*= 15.0 Hz, 2H), 3.53 (m, 2H), 4.45 (s, 2H), 6.64 (s, 1 H), 7.57 (s, 1H). APCI MS *m*/*z* 643 [C₄₀H₅₈N₄O₃ + H]⁺. m.p. 220-240°C dec. HPLC (Method A) 98.6% (214 nm) *t*_{R} = 12.3 min.

### Example 39

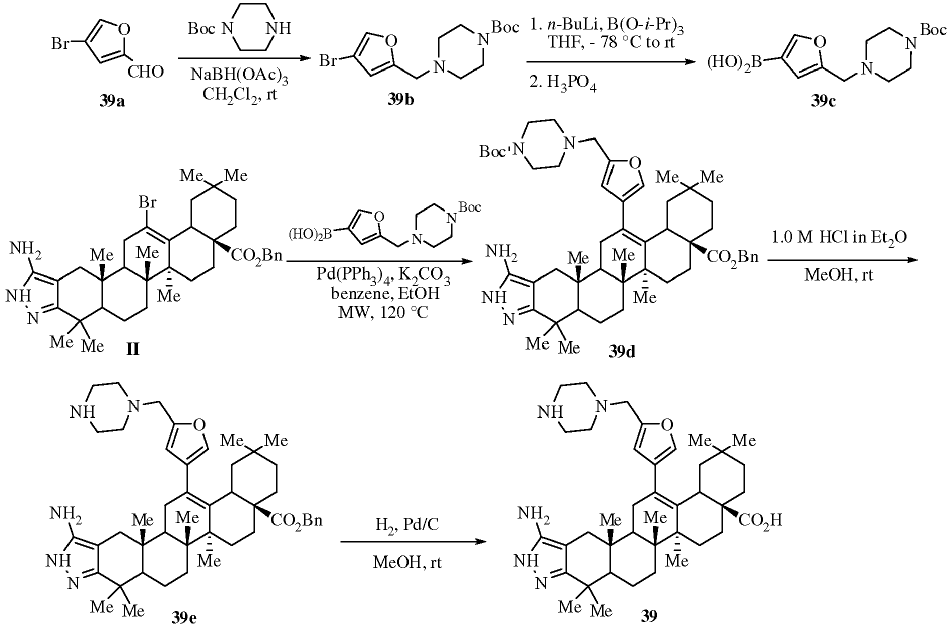

### (i) Preparation of 39b: tert-Butyl 4-((4-bromofuran-2-yl)methyl)piperazine-1-carboxylate

To a solution of 4-bromofuran-2-carbaldehyde (500 mg, 2.85 mmol) and CH₂Cl₂ (10 mL) was added *tert*-butyl piperazine-1-carboxylate (1.06 g, 5.74 mmol) and sodium triacetoxyborohydride (1.2 g, 5.74 mmol). The mixture was stirred at room temperature overnight. The resultant mixture was diluted with EtOAc (100 mL), washed with brine then dried (Na₂S0₄), filtered and concentrated under reduced pressure. The residue was purified by column chromatography (silica, 0-5% MeOH in CH₂Cl₂) to afford the sub-title compound (900 mg, 91%).
¹H NMR (300 MHz, CDCl₃) δ 1.45 (s, 9H), 2.39-2.42 (m, 4H), 3.42-3.45 (m, 4H), 3.52 (s, 2H), 6.27 (s, 1H), 7.38 (s,1H).

### (ii) Preparation of 39c: 5-((4-(tert-Butoxycarbonyl)piperazin-1-yl)methyl)furan-3-ylboronic acid

To a solution of **39b** (900 mg, 2.60 mmol) and THF (10 mL) was added n-butyllithium (1.35 mL, 3.39 mmol) at -78°C. After stirring for 10 min, triisopropylborate (2.99 mL, 13.04 mmol) was added at -78°C. The mixture was slowly warmed to room temperature and quenched with phosphoric acid. The reaction mixture was neutralized with 2 M NaOH solution and extracted with EtOAc (3 × 10 mL). The extracts were dried (Na₂SO₄), filtered and concentrated under reduced pressure. The residue was purified by column chromatography (silica, 0-5% CMA in CH₂Cl₂) to afford the sub-title compound (180 mg, 58%).
¹H NMR (300 MHz, CDCl₃) δ 1.44 (s, 9H), 3.44 (m, 4H), 3.56 (s, 2H), 3.73 (m, 4H), 6.39 (s, 1 H), 7.87 (s, 1 H).

### (iii) Preparation of 39d: (4aS,6aS,6bR,13aR)-Benzyl 12-amino-15-(5-((4-(tert-butoxycarbonyl)piperazin-1-yl)methyl)furan-3-yl)-2,2,6a,6b,9,9,13a-heptamethyl-2,3,4,4a,5,6,6a,6b,7,8,8a,9,11,13,13a,13b,14,15b-Octadecahydro-1H-chryseno[1,2-f]indazole-4a-carboxylate

To a mixture of **II** (300 mg, 0.45 mmol) and **39c** (420 mg, 1.35 mmol) in benzene (4 mL) and EtOH (1 mL) was added K₂CO₃ (249 mg, 1.80 mmol). The mixture was sparged with nitrogen and then Pd(PPh₃)₄ (105 mg, 0.09 mmol) was added. The reaction mixture was heated at 120°C for 1 hour using microwave irradiation. The solvent was removed under reduced pressure and the residue was dissolved in EtOAc (20 mL). The solution was washed with brine then dried (Na₂SO₄), filtered and concentrated. The residue was purified by column chromatography (silica, 0-10% MeOH in CH₂Cl₂) to afford the sub-title compound (260 mg, 68%).
¹H NMR (300 MHz, CDCl₃) δ 0.57-2.41 (m, 65H), 3.42-3.48 (m, 6H), 5.04 (d, *J*= 14.7 Hz, 1H), 5.14 (d, *J*= 12.3 Hz, 1H), 6.18 (s, 1H), 7.21 (s, 1H), 7.33-7.35 (m, 5H). APCI MS *m*/*z* 848 [C₅₂H₇₃N₅O₅ + H]⁺.

### (iv) Preparation of 39e: (4aS,6aS,6bR,13aR)-Benzyl 12-amino-2,2,6a,6b,9,9,13a-heptamethyl-15-(5-(piperazin-1-ylmethyl)furan-3-yl)-2,3,4,4a,5,6,6a,6b,7,8,8a,9,11,13,13a,13b,14,15b-octadecahydro-1H-chryseno[1,2-f]indazole-4a-carboxylate

To a solution of **39d** (260 mg, 0.30 mmol) and MeOH (2 mL) was added HCl (1.0 M in diethyl ether, 10 mL, 10.0 mmol). After stirring overnight at room temperature, the reaction mixture was concentrated. Purification of the residue by column chromatography (silica, 0-80% CMA in CH₂Cl₂) afforded the sub-title compound (190 mg, 83%).
¹H NMR (300 MHz, CD₃OD) δ 0.57-2.37 (m, 42H), 2.44 (s, 4H), 2.80 (s, 4H), 3.43 (s, 2H), 4.98-5.03 (m, 1H), 5.15-5.21 (m, 1H), 6.20 (s, 1H), 7.21 (s, 1H), 7.36-7.37 (m, 5H). APCI MS *m*/*z* 748 [C₄₇H₆₅N₅O₃ + H]⁺.

### (v) Preparation of 39: (4aS,6aS,6bR,13aR)-12-Amino-2,2,6a,6b,9,9,13a-heptamethyl-15-(5-(piperazin-1-ylmethyl)furan-3-yl)-2,3,4,4a,5,6,6a,6b,7,8,8a,9,11,13,13a,13b,14,15b-octadecahydro-1H-chryseno[1,2-f]indazole-4a-carboxylic acid

A solution of **39e** (190 mg, 0.25 mmol) and MeOH (10 mL) was flushed with nitrogen and then 10% Pd/C (190 mg) was added. The mixture was flushed with nitrogen followed by hydrogen. The mixture was stirred under hydrogen at atmospheric pressure overnight. The catalyst was removed by filtration through diatomaceous earth and the filtrate was concentrated under reduced pressure. The residue was purified by column chromatography (silica, 0-70% CMA in CH₂Cl₂) followed by preparative HPLC to afford the title compound (34 mg, 20%) as a solid.
*R_{f}*0.10 (32:17:1 Methylene Chloride/Methanol/concentrated Ammonium Hydroxide).
¹H NMR (300 MHz, CD₃OD) δ 0.60-2.44 (m, 42H), 3.14 (s, 4H), 3.39 (s, 4H), 4.06 (s, 2H), 6.64 (s, 1H), 7.48 (s,1H).
APCI MS *m*/*z* 658 [C₄₀H₅₉N₅O₃ + H]⁺. m.p. 220-240°C dec. HPLC (Method A) >99% (214 nm) *t*_{R} = 13.7 min.

### Example 40

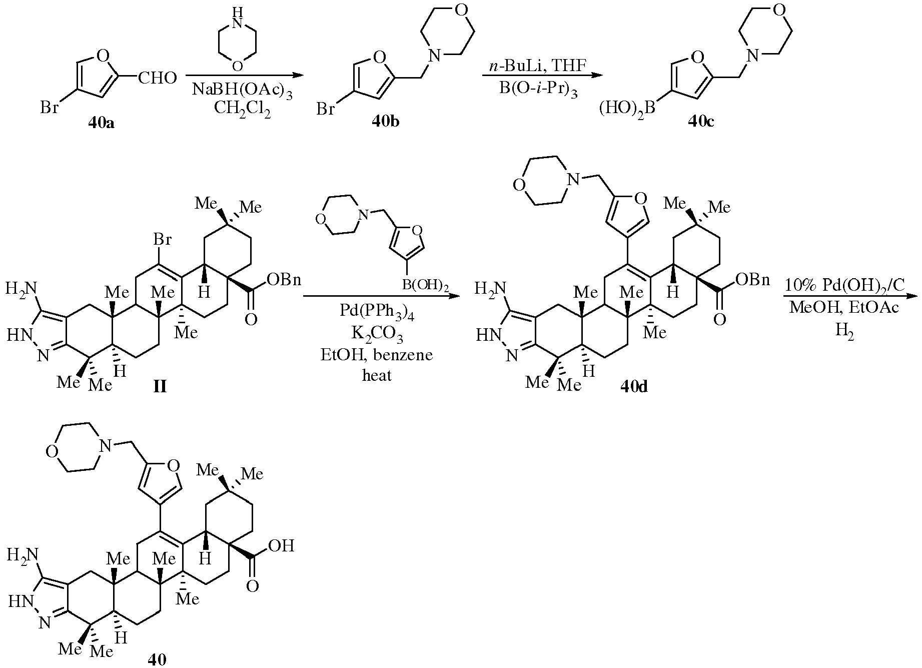

### (i) Preparation of 40b: 4-((4-Bromofuran-2-yl)methyl)morpholine

To a solution of 4-bromofuran-2-carbaldehyde (500 mg, 2.85 mmol) and morpholine (0.5 mL, 5.71 mmol) in CH₂Cl₂ (10 mL) was added sodium triacetoxyborohydride (1.2 g, 5.71 mmol). The mixture was stirred at room temperature for 12 hours. The reaction mixture was diluted with EtOAc (100 mL). The organic phase was washed with aqueous NaHCO₃ and brine then dried (MgSO₄), filtered and concentrated to dryness. The residue was purified by column chromatography (silica, 0-10% MeOH in CH₂Cl₂) to afford the sub-title compound (600 mg, 86%).
¹H NMR (300 MHz, CDCl₃) δ 2.42 (m, 4H), 3.62 (s, 2H), 3.72 (m, 4H), 6.27 (s, 1H), 7.32 (s, 1 H).

### (ii) Preparation of 40c: 5-(Morpholinomethyl)furan-3-ylboronic acid

To a solution of **40b** (300 mg, 1.20 mmol) in THF (6 mL) was added n-butyllithium (2.5 M in hexanes, 0.64 mL, 1.60 mmol) at -78°C. The mixture was stirred at -78°C for 20 min. Triisopropyl borate (1.3 mL, 6.0 mmol) was added. The reaction mixture was stirred at -78°C for 1 hour and quenched with HCl (2 M, 4 mL). The reaction mixture was stirred for 5 min and neutralized by NaOH (2 M) and extracted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (120 mg, 47%).
¹H NMR (300 MHz, CDCl₃) δ 2.40 (s, 4H), 3.65 (s, 2H), 3.68 (m, 4H), 6.58 (s, 1H), 7.95 (s, 1 H).

### (iii) Preparation of 40d: (4aS,6aS,6bR,8aR,13aR,15bS)-Benzyl 12-amino-2,2,6a,6b,9,9,13a-heptamethyl-15-(5-(morpholinomethyl)furan-3-yl)-2,3,4,4a,5,6,6a,6b,7,8,8a,9,11,13,13a,13b,14,15b-octadecahydro-1H-chryseno[1,2-f]indazole-4a-carboxylate

A mixture of **II** (100 mg, 0.18 mmol), **40c** (120 mg, 0.56 mmol), Pd(PPh₃)₄ (20 mg, 0.018 mmol) and K₂CO₃ (100 mg, 0.72 mmol) in benzene (3.5 mL) and EtOH (1.5 mL) was sealed and heated to 120°C by microwave for 1 hour. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (120 mg, 89%).
APCI MS (Positive Mode) *m*/*z* 749 [C₄₇H₆₄N₄O₄ + H]⁺.

### (iv) Preparation of 40: (4aS,6aS,6bR,8aR,13aR,15bS)-12-Amino-2,2,6a,6b,9,9,13a-heptamethyl-15-(5-(morpholinomethyl)furan-3-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **40d** (120 mg, 0.16 mmol) and 10% Pd(OH)₂/C (40 mg) in MeOH (13 mL) and EtOAc (2 mL) was stirred under a hydrogen balloon for 6 hours. The reaction mixture was filtered through a pad of diatomaceous earth and washed with CMA (25 mL). The filtrate was concentrated to dryness under reduced pressure. The residue was purified by column chromatography (silica, 0-60% CMA in CH₂Cl₂) followed by preparative HPLC to provide the title compound (25 mg, 24%) as an off-white solid.
*R_{f}* 0.25 (80:18:2 Methylene Chloride/Methanol/concentrated Ammonium Hydroxide).
¹H NMR (300 MHz, CD₃OD) δ 0.60 (s, 3H), 0.84 (s, 3H), 0.89 (s, 3H), 0.94 (s, 3H), 1.22 (s, 3H), 1.25 (s, 3H), 1.30 (s, 3H), 1.35-2.28 (m, 20H), 2.38 (d, *J*= 15.0 Hz, 1H), 3.85 (m, 3H), 4.44 (s, 2H), 6.86 (s, 1 H), 7.60 (s, 1 H). mp >300 °C. APCI MS (Positive Mode) *m*/*z* 659 [C₄₀H₅₈N₄O₄ + H]⁺.

### Example 41

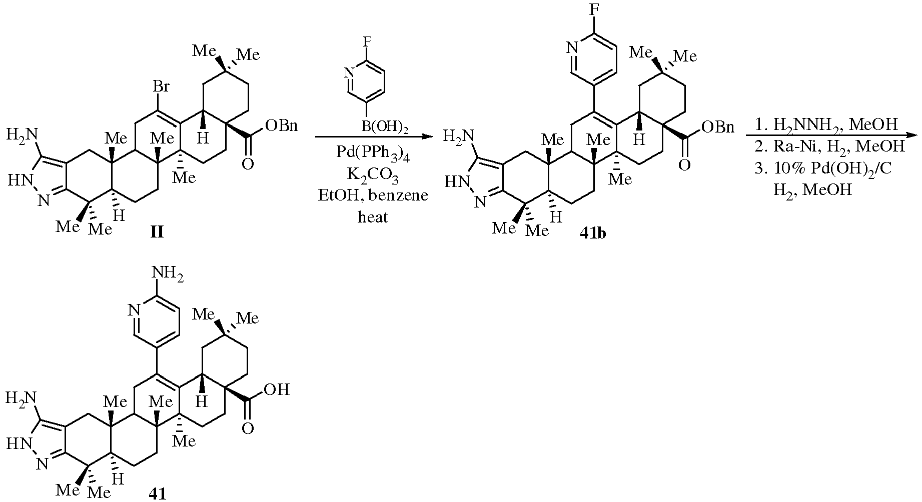

### (i) Preparation of 41b: (4aS,6aS,6bR,8aR,13aR,15bS)-Benzyl 12-amino-15-(6-fluoropyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **II** (400 mg, 2.85 mmol), 6-fluoropyridin-3-ylboronic acid (400 mg, 2.85mmol), Pd(PPh₃)₄ (105 mg, 0.090 mmol) and K₂CO₃ (600 mg, 4.34 mmol) in benzene (4.0 mL) and EtOH (1.0 mL) was sealed and heated to 120 °C by microwave for 1 hour. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (500 mg, 81%) as a brown solid.
APCI MS (Positive Mode) *m*/*z* 679 [C₄₃H₅₅FN₄O₂ + H]⁺.

### (ii) Preparation of 41: (4aS,6aS,6bR,8aR,13aR,15bS)-12-Amino-15-(6-aminopyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **41b** (100 mg, 0.14 mmol) and hydrazine (0.5 mL) in MeOH (4 mL) was sealed and heated to 140°C by microwave for 5 hours. The mixture was concentrated to dryness. The residue was dissolved in MeOH (20 mL), transferred to a Parr hydrogenation flask and Raney Ni (70 mg) was added. The mixture was placed on a Parr shaker under 40 psi of hydrogen for 12 hours. The mixture was filtered through a pad of diatomaceous earth and the filter cake washed with CMA. The filtrate was concentrated to dryness and the residue dissolved in MeOH (10 mL), transferred to a Parr hydrogenation flask and 10% Pd(OH)₂/C (70 mg) was added and the mixture placed on a Parr Shaker under 30 psi of hydrogen for 12 hours. The reaction mixture was filtered through a pad of diatomaceous earth and washed with CMA (25 mL). The filtrate was concentrated to dryness under reduced pressure. The residue was purified by column chromatography (silica, 0-60% CMA in CH₂Cl₂) to afford the title compound (8 mg, 7%) as a brown solid.
*R_{f}* 0.30 (80:18:2 Methylene Chloride/Methanol/concentrated Ammonium Hydroxide).
¹H NMR (300 MHz, CD₃OD) δ 0.50 (s, 3H), 0.82 (s, 3H), 0.90 (s, 3H), 0.98 (s, 3H), 1.22 (s, 3H), 1.31 (s, 6H), 1.35-2.30 (m, 21 H), 6.61 (d, *J*= 8.1 Hz, 1 H), 7.47 (d, *J* = 8.1 Hz, 1 H), 7.80 (s, 1 H). mp >300°C dec. APCI MS (Positive Mode) *m*/*z* 586 [C₃gH₅₁N₅O₂ + H]⁺.

### Example 42

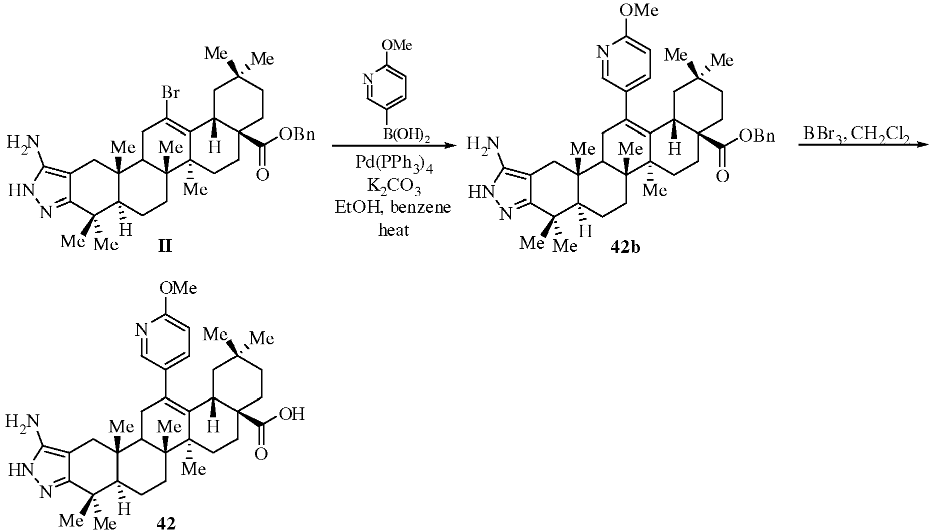

### (i) Preparation of 42b: (4aS,6aS,6bR,8aR,13aR,15bS)-Benzyl 12-amino-15-(6-methoxypyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **II** (200 mg, 0.30 mmol), 6-methoxypyridin-3-ylboronic acid (138 mg, 0.90 mmol), Pd(PPh₃)₄ (34 mg, 0.030 mmol) and K₂CO₃ (208 mg, 1.50 mmol) in benzene (4.0 mL) and EtOH (1.0 mL) was sealed and heated to 120°C by microwave for 1 hour. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (200 mg, 96%) as a brown solid.
APCI MS (Positive Mode) *m*/*z* 691 [C₄₄H₅₈N₄O₃ + H]⁺.

### (ii) Preparation of 42: (4aS,6aS,6bR,8aR,13aR,15bS)-12-Amino-15-(6-methoxypyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

To a solution of **42b** (107 mg, 0.15 mmol) in CH₂Cl₂ (2 mL) was added boron tribromide (1 M in THF, 0.93 mL, 0.93 mmol) at -78°C. The mixture was warmed to 0°C for 6 hours. The reaction mixture was quenched with MeOH (2 mL) and concentrated to dryness under reduced pressure. The residue was purified by column chromatography (silica, 0-4% CMA in CH₂Cl₂) to afford the title compound (34 mg, 38%).
*R_{f}0.75* (80:18:2 Methylene Chloride/Methanol/concentrated Ammonium Hydroxide).
¹H NMR (300 MHz, CD₃OD) δ 0.32 (s, 3H), 0.78 (s, 3H), 0.88 (s, 3H), 1.0 (s, 3H), 1.16 (s, 3H), 1.25 (s, 3H), 1.28 (s, 3H), 1.30-2.25 (m, 19H), 2.32 (d, *J* = 14.7 Hz, 1H), 3.04 (m, 1 H), 3.89 (s, 3H), 6.78 (d, *J* = 8.7 Hz, 1 H), 7.62 (d, *J* = 10.2, 1H), 8.02 (s,1H). mp >300°C. APCI MS (Positive Mode) *m*/*z* 601 [C₃₇H₅₂N₄O₃ + H]⁺.

### Example 43

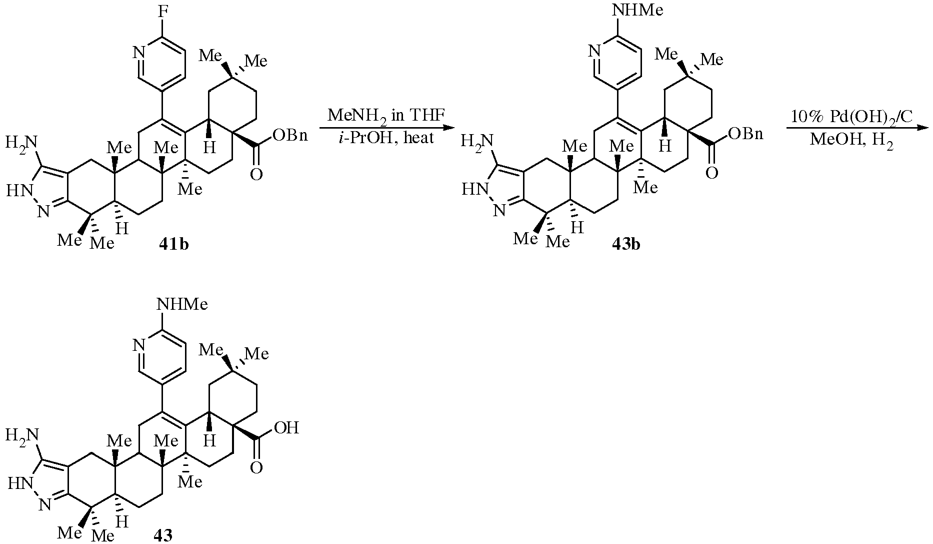

### (i) Preparation of 43b: (4aS,6aS,6bR,8aR,13aR,15bS)-Benzyl 12-amino-2,2,6a,6b,9,9,13a-heptamethyl-15-(6-(methylamino)pyridin-3-yl)-2,3,4,4a,5,6,6a,6b,7,8,8a,9,11,13,13a,13b,14,15b-octadecahydro-1H-chryseno[1,2-f]indazole-4a-carboxylate

A mixture of **41b** (100 mg, 0.14 mmol) and methylamine (1.5 mL, 1 M in THF, 1.5 mmol) in *i*-PrOH (2 mL) was sealed and heated to 160°C by microwave for 5 hours. The mixture was concentrated to dryness. The residue was purified by column chromatography (silica, 0-30% CMA in CH₂Cl₂) to afford the sub-title compound (41 mg, 42%) as a brown solid.
APCI MS (Positive Mode) *m*/*z* 690 [C₄₄H₅₉N₅O₂ + H]⁺.

### (ii) Preparation of 43: (4aS,6aS,6bR,8aR,13aR,15bS)-12-Amino-2,2,6a,6b,9,9,13a-heptamethyl-15-(6-(methylamino)pyridin-3-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **43b** (32 mg, 0.046 mmol) and 10% Pd(OH)₂/C (32 mg) in MeOH (20 mL) was stirred under a hydrogen balloon for 5 hours. The reaction mixture was filtered through a pad of diatomaceous earth and washed with CMA (25 mL). The filtrate was concentrated to dryness under reduced pressure. The residue was purified by column chromatography (silica, 0-70% CMA in CH₂Cl₂) followed by preparative HPLC to provide the title compound (10 mg, 37%) as an off-white solid.
*R_{f}* 0.60 (80:18:2 Methylene Chloride/Methanol/concentrated Ammonium Hydroxide).
¹H NMR (300 MHz, CD₃OD) δ 0.50 (s, 3H), 0.82 (s, 3H), 0.90 (s, 3H), 0.98 (s, 3H), 1.22 (s, 3H), 1.28 (s, 3H), 1.31 (s, 3H), 1.35-2.30 (m, 21 H), 3.02 (m, 1H), 3.03 (s, 3H), 7.05 (d, *J* = 9.3 Hz, 1 H), 7.83 (s, 1 H), 7.90 (d, *J* = 8.4 Hz, 1H). mp >300°C. APCI MS (Positive Mode) *m*/*z* 600 [C₃₇H₅₃N₅O₂ + H]⁺.

### Example 44

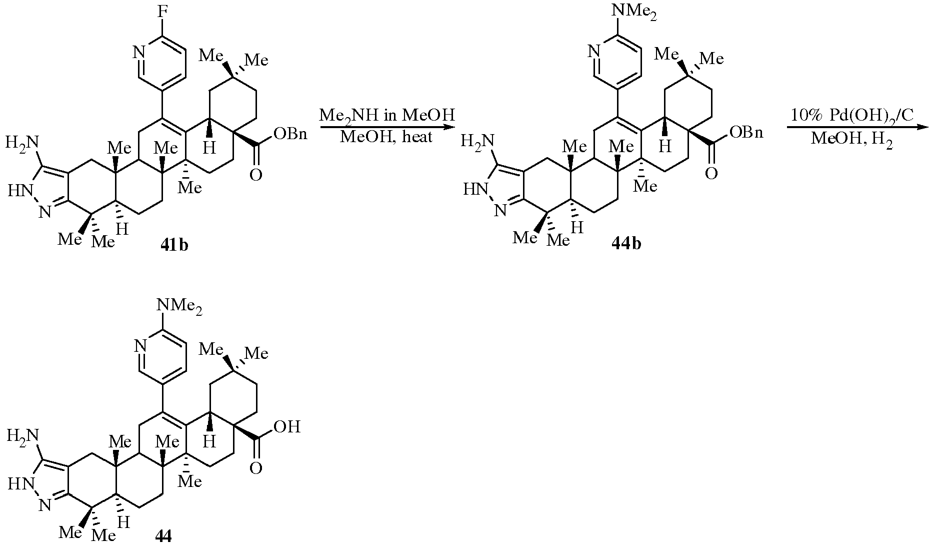

### (i) Preparation of 44b: (4aS,6aS,6bR,8aR,13aR,15bS)-Benzyl 12-amino-15-(6-(dimethylamino)pyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of **41** b (150 mg, 0.22 mmol) and dimethylamine (2.0 mL, 1 M in MeOH, 2.0 mmol) in MeOH (1 mL) was sealed and heated to 140°C by microwave for 7 hours. The mixture was concentrated to dryness. The residue was purified by column chromatography (silica, 0-10% MeOH in CH₂Cl₂) to afford the sub-title compound (88 mg, 58%) as a brown solid.
APCI MS (Positive Mode) *m*/*z* 690 [C₄₅H₆₁N₅O₂ + H]⁺.

### (ii) Preparation of 44: (4aS,6aS,6bR,8aR,13aR,15bS)-12-Amino-15-(6-(dimethylamino)pyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A mixture of **44b** (88 mg, 0.12 mmol) and 10% Pd(OH)₂/C (40 mg) in MeOH (15 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-40% CMA in CH₂Cl₂) to afford the title compound (35 mg, 26%) as an off-white solid.
*R_{f}* 0.75 (80:18:2 Methylene Chloride/Methanol/concentrated Ammonium Hydroxide).
¹H NMR (300 MHz, CD₃OD) δ 0.38 (s, 3H), 0.78 (s, 3H), 0.87 (s, 3H), 0.99 (s, 3H), 1.15 (s, 3H), 1.25 (s, 3H), 1.26 (s, 3H), 1.35-2.30 (m, 19H), 2.33 (d, *J*= 14.4 Hz, 1H), 3.06 (s, 6H), 3.07 (m, 1H), 6.66 (d, *J* = 8.7 Hz, 1 H), 7.50 (d, *J* = 8.7 Hz, 1 H), 7.94 (s, 1 H).
mp >300°C dec. APCI MS (Positive Mode) *m*/*z* 614 [C₃₈H₅₅N₅O₂ + H]⁺.

### Example 45

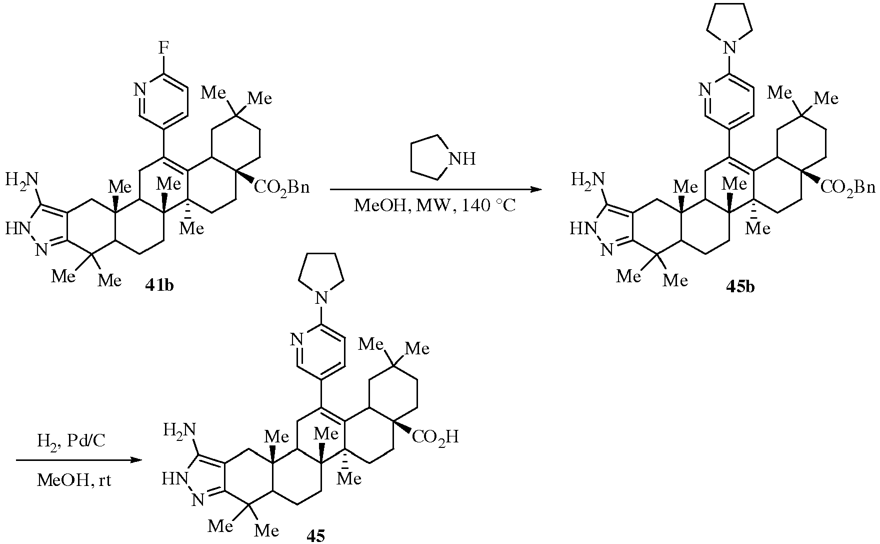

### (i) Preparation of 45b: (4aS,6aS,6bR,13aR)-Benzyl 12-amino-2,2,6a,6b,9,9,13a-heptamethyl-15-(6-(pyrrolidin-1-yl)pyridin-3-yl)-2,3,4,4a,5,6,6a,6b,7,8,8a,9,11,13,13a,13b,14,15b-octadecahydro-1H-chryseno[1,2-f]indazole-4a-carboxylate

To a solution of **41b** (150 mg, 0.22 mmol) in MeOH (4 mL) was added pyrrolidine (1 mL, 12.09 mmol). The mixture was heated at 140°C for 5 hours using microwave irradiation. The solvent was removed under reduced pressure and the residue was dissolved in EtOAc (20 mL). The solution was washed with brine then dried (Na₂SO₄), filtered and concentrated to afford the sub-title compound (162 mg, 100%) which was used without further purification.
APCI MS *m*/*z* 730 [C₄₇H₆₃N₅O₂ + H]⁺,

### (ii) Preparation of 45: (4aS,6aS,6bR,13aR)-12-Amino-2,2,6a,6b,9,9,13a-heptamethyl-15-(6-(pyrrolidin-1-yl)pyridin-3-yl)-2,3,4,4a,5,6,6a,6b,7,8,8a,9,11,13,13a,13b,14,15b-octadecahydro-1H-chryseno[1,2-f]indazole-4a-carboxylic acid

A solution of **45b** (162 mg, 0.22 mmol) and MeOH (10 mL) was flushed with nitrogen and then 10% Pd/C (200 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-40% CMA in CH₂Cl₂) to afford the title compound (52 mg, 37%).
*R_{f}* 0.48 (43:6:1 Methylene Chloride/Methanol/concentrated Ammonium Hydroxide).
¹H NMR (300 MHz, CD₃OD) δ 0.39 (s, 3H), 0.78 (s, 3H), 0.87-2.27 (m, 38H), 2.38 (d, *J* = 14.6 Hz, 1 H), 3.08 (m, 1 H), 3.43 (m, 4H), 6.50 (d, *J* = 8.7 Hz, 1 H), 7.51 (dd, *J* = 2.1 Hz, 2.9 Hz,1 H), 7.90 (s, 1H). m.p. 280-300°C dec. APCI MS *m*/*z* 640 [C₄₀H₅₇N₅O₂ + H]⁺.
HPLC (Method A) >99% (214 nm) t_{R} = 12.2 min.

### Example 46

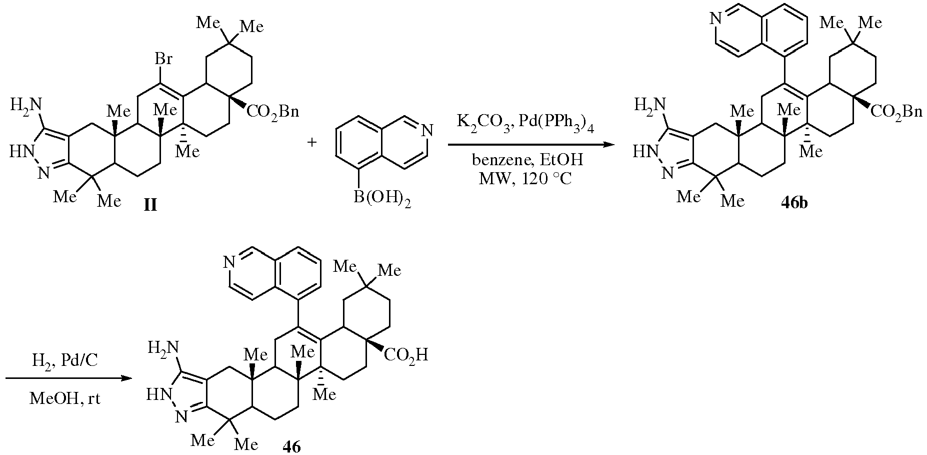

### (i) Preparation of 46b: (4aS,6aS,6bR,13aR)-Benzyl 12-amino-15-(isoquinolin-5-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a mixture of II (200 mg, 0.30 mmol) and isoquinolin-5-ylboronic acid (156 mg, 0.90 mmol) in benzene (4 mL) and EtOH (1 mL) was added K₂CO₃ (166 mg, 1.20 mmol). The mixture was sparged with nitrogen and then Pd(PPh₃)₄ (70 mg, 0.06 mmol) was added. The reaction mixture was heated at 120°C for 1 hour using microwave irradiation and then concentrated. The residue was dissolved in EtOAc (20 mL) and washed with brine. The organic solution was dried (Na₂SO₄), filtered and concentrated under reduced pressure. The residue was purified by column chromatography (silica, 0-10% MeOH in CH₂Cl₂) to afford the sub-title compound (180 mg, 84%).
APCI MS *m*/*z* 711 [C₄₇H₅₈N₄O₂ + H]⁺.

### (ii) Preparation of 46: (4aS,6aS,6bR,13aR)-12-Amino-15-(isoquinolin-5-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **46b** (180 mg, 0.25 mmol) and MeOH (15 mL) was flushed with nitrogen and then 10% Pd/C (220 mg) was added. The mixture was flushed with nitrogen followed by hydrogen. The mixture was stirred under hydrogen at atmospheric pressure overnight. The catalyst was removed by filtration through diatomaceous earth and the filtrate was concentrated under reduced pressure. The residue was purified by column chromatography (silica, 0-80% CMA in CH₂Cl₂) followed by preparative HPLC to afford the title compound (13 mg, 8%) as a solid.
*R_{f}0.73* (40:9:1 Methylene Chloride/Methanol/concentrated Ammonium Hydroxide).
¹H NMR (300 MHz, CD₃OD) δ -0.17-2.33 (m, 41 H), 2.95 (d, *J* = 11.1 Hz, 1H), 7.94-7.99 (m, 1 H), 8.13 (d, *J* = 6.3 Hz, 1 H), 8.19 (d, *J* = 7.2 Hz, 1 H), 8.32 (d, *J* = 8.4 Hz, 1 H), 8.54 (d, *J* = 6.3 Hz, 1 H), 9.61 (s, 1 H). APCI MS *m*/*z* 621 [C₄₀H₅₂N₄O₂ + H]⁺. m.p. >300°C. HPLC (Method A) 93.4% (214 nm) *t*_{R} = 11.6 min.

### Example 47

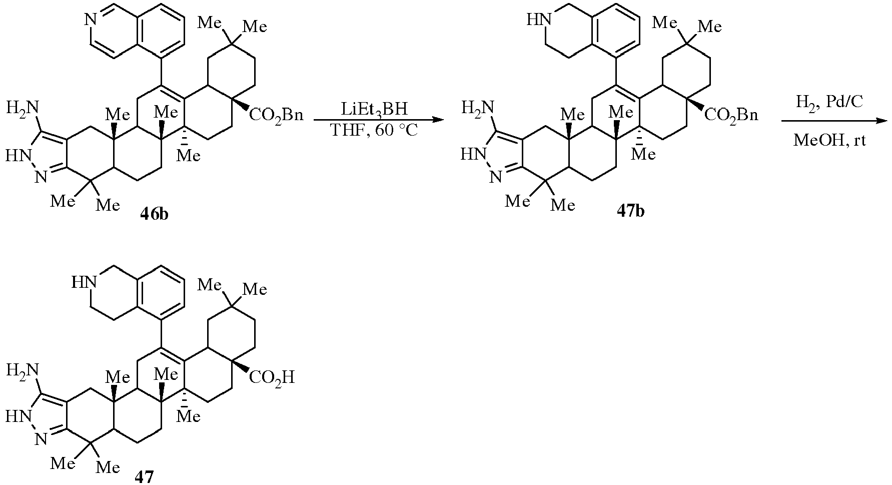

### (i) Preparation of 47b: (4aS,6aS,6bR,13aR)-Benzyl 12-amino-2,2,6a,6b,9,9,13a-heptamethyl-15-(1,2,3,4-tetrahydroisoquinolin-5-yl)-2,3,4,4a,5,6,6a,6b,7,8,8a,9,11,13,13a,13b,14,15b-octadecahydro-1H-chryseno[1,2-f]indazole-4a-carboxylate

To a solution of **46b** (150 mg, 0.21 mmol) and THF (5 mL) was added lithium triethylborohydride (1.26 mL, 1.26 mmol, 1.0 M in THF). The mixture was heated at 60°C overnight and then cooled to room temperature. The mixture was taken up in EtOAc (40 mL) and washed with brine (3 x 15 mL). The organic layer was dried (Na₂SO₄), filtered and concentrated. The residue was purified by column chromatography (silica, 0-80% CMA in CH₂Cl₂) to afford the sub-title compound (40 mg, 26%).
APCI MS *m*/*z* 715 [C₄₇H₆₂N₄O₂ + H]⁺.

### (ii) Preparation of 47: (4aS,6aS,6bR,13aR)-12-Amino-2,2,6a,6b,9,9,13a-heptamethyl-15-(1,2,3,4-tetrahydroisoquinolin-5-yl)-2,3,4,4a,5,6,6a,6b,7,8,8a,9,11,13,13a,13b,14,15b-octadecahydro-1H-chryseno[1,2-f]indazole-4a-carboxylic acid

A solution of **47b** (40 mg, 0.055 mmol) and MeOH (10 mL) was flushed with nitrogen and then 10% Pd/C (50 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-80% CMA in CH₂Cl₂) to afford the title compound (19 mg, 56%).
*R_{f}* 0.28 (40:9:1 Methylene Chloride/Methanol/concentrated Ammonium Hydroxide).
¹H NMR (400 MHz, CD₃OD) δ 0.24 (s, 3H), 0.75-2.36 (m, 38H), 2.86-3.44 (m, 5H), 4.10-4.20 (m, 2H), 6.98 (d, *J* = 7.42 Hz, 1H), 7.19 (d, *J* = 7.42 Hz, 1H), 7.40 (d, *J*= 7.42 Hz, 1H). APCI MS *m*/*z* 625 [C₄₀H₅₆N₄O₂ + H]⁺. m.p. >300°C dec. HPLC (Method A) 99.1 % (214 nm) *t*_{R} = 12.4 min

### Example 48

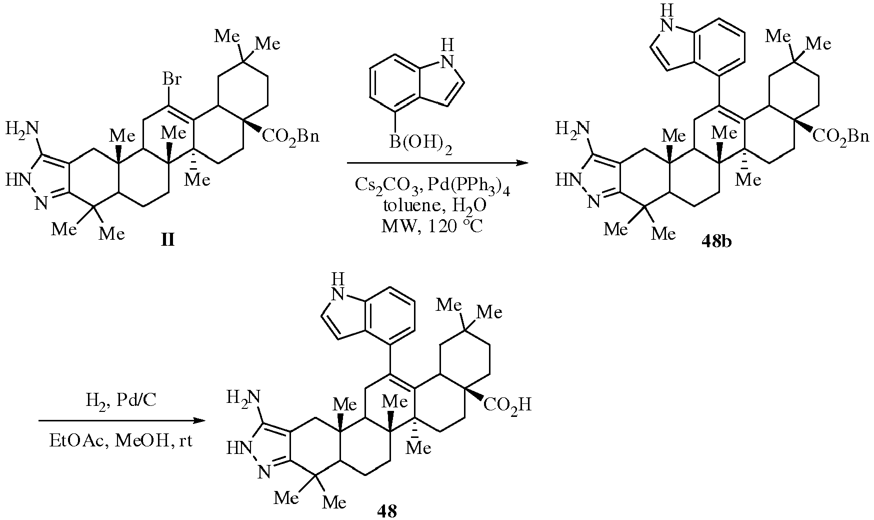

### (i) Preparation of 48b: (4aS,6aS,6bR,13aR)-Benzyl 12-amino-15-(1H-indol-4-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a mixture of **II** (200 mg, 0.302 mmol) and 1*H*-indol-4-ylboronic acid (146 mg, 0.906 mmol) in toluene (4.5 mL) and H₂O (0.5 mL) was added cesium carbonate (393 mg, 1.208 mmol). The mixture was sparged with nitrogen and then Pd(PPh₃)₄ (70 mg, 0.060 mmol) was added. The reaction mixture was heated at 120°C for 1 hour using microwave irradiation. The solvent was concentrated under reduced pressure. The residue was taken up in EtOAc (20 mL) and washed with brine (3 × 15 mL). The organic layer was dried (Na₂SO₄), filtered and concentrated. The residue was purified by column chromatography (silica, 0-10% MeOH in CH₂Cl₂) to afford the sub-title compound (180 mg, 85%).
APCI MS *m*/*z* 699 [C₄₆H₅₈N₄O₂ + H]⁺.

### (ii) Preparation of 48: (4aS,6aS,6bR,13aR)-12-Amino-15-(1H-indol-4-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of 48b (180 mg, 0.26 mmol), EtOAc (2 mL) and MeOH (8 mL) was flushed with nitrogen and then 10% Pd/C (280 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-10% MeOH in CH₂Cl₂) followed by preparative HPLC to afford the title compound (29 mg, 18%) as a solid.
*R_{f}* 0.30 (90:9:1 Methylene Chloride/Methanol/concentrated Ammonium Hydroxide).
¹H NMR (400 MHz, CD₃OD) δ -0.08 (s, 3H), 0.22 (s, 3H), 0.54 (s, 3H), 0.78-2.56 (m, 32H), 6.28 (s, 1 H), 7.01-7.24 (m, 5H). APCI MS *m*/*z* 609 [C₃₉H₅₂N₄O₂ + H]⁺. m.p. 280-300°C dec. HPLC (Method A) 99.3 % (214 nm) *t*_{R} = 16.6 min

### Example 49

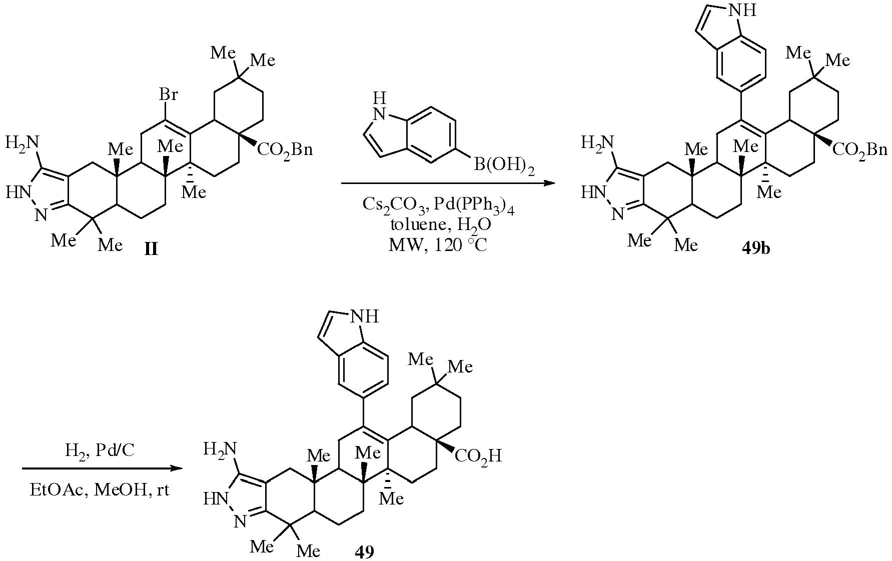

### (i) Preparation of 49b: (4aS,6aS,6bR,13aR)-Benzyl 12-amino-15-(1H-indol-5-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a mixture of **II** (200 mg, 0.302 mmol) and 1*H*-indol-5-ylboronic acid (146 mg, 0.91 mmol) in toluene (4.5 mL) and H₂O (0.5 mL) was added cesium carbonate (393 mg, 1.21 mmol). The mixture was sparged with nitrogen and then Pd(PPh₃)₄ (70 mg, 0.06 mmol) was added. The reaction mixture was heated at 120°C for 1 hour using microwave irradiation. The solvent was concentrated under reduced pressure. The residue was taken up in EtOAc (20 mL) and washed with brine (3 × 15 mL). The organic layer was dried (Na₂SO₄), filtered and concentrated. The residue was purified by column chromatography (silica, 0-10% CMA in CH₂Cl₂) to afford the sub-title compound (178 mg, 84%).
APCI MS *m*/*z* 699 [C₄₆H₅₈N₄O₂ + H]⁺.

### (ii) Preparation of 49: (4aS,6aS,6bR,13aR)-12-Amino-15-(1H-indol-5-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **49b** (175 mg, 0.25 mmol), EtOAc (5 mL) and MeOH (10 mL) was flushed with nitrogen and then 10% Pd/C (175 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-6% MeOH in CH₂Cl₂) followed by preparative HPLC to afford the title compound (18 mg, 12%) as a solid.
*R_{f}* 0.30 (90:9:1 Methylene Chloride/Methanol/concentrated Ammonium Hydroxide).
¹H NMR (400 MHz, CD₃OD) δ 0.10 (s, 3H), 0.70 (s, 3H), 0.89-2.43 (m, 35H), 3.21-3.25 (m, 1 H), 6.38-6.39 (m, 1 H), 7.04 (s, 1 H), 7.18 (s, 1 H), 7.32 (d, *J* = 8.26 Hz, 1 H), 7.39 (s, 1 H). APCI MS *m*/*z* 609 [C₃₉H₅₂N₄O₂ + H]⁺. m.p. 230-250°C dec. HPLC (Method A) 96.2 % (214 nm) *t*_{R} = 17.1 min

### Example 50

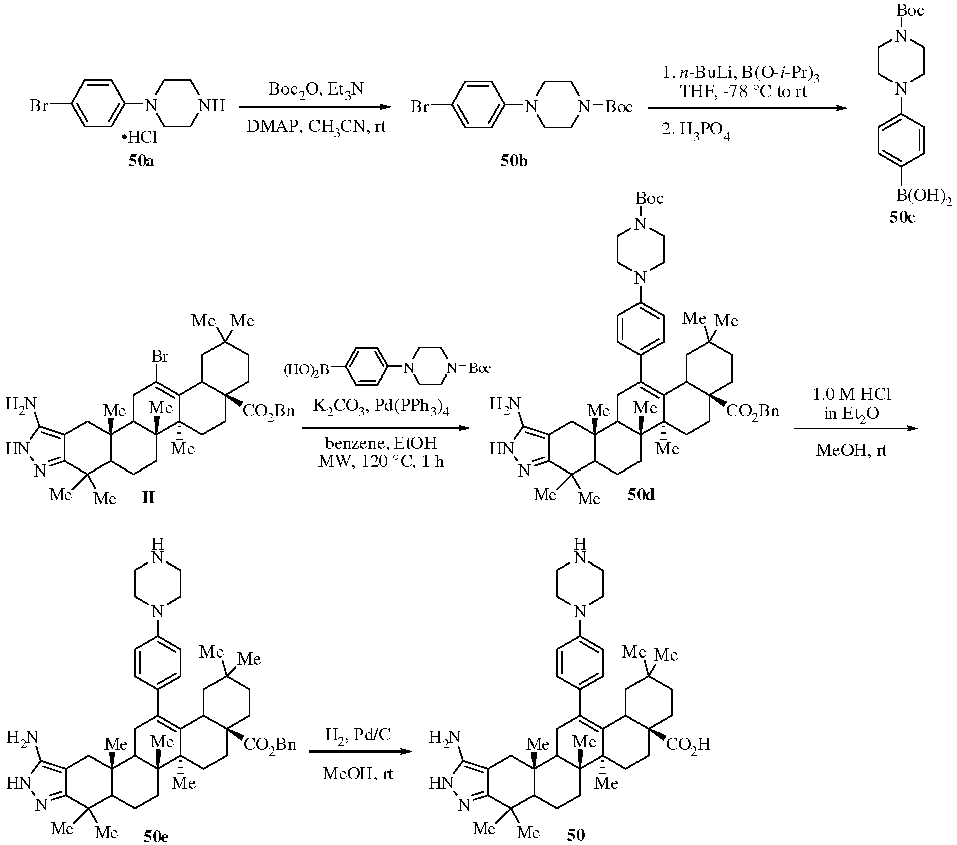

### (i) Preparation of 50b: tert-Butyl 4-(4-bromophenyl)piperazine-1-carboxylate

To a slurry of 1-(4-bromophenyl)piperazine hydrochloride (3.0 g, 10.8 mmol), DMAP (135 mg, 1.1 mmol) and triethylamine (4.5 mL, 32.4 mmol) in CH₃CN (25 mL) was added di-tert-butyl dicarbonate (2.6 g, 11.9 mmol). The mixture was stirred at room temperature overnight. Water (25 mL) was added to the resultant mixture and stirred for 30 min. The resultant solid was collected by filtration. The solid was washed with H₂O and dried in a vacuum oven at 40°C to afford the sub-title compound (3.2 g, 87%).
¹H NMR ((300 MHz, DMSO-d₆) δ 1.48 (s, 9H), 3.15 (m, 4H), 3.54 (m, 4H), 6.95 (d, *J* = 9.0 Hz, 2H), 7.37 (d, *J*= 9.0 Hz, 2H).

### (ii) Preparation of 50c: 4-(4-(tert-Butoxycarbonyl)piperazin-1-yl)phenylboronic acid

To a solution of **50b** (500 mg, 1.46 mmol) and THF (10 mL) was added n-butyllithium (0.76 mL of 2.5 M in hexanes, 1.90 mmol) at -78°C. After stirring for 30 min, triisopropylborate (1.68 mL, 7.33 mmol) was added at -78°C. The mixture was slowly warmed to room temperature and quenched with phosphoric acid. The reaction mixture was neutralized with saturated NaHCO₃ and extracted with EtOAc (10 mL × 3). The extracts were dried (Na₂SO₄), filtered and concentrated under reduced pressure. The residue was purified by column chromatography (silica, 0-10% MeOH in CH₂Cl₂) to afford the sub-title compound (180 mg, 58%).
*¹*H NMR (300 MHz, CD₃OD) δ -0.50 (s, 9H), 0.93-0.96 (m, 4H), 1.15-1.19 (m, 4H), 4.86-4.89 (m, 4H).

### (iii) Preparation of 50d: (4aS,6aS,6bR,13aR)-Benzyl 12-amino-15-(4-(4-(tert-butoxycarbonyl)piperazin-1-yl)phenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a mixture of II (130 mg, 0.19 mmol) and 50c (180 mg, 0.58 mmol) in benzene (4 mL) and EtOH (1 mL) was added K₂CO₃ (108 mg, 0.78 mmol). The mixture was sparged with nitrogen and then Pd(PPh₃)₄ (45 mg, 0.06 mmol) was added. The reaction mixture was heated at 120°C for 1 hour using microwave irradiation and then concentrated under reduced pressure. The residue was dissolved in EtOAc (20 mL) and the solution was washed with brine then dried (Na₂SO₄), filtered and concentrated. The residue was purified by column chromatography (silica, 0-5% MeOH in CH₂Cl₂) to afford the sub-title compound (100 mg, 60%).
¹H NMR (300 MHz, CD₃OD) δ 0.28-3.59 (m, 59H), 5.18 (d, 2H), 6.70-7.07 (m, 9H). APCI MS *m*/*z* 844 [C₅₃H₇₃N₅O₄ + H]⁺.

### (iv) Preparation of 50e: (4aS,6aS,6bR,13aR)-Benzyl 12-amino-2,2,6a,6b,9,9,13a-heptamethyl-15-(4-(piperazin-1-yl)phenyl)-2,3,4,4a,5,6,6a,6b,7,8,8a,9,11,13,13a,13b,14,15b-octadecahydro-1H-chryseno[1,2-f]indazole-4a-carboxylate

To a solution of **50d** (220 mg, 0.26 mmol) in MeOH (3 mL) was added HCl (1.0 M in diethyl ether, 6 mL, 6.0 mmol). The mixture was stirred at room temperature for 62 hours and then concentrated under reduced pressure. The residue was purified by column chromatography (silica, 0-50% CMA in CH₂Cl₂) to afford the sub-title compound (65 mg, 33%).
¹H NMR (300 MHz, CD₃OD) δ 0.27-2.34 (m, 42H), 2.98-3.06 (m, 8H), 5.02 (d, *J*= 12.3 Hz, 1H), 5.22 (d, *J*= 12.0 Hz, 1H), 6.78 (d, *J*= 8.7 Hz, 2H), 6.99 (d, *J*= 8.7 Hz, 2H), 7.39 (m, 5H). APCI MS *m*/*z* 744 [C₄₈H₆₅N₅O₂ + H]⁺.

### (v) Preparation of 50: (4aS,6aS,6bR,13aR)-12-Amino-2,2,6a,6b,9,9,13a-heptamethyl-15-(4-(piperazin-1-yl)phenyl)-2,3,4,4a,5,6,6a,6b,7,8,8a,9,11,13,13a,13b,14,15b-octadecahydro-1H-chryseno[1,2-f]indazole-4a-carboxylic acid

A solution of **50e** (65 mg, 0.087 mmol) and MeOH (15 mL) was flushed with nitrogen and then 10% Pd/C (90 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-70% CMA in CH₂Cl₂) followed by preparative HPLC to afford the title compound (11 mg, 20%) as a solid.
*R_{f}* 0.10 (40:9:1 Methylene Chloride/Methanol/concentrated Ammonium Hydroxide).
¹H NMR (300 MHz, CD₃OD) δ 0.28-2.42 (m, 41 H), 3.10 (d, *J*= 10.2 Hz, 1H), 3.37 (m, 8H), 7.03 (d, *J* = 9.0 Hz, 2H), 7.24 (d, *J* = 7.8 Hz, 2H). APCI MS *m*/*z* 654 [C₄₁H₅₉N₅O₂ + H]⁺ m.p. 270-290°C dec. HPLC (Method A) >99% (214 nm) *t*_{R} = 13.2 min.

### Example 51

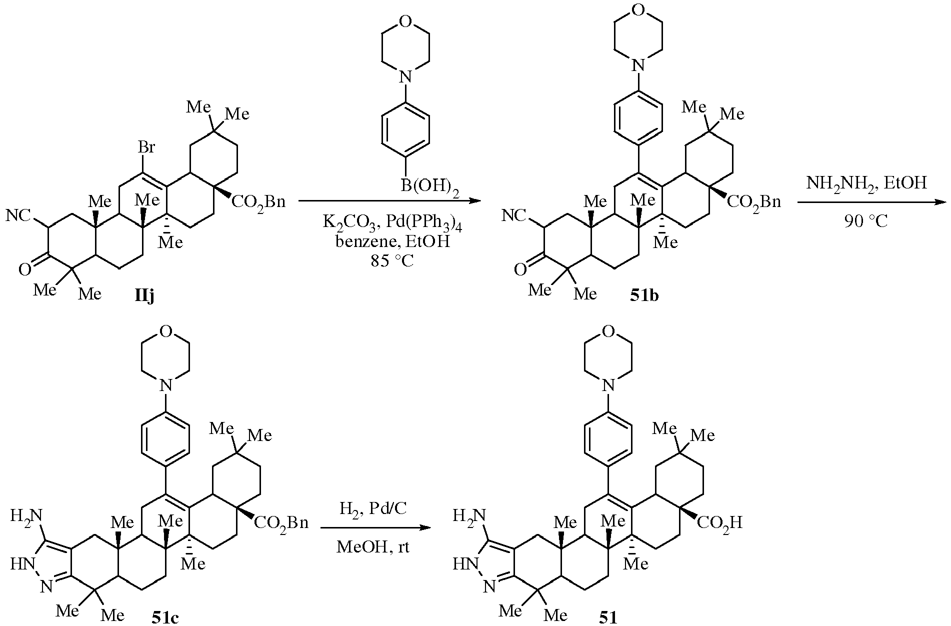

### (i) Preparation of 51b: (4aS,6aS,6bR,12aR)-Benzyl 11-cyano-2,2,6a,6b,9,9,12a-heptamethyl-14-(4-morpholinophenyl)-10-oxo-1,2,3,4,4a,5,6,6a,6b,7,8,8a,9,10,11,12,12a,12b,13,14b-icosahydropicene-4a-carboxylate

To a mixture of IIj (300 mg, 0.46 mmol) and 4-morpholinophenylboronic acid (575 mg, 2.78 mmol) in benzene (10 mL) and EtOH (5 mL) was added K₂CO₃ (511 mg, 3.70 mmol). The mixture was sparged with nitrogen and then Pd(PPh₃)₄ (107 mg, 0.09 mmol) was added. The mixture was heated at 85°C overnight and concentrated under reduced pressure. The residue was dissolved in EtOAc (50 mL) and the solution was washed with brine then dried (Na₂SO₄), filtered and concentrated. The residue was purified by column chromatography (silica, 0-30% EtOAc in hexanes) to afford the sub-title compound (200 mg, 59%).
¹H NMR (300 MHz, CDCl₃) δ 0.28-2.29 (m, 43H), 3.09-3.10 (m, 4H), 3.85-3.86 (m, 4H), 5.05 (m, 1H), 5.17 (m, 1H), 6.75-6.78 (m, 2H), 7.02-7.06 (m, 2H), 7.25-7.36 (m, 5H).

### (ii) Preparation of 51c: (4aS,6aS,6bR,13aR)-Benzyl 12-amino-2,2,6a,6b,9,9,13a-heptamethyl-15-(4-morpholinophenyl)-2,3,4,4a,5,6,6a,6b,7,8,8a,9,11,13,13a,13b,14,15b-octadecahydro-1H-chryseno[1,2-f]indazole-4a-carboxylate

To a solution of 51 b (196 mg, 0.26 mmol) and EtOH (5 mL) was added hydrazine (42 µL, 1.34 mmol). The mixture was heated at 90°C for 60 hours and then concentrated under reduced pressure. The residue was purified by column chromatography (silica, 0-20% CMA in CH₂Cl₂) to provide the sub-title compound (109 mg, 55%).
¹H NMR (300 MHz, CDCl₃) δ 0.30-2.24 (m, 43H), 3.07-3.12 (m, 4H), 3.40-3.47 (m, 2H), 3.85-3.88 (m, 4H), 5.10 (d, *J* = 12.3 Hz, 1 H), 5.22 (d, *J* = 12.6 Hz, 1 H), 6.78 (d, *J* = 8.7 Hz, 1 H), 7.06 (d, *J* = 8.4 Hz, 1 H), 7.36-7.39 (m, 5H). APCI MS m/z 745 [C₄₈H₆₄N₄O₃ + H]⁺.

### (iii) Preparation of 51: (4aS,6aS,6bR,13aR)-12-Amino-2,2,6a,6b,9,9,13a-heptamethyl-15-(4-morpholinophenyl)-2,3,4,4a,5,6,6a,6b,7,8,8a,9,11,13,13a,13b,14,15b-octadecahydro-1H-chryseno[1,2-f]indazole-4a-carboxylic acid

A solution of 51 c (108 mg, 0.14 mmol) and MeOH (15 mL) was flushed with nitrogen and then 10% Pd/C (55 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-10% MeOH in CH₂Cl₂) to afford the title compound (26 mg, 27%).
*R_{f}* 0.35 (90:9:1 Methylene Chloride/Methanol/concentrated Ammonium Hydroxide).
¹H NMR (300 MHz, CD₃OD) δ 0.30-2.37 (m, 42H), 3.10 (m, 4H), 3.84 (m, 4H), 6.95 (d, *J* = 7.8 Hz, 2H), 7.16 (d, *J* = 7.5 Hz, 2H). APCI MS *m*/*z* 655 [C₄₁H₅₈N₄O₃ + H]⁺ m.p. 250-270°C dec. HPLC (Method A) >99% (214 nm) *t*_{R} = 13.3 min.

### Example 52

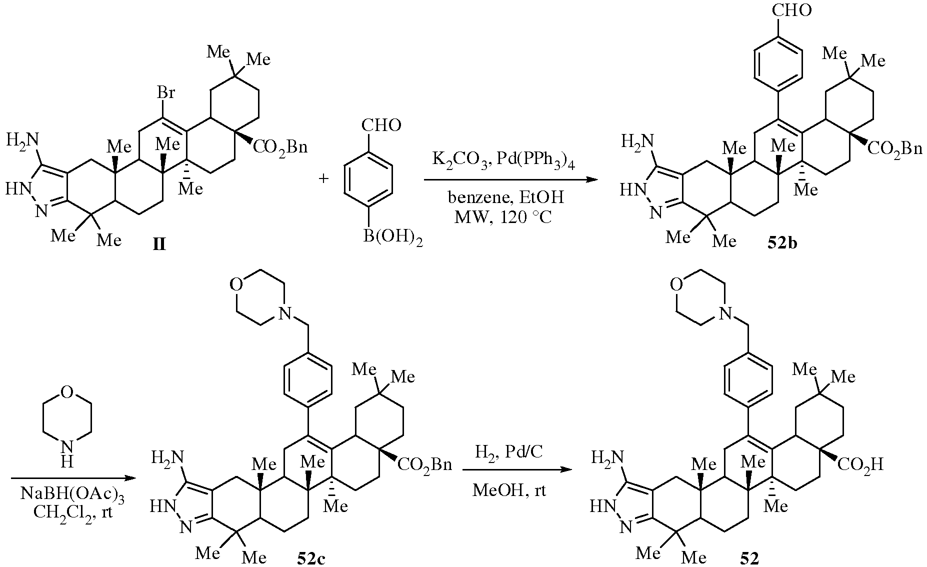

### (i) Preparation of 52b: (4aS,6aS,6bR,13aR)-Benzyl 12-amino-15-(4-formylphenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a solution of II (250 mg, 0.37 mmol), 4-formylphenylboronic acid (170 mg, 1.13 mmol), benzene (4 mL) and EtOH (1 mL) was added K₂CO₃ (208 mg, 1.50 mmol). The mixture was sparged with nitrogen and then Pd(PPh₃)₄ (87 mg, 0.07 mmol) was added. The reaction mixture was heated at 120°C for 1 hour using microwave irradiation and then concentrated under reduced pressure. The residue was dissolved in EtOAc (20 mL) and the solution was washed with brine. The organic layer was dried (Na₂SO₄), filtered and concentrated. The residue was purified by column chromatography (silica, 0-10% MeOH in CH₂Cl₂) to afford the sub-title compound (224 mg, 86%).
APCI MS *m*/*z* 688 [C₄₅H₅₇N₃O₃ + H]⁺.

### (ii) Preparation of 52c: (4aS,6aS,6bR,13aR)-Benzyl 12-amino-2,2,6a,6b,9,9,13a-heptamethyl-15-(4-(morpholinomethyl)phenyl)-2,3,4,4a,5,6,6a,6b,7,8,8a,9,11,13,13a,13b,14,15b-octadecahydro-1H-chryseno[1,2-f]indazole-4a-carboxylate

To a solution of **52b** (220 mg, 0.32 mmol), morpholine (56 µL, 0.64 mmol) and CH₂Cl₂ (10 mL) was added sodium triacetoxyborohydride (136 mg, 0.64 mmol). The mixture was stirred at room temperature overnight. The resultant mixture was diluted with EtOAc (100 mL) and the solution was washed with brine then dried (Na₂SO₄), filtered and concentrated under reduced pressure. The residue was purified by column chromatography (silica, 0-20% MeOH in CH₂Cl₂) to afford the sub-title compound (80 mg, 33%).
¹H NMR (300 MHz, CD₃OD) δ 0.28-2.24 (m, 41 H), 2.39-2.42 (m, 4H), 3.03 (m, 1 H), 3.45 (s, 2H), 3.69-3.70 (m, 4H), 5.03 (d, *J* = 12.6 Hz, 1H), 5.23 (d, *J* = 12.3 Hz, 1H), 7.15-7.18 (m, 4H), 7.37 (s, 5H).

### (iii) Preparation of 52: (4aS,6aS,6bR,13aR)-12-Amino-2,2,6a,6b,9,9,13a-heptamethyl-15-(4-(morpholinomethyl)phenyl)-2,3,4,4a,5,6,6a,6b,7,8,8a,9,11,13,13a,13b,14,15b-octadecahydro-1H-chryseno[1,2-f]indazole-4a-carboxylic acid

A solution of **52c** (75 mg, 0.09 mmol) and MeOH (10 mL) was flushed with nitrogen and then 10% Pd/C (100 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by preparative HPLC to afford the title compound (8 mg, 12%).
*R_{f}0.75* (40:9:1 Methylene Chloride/Methanol/concentrated Ammonium Hydroxide).
¹H NMR (300 MHz, CD₃OD) δ 0.25-2.39 (m, 45H), 2.99 (m, 1H), 3.73 (s, 2H), 4.02 (s, 2H), 4.38 (s, 2H), 7.47-7.52 (m, 4H). APCI MS *m*/*z* 669 [C₄₂H₆₀N₄O₃ + H]⁺. m.p. >300°C dec. HPLC (Method A) >99% (214 nm) *t*_{R} = 12.1 min.

### Example 53

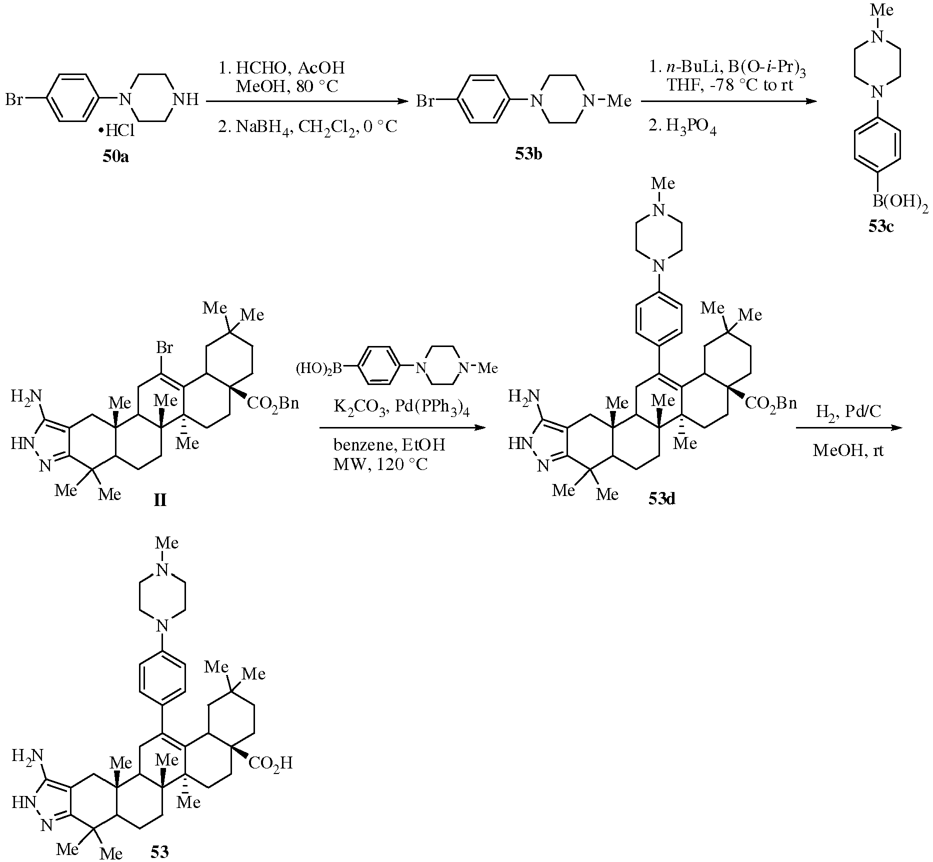

### (i) Preparation of 53b: 1-(4-Bromophenyl)-4-methylpiperazine

A mixture of 1-(4-bromophenyl)piperazine hydrochloride (1.0 g, 3.61 mmol), formaldehyde (37% aqueous, 3 mL, 39.71 mmol), AcOH (0.23 mL, 3.97 mmol) and MeOH (30 mL) was heated at 80°C under nitrogen. After 1.5 hours, the mixture was cooled to 0°C in an ice bath. Methylene chloride (5 mL) was added followed by a slow addition of sodium borohydride (1.91 g, 50.54 mmol) under nitrogen. The mixture was stirred for 1 hour and then poured into a seperatory funnel containing a saturated solution of NH₄Cl (25 mL) and CH₂Cl₂ (50 mL). The layers were separated and the aqueous layer was extracted with CH₂Cl₂ (2 × 50 mL). The extracts were dried (Na₂SO₄), filtered and concentrated to to give the sub-title compound (900 mg, 97%) which was used without further purification.
¹H NMR ((300 MHz, CDCl₃) δ 2.34 (s, 3H), 2.55 (m, 4H), 3.17 (m, 4H), 6.79 (d, *J*= 9.0 Hz, 2H), 7.32 (d, *J*= 9.0 Hz, 2H).

### (ii) Preparation of 53c: 4-(4-Methylpiperazin-1-yl)phenylboronic acid

To a solution of **53b** (390 mg, 1.52 mmol) and THF (10 mL) was added n-butyllithium (0.79 mL of 2.5 M in hexanes, 1.98 mmol) at -78°C and stirred for 10 min. Triisopropylborate (1.75 mL, 7.64 mmol) was added at-78°C. The mixture was slowly warmed to room temperature and then quenched with phosphoric acid. The reaction mixture was neutralized with saturated NaHCO₃ solution and extracted with EtOAc (3 × 10 mL). The solution was dried (Na₂SO₄), filtered and concentrated under reduced pressure. The residue was purified by column chromatography (silica, 0-10% MeOH in CH₂Cl₂) to afford the sub-title compound (180 mg, 58%).
¹H NMR ((300 MHz, CD₃OD) δ 2.32 (s, 3H), 2.55 (m, 4H), 3.03 (s, 1H), 3.21 (m, 3H), 6.69-7.59 (m, 4H).

### (iii) Preparation of 53d: (4aS,6aS,6bR,13aR)-Benzyl 12-amino-2,2,6a,6b,9,9,13a-heptamethyl-15-(4-(4-methyl pi perazi n-1-yl) phenyl)-2,3,4,4a,5,6,6a,6b,7,8,8a,9,11,13,13a,13b,14,15b-octadecahydro-1H-chryseno[1,2-f]indazole-4a-carboxylate

To a mixture of II (160 mg, 0.24 mmol) and **53c** (159 mg, 0.72 mmol) in benzene (4 mL) and EtOH (1 mL) was added K₂CO₃ (133 mg, 0.96 mmol). The mixture was sparged with nitrogen and then Pd(PPh₃)₄ (55 mg, 0.04 mmol) was added. The reaction mixture was heated at 120°C for 1 hour using microwave irradiation and then concentrated. The residue was dissolved in EtOAc (20 mL) and the solution was washed with brine then dried (Na₂SO₄), filtered and concentrated under reduced pressure. The residue was purified by column chromatography (silica, 0-10% MeOH in CH₂Cl₂) to afford the sub-title compound (80 mg, 44%).
¹H NMR (300 MHz, CDCl₃) δ 0.29-3.46 (m, 53H), 5.06 (d, *J* = 12.6 Hz, 1 H), 5.22 (d, *J* = 12.6 Hz, 1 H), 6.80 (d, *J* = 8.4 Hz, 2H), 7.05 (d, *J* = 8.7 Hz, 2H), 7.35-7.44 (m, 5H). APCI MS *m*/*z* 758 [C₄₉H₆₇N₅O₂ + H]⁺.

### (iv) Preparation of 53: (4aS,6aS,6bR,13aR)-12-Amino-2,2,6a,6b,9,9,13a-heptamethyl-15-(4-(4-methyl pi perazi n-1-yl)phenyl)-2,3,4,4a,5,6,6a,6b,7,8,8a,9,11,13,13a,13b,14,15b-octadecahydro-1H-chryseno[1,2-f]indazole-4a-carboxylic acid

A solution of **53d** (75 mg, 0.09 mmol) and MeOH (10 mL) was flushed with nitrogen and then 10% Pd/C (70 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-40% CMA in CH₂Cl₂) followed by preparative HPLC to afford the title compound (6 mg, 5%) as a solid.
*R_{f}0.37* (40:9:1 Methylene Chloride/Methanol/concentrated Ammonium Hydroxide).
¹H NMR (300 MHz, CD₃OD) δ 0.28-2.42 (m, 34H), 2.97 (s, 3H), 3.12-3.77 (m, 8H), 3.37 (s, 8H), 7.02 (d, *J* = 8.7 Hz, 2H), 7.24 (d, *J* = 8.1 Hz, 2H). APCI MS *m*/*z* 668 [C₄₂H₆₁N₅O₂ + H]⁺. m.p. >300 °C dec. HPLC (Method A) >99% (214 nm) *t*_{R} = 13.9 min.

### Example 54

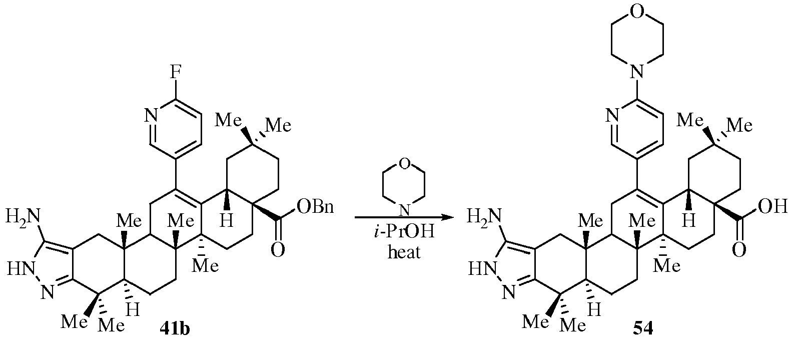

### (i) Preparation of 54: (4aS,6aS,6bR,8aR,13aR,15bS)-12-Amino-2,2,6a,6b,9,9,13a-heptamethyl-15-(6-morpholinopyridin-3-yl)-2,3,4,4a,5,6,6a,6b,7,8,8a,9,11,13,13a,13b,14,15b-octadecahydro-1H-chryseno[1,2-f]indazole-4a-carboxylic acid

A mixture of **41b** (100 mg, 0.14 mmol) and morpholine (0.5 mL) in *i*-PrOH (2 mL) was sealed and heated to 160°C by microwave for 2 hours. The mixture was concentrated to dryness. The residue was purified by column chromatography (silica, 0-30% CMA in CH₂Cl₂) followed by preparative HPLC to afford the sub-title compound (10 mg, 11%) as an off-white solid.
*R_{f}0.75* (80:18:2 Methylene Chloride/Methanol/concentrated Ammonium Hydroxide).
¹H NMR (300 MHz, CD₃OD) δ 0.46 (s, 3H), 0.82 (s, 3H), 0.90 (s, 3H), 0.99 (s, 3H), 1.22 (s, 3H), 1.25 (s, 3H), 1.29 (s, 3H), 1.35-2.30 (m, 19H), 2.37 (d, *J* = 14.4 Hz, 1H), 3.01 (s, 1 H), 3.62 (m, 4H), 3.84 (m, 4H), 7.34 (d, *J* = 9.6 Hz, 1 H), 8.00 (s, 2H). mp >300°C. APCI MS (Positive Mode) *m*/*z* 656 [C₄₀H₅₇N₅O₃ + H]⁺.

### Example 55

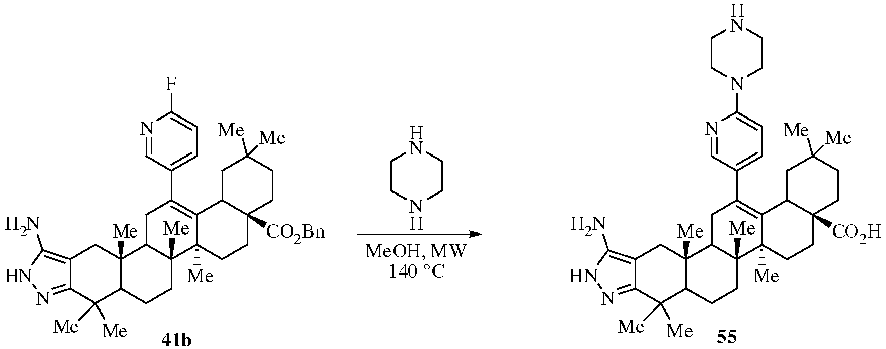

### (i) Preparation of 55: (4aS,6aS,6bR,13aR)-12-Amino-2,2,6a,6b,9,9,13a-heptamethyl-15-(6-(piperazin-1-yl)pyridin-3-yl)-2,3,4,4a,5,6,6a,6b,7,8,8a,9,11,13,13a,13b,14,15b-octadecahydro-1H-chryseno[1,2-f]indazole-4a-carboxylic acid

To **41b** (150 mg, 0.22 mmol) in MeOH (5 mL) was added piperazine (1.38 g, 20.89 mmol). The reaction mixture was heated at 140°C for 5 hours and then at 150°C for an additional 5 hours using microwave irradiation. The solvent was concentrated and then the residue was dissolved in EtOAc/i-PrOH/CH₂Cl₂ (1:1:1, 30 mL). The solution was washed with brine, dried (Na₂SO₄), filtered and concentrated. The residue was purified by column chromatography (silica, 0-40% CMA in CH₂Cl₂) followed by preparative HPLC to afford the title compound (15 mg, 10%) as a solid.
*R_{f}* 0.10 (40:9:1 Methylene Chloride/Methanol/concentrated Ammonium Hydroxide). ¹H NMR (300 MHz, CD₃OD) δ 0.40-2.43 (m, 41 H), 2.98 (m, 1 H), 3.39-3.41 (m, 4H), 3.85-3.87 (m, 4H), 7.23 (d, *J* = 9.0 Hz, 1 H), 7.89 (d, *J* = 3.4 Hz, 1 H), 8.12 (s, 1 H). APCI MS *m*/*z* 655 [C₄₀H₅₈N₆O₂ + H]⁺ m.p. 240-260 °dec. HPLC (Method A) 96.2% (214 nm) t_{R} = 10.7 min.

### Example 56

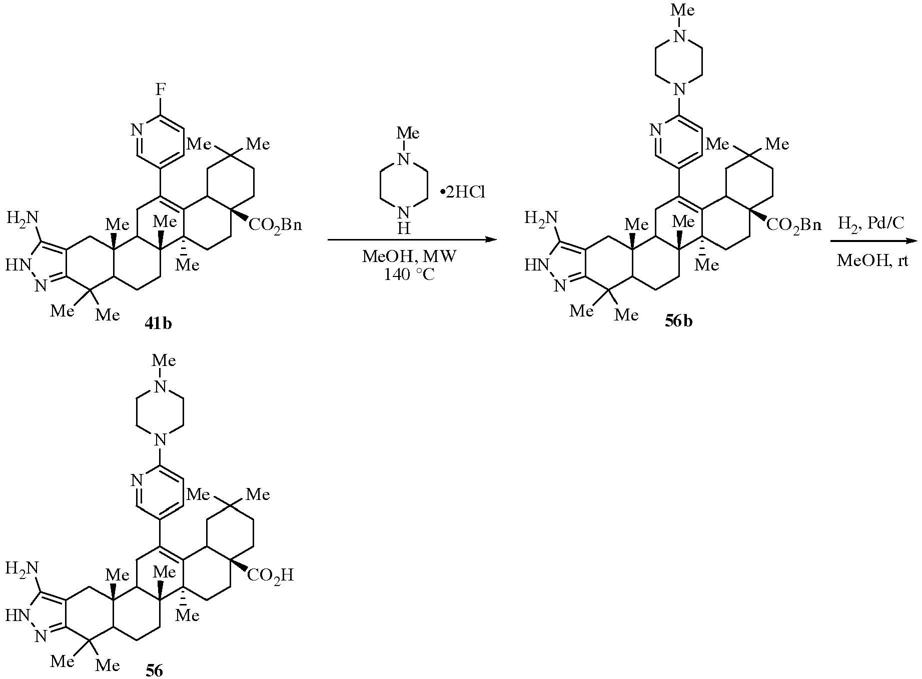

### (i) Preparation of 56b: (4aS,6aS,6bR,13aR)-Benzyl 12-amino-2,2,6a,6b,9,9,13a-heptamethyl-15-(6-(4-methylpiperazin-1-yl)pyridin-3-yl)-2,3,4,4a,5,6,6a,6b,7,8,8a,9,11,13,13a,13b,14,15b-octadecahydro-1H-chryseno[1,2-f]indazole-4a-carboxylate

To a solution of **41b** (210 mg, 0.30 mmol) in MeOH (4 mL) was added 1-methylpiperazine dihydrochloride (1.5 g, 8.67 mmol). The reaction mixture was heated at 140°C for 10 hours using microwave irradiation and then concentrated. The residue was dissolved in EtOAc (20 mL) and the solution was washed with brine then dried (Na₂SO₄), filtered and concentrated under reduced pressure. The residue was purified by column chromatography (silica, 0-30% CMA in CH₂Cl₂) to afford the sub-title compound (90 mg, 38%).
¹H NMR (300 MHz, CD₃OD) δ 0.34-2.16 (m, 43H), 2.34 (s, 4H), 2.56 (s, 4H), 3.01 (m, 1H), 3.49 (s, 4H), 5.03 (d, *J* = 12.1 Hz, 2H), 5.20 (d, *J* = 12.0 Hz, 2H), 6.65 (d, *J* = 8.4 Hz, 1 H), 6.65 (d, *J* = 4.8 Hz, 1 H), 7.36-7.38 (m, 6H), 7.98 (s, 1 H).
APCI MS *m*/*z* 759 [C₄₈H₆₆N₆O₂ + H]⁺.

### (ii) Preparation of 56: (4aS,6aS,6bR,13aR)-12-Amino-2,2,6a,6b,9,9,13a-heptamethyl-15-(6-(4-methylpiperazin-1-yl)pyridin-3-yl)-2,3,4,4a,5,6,6a,6b,7,8,8a,9,11,13,13a,13b,14,15b-octadecahydro-1H-chryseno[1,2-f]indazole-4a-carboxylic acid

A solution of **56b** (70 mg, 0.09 mmol) and MeOH (10 mL) was flushed with nitrogen and then 10% Pd/C (70 mg) was added. The mixture was flushed with nitrogen followed by hydrogen. The mixture was stirred under hydrogen at atmospheric pressure overnight. The catalyst was removed by filtration through diatomaceous earth and the filtrate was concentrated under reduced pressure. The residue was purified by column chromatography (silica, 0-60% CMA in CH₂Cl₂) to afford the title compound (42 mg, 69%).
R_{f}0.25 (40:9:1 Methylene Chloride/Methanol/concentrated Ammonium Hydroxide).
¹H NMR (300 MHz, CD₃OD) δ 0.35-2.24 (m, 40H), 2.37 (s, 3H), 2.62 (s, 4H), 3.07 (m, 1 H), 3.53 (s, 4H), 4.53 (s, 1 H), 6.86 (d, *J* = 8.4 Hz, 1 H), 7.75 (d, *J* = 8.1 Hz, 1 H), 8.03 (s, 1H). APCI MS *m*/*z* 669 [C₄₁H₆₀N₆O₂ + H]⁺. m.p. 280-300 °C dec. HPLC (Method A) >99% (214 nm) *t*_{R} = 10.3 min.

### Example 57

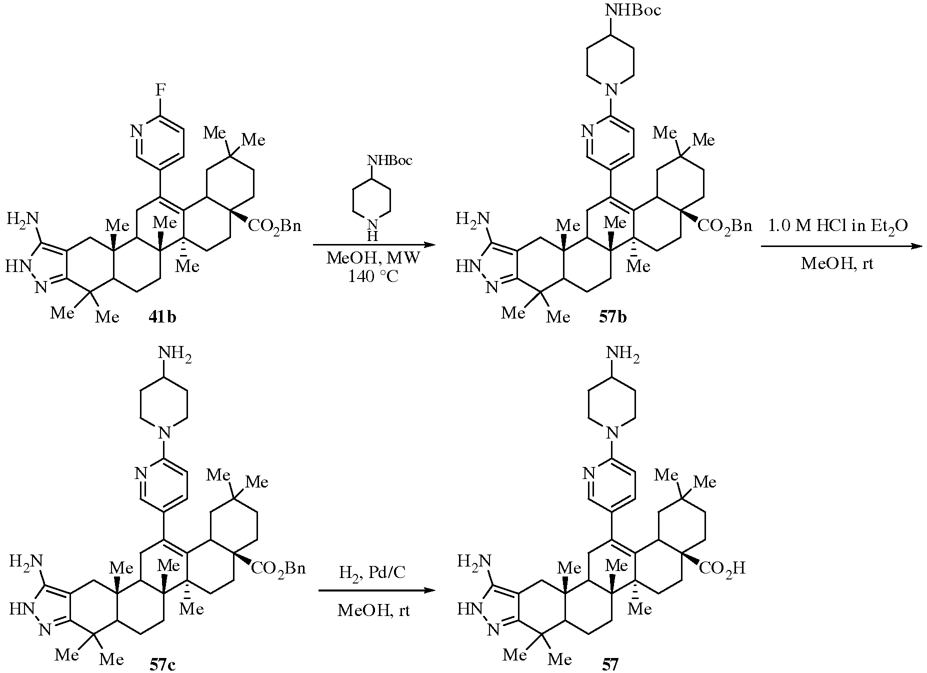

### (i) Preparation of 57b: (4aS,6aS,6bR,13aR)-Benzyl 12-amino-15-(6-(4-(tert-butoxycarbonylamino)piperidin-1-yl)pyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To 41 b (200 mg, 0.29 mmol) in MeOH (4 mL) was added tert-butyl piperidin-4-ylcarbamate (1.5 g, 7.50 mmol). The reaction mixture was heated at 140°C for 7 hours using microwave irradiation and then concentrated. The residue was dissolved in EtOAc (20 mL) and the solution was washed with brine then dried (Na₂SO₄), filtered and concentrated under reduced pressure. The residue was purified by column chromatography (0-10% MeOH in CH₂Cl₂) to afford the sub-title compound (200 mg, 79%).
APCI MS *m*/*z* 859 [C₅₃H₇₄N₆O₄ + H]⁺.

### (ii) Preparation of 57c: (4aS,6aS,6bR,13aR)-Benzyl 12-amino-15-(6-(4-aminopiperidin-1-yl)pyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a solution of **57b** (190 mg, 0.22 mmol) in MeOH (10 mL) was added HCl (1.0 M in diethyl ether; 10 mL, 10.0 mmol). The mixture was stirred at room temperature for 32 hours and then concentrated under reduced pressure. Purification of the residue by column chromatography (silica, 0-70 % CMA in CH₂Cl₂) afforded the sub-title compound (100 mg, 59%).
APCI MS *m*/*z* 759 [C₄₈H₆₆N₆O₂ + H]⁺.

### (iii) Preparation of 57: (4aS,6aS,6bR,13aR)-12-Amino-15-(6-(4-aminopiperidin-1-yl)pyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **57c** (100 mg, 0.13 mmol) and MeOH (10 mL) was flushed with nitrogen and then 10% Pd/C (100 mg) was added. The mixture was flushed with nitrogen followed by hydrogen. The mixture was stirred under hydrogen at atmospheric pressure overnight. The catalyst was removed by filtration through diatomaceous earth and the filtrate was concentrated under reduced pressure. The residue was purified by column chromatography (silica, 0-50% CMA in CH₂Cl₂) to afford the title compound (25 mg, 29%).
*R_{f}*0.11 (40:9:1 Methylene Chloride/Methanol/concentrated Ammonium Hydroxide).
¹H NMR (300 MHz, CD₃OD) δ 0.36-2.35 (m, 46H), 3.07 (m, 2 H), 3.10 (dd, *J* = 3.4 Hz, 3.9 Hz, 1 H), 4.29 (d, *J* = 13.2 Hz, 2H), 6.87 (d, *J* = 8.7 Hz, 1 H), 7.61 (m, 1 H), 8.03 (s, 1 H). APCI MS *m*/*z* 669 [C₄₁H₆₀N₆O₂ + H]⁺ m.p. 260-270°C dec. HPLC (Method A) 95.3% (214 nm) *t*_{R} = 10.8 min.

### Example 58

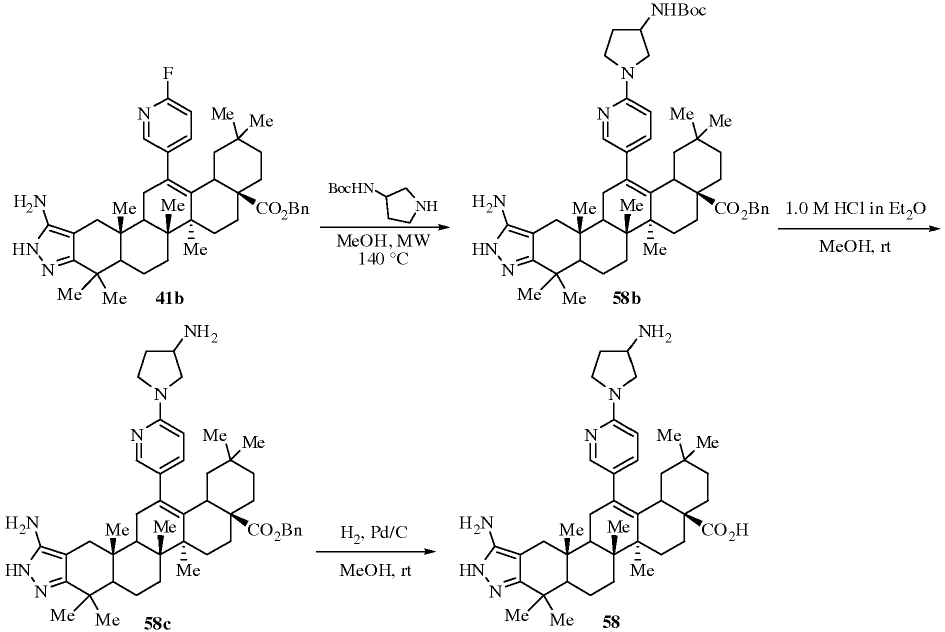

### (i) Preparation of 58b: (4aS,6aS,6bR,13aR)-Benzyl 12-amino-15-(6-(3-(tert-butoxycarbonylamino)pyrrolidin-1-yl)pyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a solution of 41 b (246 mg, 0.36 mmol) in MeOH (4 mL) was added *tert*-butyl pyrrolidin-3-ylcarbamate (1.0 g, 5.36 mmol). The reaction mixture was heated at 140°C for 6 hours using microwave irradiation. The solvent was removed under reduced pressure and the residue was dissolved in EtOAc (20 mL). The solution was washed with brine then dried (Na₂SO₄), filtered and concentrated. The residue was purified by column chromatography (silica, 0-10% MeOH in CH₂Cl₂) to afford the sub-title compound (306 mg, 100%).
APCI MS *m*/*z* 845 [C₅₂H₇₂N₆O₄ + H]⁺.

### (ii) Preparation of 58c: (4aS,6aS,6bR,13aR)-Benzyl 12-amino-15-(6-(3-aminopyrrolidin-1-yl)pyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

To a solution of **58b** (300 mg, 0.35 mmol) in MeOH (10 mL) was added HCl (1.0 M in diethyl ether, 7.8 mL, 7.80 mmol). The reaction mixture was stirred at room temperature for 24 hours and then concentrated. The residue was purified by column chromatography (silica, 0-40% CMA in CH₂Cl₂) to afford the sub-title compound (50 mg, 19%).
¹H NMR (300 MHz, CD₃OD) δ 0.36-2.35 (m, 42H), 3.08-3.68 (m, 7H), 5.05 (d, *J*= 12.0 Hz, 1H), 5.21 (d, *J* = 12.0 Hz, 1 H), 6.32 (d, *J* = 8.7 Hz, 1 H), 7.30-7.39 (m, 6H), 7.89 (s, 1H).
APCI MS m/z 745 [C₄₇H₆₄N₆O₂ + H]⁺.

### (iii) Preparation of 58: (4aS,6aS,6bR,13aR)-12-Amino-15-(6-(3-aminopyrrolidin-1-yl)pyridin-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid

A solution of **58c** (50 mg, 0.067 mmol) and MeOH (15 mL) was flushed with nitrogen and then 10% Pd/C (50 mg) was added. The mixture was flushed with nitrogen followed by hydrogen. The mixture was stirred under hydrogen at atmospheric pressure overnight. The catalyst was removed by filtration through diatomaceous earth and the filtrate was concentrated. The residue was purified by column chromatography (silica, 0-70% CMA in CH₂Cl₂) to afford the title compound (33 mg, 76%).
*R_{f}* 0.14 (40:9:1 Methylene Chloride/Methanol/concentrated Ammonium Hydroxide).
¹H NMR (300 MHz, CD₃OD) δ 0.37-2.36 (m, 44H), 3.10-3.80 (m, 5H), 6.53 (d, *J* = 8.7 Hz, 1 H), 7.61 (d, *J* = 8.4 Hz, 1 H), 7.93 (s, 1H). APCI MS *m*/*z* 655 [C₄₀H₅₈N₆O₂ + H]⁺. m.p. >300°C. HPLC (Method A) 97.4% (214 nm) *t*_{R} = 10.8 min.

### Example 59

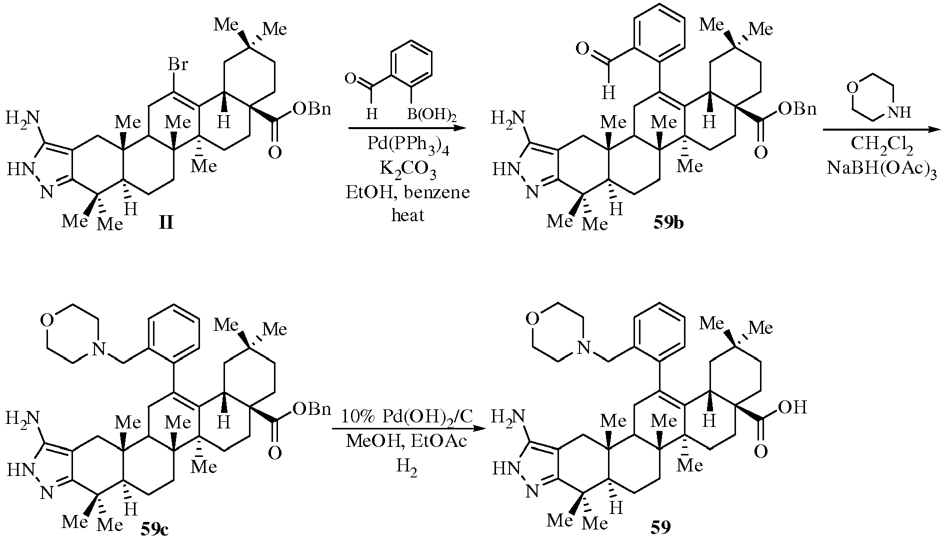

### (i) Preparation of 59b: (4aS,6aS,6bR,8aR,13aR,15bS)-benzyl 12-amino-15-(2-formylphenyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylate

A mixture of II (500 mg, 0.75 mmol), 2-formylphenylboronic acid (337 mg, 2.24 mmol) Pd(PPh₃)₄ (80 mg, 0.069 mmol) and K₂CO₃ (414 mg, 3.0 mmol) in benzene (3.5 mL) and EtOH (1.5 mL) was sealed and heated to 120°C by microwave for 1 hour. The reaction mixture was diluted with EtOAc (100 mL). The organic phase was washed with brine then dried (MgSO₄), filtered and concentrated to dryness. The residue was purified by column chromatography (silica, 0-10% MeOH in CH₂Cl₂) to afford the sub-title compound (540 mg) as a brown solid.
APCI MS (Positive Mode) *m*/*z* 688 [C₄₅H₅₇N₃O₃ + H]⁺.

### (ii) Preparation of 59c: (4aS,6aS,6bR,8aR,13aR,15bS)-Benzyl 12-amino-2,2,6a,6b,9,9,13a-heptamethyl-15-(2-(morpholinomethyl)phenyl)-2,3,4,4a,5,6,6a,6b,7,8,8a,9,11,13,13a,13b,14,15b-octadecahydro-1H-chryseno[1,2-f]indazole-4a-carboxylate

To a solution of **59b** (140 mg, 0.20 mmol) and morpholine (0.035 mL, 0.40 mmol) in CH₂Cl₂ (5 mL) was added sodium triacetoxyborohydride (85 mg, 0.40 mmol). The mixture was stirred at room temperature for 12 hours. The reaction mixture was diluted with EtOAc (150 mL). The organic phase was washed with brine then dried (MgSO₄), filtered and concentrated to dryness. The residue was purified by column chromatography (silica, 0-10% MeOH in CH₂Cl₂) to afford the sub-title compound (71 mg, 47%) as a brown solid.
APCI MS (Positive Mode) *m*/*z* 759 [C₄₉H₆₆N₄O₃ + H]⁺.

### (iii) Preparation of 59: (4aS,6aS,6bR,8aR,13aR,15bS)-12-Amino-2,2,6a,6b,9,9,13a-heptamethyl-15-(2-(morpholinomethyl)phenyl)-2,3,4,4a,5,6,6a,6b,7,8,8a,9,11,13,13a,13b,14,15b-octadecahydro-1H-chryseno[1,2-f]indazole-4a-carboxylic acid

A mixture of **59c** (71 mg, 0.093 mmol) and 10% Pd(OH)₂/C (50 mg) in MeOH (12 mL) and EtOAc (3 mL) was stirred under a hydrogen balloon for 12 hours. The reaction mixture was filtered through a pad of diatomaceous earth and washed with CMA (25 mL). The filtrate was concentrated to dryness under reduced pressure. The residue was purified by column chromatography (silica, 0-70% CMA in CH₂Cl₂) to afford the title compound (20 mg, 33%) as a mixture of rotamers.
*R_{f}* 0.80 (80:18:2 Methylene Chloride/Methanol/concentrated Ammonium Hydroxide).
¹H NMR (400 MHz, CD₃OD) δ 0.25 (s, 3H), 0.78 (s, 3H), 0.89 (s, 3H), 1.08 (s, 3H), 1.12 (s, 3H), 1.28 (s, 6H), 1.35-2.30 (m, 23H), 2.28 (d, *J* = 14.8 Hz, 1H), 2.40 (m, 1H), 2.90 (m, 1H), 3.95-4.50 (m, 5H), 7.20-7.62 (m, 4H). mp >300°C dec. APCI MS (Positive Mode) *m*/*z* 669 [C₄₂H₆₀N₄O₃ + H]⁺.

### Example 60

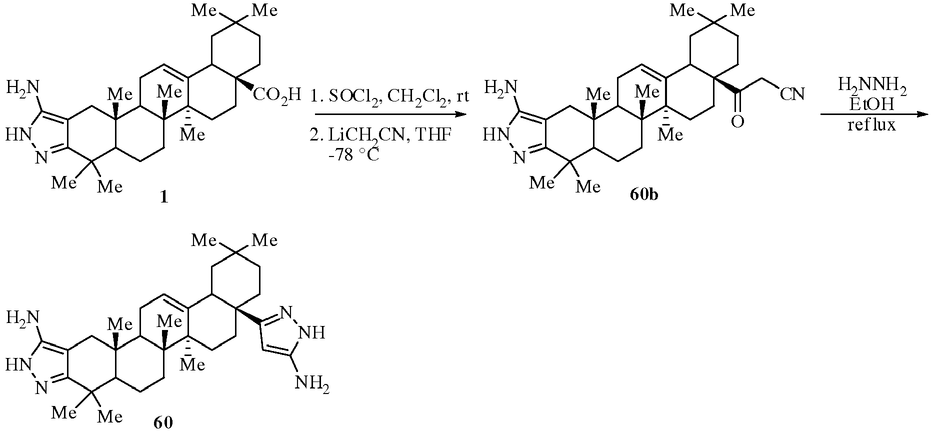

### (i) Preparation of 60b: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-3-oxopropanenitrile

To a suspension of 1 (150 mg, 0.30 mmol) and CH₂Cl₂ (7 mL) was added thionyl chloride (0.22 mL, 3.0 mmol) at room temperature. The mixture was stirred for 1 hour, after which time the solvent was removed under reduced pressure. The residue was taken up in toluene (5 mL), concentrated and placed under vacuum at room temperature. A solution of CH₃CN (0.16 mL, 3.0 mmol) and THF (5 mL) was cooled to - 78°C under nitrogen. The solution was treated with n-butyllithium (2.5 M in hexanes, 1.27 mL, 3.2 mmol) while maintaining the internal temperature below-70°C. The previously prepared acid chloride was taken up in THF (2 mL), cooled to -78°C and slowly added to the lithium salt mixture. The reaction mixture was stirred for 80 minutes and then quenched with 1 N HCl (10 mL). The layers were separated and the aqueous layer was extracted with EtOAc (15 mL) and the combined organic layers were dried (Na₂SO₄) and filtered. The solvent was removed under reduced pressure and the residue was purified by column chromatography (silica, 0-50% CMA in CH₂Cl₂) to provide the sub-title compound (115 mg, 74%) as an off-white solid.
¹H NMR (300 MHz, CD₃OD) 0.80 (s, 3H), 0.91-0.96 (m, 9H), 1.12-1.17 (m, 12H), 1.31-1.91 (m, 15H), 2.08-2.12 (m, 3H), 2.39-2.45 (m, 1H), 2.71-2.81 (m, 1H), 5.43-5.45 (m, 1 H). ESI MS *m*/*z* 517 [C₃₃H₄₈N₄O + H]⁺. HPLC 97.6% (area %), *t*_{R} = 16.2 min.

### (ii) Preparation of 60: (4aS,6aS,6bR,13aR)-4a-(5-Amino-1 H-pyrazol-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-12-amine

A solution of **60b** (100 mg, 0.19 mmol), hydrazine (31 mg, 0.97 mmol) and EtOH (5 mL) was heated at reflux under nitrogen for 16 hours. A second portion of hydrazine (91 mg, 2.85 mmol) was added and the reaction continued for 72 hours at reflux. The solvent was removed under reduced pressure and the residue was purified by column chromatography (silica, 0-50% CMA in CH₂Cl₂) to provide the title compound (43 mg, 43%) as an off-white solid.
¹H NMR (300 MHz, CD₃OD) 0.58 (s, 3H), 0.82 (s, 3H), 0.91-1.35 (m, 18H), 1.39-1.91 (m, 15H), 2.13-2.35 (m, 2H), 2.73-2.81 (m, 1H), 5.43-5.48 (m, 2H). ESI MS *m*/*z* 531 [C₃₃H₅₀N₆+ H]⁺. HPLC 97.9% (area %), t_{R} = 14.2 min.

### Example 61

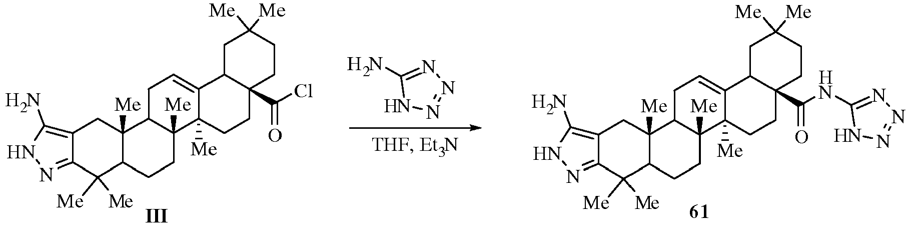

### (i) Preparation of 61: (4aS,6aS,6bR,13aR)-12-Amino-2,2,6a,6b,9,9,13a-heptamethyl-N-(1H-tetrazol-5-yl)-2,3,4,4a,5,6,6a,6b,7,8,8a,9,11,13,13a,13b,14,15b-octadecahydro-1H-chryseno[1,2-f]indazole-4a-carboxamide

To a solution of 1*H*-tetrazol-5-amine hydrate (103 mg, 1.0 mmol) and triethylamine (0.27 mL, 2.0 mmol) in THF (3 mL) was added **III** (100 mg, 0.20 mmol). The mixture was stirred at room temperature overnight. The reaction mixture was diluted with EtOAc (60 mL). The organic phase was washed with brine, dried (MgSO₄), filtered and concentrated to dryness under reduced pressure. The residue was purified by column chromatography (silica, 0-80% CMA in CH₂Cl₂) followed by preparative HPLC to afford the title compound (35 mg, 32%) as an off-white solid.
*R_{f}* 0.40 (80:18:2 Methylene Chloride/Methanol/concentrated Ammonium Hydroxide).
¹H NMR (300 MHz, CD₃OD) δ 0.73 (s, 3H), 0.85 (s, 3H), 0.92 (s, 3H), 0.97 (2, 3H), 1.15 (s, 3H), 1.20 (s, 3H), 1.25 (s, 3H), 1.30-1.90 (m, 16H), 2.10 (m, 2H), 2.20 (m, 1H), 2.41 (d, *J* = 15.0 Hz, 1 H), 3.05 (m, 1 H), 5.46 (s, 1 H). mp >300°C. ESI MS (Positive Mode) *m*/*z* 561 [C₃₂H₄₈N₈O + H]⁺.

### Example 62

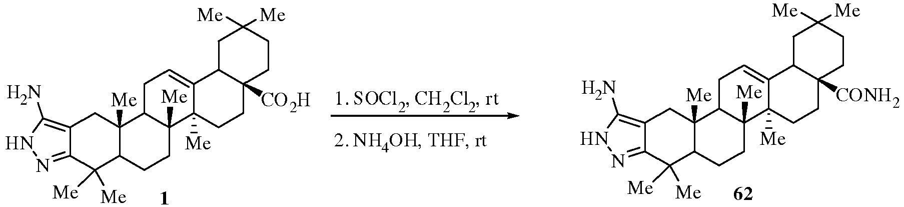

### (i) Preparation of 62: (4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxamide

To a suspension of 1 (250 mg, 0.51 mmol) and CH₂Cl₂ (5 mL) was added thionyl chloride (0.4 mL, 5.1 mmol) at room temperature. The mixture was stirred for 3 hours, after which time the solvent was removed under reduced pressure. The residue was taken up in toluene (5 mL), concentrated and placed under vacuum at room temperature for 1 hour. The residue was taken up in THF (5 mL) and concentrated NH₄OH (1.4 mL) was added. The mixture was stirred for 1 hour and another portion of NH₄OH (1.4 mL) was added. The mixture was stirred at room temperature over night before being quenched with saturated NaHCO₃ solution (10 mL). The mixture was extracted with CH₂Cl₂ (3 × 15 mL), dried (Na₂SO₄) and filtered. The solvent was removed under reduced pressure and the residue was purified by column chromatography (silica, 0-3% MeOH in CH₂Cl₂) to provide the title compound (144 mg, 58%) as an off-white solid.
¹H NMR (500 MHz, CD₃OD) δ 0.89-0.98 (m, 12H), 1.02-1.31 (m, 12H), 1.39-1.91 (m, 12H), 2.03-2.11 (m, 3H), 2.42-2.45 (m, 1 H), 2.76-2.83 (m, 1 H), 5.43-5.45 (m, 1 H).
ESI MS *m*/*z* 493 [C₃₁H₄₈N₄O + H]⁺.HPLC 97.9% (area %), *t*_{R} = 14.2 min.

### Example 63

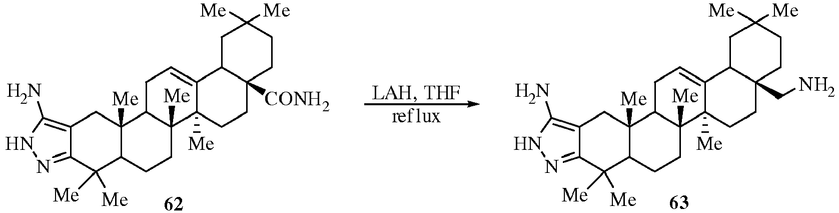

### (i) Preparation of 63: (4aS,6aS,6bR,13aR)-4a-(Aminomethyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-12-amine

To a solution of **62** (300 mg, 0.61 mmol) and THF (20 mL) was added lithium aluminum hydride (230 mg, 6.1 mmol) at room temperature. The mixture was heated at reflux for 24 hours, cooled to room temperature, carefully quenched with H₂O and EtOAc then extracted with CH₂Cl₂ (3 × 20 mL). The organics were dried (Na₂SO₄) and filtered. The solvent was removed under reduced pressure and the residue was purified by column chromatography (silica, 0-60% CMA in CH₂Cl₂) to provide the title compound (94 mg, 30%).
¹H NMR (300 MHz, CDCl₃) δ 0.82-1.03 (m, 12H), 1.15-1.85 (m, 14H), 1.91-2.11 (m, 15H), 2.52-2.60 (m, 2H), 2.83-2.91 (m, 1H), 3.25-3.30 (m, 1H), 3.56-3.63 (m, 1H), 5.23-5.39 (m, 2H), 7.92-7.94 (m, 2H). ESI MS *m*/*z* 479 [C₃₁H₅₀N₄ + H]⁺. HPLC 96.6% (area %), *t*_{R} = 11.3 min.

### Example 64

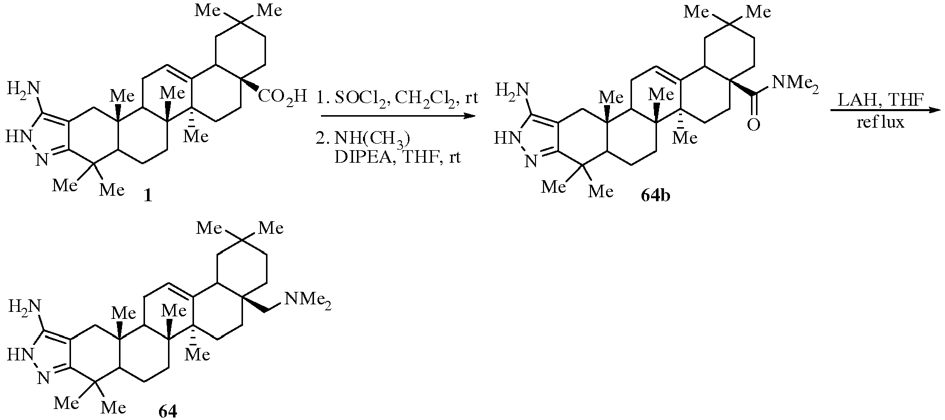

### (i) Preparation of 64b: (4aS,6aS,6bR,13aR)-12-Amino-N,N,2,2,6a,6b,9,9,13a-nonamethyl-2,3,4,4a,5,6,6a,6b,7,8,8a,9,11,13,13a,13b,14,15b-octadecahydro-1H-chryseno[1,2-f]indazole-4a-carboxamide

To a suspension of 1 (200 mg, 0.41 mmol) and CH₂Cl₂ (4 mL) was added thionyl chloride (0.3 mL, 4.1 mmol) at room temperature. The mixture was stirred for 0.5 hours, after which time the solvent was removed under reduced pressure. The residue was taken up in toluene (5 mL), concentrated and placed under vacuum at room temperature for 1 hour. The residue was taken up in THF (4 mL) and *N,N-*diisopropylethylamine (0.23 mL, 1.2 mmol) was added followed by diethylamine solution (2.0 M in THF, 2.05 mL, 4.1 mmol). The solution was stirred overnight and then quenched with a 2% solution of citric acid. The layers were separated and the aqueous solution was extracted with CH₂Cl₂ (2 × 10 mL). The combined extracts were dried (Na₂SO₄) and filtered. The solvent was removed under reduced pressure and the residue was purified by column chromatography (silica, 0-7% MeOH in CH₂Cl₂) to provide the sub-title compound (153 mg, 73%) as an off-white solid.
¹H NMR (300 MHz, CD₃OD) δ 0.83 (s, 3H), 0.91-0.98 (m, 9H), 1.17-1.26 (m, 13H), 1.39-2.25 (m, 16H), 3.01-3.20 (m, 7H), 5.29 (m, 1 H). ESI MS *m*/*z* 521 [C₃₃H₅₂N₄O + H]⁺. HPLC 98.4% (area %), *t*_{R} = 17.2 min.

### (ii) Preparation of 64: (4aS,6aS,6bR,13aR)-4a-((Dimethylamino)methyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-12-amine

A solution of **64b** (129 mg, 0.25 mmol) and THF (12.5 mL) was slowly added to a suspension of lithium aluminum hydride (94 mg, 2.5 mmol) and THF (6.5 mL) at room temperature. The mixture was stirred overnight and then quenched with EtOAc followed by H₂O. The layers were separated and the aqueous layer was extracted with EtOAc (3 × 15 mL). The organics were combined, dried (Na₂SO₄) and filtered. The solvent was removed under reduced pressure and the residue was purified by column chromatography (silica, 0-7% MeOH in CH₂Cl₂) to provide the title compound (54 mg, 43%) as a white solid.
¹H NMR (300 MHz, CDCl₃) δ 0.83-0.95 (m, 12H), 1.02-1.33 (m, 20H), 1.35-2.10 (m, 16H), 2.30-2.41 (m, 3H), 5.21-5.25 (m, 1H). ESI MS *m*/*z* 507 [C₃₃H₅₄N₄ + H]⁺. HPLC 96.4% (area %), *t*_{R} = 11.6 min

### Example 65

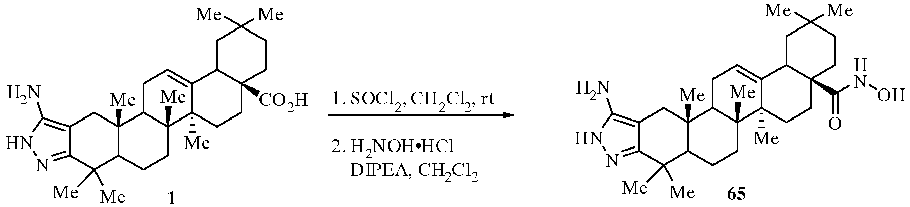

### (i) Preparation of 65: (4aS,6aS,6bR,13aR)-12-Amino-N-hydroxy-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxamide

To a suspension of 1 (75 mg, 0.15 mmol) in CH₂Cl₂ (5 mL) was added thionyl chloride (0.1 mL, 1.5 mmol) at room temperature. The mixture was stirred for 0.5 hours, after which time the solvent was removed under reduced pressure. The residue was taken up in toluene (5 mL), concentrated and placed under vacuum at room temperature overnight. The residue was taken up in CH₂Cl₂ (5 mL) at room temperature. *N,N-*Diisopropylethylamine (0.2 mL, 1.05 mmol) was added followed by hydroxylamine hydrochloride (52 mg, 0.75 mmol). The mixture was stirred at room temperature for 14 hours. A solution of citric acid (2%, 5 mL) was added and the mixture was extracted with CH₂Cl₂ (2 × 5 mL). The combined organic layers were dried (Na₂SO₄), filtered and concentrated under reduced pressure. The residue was purified by column chromatography (silica, 0-6% MeOH in CH₂Cl₂) to provided the title compound (144 mg, 58%) as an off-white solid.
¹H NMR (500 MHz, CD₃OD) 0.87-0.95 (m, 12H), 1.05-1.33 (m, 12H), 1.38-1.88 (m, 13H), 2.08-2.15 (m, 3H), 2.43-2.46 (m, 1H), 2.75-2.82 (m, 1H), 5.43-5.45 (m, 1H). APCI MS *m*/*z* 509 [C₃₁H₄₈N₄O + H]⁺. HPLC 97.4% (area %), *t*_{R} = 13.5 min.

### Example 66

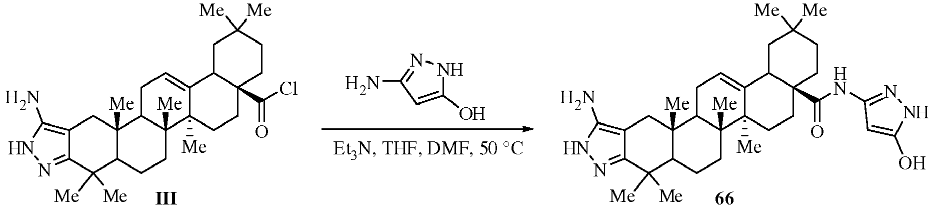

### (i) Preparation of 66: (4aS,6aS,6bR,13aR)-12-Amino-N-(5-hydroxy-1H-pyrazol-3-yl)-2,2,6a,6b,9,9,13a-heptamethyl-2,3,4,4a,5,6,6a,6b,7,8,8a,9,11,13,13a,13b, 14,15b-octadecahydro-1H-chryseno[1,2-f]indazole-4a-carboxamide

To a solution of **III** (250 mg, 0.48 mmol) and THF (2 mL) was added triethylamine (0.68 mL, 4.88 mmol) and the solution was stirred for 5 min. To the resultant mixture was added a solution of 3-amino-5-hydroxypyrazole (241 mg, 2.44 mmol) in DMF (6 mL) and the mixture was heated at 50°C for 3 hours. The mixture was poured into H₂O (20 mL) and the precipitate was removed by filtration. The filtrate was concentrated and the residue was purified by column chromatography (silica, 0-50% CMA in CH₂Cl₂) followed by preparative HPLC to afford the title compound (48 mg, 17%) as a solid.
*R_{f}*0.28 (90:9:1 Methylene Chloride/Methanol/concentrated Ammonium Hydroxide).
¹H NMR (300 MHz, CDCl₃) δ 0.49-2.97 (m, 42H), 5.36 (s, 1 H), 5.76 (s, 1 H).
APCI MS *m*/*z* 575 [C₃₄H₅₀N₆O₂ + H]⁺. m.p. 275-295°C dec. HPLC (Method A) 97.7% (214 nm) *t*_{R} = 11.5 min.

### Example 67

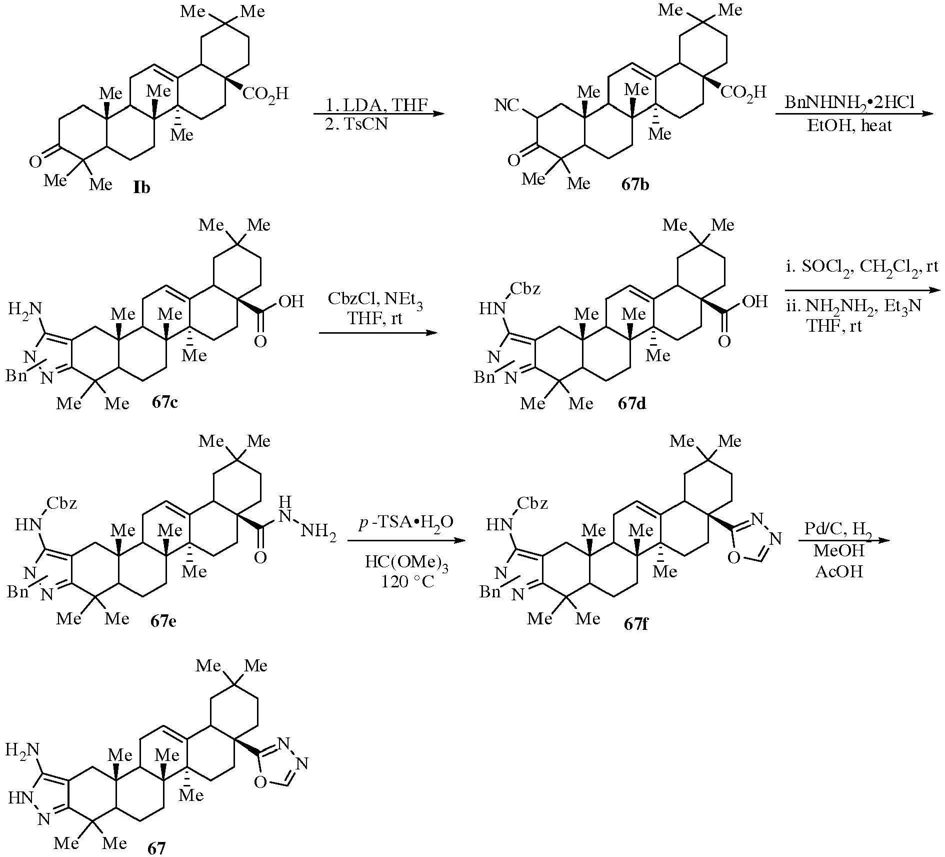

### (i) Preparation of 67b: (4aS,6aS,6bR,12aR)-11-Cyano-2,2,6a,6b,9,9,12a-heptamethyl-10-oxo-1,2,3,4,4a,5,6,6a,6b,7,8,8a,9,10,11,12,12a,12b,13,14b-icosahydropicene-4a-carboxylic acid

A solution of diisopropylamine (1.4 mL, 10.2 mmol) and THF (20 mL) was cooled to - 78°C under nitrogen. A solution of *n*-butyllithium (2.5 M in hexanes, 4.4 mL, 11.0 mmol) was slowly added, maintaining the internal temperature below -70°C. The solution was allowed to stir for 30 min and was then slowly added to a solution of **Ib** (2.0 g, 4.4 mmol) and THF (250 mL) at -78°C under nitrogen. This solution was stirred for 30 min after which time a suspension of *p*-toluenesulfonyl cyanide (1.6 g, 8.8 mmol) and THF (25 mL) was added over 45 min. The solution was stirred for 1 hour and then quenched by the addition of saturated ammonium chloride solution (100 mL) at -78°C. The mixture was allowed to warm to room temperature overnight. The organic layer was separated and the aqueous layer was extracted with EtOAc (3 × 100 mL). The combined organic layers were dried (Na₂SO₄) and filtered. The solvent was removed under reduced pressure and the residue was purified by column chromatography (silica, 0-35% EtOAc in hexanes) to provide the sub-title compound (1.3 g, 60%) as a white solid.
¹H NMR (300 MHz, CDCl₃) δ 0.77-0.82 (m, 9H), 1.06-1.11 (m , 13H), 1.12-2.03 (m, 19H), 2.09-2.38 (m, 1H), 2.81-2.89 (m, 1H), 5.26-5.35 (m, 1H).

### (ii) Preparation of 67c: (4aS,6aS,6bR,13aR)-12-Amino-11-benzyl-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid (and N-benzyl structural isomer)

A solution of **67b** (1.3 g, 2.6 mmol), benzylhydrazine dihydrochloride (1.2 g, 6.2 mmol) and EtOH (15 mL) was heated at reflux for 58 hours. The solvent was removed under reduced pressure and the residue was purified by column chromatography (silica, 0-5% MeOH in CH₂Cl₂) to provide the sub-title compound (1.1 g, 72%) as yellow solid.
¹H NMR (300 MHz, CDCl₃) δ 0.78-0.95 (m, 15H), 1.05-2.03 (m, 25H), 2.25-2.35 (m, 1 H), 2.81-2.91 (m, 1 H), 5.21-5.36 (m, 3H), 7.04-7.11 (m, 2H), 7.21-7.35 (m, 3H). ESI MS *m*/*z* 584 [C₃₈H₅₃N₃O₂ + H]⁺.

### (iii) Preparation of 67d: (4aS,6aS,6bR,13aR)-11-Benzyl-12-(benzyloxycarbonylamino)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxylic acid (and N-benzyl structural isomer)

To a solution of **67c** (560 mg, 0.96 mmol) in THF (50 mL) were added triethylamine (0.27 mL, 1.92 mmol) and benzyl chloroformate (0.16 mL, 1.15 mmol) under nitrogen. The mixture was stirred at room temperature for 16 hours and then quenched with brine (40 mL). The layers were separated and the aqueous layer was extracted with EtOAc (2 × 20 mL). The solution was dried (Na₂SO₄), filtered and concentrated to **afford** the sub-title compound (108 mg, quant) which was used without further purification.
ESI MS *m*/*z* 718 [C₄₆H₅₉N₃O₄ + H]⁺.

### (iv) Preparation of 67e: Benzyl (4aS,6aS,6bR,13aR)-11-benzyl-4a-(hydrazinecarbonyl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-12-ylcarbamate (and N-benzyl structural isomer)

To a solution of 67d (744 mg, 1.03 mmol) in CH₂Cl₂ (100 mL) was added thionyl chloride (0.76 mL, 10.37 mmol). The mixture was stirred at room temperature for 1.5 hours and then the solvent was removed under reduced pressure. The residue was azeotroped with toluene and then dried under vacuum overnight to afford the intermediate acid chloride (687 mg, 93%). The crude acid chloride (364 mg, 0.49 mmol) was dissolved in THF (10 mL) and then triethylamine (0.34 mL, 2.47 mmol) and hydrazine (0.15 mL, 4.94 mmol) were added. The mixture was stirred at room temperature for 20 hours and concentrated under reduced pressure. The residue was partitioned between EtOAc (20 mL) and H₂O (10 mL). The layers were separated and the organic layer was washed with H₂O and brine. The solution was dried (Na₂SO₄), filtered and concentrated. The residue was purified by column chromatography (silica, 0-40 % CMA in CH₂Cl₂) to afford the sub-title compound (224 mg, 62%).
ESI MS (*m*/*z* 733 [C₄₆H₆₁ N₅O₃ + H]⁺.

### (v) Preparation of 67f: Benzyl (4aS,6aS,6bR,13aR)-11-benzyl-2,2,6a,6b,9,9,13a-heptamethyl-4a-(1,3,4-oxadiazol-2-yl)-2,3,4,4a,5,6,6a,6b,7,8,8a,9,11,13,13a,13b,14,15b-octadecahydro-1H-chryseno[1,2-f]indazol-12-ylcarbamate (and N-benzyl structural isomer)

A mixture of **67e** (100 mg, 0.13 mmol), trimethoxymethane (0.22 mL, 2.04 mmol) and *p*-toluene sulfonic acid monohydrate (4 mg, 0.02 mmol) was heated at 120°C overnight. The mixture was concentrated under reduced pressure and the residue was purified by column chromatography (silica, 2:1 hexanes/EtOAc) to afford the sub-title compound (75 mg, 74%).
ESI MS *m*/*z* 651 [C₄₇H₅₉N₅O₃-C₇H₇ + H]⁺.

### (vi) Preparation of 67: (4aS,6aS,6bR,13aR)-2,2,6a,6b,9,9,13a-Heptamethyl-4a-(1,3,4-oxadiazol-2-yl)-2,3,4,4a,5,6,6a,6b,7,8,8a,9,11,13,13a,13b,14,15b-octadecahydro-1H-chryseno[1,2-]indazol-12-amine

A solution of **67d** (70 mg, 0.09 mmol), acetic acid (0.2 mL) and MeOH (10 mL) was flushed with nitrogen. To the resultant mixture was added 10% Pd/C (150 mg) and the mixture was flushed with nitrogen followed by hydrogen. The mixture was stirred under hydrogen at atmospheric pressure for 23 hours. The catalyst was removed by filtration through diatomaceous earth and the filtrate was concentrated under reduced pressure. The residue was purified by column chromatography (silica, 0-40% CMA in CH₂Cl₂) to afford the title compound (6 mg, 12%).
*R_{f}*0.42 (90:9:1 Methylene Chloride/Methanol/concentrated Ammonium Hydroxide).
¹H NMR (300 MHz, CD₃OD) δ 0.45-2.41 (m, 41 H), 3.14 (d, *J* = 10.8 Hz, 1 H), 5.44 (s, 1H), 8.84 (s, 1H). ESI MS *m*/*z* 518 [C₃₂H₄₇N₅O + H]⁺. m.p. 255-270 °C dec. HPLC (Method A) 89.0% (214 nm) *t*_{R} = 16.1 min.

### Example 68

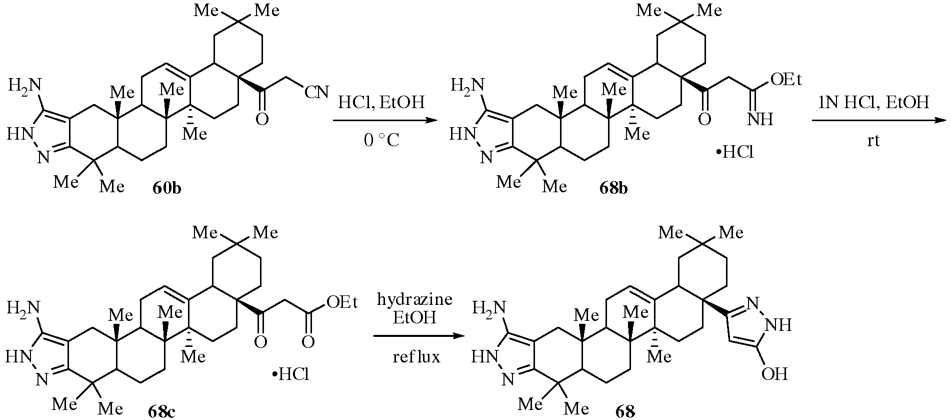

### (i) Preparation of 68b: Ethyl 3-((4aS,6aS,6bR,13aR)-12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-3-oxopropanimidate hydrochloride

A solution of **60b** (200 mg, 0.39 mmol) and EtOH (15 mL) was saturated with anhydrous HCl at -5°C. The solution was kept at that temperature for 10 d before removal of the solvent and excess HCl under reduced pressure. The residue was coevaporated with EtOH followed by diethyl ether to provide the sub-title compound (220 mg, 100%) as a tan solid.
APCI MS *m*/*z* 563 [C₃₃H₅₄N₄O₂ + H]⁺.

### (ii) Preparation of 68c: Ethyl 3-((4aS,6aS,6bR,13aR)-12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-3-oxopropanoate hydrochloride

To a solution of **68b** (220 mg, 0.39 mmol) and EtOH (3 mL) was added 1 N HCl (2 mL) at room temperature. After 24 hours, the solvent was removed under reduced pressure and the residue was purified by column chromatography (silica, 0-65% CMA in CH₂Cl₂) to provide the sub-title compound (135 mg, 58%).
¹H NMR (500 MHz, CDCl₃) δ 0.71-0.95 (m, 12H), 1.13-1.31 (m, 16H), 1.34-2.15 (m, 17H), 2.29-2.33 (m, 1H), 2.71-2.74 (m, 1H), 3.49-3.71 (m, 2H), 4.13-4.21 (m, 2H), 5.43-5.45 (m, 1 H).

### (iii) Preparation of 68: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-1H-pyrazol-5-ol

A solution of **68c** (135 mg, 0.24 mmol), hydrazine (38 mg, 1.2 mmol) and EtOH (5 mL) was heated at reflux under nitrogen for 66 hours and then cooled to room temperature. The solvent was removed under reduced pressure and the residue was purified by column chromatography (silica, 0-55% CMA in CH₂Cl₂) to provide the title compound (11 mg, 9%) as a solid.
¹H NMR (300 MHz, CD₃OD) δ 0.62 (s, 3H), 0.86-1.01 (m, 9H), 1.03-1.39 (m, 15H), 1.41-1.99 (m, 11H), 2.03-2.11 (m, 2H), 2.25-2.45 (m, 2H), 2.74-2.81 (m, 1H), 5.43-5.45 (m, 1 H). APCI MS *m*/*z* 532 [C₃₃H₄₉N₅O + H]⁺. HPLC 98.6% (area %), *t*_{R} = 10.2 min.

### Example 69

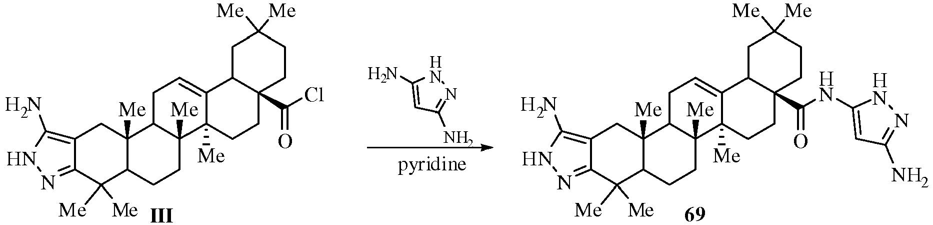

### (i) Preparation of 69: (4aS,6aS,6bR,13aR)-12-amino-N-(3-amino-1H-pyrazol-5-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxamide

To a solution of 1*H*-pyrazole-3,5-diamine (70 mg, 0.54 mmol) prepared by the procedure described in the literature *(*US82902, 2007) and pyridine (2.0 mL) was added III (100 mg, 0.20 mmol). The mixture was stirred at room temperature overnight. The reaction mixture was concentrated to dryness under reduced pressure. The residue was purified by column chromatography (silica, 0-40% CMA in CH₂Cl₂) followed by preparative HPLC to afford the title compound (25 mg, 25%) as an off-white solid.
*R_{f}*0.20 (180:18:2 Methylene Chloride/Methanol/concentrated Ammonium Hydroxide).
¹H NMR (300 MHz, DMSO-d₆) δ 0.83 (s, 3H), 0.92 (s, 3H), 0.95 (s, 3H), 1.0 (s, 3H), 1.25 (s, 6H), 1.30 (s, 3H), 1.30-1.90 (m, 23H), 2.06 (m, 3H), 2.25 (m, 1 H), 2.44 (d, *J* = 15.0 Hz, 1H), 2.62 (m,1H), 5.35 (s, 1 H). mp >300 °C. APCI MS (Positive Mode) *m*/*z* 574 [C₃₄H₅₁N₇O + H]⁺.

### Example 70

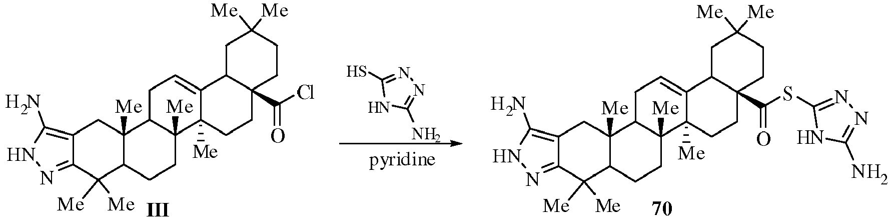

### (i) Preparation of 70: (4aS,6aS,6bR,13aR)-S-5-Amino-4H-1,2,4-triazol-3-yl 12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carbothioate

To a solution of 5-amino-4*H-*1,2,4-triazole-3-thiol (1.42 g, 12.2 mmol) in pyridine (30 mL was added **III** (2.0 g, 3.91 mmol). The mixture was stirred at room temperature for 3 hours. The reaction mixture was concentrated to remove pyridine under reduced pressure. The residue was purified by column chromatography (silica, 0-15% MeOH in CH₂Cl₂) to afford the title compound (1.41 g, 59%) as an off-white solid.

*R_{f}*0.12 (89:10:1 Methylene Chloride/Methanol/concentrated Ammonium Hydroxide). ¹H NMR (300 MHz, CD₃OD) δ 0.87 (s, 6H), 0.90 (s, 3H), 0.95 (s, 3H), 1.10 (s, 3H), 1.15 (s, 3H), 1.20 (s, 3H), 1.30-2.20 (m, 19H), 2.38 (d, *J* = 15.0 Hz, 1H), 2.88 (m, 1H), 5.39 (s, 1H). mp 278-296°C. ESI MS (Positive Mode) *m*/*z* 59 [C₃₃H₄₉N₇OS - H]⁻.

### Example 71

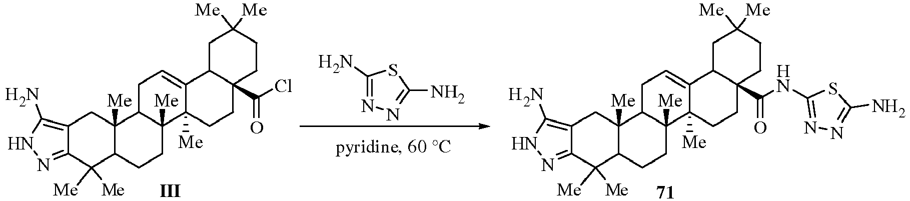

### (i) Preparation of 71: (4aS,6aS,6bR,13aR)-12-Amino-N-(5-amino-1,3,4-thiadiazol-2-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxamide

To a solution of **III** (500 mg, 0.97 mmol) and pyridine (4 mL) was added 1,3,4-thiadiazole-2,5-diamine (340 mg, 2.92 mmol). The reaction mixture was heated at 60°C for 20 hours and then poured into H₂O (50 mL). The precipitate was collected by filtration and dissolved in CH₂Cl₂/*i*-PrOH (2:1, 20 mL). The aqueous layer was extracted with CH₂Cl₂/*i*-PrOH (2:1, 2 x25 mL). The combined extracts were dried (Na₂SO₄), filtered and concentrated under reduced pressure. The residue was purified by column chromatography (silica, 0-40% CMA in CH₂Cl₂) followed by preparative HPLC to afford the title compound (32 mg, 6%) as a solid.
*R_{f}*0.20 (90:9:1 Methylene Chloride/Methanol/concentrated Ammonium Hydroxide)
¹H NMR (300 MHz, DMSO-*d*₆) δ 0.77-2.43 (m, 41 H), 2.94 (d, *J* = 12 Hz, 1 H), 5.32 (s, 1 H), 7.46 (s, 1 H), 11.50 (s, 1 H), 13.20 (s, 1 H).
APCI MS *m*/*z* 592 [C₃₃H₄₉N₇OS + H]⁺. m.p. 280-300°C dec. HPLC (Method A) 95.4% (214 nm) *t*_{R} = 10.2 min.

### Example 72

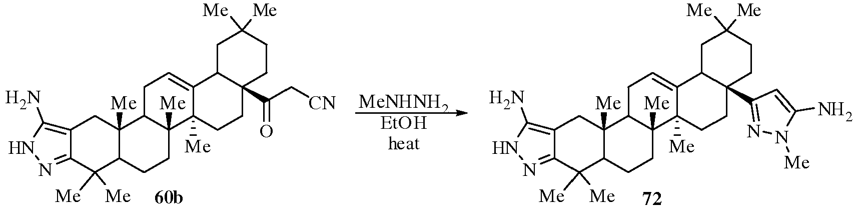

### (i) Preparation of 72: (4aS,6aS,6bR,8aR,13aR,15bS)-4a-(5-Amino-1-methyl-1H-pyrazol-3-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-12-amine

A mixture of **60b** (282 mg, 0.54 mmol) and methylhydrazine (0.14 mL, 2.73 mmol) in EtOH (4 mL) was sealed and heated to 160°C by microwave for 3 hours. The reaction mixture was concentrated to dryness. The residue was purified by column chromatography (silica, 0-10% MeOH in CH₂Cl₂) followed by preparative HPLC to afford title compound (35 mg, 12%) as a white solid.
*R_{f}*0.34 (89:10:1 Methylene Chloride/Methanol/concentrated Ammonium Hydroxide).
¹H NMR (300 MHz, CD₃OD) δ 0.65 (s, 3H), 0.88 (s, 3H), 0.95 (s, 3H), 0.97 (s, 3H), 1.15 (s, 3H), 1.20 (s, 3H), 1.24 (s, 3H), 1.25-2.10 (m, 19H), 2.38 (m, 2H), 2.78 (m, 1 H), 3.68 (s, 3H), 5.47 (m, 1H). mp 195-210°C dec. APCI MS (Positive Mode) *m*/*z* 545 [C₃₄H₅₂N₆ + H]⁺.

### Example 73

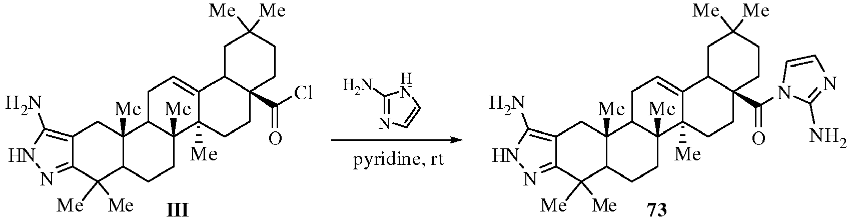

### (i) Preparation of 73: (2-Amino-1H-imidazol-1-yl)((4aS,6aS,6bR,13aR)-12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)methanone

To a solution of **III** (200 mg, 0.39 mmol) and pyridine (2 mL) was added 1*H*-imidazol-2-amine (70 mg, 0.84 mmol). The reaction mixture was stirred at room temperature for 3.5 hours and then poured into H₂O (20 mL). The precipitate was collected by filtration and dissolved in CH₂Cl₂/*i*-PrOH (2:1, 10 mL). The aqueous layer was extracted with CH₂Cl₂/*i*-PrOH (2:1, 2 × 10 mL). The combined organics were dried (Na₂SO₄), filtered and concentrated under reduced pressure. The residue was purified by column chromatography (silica, 0-30% CMA in CH₂Cl₂) to afford the title compound (96 mg, 44%).
*R_{f}*0.50 (90:9:1 Methylene Chloride/Methanol/concentrated Ammonium Hydroxide).
¹H NMR (300 MHz, DMSO-*d*₆) δ 0.91-2.49 (m, 41 H), 3.00 (d, *J* = 11.1 Hz, 1 H), 4.12 (s, 2H), 5.29 (s, 1 H), 6.46 (s, 1 H), 6.64 (s, 2H), 7.39 (s, 1 H), 10.95 (s, 1 H). APCI MS *m*/*z* 559 [C₃₄H₅₀N₆O + H]⁺. m.p. 230-250°C dec. HPLC (Method A) 97.6% (214 nm) *t*_{R} = 9.9 min.

### Example 74

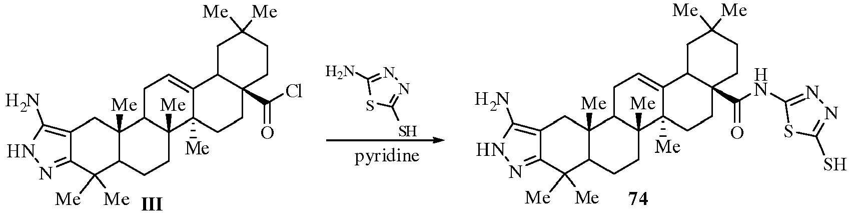

### (i) Preparation of 74: (4aS,6aS,6bR,13aR)-12-Amino-N-(5-mercapto-1,3,4-thiadiazol-2-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxamide

To a solution of 5-amino-1,3,4-thiadiazole-2-thiol (200 mg, 2.1 mmol) prepared by the procedure described in the literature *(*US3940409, 1976) and pyridine (10.0 mL) was added **III** (215 mg, 0.42 mmol). The mixture was stirred at room temperature overnight. The reaction mixture was concentrated to dryness under reduced pressure. The residue was purified by column chromatography (silica, 0-70% CMA in CH₂Cl₂) followed by preparative HPLC to afford the title compound (30 mg, 12%) as an off-white solid.
*R_{f}* 0.60 (80:18:2 Methylene Chloride/Methanol/concentrated Ammonium Hydroxide).
¹H NMR (300 MHz, CD₃OD) δ 0.89 (s, 3H), 0.90 (s, 3H), 0.92 (s, 3H), 0.97 (s, 3H), 1.29 (s, 6H), 1.36 (s, 3H), 1.43-2.30 (m, 19H), 2.43 (d, *J* = 14.7 Hz, 1H), 2.84 (m, 1H), 5.42 (s, 1 H). mp >300°C. ESI MS (Positive Mode) *m*/*z* 609 [C₃₃H₄₈N₆OS₂ + H]⁺.

### Example 75

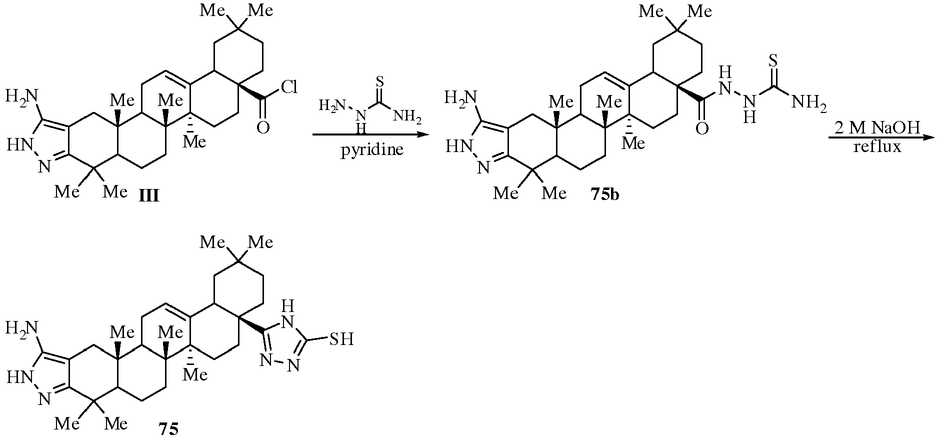

### (i) Preparation of 75b: 2-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carbonyl)hydrazinecarbothioamide

To a solution of hydrazinecarbothioamide (914 mg, 10.0 mmol) and pyridine (50.0 mL) was added **III** (1.0 g, 2.0 mmol). The mixture was stirred at room temperature for 2 hours. The reaction mixture was poured into H₂O. Brown precipitates were collected by filtration and washed with H₂O, dried in an oven at 40°C to provide the sub-title compound (900 mg). The crude compound was used without further purification.
ESI MS (Positive Mode) *m*/*z* 567 [C₃₂H₅₀N₆OS + H]⁺.

### (ii) Preparation of 75: 5-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-4H-1,2,4-triazole-3-thiol

A mixture of **75b** (100 mg) and NaOH (2 M, 2.0 mL) was heated at reflux for 7 d. The reaction mixture was concentrated to dryness under reduced pressure. The residue was triturated with MeOH and CH₂Cl₂. The filtrate was concentrated and purified by preparative HPLC to provide the title compound (61 mg, 66%) as an off-white solid.
*R_{f}*0.60 (180:18:2 Methylene Chloride/Methanol/concentrated Ammonium Hydroxide).
¹H NMR (300 MHz, CD₃OD) δ 0.60 (s, 3H), 0.82 (s, 3H), 0.95 (s, 3H), 0.98 (s, 3H), 1.21 (s, 3H), 1.25 (s, 3H), 1.46 (s, 3H), 1.50-2.05 (m, 18H), 2.25 (m, 1H), 2.40 (d, J = 15.0 Hz, 1 H), 2.98 (m, 1 H), 5.40 (s, 1 H). mp >300°C. ESI MS (Positive Mode) *m*/*z* 549 [C₃₂H₄₈N₆S + H]⁺.

### Example 76

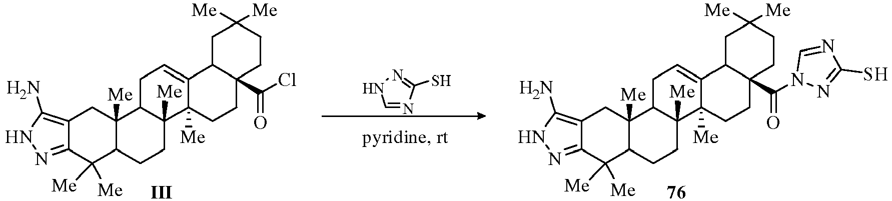

### (i) Preparation of 76: ((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)(3-mercapto-1H-1,2,4-triazol-1-yl)methanone

To a solution of **III** (300 mg, 0.58 mmol) and pyridine (3 mL) was added 1*H*-1,2,4-triazole-3-thiol (177 mg, 1.75 mmol). The reaction mixture was stirred at room temperature for 1 hour and then poured into H₂O (30 mL). The precipitate was dissolved in CH₂Cl₂/*i*-PrOH (2:1, 20 mL). The aqueous layer was extracted with CH₂Cl₂/*i*-PrOH (2:1, 3 × 10 mL) and the combined organics were dried (Na₂SO₄), filtered and concentrated under reduced pressure. The residue was purified by column chromatography (silica, 0-8% MeOH in CH₂Cl₂) to afford the title compound (110 mg, 33%).
*R_{f}*0.27 (90:9:1 Methylene Chloride/Methanol/concentrated Ammonium Hydroxide)
¹H NMR (300 MHz, CD₃OD) δ 0.89-2.23 (m, 40H), 2.41 (d, *J* = 14.7 Hz, 1 H), 3.00 (dd, *J* = 3.6, 12.9 Hz, 1H), 5.40 (s, 1H), 8.46 (s, 1H). APCI MS *m*/*z* 577 [C₃₃H₄₈N₆OS + H]⁺ m.p. 260-280°C dec. HPLC (Method A) 96.2% (214 nm) *t*_{R} = 14.3 min.

### Example 77

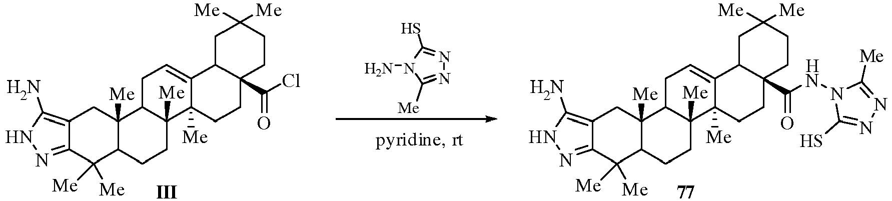

### (i) Preparation of 77: (4aS,6aS,6bR,13aR)-12-Amino-N-(3-mercapto-5-methyl-4H-1,2,4-triazol-4-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxamide

To a solution of **III** (300 mg, 0.58 mmol) and pyridine (3 mL) was added 4-amino-5-methyl-4*H-*1,2,4-triazole-3-thiol (228 mg, 1.75 mmol). The reaction mixture was stirred at room temperature for 1 hour and then poured into H₂O (30 mL). The precipitate was collected by filtration and dried under vacuum overnight. The residue was purified by column chromatography (silica, 0-30% CMA in CH₂Cl₂) followed by preparative HPLC to afford the title compound (30 mg, 8%) as a solid.
*R_{f}*0.25 (90:9:1 Methylene Chloride/Methanol/concentrated Ammonium Hydroxide)
¹H NMR (300 MHz, CD₃OD) δ 0.89-2.50 (m, 44H), 2.93-2.97 (m, 1 H), 5.36 (s, 1 H). APCI MS *m*/*z* 606 [C₃₄H₅₁N₇OS + H]⁺. m.p. 250-270°C dec. HPLC (Method A) 92.7% (214 nm) *t*_{R} = 13.2 min.

### Example 78

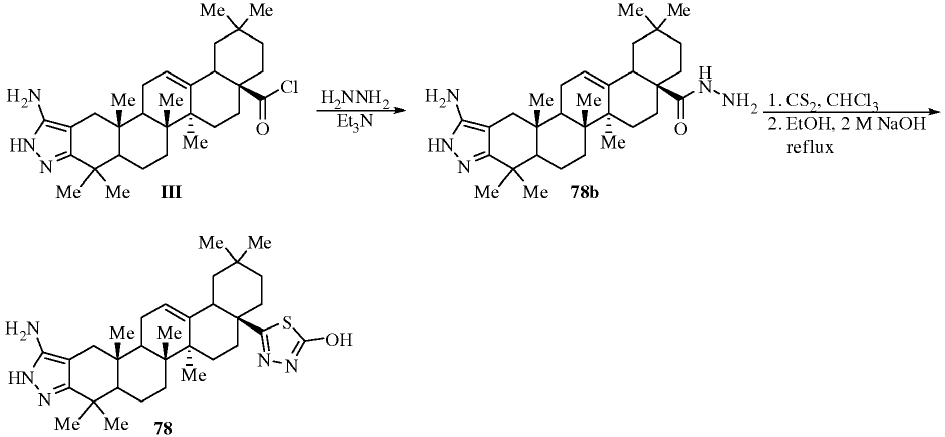

### (i) Preparation of 78b: (4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carbohydrazide

To a solution of hydrazine (0.15 mL, 4.5 mmol) and triethylamine (0.77 mL) was added **III** (500 mg, 0.91 mmol). The mixture was stirred at room temperature overnight. The reaction mixture was diluted with EtOAc (100 mL). The organic phase was washed with brine then dried (MgSO₄), filtered and concentrated to dryness. The residue was purified by column chromatography on (silica, 0-50% CMA in CH₂Cl₂) to afford the sub-title compound (360 mg, 78%) as a brown solid.

### (ii) Preparation of 78: 5-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-1,3,4-thiadiazol-2-ol

To a solution of **78b** (140 mg, 0.27 mmol) in CHCl₃ (5 mL) was added carbon disulfide (0.58 mL, 0.96 mmol). The mixture was stirred at room temperature for 4 hours. The reaction mixture was concentrated to dryness under reduced pressure. The residue was dissolved in EtOH (5 mL) and NaOH (2 M, 5 mL) was added. The mixture was heated at reflux for 12 hours. The reaction mixture was concentrated to remove EtOH. Brown solids were collected by filtration and washed with H₂O. The residue was purified by column chromatography (silica, 0-50% CMA in CH₂Cl₂) followed by preparative HPLC to afford the title compound (30 mg, 12%) as an off-white solid.
*R_{f}*0.30 (10:1 Methylene Chloride/Methanol).
¹H NMR (300 MHz, DMSO-*d*6) δ 0.58 (s, 3H), 0.75 (s, 3H), 0.92 (s, 6H), 1.16 (s, 3H), 1.21 (s, 3H), 1.26 (s, 3H), 1.31-1.97 (m, 22H), 2.22 (m, 1 H), 2.43 (d, *J* = 15.0 Hz, 1 H), 2.83 (m, 1 H), 5.35 (s, 1 H).mp >300°C. ESI MS (Positive Mode) *m*/*z* 550 [C₃₂H₄₇N₅OS + H]⁺.

### Example 79

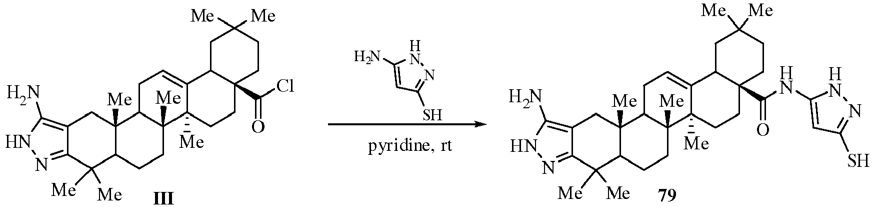

### (i) Preparation of 79: (4aS,6aS,6bR,13aR)-12-Amino-N-(3-mercapto-1H-pyrazol-5-yl)-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carboxamide

To a solution of 5-amino-1*H*-pyrazole-3-thiol (459 mg, 4.0 mmol) prepared by the procedure described in the literature (J. Med. Chem. 2008, 51(15), 4672-4684) and pyridine (10.0 mL) was added **III** (490 mg, 0.95 mmol). The mixture was stirred at room temperature overnight. The reaction mixture was concentrated to dryness under reduced pressure. The residue was purified by column chromatography (silica, 0-70% CMA in CH₂Cl₂) followed by preparative HPLC to afford the title compound (115 mg, 17%) as an off-white solid.
*R_{f}*0.75 (80:18:2 Methylene Chloride/Methanol/concentrated Ammonium Hydroxide).
¹H NMR (300 MHz, CD₃OD) δ 0.89 (s, 6H), 0.91 (s, 3H), 0.95 (s, 3H), 1.28 (s, 3H), 1.40 (s, 3H), 1.48 (s, 3H), 1.55-2.20 (m, 19H), 2.43 (d, *J* = 14.4 Hz, 1H), 2.93 (m, 1H), 5.39 (m, 1H), 5.57 (m, 1H). mp >300°C. ESI MS (Positive Mode) *m*/*z* 591 [C₃₄H₅₀N₆OS + H]⁺.

### Example 80

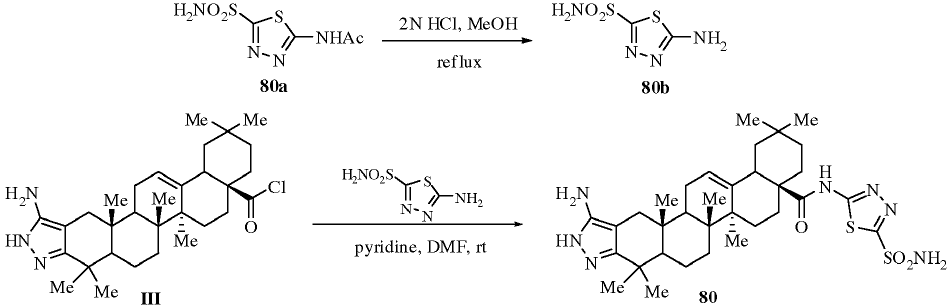

### (i) Preparation of 80b: 5-Amino-1,3,4-thiadiazole-2-sulfonamide

To a solution of *N*-(5-sulfamoyl-1,3,4-thiadiazol-2-yl)acetamide (1.0 g, 4.50 mmol) and MeOH (15 mL) was added HCl (2 N, 10 mL). The reaction mixture was heated at reflux for 18 hours and then concentrated under reduced pressure. The residue was purified by column chromatography (silica, 0-10% MeOH in CMA) to afford the sub-title compound (800 mg, 98%).

### (ii) Preparation of 80: (4aS,6aS,6bR,13aR)-12-Amino-2,2,6a,6b,9,9,13a-heptamethyl-N-(5-sulfamoyl-1,3,4-thiadiazol-2-yl)-2,3,4,4a,5,6,6a,6b,7,8,8a,9,11,13,13a,13b,14,15b-octadecahydro-1H-chryseno[1,2-flindazole-4a-carboxamide

To a solution of **III** (400 mg, 0.78 mmol), pyridine (15 mL) and DMF (1 mL) was added **80b** (281 mg, 1.56 mmol). The reaction mixture was stirred at room temperature for 24 hours and then poured into H₂O (15 mL). The precipitate was collected by filtration and dried under vacuum overnight. The residue was purified by column chromatography (silica, 0-60% CMA in CH₂Cl₂) followed by preparative HPLC to afford the title (8 mg, 2%) as a solid.
*R_{f}*0.35 (90:9:1 Methylene Chloride/Methanol/concentrated Ammonium Hydroxide).
¹H NMR (300 MHz, Acetone-*d*₆) δ 0.68-2.27 (m, 40H), 2.45 (d, *J* = 15 Hz, 1H), 3.05-3.08 (m, 1 H), 5.47 (s, 1H). APCI MS *m*/*z* 656 [C₃₃H₄₉N₇O₃S₂ + H]⁺. m.p. 280-300°C dec. HPLC (Method A) >99% (214 nm) *t*_{R} = 14.0 min.

### Example 81

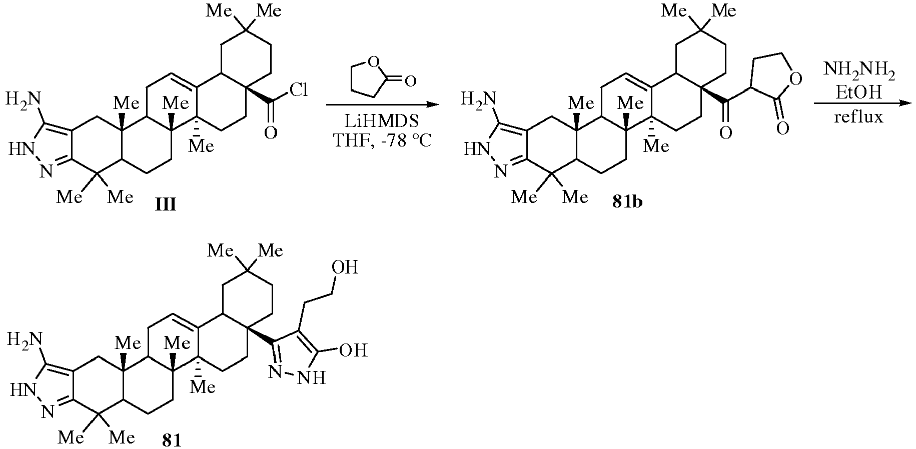

### (i) Preparation of 81b: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1Hchryseno[1,2-f]indazole-4a-carbonyl)dihydrofuran-2(3H)-one

To a solution of lithium bis(trimethylsilyl)amide (2.3 mL, 1 M in THF, 2.3 mmol) was added dihydrofuran-2(3*H*)-one (0.15 mL, 1.9 mmol) at -78°C. The mixture was stirred for 15 min. The anion solution was added to a solution of III (100 mg, 0.19 mmol) in THF (7 mL) precooled to -78°C. The mixture was stirred at -78°C for 30 min and deemed incomplete. To a second solution of lithium bis(trimethylsilyl)amide (1.1 mL, 1 M in THF, 1.1 mmol) was added dihydrofuran-2(3*H*)-one (0.075 mL, 1.0 mmol) at - 78°C. The second anion mixture was stirred for 15 min. The anion solution was added to the above reaction mixture at -78°C. The mixture was continued to stir at -78°C for 30 min. The reaction mixture was quenched with saturated NH₄Cl (60 mL) and extracted with EtOAc (100 mL). The organic phase was washed with brine, dried (MgSO₄), filtered and concentrated to dryness. The residue was purified by column chromatography (silica, 0-30% CMA in CH₂Cl₂) to afford the sub-title compound (150 mg) as a brown solid.
ESI MS (Positive Mode) *m*/*z* 562 [C₃₅H₅₁N₃O₃ + H]⁺

### (ii) Preparation of 81: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-4-(2-hydroxyethyl)-1H-pyrazol-5-ol

A mixture of **81b** (150 mg) and hydrazine (0.037 mL) in EtOH (2 mL) was heated at reflux overnight. The reaction mixture was concentrated to dryness under reduced pressure. The residue was purified by column chromatography (silica, 0-60% CMA in CH₂Cl₂) followed by preparative HPLC to afford the title compound (12 mg, 8%) as an off-white solid.
*R_{f}*0.70 (80:18:2 Methylene Chloride/Methanol/concentrated Ammonium Hydroxide).
¹H NMR (300 MHz, CD₃OD) δ 0.50 (s, 3H), 0.86 (s, 3H), 0.95 (s, 3H), 0.98 (s, 3H), 1.23 (s, 3H), 1.28 (s, 3H), 1.32 (s, 3H), 1.35-2.08 (m, 18H), 2.25 (m, 1H), 2.36 (d, *J* = 15.0 Hz, 1 H), 2.74 (m, 2H), 2.82 (m, 1 H), 3.72 (t, *J* = 6.6 Hz, 2H), 5.51 (s, 1H). mp 251-255°C. APCI MS (Positive Mode) *m*/*z* 576 [C₃₅H₅₃N₅O₂ + H]⁺.

### Example 82

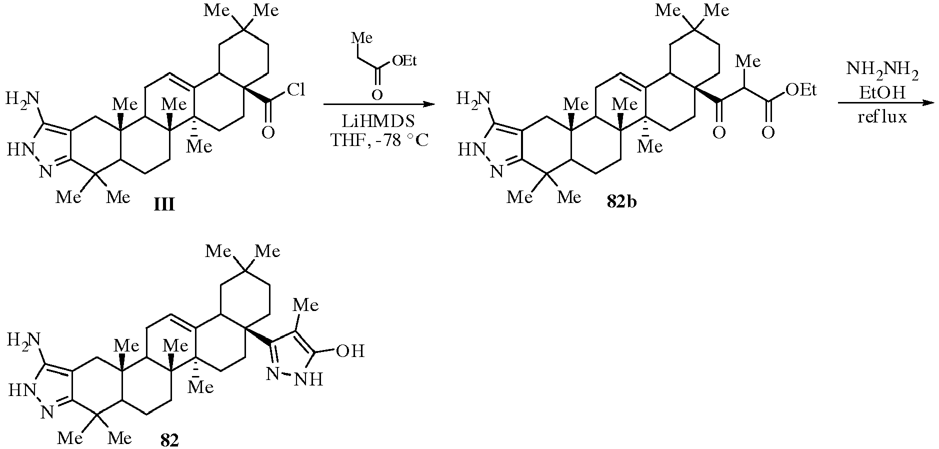

### (i) Preparation of 82b: Ethyl 3-((4aS,6aS,6bR,13aR)-12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-2-methyl-3-oxopropanoate

To a solution of lithium bis(trimethylsilyl)amide (3.8 mL, 1 M in THF, 3.8 mmol) was added ethyl propionate (0.22 mL, 1.9 mmol). The mixture was stirred for 30 min. The anion solution was added to a solution of III (100 mg, 0.19 mmol) in THF (3 mL) precooled to -78°C. The mixture was stirred at -78°C for 1 hour and the reaction was deemed incomplete. To a second solution of lithium bis(trimethylsilyl)amide (1.9 mL, 1 M in THF, 1.9 mmol) was added ethyl propionate (0.33 mL, 1.9 mmol). The second anion mixture was stirred for 30 min. The anion solution was added to the above reaction mixture at -78°C. The mixture was continued to stir at -78°C for 30 min. The reaction mixture was quenched with saturated NH₄Cl (20 mL) and extracted with EtOAc (100 mL). The organic phase was washed brine, dried (MgSO₄), filtered and concentrated to dryness. The residue was purified by column chromatography (silica, 0-30% CMA in CH₂Cl₂) to afford the sub-title compound (100 mg) as a brown solid. APCI MS (Positive Mode) *m*/*z* 578 [C₃₆H₅₅N₃O₃ + H]⁺

### (ii) Preparation of 82: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-4-methyl-1H-pyrazol-5-ol

A mixture of **82b** (100 mg) and hydrazine (0.06 mL) in EtOH (2 mL) was heated at reflux overnight. The reaction mixture was concentrated to dryness under reduced pressure. The residue was purified by column chromatography (silica, 0-50% CMA in CH₂Cl₂) followed by preparative HPLC to the title compound (7 mg, 7%) as an off-white solid.
*R_{f}*0.70 (80:18:2 Methylene Chloride/Methanol/concentrated Ammonium Hydroxide).
¹H NMR (300 MHz, CD₃OD) δ 0.55 (s, 3H), 0.85 (s, 3H), 0.96 (s, 3H), 1.00 (s, 3H), 1.19 (s, 3H), 1.23 (s, 3H), 1.28 (s, 3H), 1.32-2.08 (m, 21 H), 2.39 (m, 1 H), 2.44 (d, *J* = 15.0 Hz, 1H), 2.90 (m, 1H), 5.48 (s, 1H). mp >300°C. APCI MS (Positive Mode) *m*/*z* 546 [C₃₄H₅₁N₅O + H]⁺.

### Example 83

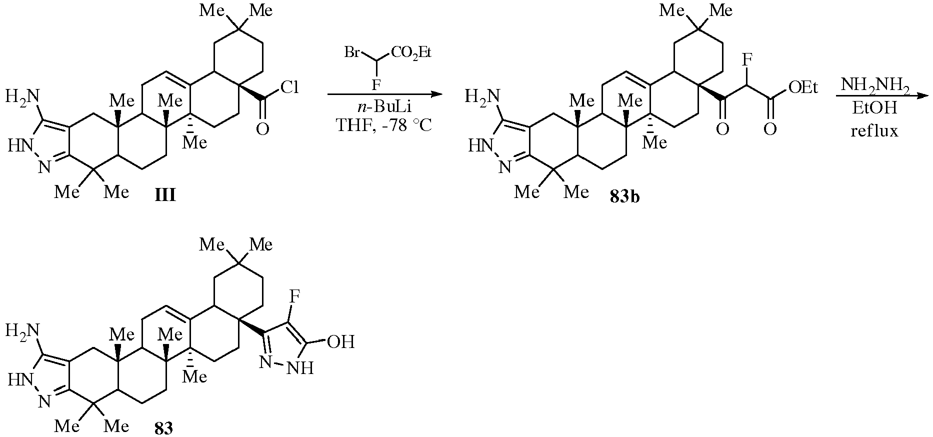

### (i) Preparation of 83b: Ethyl 3-((4aS,6aS,6bR,13aR)-12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-2-fluoro-3-oxopropanoate

To a solution of lithium bis(trimethylsilyl)amide (5.9 mL, 1 M in THF, 5.9 mmol) was added ethyl 2-bromo-2-fluoroacetate (0.46 mL, 3.9 mmol) at -78°C. The mixture was stirred for 10 min. The anion solution was added to a solution of **III** (200 mg, 0.39 mmol) in THF (4 mL) precooled to -78°C. The mixture was stirred at -78°C for 1 hour and the reaction was deemed incomplete. To a second solution of *n*-butyllithium (1.8 mL, 2.5 M in hexanes, 5.9 mmol) was added ethyl 2-bromo-2-fluoroacetate (0.46 mL, 3.9 mmol) at -78°C. The second anion mixture was stirred for 10 min. The anion solution was added to the above reaction mixture at -78°C. The mixture was stirred at -78°C for 1 hour and warmed to -20°C over 1 hour. The reaction mixture was quenched with saturated NH₄Cl (20 mL) and extracted with EtOAc (100 mL). The organic phase was washed with brine, dried (MgSO₄), filtered and concentrated to dryness. The residue was purified by column chromatography (silica, 0-30% CMA in CH₂Cl₂) to afford the sub-title compound (110 mg) as a brown solid.
APCI MS (Positive Mode) *m*/*z* 582 [C₃₅H₅₂FN₃O₃ + H]⁺.

### (ii) Preparation of 83: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-4-fluoro-1H-pyrazol-5-ol

A mixture of **83b** (110 mg) and hydrazine (0.05 mL) in EtOH (2 mL) was heated at reflux overnight. The reaction mixture was concentrated to dryness under reduced pressure. The residue was purified by column chromatography (silica, 0-50% CMA in CH₂Cl₂) followed by preparative HPLC to afford the title compound (9 mg, 10%) as an off-white solid.

*R_{f}* 0.50 (80:18:2 Methylene Chloride/Methanol/concentrated Ammonium Hydroxide). ¹H NMR (300 MHz, CD₃OD) δ 0.52 (s, 3H), 0.85 (s, 3H), 0.96 (s, 3H), 1.00 (s, 3H), 1.19 (s, 3H), 1.23 (s, 3H), 1.28 (s, 3H), 1.32-2.08 (m, 18H), 2.39 (m, 1H), 2.40 (d, *J* = 15.0 Hz, 1H), 2.90 (m, 1H), 5.42 (s, 1H). mp >300°C. APCI MS (Positive Mode) *m*/*z* 550 [C₃₃H₄₈FN₅O + H]⁺.

### Example 84

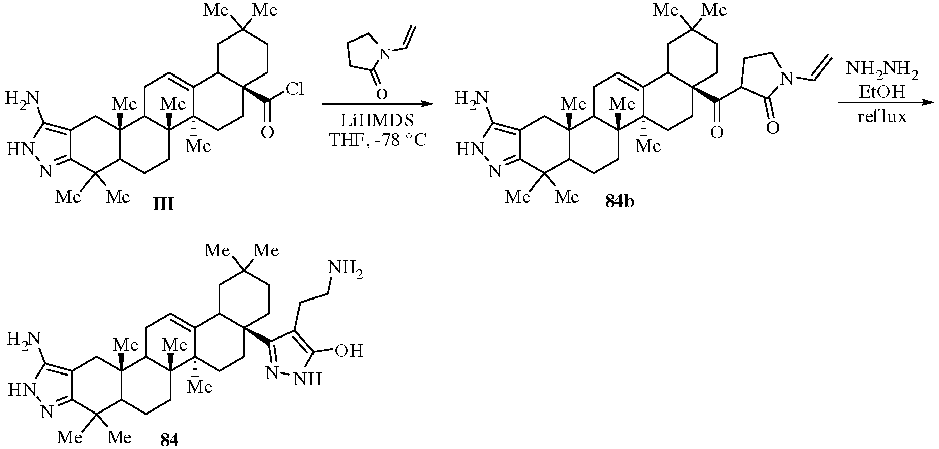

### (i) Preparation of 84b: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazole-4a-carbonyl)-1-vinylpyrrolidin-2-one

To a solution of lithium bis(trimethylsilyl)amide (38 mL, 1 M in THF, 38.0 mmol) was added 1-vinyl-2-pyrrolidinone (2.1 mL, 19.0 mmol) at-78°C. The mixture was stirred for 10 min. The anion solution was added to a solution of III (1.0 g, 1.9 mmol) in THF (20 mL) precooled to -78°C. The mixture was stirred at -78°C for 1 hour. The reaction mixture was quenched with saturated NH₄Cl (60 mL) and extracted with EtOAc (100 mL). The organic phase was washed with brine, dried (MgSO₄), filtered and concentrated to dryness. The residue was purified by column chromatography (silica, 0-10% MeOH in CH₂Cl₂) to afford the sub-title compound (350 mg) as a brown solid.
APCI MS (Positive Mode) *mlz* 587 [C₃₇H₅₄N₄O₂ + H]⁺.

### (ii) Preparation of 84: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-4-(2-aminoethyl)-1H-pyrazol-5-ol

A mixture of **84b** (100 mg) and hydrazine (0.05 mL) in EtOH (2 mL) was sealed and heated to 160°C by microwave for 3 hours. The reaction mixture was concentrated to dryness under reduced pressure. The residue was purified by column chromatography (silica, 0-80% CMA in CH₂Cl₂) to afford the title compound (50 mg, 30%) as a brown solid.
*R_{f}*0.27 (80:18:2 Methylene Chloride/Methanol/concentrated Ammonium Hydroxide). ¹H NMR (300 MHz, CD₃OD) δ 0.50 (s, 3H), 0.86 (s, 3H), 0.95 (s, 3H), 0.98 (s, 3H), 1.09 (s, 3H), 1.21 (s, 6H), 1.35-2.40 (m, 21 H), 2.72 (m, 2H), 2.96 (m, 2H), 5.47 (s, 1H).
mp 250-260°C dec. APCI MS (Positive Mode) *m*/*z* 575 [C₃₅H₅₄N₆O + H]⁺.

### Example 85 and Example 86

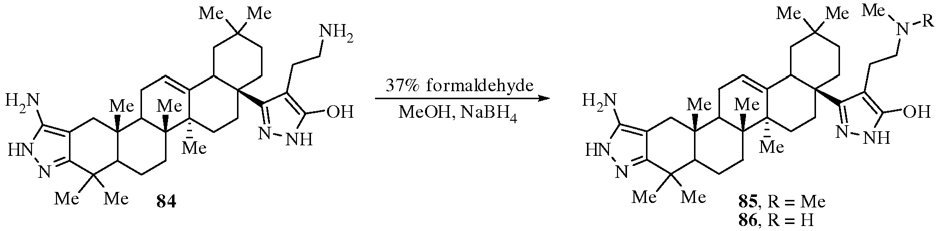

### (i) Preparation of 85 and 86: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-4-(2-(dimethylamino)ethyl)-1H-pyrazol-5-ol and 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-4-(2-(methylamino)ethyl)-1H-pyrazol-5-ol

To a solution of **84** (100 mg, 0.17 mmol) in MeOH (8 mL) was added 37% formaldehyde (0.021 mL, 0.36 mmol) in an ice bath. The mixture was stirred for 5 min. Sodium borohydride (32 mg, 0.84 mmol) was added. The reaction mixture was stirred at 0°C for 1 hour and concentrated to dryness. The residue was purified by column chromatography (silica, 0-80% CMA in CH₂Cl₂) to afford **85** (35 mg, 20%) as an off-white solid and **86** (18 mg, 20%) as an off-white solid.
Data for **85:**
   *R_{f}*0.35 (80:18:2 Methylene Chloride/Methanol/concentrated Ammonium Hydroxide).
   ¹H NMR (300 MHz, CD₃OD) δ 0.50 (s, 3H), 0.86 (s, 3H), 0.95 (s, 3H), 0.98 (s, 3H), 1.15 (s, 3H), 1.21 (s, 6H), 1.35-2.35 (m, 20H), 2.35 (s, 6H), 2.67 (m, 4H), 2.90 (m, 1 H), 5.48 (s, 1H). mp 260-270°C dec. APCI MS (Positive Mode) *m*/*z* 603 [C₃₇H₅₈N₆O + H]⁺.
Data for **86:**
   *R_{f}*0.30 (80:18:2 Methylene Chloride/Methanol/concentrated Ammonium Hydroxide).
   ¹H NMR (300 MHz, CD₃OD) δ 0.50 (s, 3H), 0.86 (s, 3H), 0.95 (s, 3H), 0.98 (s, 3H), 1.15 (s, 3H), 1.21 (s, 6H), 1.35-2.08 (m, 20H), 2.30 (s, 2H), 2.71 (m, 3H), 2.83 (m, 3H), 5.49 (s, 1H). mp 260-270°C dec. APCI MS (Positive Mode) *m*/*z* 589 [C₃₆H₅₆N₆O + H]⁺.

### Example 87

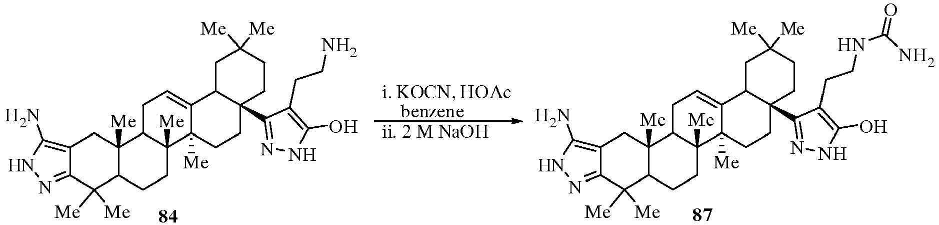

### (i) Preparation of 87:1-(2-(3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-5-hydroxy-1H-pyrazol-4-yl)ethyl)urea

To a solution of **84** (66 mg, 0.11 mmol) and acetic acid (0.2 mL) in THF (2 mL) and benzene (3 mL) was added potassium cyanate (22 mg, 0.26 mmol). The mixture was stirred at room temperature for 12 hours. Sodium hydroxide (2 mL, 2.0 M) was added. The reaction mixture was stirred at room temperature for 3 hours and concentrated to dryness. The residue was purified by column chromatography (silica, 0-10% MeOH in CMA) to afford the title compound (7 mg, 10%) as a brown solid.
*R_{f}*0.50 (80:28:2 Methylene Chloride/Methanol/concentrated Ammonium Hydroxide).
¹H NMR (300 MHz, CD₃OD) δ 0.51 (s, 3H), 0.87 (s, 3H), 0.96 (s, 3H), 1.00 (s, 3H), 1.12 (s, 3H), 1.28 (s, 3H), 1.32 (s, 3H), 1.35-2.12 (m, 20H), 2.28 (s, 1H), 2.30 (d, *J* = 15.0 Hz, 1 H), 2.60 (m, 2H), 2.85 (m, 1 H), 5.52 (s, 1 H). mp >300°C. APCI MS (Positive Mode) *m*/*z* 618 [C₃₆H₅₅N₇O₂ + H]⁺.

### Example 88

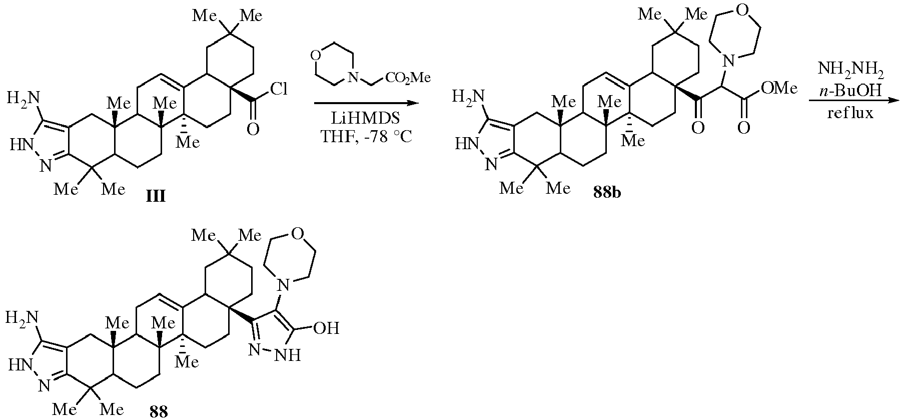

### (i) Preparation of 88b: Methyl 3-((4aS,6aS,6bR,8aR,13aR,15bS)-12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-2-morpholino-3-oxopropanoate

To a solution of lithium bis(trimethylsilyl)amide (15.6 mL, 1 M in THF, 15.6 mmol) was added methyl morpholinoacetate (1.8 g, 11.7 mmol) at -78°C. The mixture was stirred for 10 min. The anion solution was added to a solution of III (400 mg, 0.78 mmol) in THF (10 mL) precooled to -10°C. The mixture was stirred at -10°C for 1 hour. The reaction mixture was quenched with saturated NH₄Cl (60 mL) and extracted with EtOAc (100 mL). The organic phase was washed with brine, dried (MgSO₄), filtered and concentrated to dryness. The residue was purified by column chromatography (silica, 0-20% CMA in CH₂Cl₂) to afford the sub-title compound (120 mg) as a brown solid.
APCI MS (Positive Mode) *m*/*z* 635 [C₃₈H₅₈N₄O₄ + H]⁺.

### (ii) Preparation of 88: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-4-morpholino-1H-pyrazol-5-ol

A mixture of **88b** (120 mg) and hydrazine (0.05 mL) in *n*-BuOH (2 mL) was heated at reflux overnight. The reaction mixture was concentrated to dryness under reduced pressure. The residue was purified by column chromatography (silica, 0-30% CMA in CH₂Cl₂) to afford the the title compound (6 mg, 5%) as a brown solid.
*R_{f}*0.42 (80:18:2 Methylene Chloride/Methanol/concentrated Ammonium Hydroxide). ¹H NMR (300 MHz, CD₃OD) δ 0.53 (s, 3H), 0.84 (s, 3H), 0.94 (s, 3H), 1.12 (s, 3H), 1.21 (s, 3H), 1.23 (s, 6H), 1.32-2.37 (m, 24H), 2.53 (m, 1 H), 3.68 (m, 4H), 5.42 (s, 1H). mp >300°C. ESI MS (Positive Mode) *m*/*z* 617 [C₃₇H₅₆N₆O₂ + H]⁺.

### Example 89

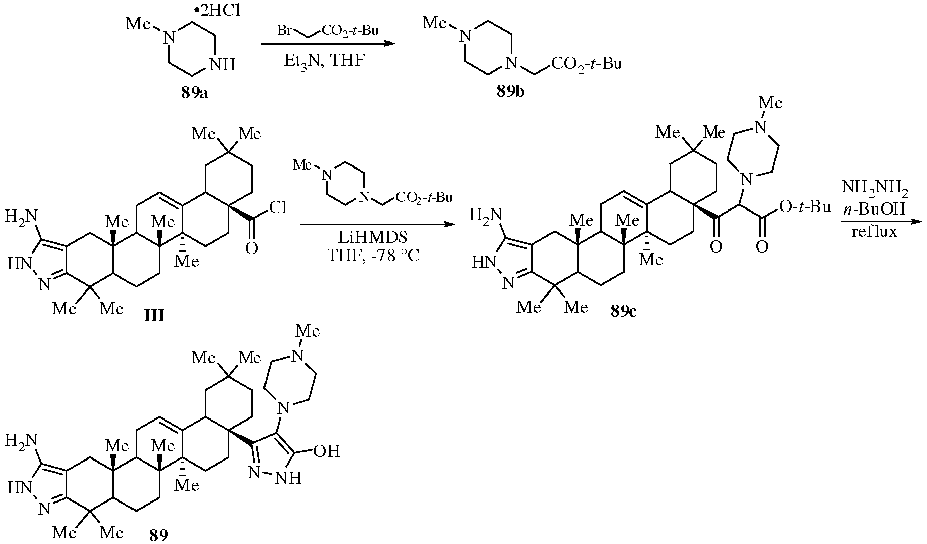

### (i) Preparation of 89b: tert-Butyl 2-(4-methylpiperazin-1-yl)acetate

To a suspension of 1-methylpiperazine dihydrochloride (8.5 g, 49.2 mmol) and triethylamine (22.8 mL, 164.0 mmol) in THF (150 mL) was added *tert*-butyl 2-bromoacetate (8.0 g, 41.0 mmol). The mixture was stirred at room temperature for 12 hours. The reaction mixture was diluted with EtOAc (200 mL) and H₂O (100 mL). The organic phase was washed with brine, dried (MgSO₄), filtered and concentrated to dryness. The residue was purified by column chromatography (silica, 0-10% MeOH in CH₂Cl₂) to afford the sub-title compound (6.0 g, 69%).
¹H NMR (400 MHz, CDCl₃) δ 1.42 (s, 9H), 2.30 (s, 3H), 2.41-2.62 (m, 8H), 3.10 (s, 2H).

### (ii) Preparation of 89c: tert-Butyl 3-((4aS,6aS,6bR,13aR)-12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-2-(4-methylpiperazin-1-yl)-3-oxopropanoate

To a solution of lithium bis(trimethylsilyl)amide (15.6 mL, 1 M in THF, 15.6 mmol) was added **89b** (2.5 g, 11.7 mmol) at -78°C. The mixture was stirred for 10 min. The anion solution was added to a solution of **III** (400 mg, 0.78 mmol) in THF (10 mL) precooled to -10°C. The mixture was stirred at 0°C for 1.5 hours. The reaction mixture was quenched with saturated NH₄Cl (60 mL) and extracted with EtOAc (100 mL). The organic phase was washed with brine, dried (MgSO₄), filtered and concentrated to dryness. The residue was purified by column chromatography (silica, 0-20% CMA in CH₂Cl₂) to afford the sub-title compound (200 mg) as a brown solid.
APCI MS (Positive Mode) *m*/*z* 690 [C₄₂H₆₇N₅O₃ + H]⁺.

### (ii) Preparation of 89: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-4-(4-methylpiperazin-1-yl)-1H-pyrazol-5-ol

A mixture of **89c** (100 mg) and hydrazine (0.02 mL) in *n*-BuOH (2 mL) was heated at reflux overnight. The reaction mixture was concentrated to dryness under reduced pressure. The residue was purified by column chromatography (silica, 0-40% CMA in CH₂Cl₂) to afford the title compound (16 mg, 18%) as a brown solid.
*R_{f}*0.70 (80:18:2 Methylene Chloride/Methanol/concentrated Ammonium Hydroxide).
¹H NMR (400 MHz, CD₃OD) δ 0.54 (s, 3H), 0.84 (s, 3H), 0.91 (s, 3H), 0.95 (s, 3H), 1.01 (s, 3H), 1.10 (s, 3H), 1.11-2.06 (m, 16H), 2.20 (m, 1H), 2.41 (m, 1H), 2.84 (s, 3H), 2.91-3.70 (m, 14H), 5.45 (s, 1H). mp >300°C. APCI MS (Positive Mode) *m*/*z* 630 [C₃₈H₅₉N₇O + H]⁺.

### Example 90

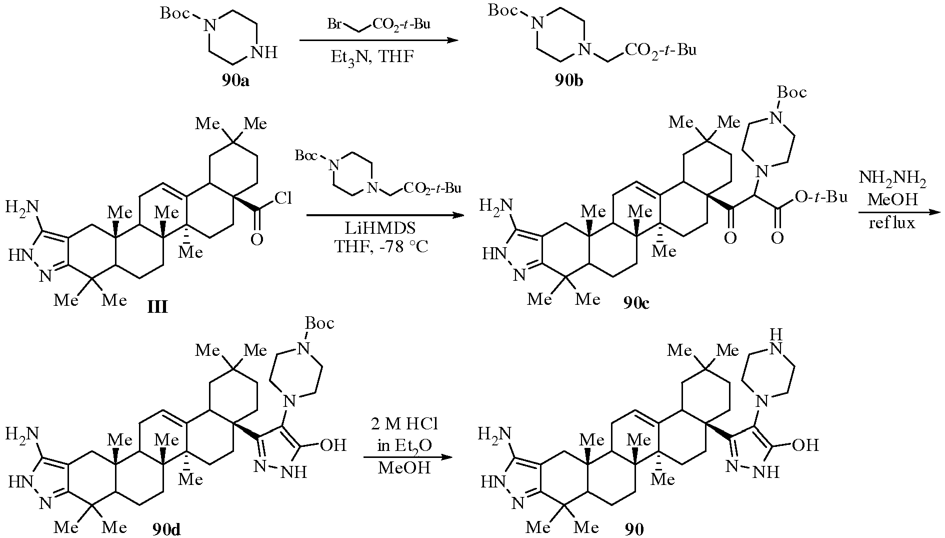

### (i) Preparation of 90b: tert-Butyl 4-(2-tert-butoxy-2-oxoethyl)piperazine-1-carboxylate

To a suspension of *tert-*butyl piperazine-1-carboxylate (3.0 g, 16.1 mmol) and triethylamine (4.5 mL, 32.2 mmol) in THF (80 mL) was added *tert*-butyl 2-bromoacetate (2.4 mL, 16.1 mmol). The mixture was stirred at room temperature for 12 hours. The reaction mixture was diluted with EtOAc (200 mL) and H₂O (100 mL). The organic phase was washed with brine, dried (MgSO₄), filtered and concentrated to dryness. The residue was purified by column chromatography (silica, 0-5% MeOH in CH₂Cl₂) to afford the sub-title compound (4.2 g, 87%).
¹H NMR (400 MHz, CDCl₃) δ 1.42 (s, 18H), 2.50 (m, 4H), 3.10 (s, 2H), 3.47 (m, 4H).

### (ii) Preparation of 90c: tert-Butyl 4-(1-((4aS,6aS,6bR,13aR)-12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-3-tert-butoxy-1,3-dioxopropan-2-yl)piperazine-1-carboxylate

To a solution of lithium bis(trimethylsilyl)amide (19.4 mL, 1 M in THF, 19.4 mmol) was added **90b** (3.5 g, 11.7 mmol) at -78°C. The mixture was stirred for 10 min. The anion solution was added to a solution of **III** (500 mg, 0.97 mmol) in THF (10 mL) precooled to -78°C. The mixture was stirred at 0°C for 1.5 hours. The reaction mixture was quenched with saturated NH₄Cl (60 mL) and extracted with EtOAc (100 mL). The organic phase was washed brine, dried (MgSO₄), filtered and concentrated to dryness. The residue was purified by column chromatography (silica, 0-20% CMA in CH₂Cl₂) to afford the sub-title compound (237 mg) as a brown solid.
ESI MS (Positive Mode) *mlz* 776 [C₄₆H₇₃N₅O₅ + H]⁺.

### (iii) Preparation of 90d: tert-Butyl 4-(3-((4aS,6aS,6bR,13aR)-12-amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-5-hydroxy-1H-pyrazol-4-yl)piperazine-1-carboxylate

A mixture of **90c** (237 mg) and hydrazine (0.059 mL) in *n*-BuOH (5 mL) was heated at reflux overnight. The reaction mixture was concentrated to dryness under reduced pressure. The residue was purified by column chromatography (silica, 0-40% CMA in CH₂Cl₂) to afford the sub-title compound (15 mg, 7%) as a brown solid.

### (iv) Preparation of 90: 3-((4aS,6aS,6bR,13aR)-12-Amino-2,2,6a,6b,9,9,13a-heptamethyl-2,3,4,4a,5,6,6a,6b,7,8,8a,9,11,13,13a,13b,14,15b-octadecahydro-1H-chryseno[1,2-f]indazol-4a-yl)-4-(piperazin-1-yl)-1H-pyrazol-5-ol

To a solution of **90d** (15 mg) in MeOH (2 mL) was added HCl (0.8 mL, 2 M in diethyl ether) and stirred at room temperature for 5 hours. The reaction mixture was concentrated and purified by preparative HPLC to provide the title compound (4.5 mg, 30%) as an off-white solid.
*R_{f}*0.12 (80:18:2 Methylene Chloride/Methanol/concentrated Ammonium Hydroxide). ¹H NMR (400 MHz, CD₃OD) δ 0.55 (s, 3H), 0.86 (s, 3H), 0.89 (s, 3H), 0.91 (s, 3H), 1.14 (s, 3H), 1.19 (s, 3H), 1.28 (s, 3H), 1.30-2.06 (m, 23H), 2.20 (m, 1 H), 2.41 (d, *J* = 15.0 Hz, 1 H), 2.85-3.65 (m, 4H), 5.45 (s, 1H). mp >300°C. APCI MS (Positive Mode) *m*/*z* 616 [C₃₇H₅₇N₇O + H]⁺.

### Example 91

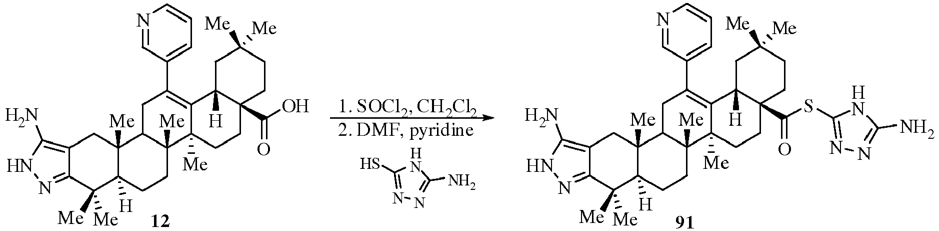

### (i) Preparation of 91: (4aS,6aS,6bR,8aR,13aR,15bS)-S-5-Amino-4H-1,2,4-triazol-3-yl 12-amino-2,2,6a,6b,9,9,13a-heptamethyl-15-(pyridin-3-yl)-2,3,4,4a,5,6,6a,6b,7,8,8a,9,11,13,13a,13b,14,15b-octadecahydro-1H-chryseno[1,2-f]indazole-4a-carbothioate

To a solution of 12 (150 mg, 0.26 mmol) in CH₂Cl₂ (10 mL) was added thionyl chloride (0.19 mL, 2.6 mmol). The mixture was stirred at room temperature for 2 hours. The reaction mixture was concentrated to dryness under reduced pressure. The residue was dissolved in pyridine (10 mL) and DMF (2 mL). 5-Amino-4*H-*1,2,4-triazole-3-thiol (71 mg, 0.60 mmol) was added and stirred at room temperature for 1 hour. The reaction mixture was concentrated to dryness under reduced pressure. The residue was purified by column chromatography (silica, 0-50% CMA in CH₂Cl₂) followed by preparative HPLC to afford the title compound (19 mg, 14%) as an off-white solid.
*R_{f}*0.20 (80:18:2 Methylene Chloride/Methanol/concentrated Ammonium Hydroxide). ¹H NMR (300 MHz, CD₃OD) δ 0.30 (s, 3H), 0.82 (s, 3H), 0.90 (s, 3H), 0.98 (s, 3H), 1.22 (s, 3H), 1.32 (s, 3H), 1.34 (s,3H), 1.35-2.30 (m, 19H), 2.35 (d, *J* = 15.0 Hz, 1H), 2.75 (m, 1H), 7.91 (dd, *J* = 5.7, 7.5 Hz, 1 H), 8.40 (d, *J* = 7.8 Hz, 1 H), 8.68 (d, *J* = 4.8 Hz, 1H), 8.79 (s, 1H). mp >300°C. ESI MS (Positive Mode) *m*/*z* 669 [C₃₈H₅₂N₈OS + H]⁺.

### Example 92

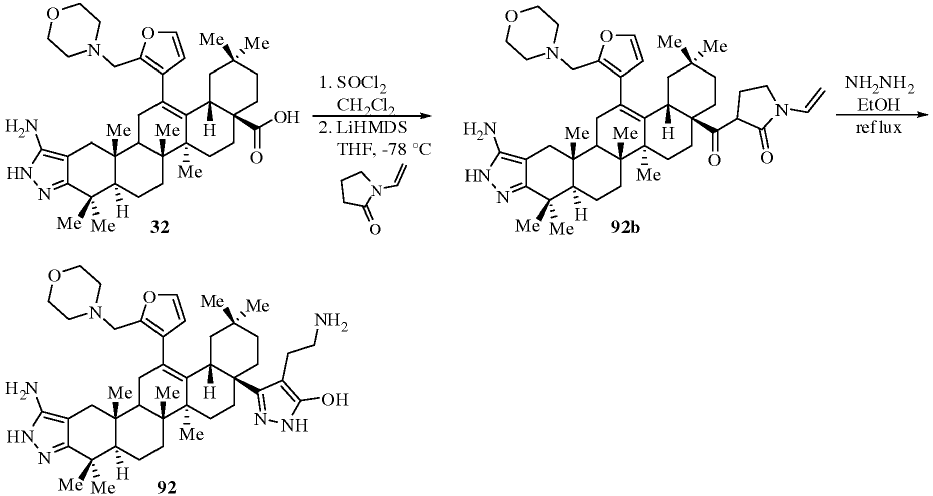

### (i) Preparation of 92b: 3-((4aS,6aS,6bR,8aR,13aR,15bS)-12-Amino-2,2,6a,6b,9,9,13a-heptamethyl-15-(2-(morpholinomethyl)furan-3-yl)-2,3,4,4a,5,6,6a,6b,7,8,8a,9,11,13,13a,13b,14,15b-octadecahydro-1H-chryseno[1,2-f]indazole-4a-carbonyl)-1-vinylpyrrolidin-2-one

To a solution of **32** (250 mg, 0.37 mmol) in CH₂Cl₂ (10 mL) was added thionyl chloride (0.27 mL, 3.7 mmol). The mixture was stirred at room temperature for 2 hours. The reaction mixture was concentrated to dryness under reduced pressure. The residue was dissolved in THF (5 mL) and added to a solution of 1-vinylpyrrolidin-2-one (0.39 mL, 3.7 mmol) and lithium bis(trimethylsilyl)amide (7.4 mL, 1 M in THF, 7.4 mmol) in THF (5 mL) at -78°C. The reaction mixture was stirred at -78°C for 1 hour. The reaction mixture was quenched with saturated NH₄Cl (10 mL) and extracted with EtOAc (100 mL x 2). The organic phase was washed with brine then dried (MgSO₄), filtered and concentrated to dryness under reduced pressure. The residue was purified by column chromatography (silica, 0-10% MeOH in CH₂Cl₂) to afford the sub-title compound (110 mg, 40%).
¹H NMR (300 MHz, CDCl₃) δ 0.51 (s, 3H), 0.81 (s, 3H), 0.87 (s, 6H), 1.10 (s, 3H), 1.18 (s, 3H), 1.20 (s, 3H), 1.21-2.60 (m, 29H), 2.95 (m, 1H), 3.35-3.75 (m, 8H), 4.05 (m, 1 H), 4.45 (m, 2H), 6.40 (m, 1 H), 7.01 (m, 1 H), 7.37 (m, 1 H).

### (ii) Preparation of 92: 3-((4aS,6aS,6bR,8aR,13aR,15bS)-12-Amino-2,2,6a,6b,9,9,13a-heptamethyl-15-(2-(morpholinomethyl)furan-3-yl)-2,3,4,4a,5,6,6a,6b,7,8,8a,9,11,13,13a,13b,14,15b-octadecahydro-1H-chryseno[1,2-f]indazol-4a-yl)-4-(2-aminoethyl)-1H-pyrazol-5-ol

A mixture of **92b** (110 mg, 0.14 mmol) and hydrazine (0.5 mL) in EtOH (2 mL) was sealed and heated to 160 °C by microwave for 3 hours. The reaction mixture was concentrated to dryness. The residue was purified by column chromatography (silica, 0-70% CMA in CH₂Cl₂) to afford the title compound (25 mg, 25%).
*R_{f}*0.54 (80:28:2 Methylene Chloride/Methanol/concentrated Ammonium Hydroxide).
¹H NMR (300 MHz, CD₃OD) δ 0.60 (s, 6H), 0.84 (s, 3H), 0.86 (s, 3H), 1.12 (s, 3H), 1.22 (s, 3H), 1.28 (s, 3H), 1.35-2.0 (m, 19H), 2.18 (m, 2H), 2.27 (d, *J* = 15.0 Hz, 2H), 2.61-2.72 (m, 6H), 2.97 (m, 2 H), 3.50-3.70 (m, 7H), 6.41 (s, 1H), 7.51 (s, 1H). mp 238-240°C. APCI MS (Positive Mode) *m*/*z* 740 [C₄₄H₆₅N₇O₃ + H]⁺.

### EXAMPLE IV

Table 6, below, lists the inhibitory concentrations of select compounds and antibiotics in the biofilm growth assay against various gram-negative bacterial biofilms. "N" means the number of isolates of bacteria tested. The biofilm growth assay procedure detailed in Example I, above, was followed. *Burkholderia cepacia* used media consisting of M9 salts, 100 µM CaCl2, 1 mM MgS04, and 0.7% citrate in 0.4% Noble agar. *Salmonella* spp. used media consisting of Nutrient Broth with 0.5% glucose in 0.5% Noble agar. Compound 1 of the invention is the most active compound in Table 6. Compound 1 is superior to tobramycin, ceftazidime, and azithromycin in the biofilm growth assay.

A successful research lead optimization strategy requires examining multiple clinical isolates in parallel based upon the inherent risks of antibacterial development and heterogeneity of biofilms. Past medicinal chemistry efforts focused on identifying superior analogs of approved classes of antibiotics have typically demonstrated that closely related analogs can exhibit varying degrees of antibacterial activities against different isolates and species of bacteria in an unpredictable trend. Hence, accurately selecting the next set of synthetic targets requires microbiological activities from a broad group of bacterial isolates and species of bacteria that would be encountered in clinical and community settings. In addition, significant variability of biofilm formation among different clinical isolates of *P. aeruginosa* has been shown to exist. Therefore, the lead optimization of a biofilm inhibitor may be detrimentally misguided if an unrepresentative group of clinical isolates not exhibiting these different biofilms are used to generate structure activity relationships.

**Table 6.**

| **Compound** | **P. aeruginosa (n=10)** | **E. coli (n=10)** | **B. cepacia (n=10)** | **Salmonella spp. (n=6)** |
|---|---|---|---|---|
| **Oleanolic Acid** | 8 | 16 | 0.5 | > 64 |
| **C644** | 2 | > 16 | 0.5 | 16 |
| **C649** | 16 | > 16 | 1 | > 16 |
| **Compound 1** | 1 | 1 | 0.25 | 1 |
| **Tobramycin** | 4 | 16 | > 64 | 8 |
| **Ceftazidime** | 4 | 0.25 | > 8 | 0.5 |
| **Azithromycin** | > 16 | - | > 16 | > 16 |

### EXAMPLE V

The bioavailabilities of certain compounds were examined in mice. Administration of the compounds was performed orally and by intraperitoneal (IP) injection using a vehicle as known to those skilled in the art. Many vehicles can be used to examine bioavailability. Prior to administration, each vehicle was optimized based on compound solubility according to the formulation research conducted by Uckun et al. (Arznelmittel-Forschung (Drug Research) 2007; 57(4):218-226). Based upon this publication, vehicles containing approximate ratios of 2:1:1 of propylene glycol:Tween20:PEG400 (Tween20 being a common emulsier used in formulations and food products) and less than 5% ethanol upon administration exhibit good solubility properties and increase serum bioavailability. During these experiments, PEG400 demonstrated a critical role in serum bioavailability. The concentration of PEG400 is modulated depending upon solubility of the compounds and the amount of aqueous phase added (0.02 M citrate and 0.9% NaCl).

Compounds 1, 12, 64, and 68 demonstrated good bioavailability when administered orally or via IP injection at approximately 20 mg/kg to 50 mg/kg exceeding approximately 5 µg/ml in the serum of mice at 30 minutes or 1 hour after administration This example demonstrates that the compounds of the invention can be formulated into tablets, capsules, suppositories, and sterile liquids for parenteral administration as known to those skilled in the art.

### EXAMPLE VI

The biofilm growth assay as described in Example I, as amended by the details that follow, was performed using *Pseudomonas syringae, Xanthomonas campestris,* and *Pectobacterium atrosepticum* (gram-negative bacterial plant pathogens). A freezer stock of each plant pathogen was grown separately overnight on 1.5% agar plates containing TSB at 30°C. The next day a pipette tip was used to inoculate a round plate composed of M8 salts and 0.7% glucose on 0.5% Noble agar from the overnight TSB plates. Plates were allowed to incubate at 30°C for approximately 24 to 48 hours. Compound 1 of the invention was examined against these plant pathogens and found to inhibit the spreading biofilms at 0.03 µg/ml against *Pseudomonas syringae,* 0.5 µg/ml against *Xanthomonas campestris,* and 0.06 µg/ml against *Pectobacterium atrosepticum.* These data demonstrate that the compounds of the invention inhibit spreading biofilms of plant pathogens.

### EXAMPLE VII

INHIBITION OF THE GROWTH OF PREFORMED BIOFILMS. The Biofilm Growth Assay described in Example I was performed in round plates with the compounds of the invention only in agar on half of the plate. The agar not containing compound was inoculated with bacteria and the formed biofilm moved toward the agar containing compound on the other half of the round plate. Once the spreading biofilm reached the agar containing compound, the biofilm was inhibited from moving or spreading onto the agar with compound. This Biofilm Growth Assay performed on these agar plates demonstrates that the compounds of the invention inhibit the growth of preformed biofilms.

Approximately 0.5% agar not containing a compound of the invention was poured and allowed to dry. Agar on half of the plate was removed and then agar containing a compound of the invention was poured on the empty half of the plate and allowed to dry. Bacteria is inoculated onto the agar not containing compound and allowed in incubate overnight as described in Example I. During incubation bacteria spread as a biofilm until they reach the agar containing compound. The compounds of the invention inhibit the spreading biofilm at the same concentrations at shown in Examples I, II, and III, above.

### EXAMPLE VIII

INHIBITION OF THE GROWTH OF PREFORMED BIOFILMS. The Biofilm Growth Assay as described in Example VII was performed in round plates with the compounds of the invention in agar on half of the plate and antibiotic disks placed onto the agar containing the compounds of the invention. This assay demonstrates that the compounds of the invention are synergistic with antibiotics like tobramycin and colistin at inhibiting spreading biofilms across the agar.

The Biofilm Growth Assay was performed as described in Example VIII. 2 to 4 antibiotic disks were placed onto the agar containing a compound of the invention in a line parallel to the line that separates the two agars in one round plate. Appropriate negative and positive control plates were performed as known to those skilled in the art. This assay demonstrates that the compounds of the invention in combination with antibiotics inhibit spreading biofilms at approximately 4 times less the concentration than when performed alone as described in Example I.

### EXAMPLE IX

### EVALUATION OF SALTS

Evaluation of Compound 62 (Table 4) salt formation was performed. HCl, HBr, H₃PO₄, p-toluenesulfonic acid, benzenesulfonic acid, methanesulfonic acid, L-tartaric acid, acetic acid, xinafoic acid, L-lactic acid, benzoic acid, adipic acid, oxalic acid, pamoic acid, maleic acid, and laurylsulfuric acid indicated crystallinity. p-toluenesulfonic acid (mono) and H₃PO₄ (bis) provided the most desirable physical properties compared to freebase. As stated herein, the invention also includes compounds and their salts.

Evaluation of Compound 64 (Table 4) salt formation was performed. HCl (mono and bis), p-TSA (bis), BSA (bis), MSA (bis), H₂SO₄ (mono), and H₃PO₄ (mono) provided salts with crystallinity. Bis-MSA and mono-H₃PO₄ increased aqueous solubility compared to freebase. As stated herein, the invention also includes compounds and their salts.

### EXAMPLE X

A topical gel containing 2% by weight of the compound of the invention with azithromycin for use in treating skin infections can be prepared.

0.25 gram of the compound of the invention is dissolved in 6.75 grams of ethanol. 0.2 grams of azithromycin is dissolved in this solution. 0.25 grams of hydroxypropyl methylcellulose is added with gentle stirring until a homogenous solution is obtained. 4.8 grams of water is then added with gentle shaking.

A formulation without antibiotic can also be prepared using this same procedure.

### EXAMPLE XI

### PHARMACEUTICAL FORMULATION FOR NEBULIZATION OF A COMPOUND OF THE INVENTION

Solutions were prepared comprising 2 mg/ml and 10 mg/ml of the compound of the invention in ethanol/propylene glycol/water (85:10:5). These solutions were nebulized separately by a ProNeb Ultra nebulizer manufactured by PARI. The nebulized solutions were collected in a cold trap, processed appropriately, and were detected by mass spectrometry. The compounds is recovered from both formulations and these latters aren suitable for the treatment or prophylaxis of lung infections.

### EXAMPLE XII

The compounds shown in the table below were prepared semi-synthetically and tested in the biofilm growth assay as described herein. These compounds with substitutions at R¹ and R² did not inhibit the biofilms of *P. aeruginosa* or *E*. *coli* at 1 or 2 µg/ml sufficiently to warrant further investigation relative to the compounds noted above.

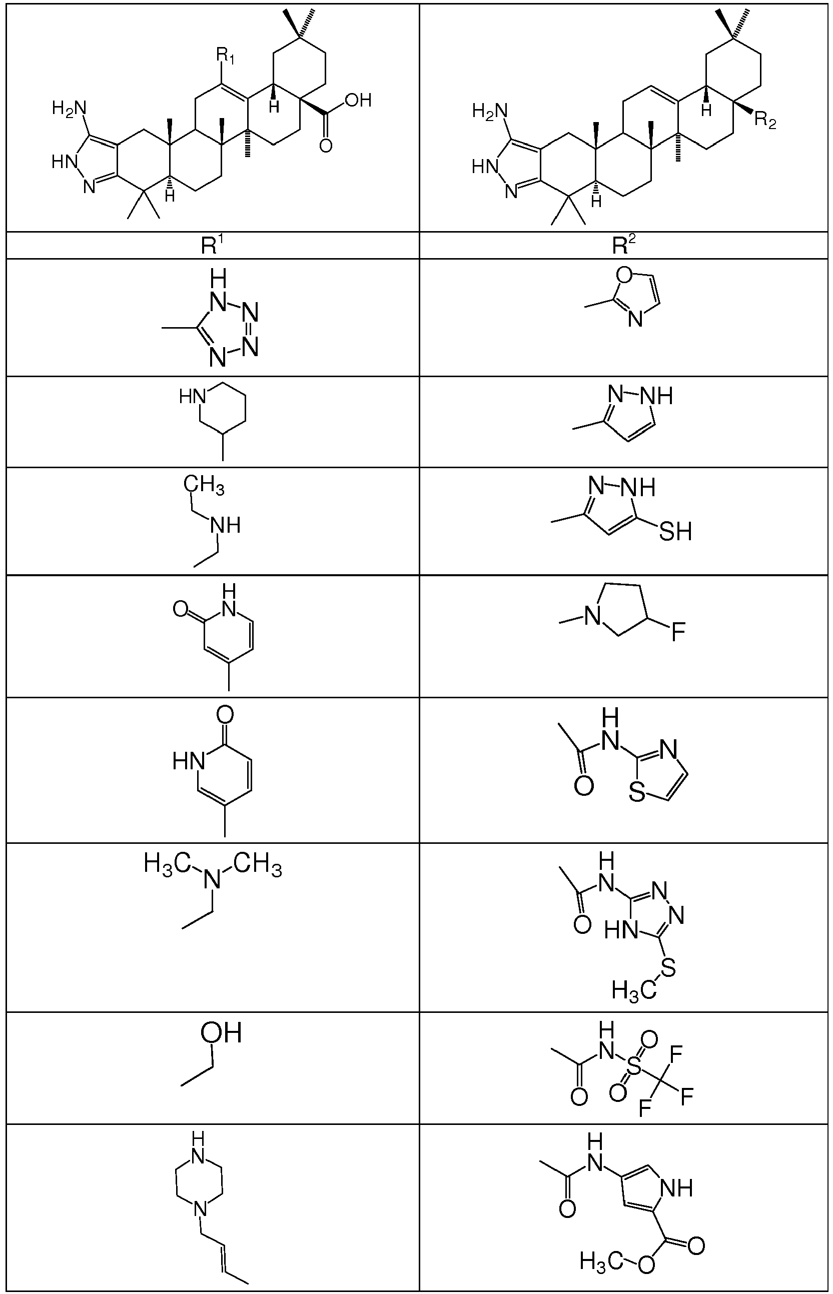

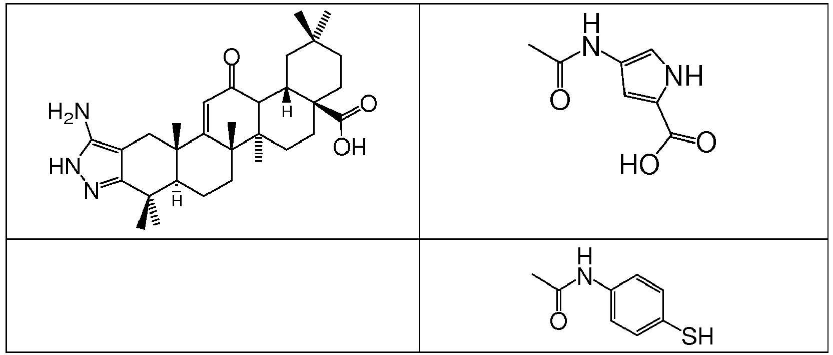

## Claims

1. A compound corresponding to the following chemical structure: 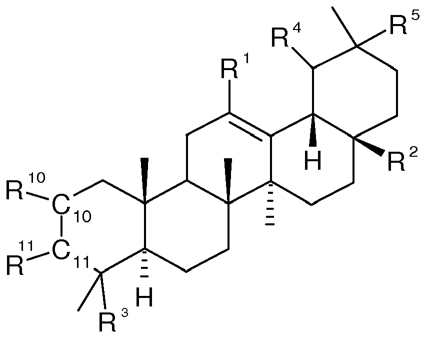
wherein R¹⁰ and R¹¹ together with the carbon atoms to which they are attached form 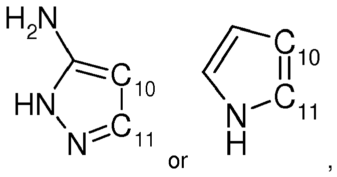
and when R¹⁰ and R¹¹ together with the carbon atoms to which they are attached
form 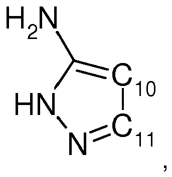
R¹ is selected from the group consisting of hydrogen, methyl, halide, C₁-C₅ alkyl wherein one or more hydrogen atoms attached to a member atom within the C₁-C₅ alkyl group is replaced with 1, 2, 3 or 4 halide atoms, nitrile, C₁-C₅ alkyl or C₂-C₅ alkenyl with one nitrile group replacing one terminal carbon atom in the unbranched or branched chain, substituted or not C₁-C₅ alkyl, substituted or not C₂-C₅ alkenyl, substituted or not C₂-C₅ alkynyl, cyclopropyl, cyclobutyl, cyclopentyl, cyclohexyl, norbornane, adamantine, C₅-C₆ cycloalkenyl, substituted or not C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together; wherein R² is selected from the group consisting of carboxyl, amide, hydroxyamide, methylamide, -CH₂N(CH₃)₂, -CH₂NR⁶R⁷, 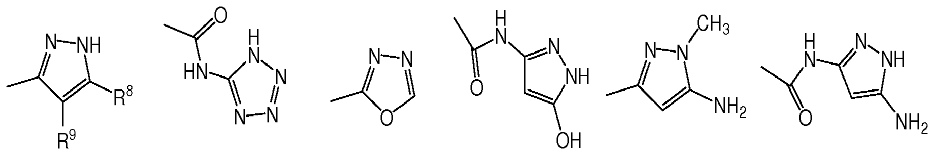 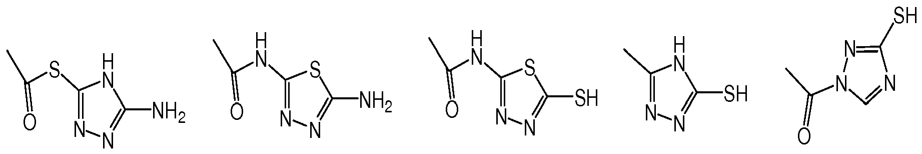 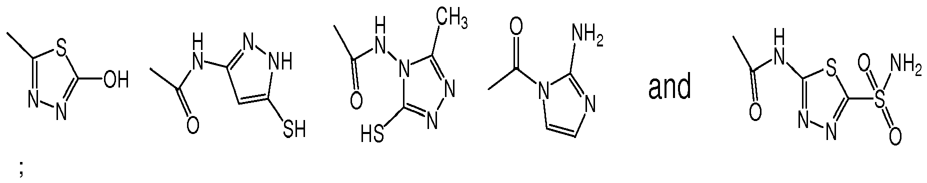
R³ is selected from the group consisting of hydrogen and methyl; one of R⁴ and R⁵ is hydrogen and the other is methyl; R⁶ and R⁷ are independently selected from the group consisting of hydrogen, methyl, ethyl, propyl, isopropyl, and butyl; wherein R⁸ is selected from the group consisting of hydroxyl, amino, -N(CH₃)₂, and -NHCH₃; and wherein R⁹ is selected from the group consisting of hydrogen, halide, C₁-C₅ alkyl, C₂-C₅ alkenyl, C₂-C₅ alkynyl, morpholinyl, piperazinyl, C₁-C₅ alkyl piperazinyl, heterocycloalkyl, cyclopropyl, cyclobutyl, cyclopentyl, cyclohexyl, norbornane, adamantine, C₅-C₆ cycloalkenyl; and C₁-C₅ alkyl, C₂-C₅ alkenyl, and C₂-C₅ alkynyl optionally substituted with moieties selected from the group consisting of hydroxyl, amino, C₁-C₅ alkyl or C₂-C₅ alkenyl with one nitrogen atom replacing one terminal carbon atom in the unbranched or branched chain, halide, C₁-C₅ alkyl with one carbon atom replaced with an oxygen atom, C₁-C₅ alkyl with a carbonylamino or aminocarbonyl replacing two carbon atoms in the unbranched or branched chain, carboxyl, amide, hydroxyamide, -CONHCH₃, -NHCONH₂, -SO₂NH₂, -SO₂CH₃,-NHCOCH₃, -NHCSNH₂, -NHSO₂CH₃, and a radical of formula -OR¹² or a radical of formula -C(O)-R¹² wherein R¹² is selected from the group consisting of C₁-C₅ alkyl, C₂-C₅ alkenyl, cyclopropyl, cyclobutyl, cyclopentyl, cyclohexyl, norbornane, adamantine, and C₅-C₆ cycloalkenyl; and pharmaceutically acceptable salts thereof,
or
when R¹⁰ and R¹¹ together with the carbon atoms to which they are attached form 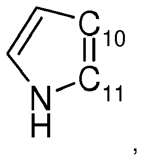
R¹ is selected from the group consisting of hydrogen, methyl, halide, nitrile, substituted or not C₁-C₅ alkyl, substituted or not C₂-C₅ alkenyl, substituted or not C₂-C₅ alkynyl, substituted or not C₂-C₅ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together; R² is a carboxyl; R³ is selected from the group consisting of hydrogen and methyl; and one of R⁴ and R⁵ is hydrogen and the other is methyl; and pharmaceutically acceptable salts thereof.

2. A compound according to claim 1 wherein the compound corresponds to the following chemical structure 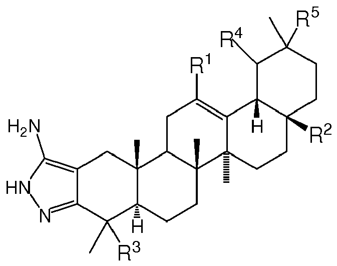 wherein R¹ is selected from the group consisting of methyl, halide, C₁-C₅ alkyl wherein one or more hydrogen atoms attached to a member atom within the C₁-C₅ alkyl group is replaced with 1, 2, 3 or 4 halide atoms, nitrile, C₁-C₅ alkyl or C₂-C₅ alkenyl with one nitrile group replacing one terminal carbon atom in the unbranched or branched chain, substituted or not C₁-C₅ alkyl, substituted or not C₂-C₅ alkenyl, substituted or not C₂-C₅ alkynyl, cyclopropyl, cyclobutyl, cyclopentyl, cyclohexyl, norbornane, adamantine, C₅-C₆ cycloalkenyl, substituted or not C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl, pyrrolopyridinyl, and tetrahydropyrrolopyridinyl optionally substituted from moieties selected from the group consisting of C₁-C₅ alkyl, halide, C₁-C₅ alkyl wherein one or more hydrogen atoms attached to a member atom within the C₁-C₅ alkyl group is replaced with 1, 2, 3 or 4 halide atoms, hydroxyl, amino, C₁-C₅ alkyl or C₂-C₅ alkenyl with one nitrogen atom replacing one terminal carbon atom in the unbranched or branched chain, C₁-C₅ alkyl or C₂-C₅ alkenyl wherein one or more hydrogen atoms attached to a member atom within the C₁-C₅ alkyl or C₂-C₅ alkenyl group is replaced with one or more hydroxyls, nitrile, C₁-C₅ alkyl or C₂-C₅ alkenyl with one nitrile group replacing one terminal carbon atom in the unbranched or branched chain, a radical of formula R'OR" wherein R' is C₁-C₅ alkyl, C₂-C₅ alkenyl, or C₂-C₅ alkynyl and R" is C₁-C₅ alkyl, C₂-C₅ alkenyl, C₂-C₅ alkynyl, C₆-C₁₂ aryl, pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together or heterocycloalkyl; a radical of formula -OR¹² or a radical of formula -C(O)-R¹² wherein R² is selected from the group consisting of C₁-C₅ alkyl, C₂-C₅ alkenyl, cyclopropyl, cyclobutyl, cyclopentyl, cyclohexyl, norbornane, adamantine, and C₅-C₆ cycloalkenyl; C₁-C₅ alkyl with one carbon atom replaced with an oxygen atom, C₁-C₅alkyl with a carbonylamino or aminocarbonyl replacing two carbon atoms in the unbranched or branched chain, and thioalkyl.

3. A compound according to claim 1 wherein the compound corresponds to the following chemical structure: 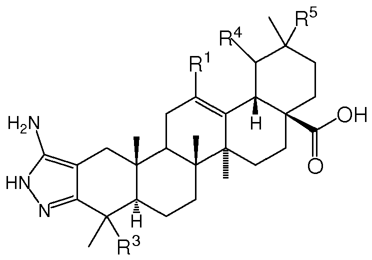 wherein R¹ is selected from the group consisting of hydrogen, halide, substituted or not C₁-C₅ alkyl, substituted or not C₂-C₅ alkenyl, cyclopropyl, cyclobutyl, cyclopentyl, cyclohexyl, norbornane, adamantine, C₅-C₆ cycloalkenyl, substituted or not C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together, preferably R¹ is hydrogen.

4. A compound according to claim 3 wherein R¹ is selected from the group consisting of halide, substituted or not C₁-C₅ alkyl, substituted or not C₂-C₅ alkenyl, cyclopropyl, cyclobutyl, cyclopentyl, cyclohexyl, norbornane, adamantine, C₅-C₆ cycloalkenyl, substituted or not C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together, preferably R¹ is selected from the group consisting of pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl, pyrrolopyridinyl, and tetrahydropyrrolopyridinyl optionally substituted from moieties selected from the group consisting of C₁-C₅ alkyl, halide, C₁-C₅ alkyl wherein one or more hydrogen atoms attached to a member atom within the C₁-C₅ alkyl group is replaced with 1, 2, 3 or 4 halide atoms, hydroxyl, amino, C₁-C₅ alkyl or C₂-C₅ alkenyl with one nitrogen atom replacing one terminal carbon atom in the unbranched or branched chain, C₁-C₅ alkyl or C₂-C₅ alkenyl wherein one or more hydrogen atoms attached to a member atom within the C₁-C₅ alkyl or C₂-C₅ alkenyl group is replaced with one or more hydroxyls, nitrile, C₁-C₅ alkyl or C₂-C₅ alkenyl with one nitrile group replacing one terminal carbon in the unbranched or branched chain, a radical of formula R'OR" wherein R' is C₁-C₅ alkyl, C₂-C₅ alkenyl or C₂-C₅ alkynyl and R" is C₁-C₅ alkyl, C₂-C₅ alkenyl, C₂-C₅ alkynyl, C₆-C₁₂ aryl, pyrrolyl, pyrazolyl, imidazolyl,
oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together or heterocycloalkyl, C₁-C₅ alkyl with one carbon atom replaced with an oxygen atom, C₁-C₅ alkyl with a carbonylamino or aminocarbonyl replacing two carbon atoms in the unbranched or branched chain, a radical of formula -OR¹² or a radical of formula -C(O)-R¹² wherein R¹² is selected from the group consisting of a C₁-C₅ alkyl, C₂-C₅ alkenyl, cyclopropyl, cyclobutyl, cyclopentyl, cyclohexyl, norbornane, adamantine, and C₅-C₆ cycloalkenyl; and thioalkyl.

5. A compound according to claim 1 wherein the compound corresponds to the following chemical structure 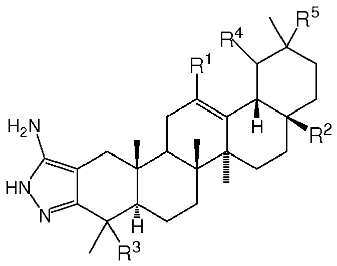 wherein R¹ is selected from the group consisting of hydrogen, halide, substituted or not C₁-C₅ alkyl, substituted or not C₂-C₅ alkenyl, C₅-C₆ cycloalkenyl, substituted or not C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together; and wherein R² is selected from the group consisting of amide, hydroxyamide, methylamide, -CH₂N(CH₃)₂, -CH₂NR⁶R⁷,

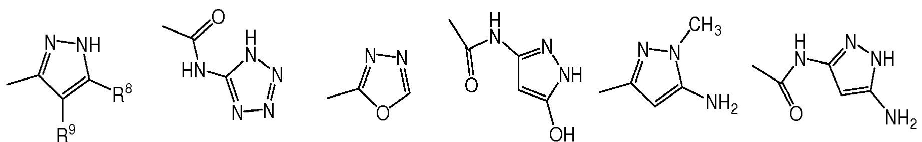

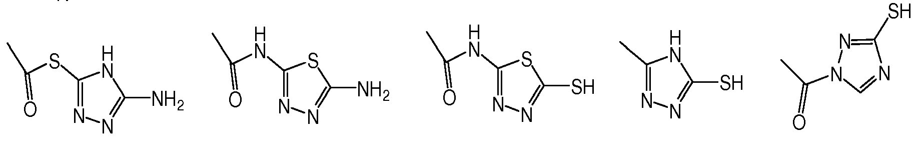

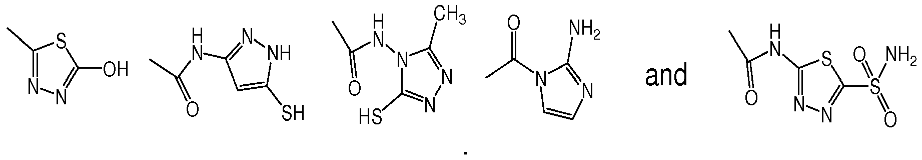

6. A compound according to claim 5 wherein R¹ is selected from the group consisting of pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, and tetrahydroindolyl optionally substituted from moieties selected from the group consisting of C₁-C₅ alkyl, halide, C₁-C₅ alkyl wherein one or more hydrogen atoms attached to a member atom within the C₁-C₅ alkyl group is replaced with 1, 2, 3 or 4 halide atoms, hydroxyl, amino, C₁-C₅ alkyl or C₂-C₅ alkenyl with one nitrogen atom replacing one terminal carbon atom in the unbranched or branched chain, C₁-C₅ alkyl or C₂-C₅ alkenyl wherein one or more hydrogen atoms attached to a member atom within the C₁-C₅ alkyl or C₂-C₅ alkenyl group is replaced with one or more hydroxyls, nitrile, C₁-C₅ alkyl or C₂-C₅ alkenyl with one nitrile group replacing one terminal carbon in the unbranched or branched chain, a radical of formula R'OR" wherein R' is C₁-C₅ alkyl, C₂-C₅ alkenyl or C₂-C₅ alkynyl and R" is a C₁-C₅ alkyl, a C₂-C₅ alkenyl, a C₂-C₅ alkynyl, aryl, pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together or heterocycloalkyl, a radical of formula -OR¹² or a radical of formula -C(O)-R¹² wherein R² is selected from the group consisting of C₁-C₅ alkyl, C₂-C₅ alkenyl, cyclopropyl, cyclobutyl, cyclopentyl, cyclohexyl, norbornane, adamantine, or C₅-C₆ cycloalkenyl; C₁-C₅ alkyl with one carbon atom replaced with an oxygen atom, C₁-C₅ alkyl with a carbonylamino or aminocarbonyl replacing two carbon atoms in the unbranched or branched chain, and thioalkyl.

7. A compound according to claim 1 wherein the compound corresponds to the following chemical structure 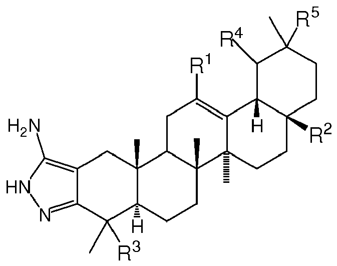
wherein R² is 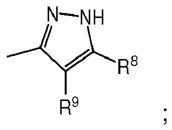
wherein R⁸ is selected from the group consisting of hydroxyl and amino; and wherein R⁹ is selected from the group consisting of hydrogen, halide, C₁-C₅ alkyl, C₂-C₅ alkenyl, C₂-C₅ alkynyl, morpholinyl, piperazinyl, C₁-C₅ alkyl piperazinyl, heterocycloalkyl cyclopropyl, cyclobutyl, cyclopentyl, cyclohexyl, norbornane, adamantine, C₅-C₆ cycloalkenyl; and C₁-C₅ alkyl, C₂-C₅ alkenyl, and C₂-C₅ alkynyl optionally substituted with moieties selected from the group consisting of hydroxyl, amino, C₁-C₅ alkyl or C₂-C₅ alkenyl with one nitrogen atom replacing one terminal carbon atom in the unbranched or branched chain, halide, a radical of formula-OR¹² or a radical of formula -C(O)-R¹² wherein R¹² is selected from the group consisting of C₁-C₅ alkyl, C₂-C₅ alkenyl, cyclopropyl, cyclobutyl, cyclopentyl, cyclohexyl, norbornane, adamantine, and C₅-C₆ cycloalkenyl; C₁-C₅ alkyl with one carbon atom replaced with an oxygen atom, C₁-C₅ alkyl with a carbonylamino or aminocarbonyl replacing two carbon atoms in the unbranched or branched chain, carboxyl, amide, hydroxyamide, -CONHCH₃,-NHCONH₂, -SO₂NH₂, -SO₂CH₃,-NHCOCH₃, -NHCSNH₂, and -NHSO₂CH₃ preferably R⁹ is hydrogen; and pharmaceutically acceptable salts thereof.

8. A compound according to claim 7 wherein R¹ is selected from the group consisting of substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together, wherein R² is 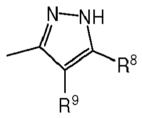 wherein R⁸ is selected from the group consisting of hydroxyl and amino; and wherein R⁹ is hydrogen.

9. A compound according to claim 1 wherein the compound corresponds to the following chemical structure: 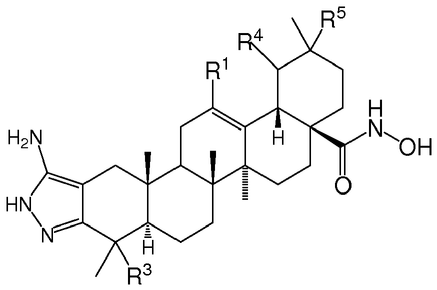 wherein R¹ is selected from the group consisting of hydrogen, methyl, halide, substituted or not C₁-C₅ alkyl, substituted or not C₂-C₅ alkenyl, substituted or not C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together.

10. A method for inhibiting or reducing the formation or growth of a biofilm comprising contacting said biofilm or a cell capable of biofilm formation or growth with an effective amount of a compound or a composition comprising the compound corresponding to the following chemical structure: 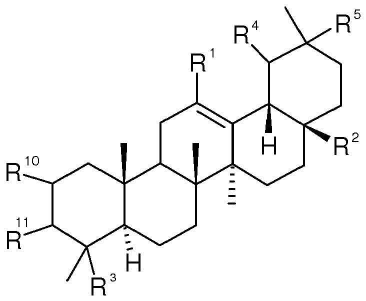
wherein R¹ is selected from the group consisting of hydrogen, methyl, halide, C₁-C₅ alkyl wherein one or more hydrogen atoms attached to a member atom within the C₁-C₅ alkyl group is replaced with 1, 2, 3 or 4 halide atoms, nitrile, C₁-C₅ alkyl or C₂-C₅ alkenyl with one nitrile group replacing one terminal carbon atom in the unbranched or branched chain, substituted or not C₁-C₅ alkyl, substituted or not C₂-C₅ alkenyl, substituted or not C₂-C₅ alkynyl, cyclopropyl, cyclobutyl, cyclopentyl, cyclohexyl, norbornane, adamantine, C₅-C₆ cycloalkenyl, substituted C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together,
wherein R² is selected from the group consisting of carboxyl, amide, hydroxyamide, methylamide, -CH₂N(CH₃)₂, -CH₂NR⁶R⁷, 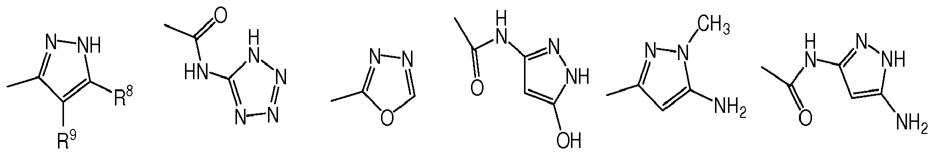 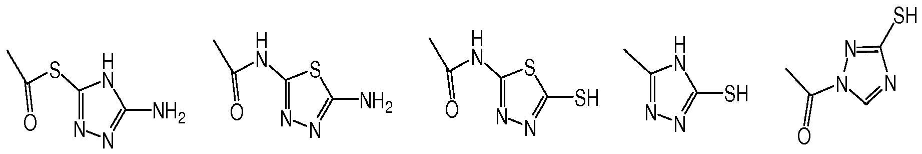 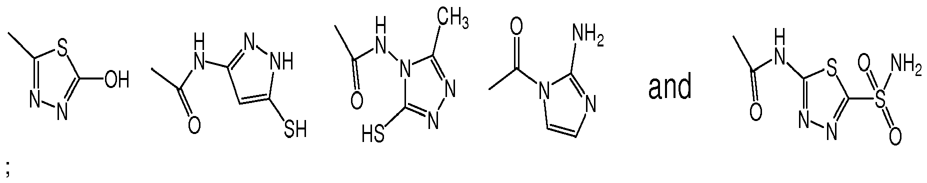
wherein R³ is selected from the group consisting of hydrogen and methyl; wherein one of R⁴ and R⁵ is hydrogen and the other is methyl; wherein R⁶ and R⁷ are independently selected from the group consisting of hydrogen, methyl, ethyl, propyl, isopropyl, and butyl; wherein R⁸ is selected from the group consisting of hydroxyl, amino, -N(CH₃)₂, and -NHCH₃; wherein R⁹ is selected from the group consisting of hydrogen, halide, C₁-C₅ alkyl, C₂-C₅ alkenyl, C₂-C₅ alkynyl, morpholinyl, piperazinyl, C₁-C₅ alkyl piperazinyl, heterocycloalkyl, cyclopropyl, cyclobutyl, cyclopentyl, cyclohexyl, norbornane, adamantine, C₅-C₆ cycloalkenyl; and C₁-C₅ alkyl, C₂-C₅ alkenyl, and C₂-C₅ alkynyl optionally substituted with moieties selected from the group consisting of hydroxyl, amino, C₁-C₅ alkyl or C₂-C₅ alkenyl with one nitrogen atom replacing one terminal carbon atom in the unbranched or branched chain, halide, C₁-C₅ alkyl with one carbon atom replaced with an oxygen atom , C₁-C₅ alkyl with a carbonylamino or aminocarbonyl replacing two carbon atoms in the unbranched or branched chain, carboxyl, amide, hydroxyamide, -CONHCH₃, -NHCONH₂, -SO₂NH₂, -SO₂CH₃, -NHCOCH₃,-NHCSNH₂, -NHSO₂CH₃, a radical of formula -OR¹² or a radical of formula -C(O)-R¹² wherein R¹² is selected from the group consisting of C₁-C₅ alkyl, C₂-C₅ alkenyl, cyclopropyl, cyclobutyl, cyclopentyl, cyclohexyl, norbornane, adamantine, a C₅-C₆ cycloalkenyl; and wherein R¹⁰ and R¹¹ together with the carbon atoms to which they are attached form an aromatic N-heterocycle which is substituted or not selected from the group consisting of indole, imidazole, quinoline, isoquinoline, pyridine, triazine, pyrazine, pyrimidine, pyridazine, isoindole, pyrrole, benzimidazole, purine, pyrazole, benzopyrazole, indazole, quinoxaline, acridine, quinazoline, indolizine, carbazole and cinnoline; and pharmaceutically acceptable salts thereof, with the proviso that the method is not performed on a human or animal body.

11. A method according to claim 10 wherein the compound corresponds to the following chemical structure: 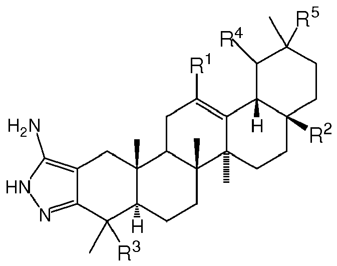
wherein R¹ is selected from the group consisting of hydrogen, methyl, halide, C₁-C₅ alkyl group wherein one or more hydrogen atoms attached to a member atom within the C₁-C₅ alkyl group is replaced with 1, 2, 3, or 4 halide atoms, nitrile, C₁-C₅ alkyl nitrile, substituted or not C₁-C₅ alkyl, substituted or not C₂-C₅ alkenyl, substituted or not C₂-C₅ alkynyl, cyclopropyl, cyclobutyl, cyclopentyl, cyclohexyl, norbornane, adamantine, C₅-C₆ cycloalkenyl, substituted or not C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together; wherein R² is selected from the group consisting of carboxyl, amide, hydroxyamide, methylamide, -CH₂N(CH₃)₂, -CH₂NR⁶R⁷, 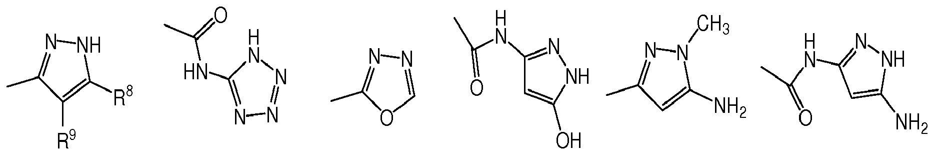 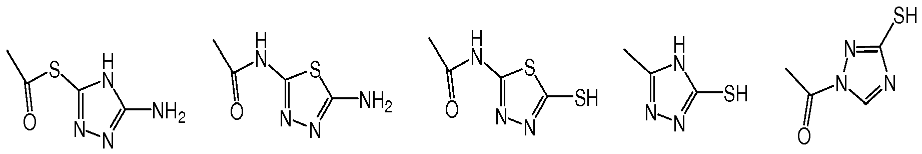 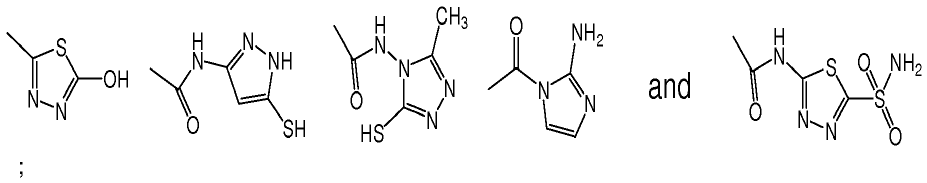
wherein R³ is selected from the group consisting of hydrogen and methyl; wherein one of R⁴ and R⁵ is hydrogen and the other is methyl; wherein R⁶ and R⁷ are independently selected from the group consisting of hydrogen, methyl, ethyl, propyl, isopropyl, and butyl; wherein R⁸ is selected from the group consisting of hydroxyl, amino, -N(CH₃)₂, and -NHCH₃; and wherein wherein R⁹ is selected from the group consisting of hydrogen, halide, C₁-C₅ alkyl, C₂-C₅ alkenyl, C₂-C₅ alkynyl, morpholinyl, piperazinyl, C₁-C₅ alkyl piperazinyl, heterocycloalkyl, cyclopropyl, cyclobutyl, cyclopentyl, cyclohexyl, norbornane, adamantine, C₅-C₆ cycloalkenyl; and C₁-C₅ alkyl, C₂-C₅ alkenyl, and C₂-C₅ alkynyl optionally substituted with moieties selected from the group consisting of hydroxyl, amino, C₁-C₅ alkyl or C₂-C₅ alkenyl with one nitrogen atom replacing one terminal carbon atom in the unbranched or branched chain, halide, C₁-C₅ alkyl with one carbon atom replaced with an oxygen atom , C₁-C₅ alkyl with a carbonylamino or aminocarbonyl replacing two carbon atoms in the unbranched or branched chain, carboxyl, amide, hydroxyamide, -CONHCH₃, -NHCONH₂, -SO₂NH₂, -SO₂CH₃, -NHCOCH₃,-NHCSNH₂, -NHSO₂CH₃, a radical of formula -OR¹² or a radical of formula -C(O)-R¹² wherein R¹² is selected from the group consisting of C₁-C₅ alkyl, C₂-C₅ alkenyl, cyclopropyl, cyclobutyl, cyclopentyl, cyclohexyl, norbornane, adamantine, and C₅-C₆ cycloalkenyl; and pharmaceutically acceptable salts thereof.

12. A method according to claim 10 wherein the compound corresponds to the following chemical structure: 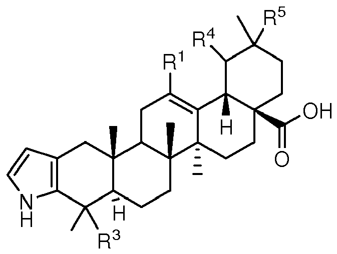 wherein R¹ is selected from the group consisting of hydrogen, methyl, halide, C₁-C₅ alkyl group wherein one or more hydrogen atoms attached to a member atom within the C₁-C₅ alkyl group is replaced with 1, 2, 3, or 4 halide atoms, nitrile, C₁-C₅ alkyl nitrile, substituted or not C₁-C₅ alkyl, substituted or not C₂-C₅ alkenyl, substituted or not C₂-C₅ alkynyl, cyclopropyl, cyclobutyl, cyclopentyl, cyclohexyl, norbornane, adamantine, C₅-C₆ cycloalkenyl, substituted or not C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together; wherein R³ is selected from the group consisting of hydrogen and methyl; and wherein one of R⁴ and R⁵ is hydrogen and the other is methyl; and salts thereof.

13. A method according to claim 10, wherein the compound corresponds to the followings chemical structure:

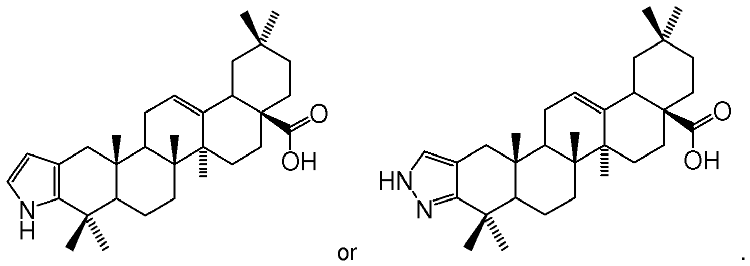

14. A compound for use in the reduction of virulence of gram-negative bacteria, the compound corresponding to the following chemical structure: 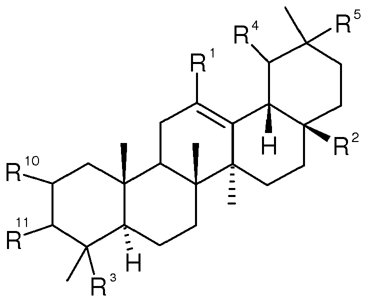
wherein R¹ is selected from the group consisting of hydrogen, methyl, halide, C₁-C₅ alkyl wherein one or more hydrogen atoms attached to a member atom within the C₁-C₅ alkyl group is replaced with 1, 2, 3 or 4 halide atoms, nitrile, C₁-C₅ alkyl or C₂-C₅ alkenyl with one nitrile group replacing one terminal carbon atom in the unbranched or branched chain, substituted or not C₁-C₅ alkyl, substituted or not C₂-C₅ alkenyl, substituted or not C₂-C₅ alkynyl, cyclopropyl, cyclobutyl, cyclopentyl, cyclohexyl, norbornane, adamantine, C₅-C₆ cycloalkenyl, substituted C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together; wherein R² is selected from the group consisting of carboxyl, amide, hydroxyamide, methylamide, -CH₂N(CH₃)₂, -CH₂NR⁶R⁷, 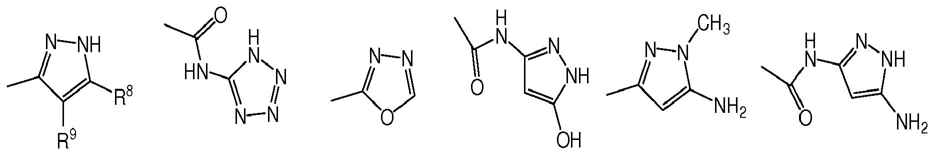 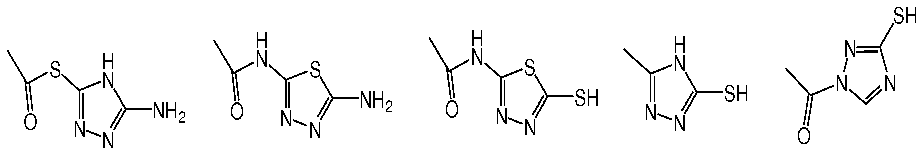 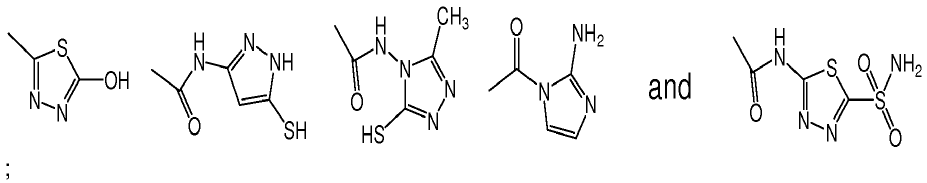
wherein R³ is selected from the group consisting of hydrogen and methyl; wherein one of R⁴ and R⁵ is hydrogen and the other is methyl; wherein R⁶ and R⁷ are independently selected from the group consisting of hydrogen, methyl, ethyl, propyl, isopropyl, and butyl; wherein R⁸ is selected from the group consisting of hydroxyl, amino, -N(CH₃)₂, and -NHCH₃; wherein R⁹ is selected from the group consisting of hydrogen, halide, C₁-C₅ alkyl, C₂-C₅ alkenyl, C₂-C₅ alkynyl, morpholinyl, piperazinyl, C₁-C₅ alkyl piperazinyl, heterocycloalkylcyclopropyl, cyclobutyl, cyclopentyl, cyclohexyl, norbornane, adamantine, C₅-C₆ cycloalkenyl; and C₁-C₅ alkyl, C₂-C₅ alkenyl, and C₂-C₅ alkynyl optionally substituted with moieties selected from the group consisting of hydroxyl, amino, C₁-C₅ alkyl or C₂-C₅ alkenyl with one nitrogen atom replacing one terminal carbon atom in the unbranched or branched chain, halide, C₁-C₅ alkyl with one carbon atom replaced with an oxygen atom , C₁-C₅ alkyl with a carbonylamino or aminocarbonyl replacing two carbon atoms in the unbranched or branched chain, carboxyl, amide, hydroxyamide, -CONHCH₃,-NHCONH₂, -SO₂NH₂, -SO₂CH₃, -NHCOCH₃,-NHCSNH₂, -NHSO₂CH₃, a radical of formula -OR¹² or a radical of formula -C(O)-R¹² wherein R¹² is selected from the group consisting of C₁-C₅ alkyl, C₂-C₅ alkenyl, cyclopropyl, cyclobutyl, cyclopentyl, cyclohexyl, norbornane, adamantine, and C₅-C₆ cycloalkenyl; and wherein R¹⁰ and R¹¹ together with the carbon atoms to which they are attached form an aromatic N-heterocycle which is substituted or not selected from the group consisting of indole, imidazole, quinoline, isoquinoline, pyridine, triazine, pyrazine, pyrimidine, pyridazine, isoindole, pyrrole, benzimidazole, purine, pyrazole, benzopyrazole, indazole, quinoxaline, acridine, quinazoline, indolizine, carbazole and cinnoline and pharmaceutically acceptable salts thereof.

15. A compound for use in the reduction of virulence of gram-negative bacteria according to claim 14 wherein the compound corresponds to the following chemical structure: 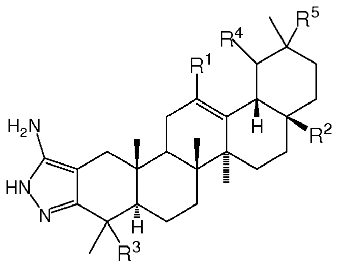
wherein R¹ is selected from the group consisting of hydrogen, methyl, halide, C₁-C₅ alkyl group wherein one or more hydrogen atoms attached to a member atom within the C₁-C₅ alkyl group is replaced with 1, 2, 3, or 4 halide atoms, nitrile, C₁-C₅ alkyl nitrile, substituted or not C₁-C₅ alkyl, substituted or not C₂-C₅ alkenyl, substituted or not C₂-C₅ alkynyl, cyclopropyl, cyclobutyl, cyclopentyl, cyclohexyl, norbornane, adamantine, C₅-C₆ cycloalkenyl, substituted or not C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together; wherein R² is selected from the group consisting of carboxyl, amide, hydroxyamide, methylamide, -CH₂N(CH₃)₂, -CH₂NR⁶R⁷, 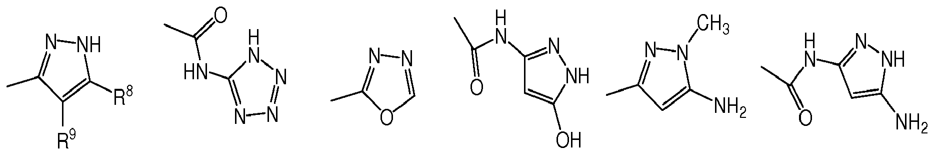 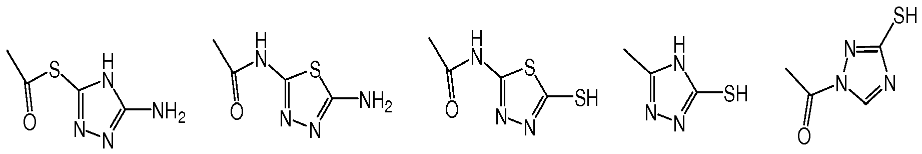 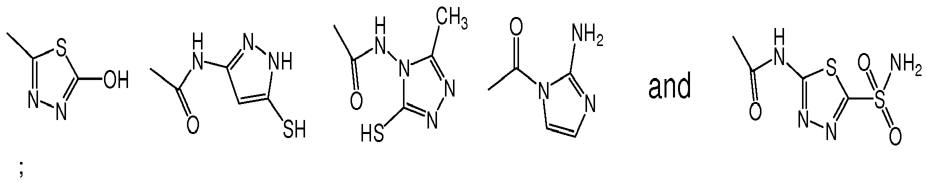
wherein R³ is selected from the group consisting of hydrogen and methyl; wherein one of R⁴ and R⁵ is hydrogen and the other is methyl; wherein R⁶ and R⁷ are independently selected from the group consisting of hydrogen, methyl, ethyl, propyl, isopropyl, and butyl; wherein R⁸ is selected from the group consisting of hydroxyl, amino, -N(CH₃)₂, and -NHCH₃; and wherein wherein R⁹ is selected from the group consisting of hydrogen, halide, C₁-C₅ alkyl, C₂-C₅ alkenyl, C₂-C₅ alkynyl, morpholinyl, piperazinyl, C₁-C₅ alkyl piperazinyl, heterocycloalkyl cyclopropyl, cyclobutyl, cyclopentyl, cyclohexyl, norbornane, adamantine, C₅-C₆ cycloalkenyl; and C₁-C₅ alkyl, C₂-C₅ alkenyl, and C₂-C₅ alkynyl optionally substituted with moieties selected from the group consisting of hydroxyl, amino, C₁-C₅ alkyl or C₂-C₅ alkenyl with one nitrogen atom replacing one terminal carbon atom in the unbranched or branched chain, halide, C₁-C₅ alkyl with one carbon atom replaced with an oxygen atom, C₁-C₅ alkyl with a carbonylamino or aminocarbonyl replacing two carbon atoms in the unbranched or baranched chain, carboxyl, amide, hydroxyamide, -CONHCH₃,-NHCONH₂, -SO₂NH₂, -SO₂CH₃, -NHCOCH₃,-NHCSNH₂, -NHSO₂CH₃, a radical of formula -OR¹² or a radical of formula -C(O)-R¹² wherein R¹² is selected from the group consisting of C₁-C₅ alkyl, C₂-C₅ alkenyl, cyclopropyl, cyclobutyl, cyclopentyl, cyclohexyl, norbornane, adamantine, and C₅-C₆ cycloalkenyl; and pharmaceutically acceptable salts thereof.

16. A compound for use in the reduction of virulence of gram-negative bacteria according to claim 14 wherein the compound corresponds to the following chemical structure: 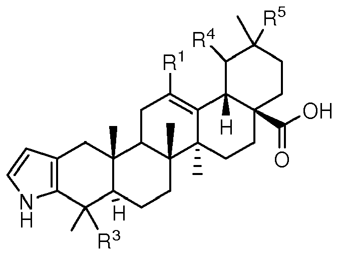 wherein R1 is selected from the group consisting of hydrogen, methyl, halide, nitrile, substituted or not C₁-C₅ alkyl, substituted or not C₂-C₅ alkenyl, substituted or not C₂-C₅ alkynyl, substituted or not C₆-C₁₂ aryl, and substituted or not pyrrolyl, pyrazolyl, imidazolyl, oxazolyl, isoxazolyl, oxadiazolyl, thiazolyl, isothiazolyl, thienyl, furanyl, furazanyl, pyridinyl, pyrimidinyl, pyridazinyl, indolyl, 3H-indolyl, isoindolyl, indolinyl, indolizinyl, indazolyl, dihydroindolyl, tetrahydroindolyl, purinyl, pyrazinyl, quinolinyl, isoquinolinyl, tetrahydroisoquinolinyl, quinoxalinyl, quinazolinyl, cinnolinyl, pteridinyl, benzimidazolyl, benzopyranyl, benzoxazolyl, benzisoxazolyl, benzofuranyl, isobenzofuranyl, benzothiazolyl, benzisothiazolyl, benzothienyl, furopyridinyl, phthalazinyl, napthyridinyl, pyrazolopyridyl, pyrazolopyrimidinyl; and their partially reduced forms; or a heteroaryl of 6 members or less fused with an aryl of 6 members or less or separately two heteroaryls of 6 members or less fused together; wherein R³ is selected from the group consisting of hydrogen and methyl; and wherein one of R⁴ and R⁵ is hydrogen and the other is methyl; and pharmaceutically acceptable salts thereof.

## Patentansprüche

1. Eine Verbindung der folgenden chemischen Struktur : 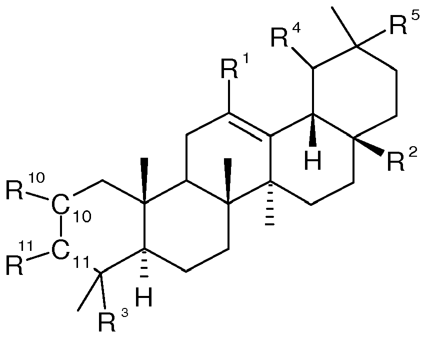
in der R¹⁰ und R¹¹ zusammen mit den Kohlenstoffatomen, an die sie gebunden sind 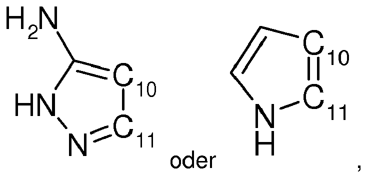
und wenn R¹⁰ und R¹¹ zusammen mit den Kohlenstoffatomen, an die sie gebunden sind 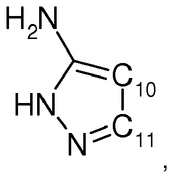
R¹ ist ausgewählt aus der Gruppe bestehend aus Wasserstoff, Methyl, Halogen, C₁-C₅-Alkyl wobei ein oder mehrere Wasserstoffatome, die an einem Atom innerhalb der C₁-C₅-Alkylgruppe gebunden sind, durch 1, 2, 3 oder 4 Halogenatome, Nitril, C₁-C₅-Alkyl oder C₂-C₅-Alkenyl mit einer Nitrilgruppe, ein endständiges Kohlenstoffatom in der unverzweigten oder verzweigten Kette ersetzt, substituierten oder nicht substituierten C₁-C₅-Alkylgruppe, substituierten oder nicht substituierten C₂-C₅-Alkenylgruppe, substituierten oder nicht substituierten C₂-C₅-Alkinylgruppe, Cyclopropyl-, Cyclobutyl-, Cyclopentyl-, Cyclohexyl-, Norbornan-, Diamantene, C₅-C₆-Cycloalkenyl-, substituierten oder nicht substituierten C₆-C₁₂ Arylgruppe und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl-, Indolizinyl-, Indazolyl-, Dihydroindolyl-, Tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen ersetzt ist; und deren partiell reduzierten Formen; oder eine Heteroarylgruppe von 6 oder weniger Mitgliedern, die mit einer Arylgruppe von 6 oder weniger Mitgliedern fusioniert ist, oder zwei separate Heteroarylgruppen von 6 oder weniger Mitglieder, die fusioniert sind; wobei R² ausgewählt ist aus der Gruppe bestehend aus Carboxyl-, Amid-, Hydroxyamid,-methylamid, CH₂N(CH₃)₂, -CH₂NR⁶R⁷, 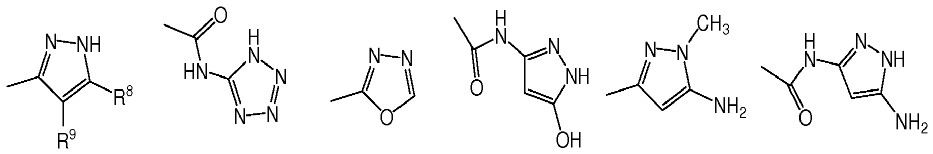 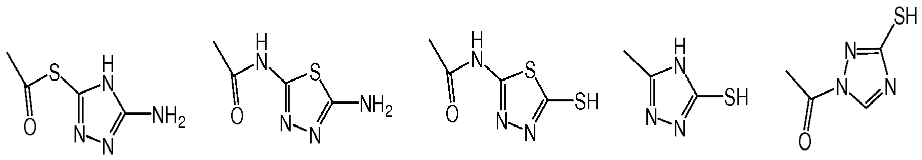 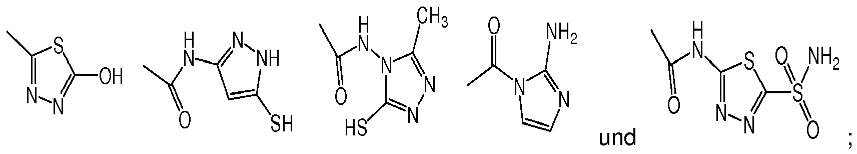
R³ ist ausgewählt aus der Gruppe, bestehend aus Wasserstoff und Methyl ; wobei die eine der Gruppen R⁴ und R⁵ Wasserstoff ist und die andere Methyl; R⁶ und R⁷ sind unabhängig voneinander ausgewählt aus der Gruppe bestehend aus Wasserstoff, Methyl-, Ethyl-, Propyl-, Isopropyl und Butyl; wobei R⁸ ausgewählt ist aus der Gruppe bestehend aus Hydroxyl-, Amin, -N(CH₃)₂, and -NHCH₃; und wobei R⁹ ausgewählt ist aus der Gruppe bestehend aus Wasserstoff, Halogen, C₁-C₅-Alkyl-, C₂-C₅-Alkenyl-, C₂-C₅-Alkinyl-, Morpholinyl-, Piperazinyl-, C₁-C₅-Alkyl-piperazinyl-, Heterocycloalkyl-, Cyclopropyl-, Cyclobutyl-, Cyclopentyl-, Cyclohexyl-, Norbornan-, Adamantan-, C₅-C₆-Cycloalkenylgruppen; und C₁-C₅-Alkyl-, C₂-C₅-Alkenyl- und C₂-C₅-Alkinylgruppen, gegebenenfalls substituiert durch Gruppen, die aus ausgewählt sind aus der Gruppe bestehend aus Hydroxyl, Amino, C₁-C₅-Alkyl- oder C₂-C₅-Alkenylgruppen mit einem Stickstoffatom, das ein endständiges Kohlenstoffatom in der substituierten unverzweigte oder verzweigte Kette ersetzt, Halogen, C₁-C₅-Alkylgruppe mit einem Kohlenstoffatom, das durch ein Sauerstoffatom ersetzt wird, C₁-C₅-Alkylgruppe mit einer Carbonylamino gruppe oder Aminocarbonyl gruppe, die zwei Kohlenstoffatome in der verzweigten oder unverzweigten Kette ersetzt, Carboxyl-, Amid-, Hydroxyamid, -CONHCH₃,-NHCONH₂, -SO₂NH₂, -SO₂CH₃, -NHCOCH₃, -NHCSNH₂, -NHSO₂CH₃, und ein Radikal der Formel-OR¹² oder ein Radikal der Formel -C (O)-R¹², wobei R¹² ausgewählt ist aus der Gruppe bestehend aus C₁-C₅-Alkyl-, C₂-C₅-Alkenyl-, Cyclopropyl-, Cyclobutyl-, Cyclopentyl-, Cyclohexyl-, Norbornan, diamantene, und C₅-C₆-Cycloalkenylgruppen; und pharmazeutisch akzeptable Salze von diesen, oder
wenn R¹⁰ und R¹¹ zusammen mit den Kohlenstoffatomen an die sie gebunden
sind, die folgende Struktur aufweist 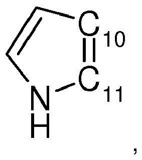
R¹ ist ausgewählt aus der Gruppe bestehend aus Wasserstoff, Methyl-, Halogen, Nitril, substituierten oder nicht substituierten C₁-C₅- Alkylgruppe, substituierten oder nicht substituierten C₂-C₅- Alkenylgruppe, substituierten oder nicht substituierten C2-C5- Alkinylgruppe, substituierten oder nicht substituierten C₂-C₅-Arylgruppe, und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H- Indolyl-, Isoindolyl-, Indolinyl-, Indolizinyl-, Indazolyl-, Dihydroindolyl-, tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen ; und ihre teilweise reduzierten Formen; oder eine Heteroarylgruppe mit 6 oder weniger Mitglieder mit einer Arylgruppe von 6 oder weniger Mitgliedern fusioniert oder separat zwei Heteroaryle von 6 oder weniger Mitglieder miteinander verschmolzen ; R² eine Carboxylgruppe ist ; R³ ausgewählt ist aus der Gruppe, bestehend aus Wasserstoff und Methyl; und einer von R⁴ und R⁵ Wasserstoff und das andere Methyl ; und pharmazeutisch verträgliche Salze davon.

2. Verbindung nach Anspruch 1, worin die Verbindung der folgenden chemischen
Struktur entspricht 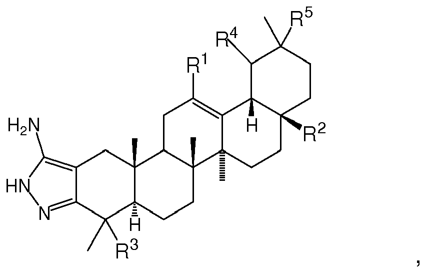 R¹ ist ausgewählt aus der Gruppe bestehend aus Methyl-, Halogen, C₁-C₅-Alkyl wobei ein oder mehrere Wasserstoffatome, die an einem Atom innerhalb der C₁-C₅-Alkylgruppe gebunden sind, durch 1, 2, 3 oder 4 Halogenatome, Nitril, C₁-C₅-Alkyl oder C₂-C₅-Alkenyl mit einer Nitrilgruppe, ein endständiges Kohlenstoffatom in der unverzweigten oder verzweigten Kette ersetzt, substituierten oder nicht substituierten C₁-C₅-Alkylgruppe, substituierten oder nicht substituierten C₂-C₅-Alkenylgruppe, substituierten oder nicht substituierten C₂-C₅-Alkinylgruppe, Cyclopropyl-, Cyclobutyl-, Cyclopentyl-, Cyclohexyl-, Norbornan-, Diamantene, C₅-C₆-Cycloalkenyl-, substituierten oder nicht substituierten C₆-C₁₂Arylgruppe und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl-, Indolizinyl-, Indazolyl-, Dihydroindolyl-, Tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinyl- -, Pyrrolopyridinyl- und tetrahydropyrrolopyridinylgruppen ersetzt ist, optional von Einheiten, ausgewählt aus der Gruppe bestehend aus C₁-C₅ -Alkyl-, Halogen, C₁-C₅- Alkyl-, worin ein oder mehrere Wasserstoff substituiert Atome an ein Mitglied Atom in der C₁-C₅- Alkylgruppe gebunden ist mit 1, 2, 3 oder 4 Halogenatome, Hydroxyl-, Amino-, C₁-C₅ -Alkyl oder C₂-C₅- Alkenyl mit einem Stickstoffatom ersetzt einem endständigen Kohlenstoffatom in der Fassung unverzweigte oder verzweigte C₁-C₅-Alkylgruppe oder C₂-C₅-Alkenylgruppe, worin ein oder mehrere Wasserstoffatome an ein Mitglied Atom in der C₁-C₅-Alkyl oder C₂-C₅-Alkenylrest verbunden ist mit einer oder mehreren Hydroxylgruppen, Nitril-, C₁-C₅ ersetzt Alkyl oder C₂-C₅- Alkenyl mit einer Nitrilgruppe ersetzen einer endständigen Kohlenstoffatom in der unverzweigten oder verzweigten Kette, einen Rest der Formel R'OR ", worin R' ist C₁-C₅-Alkyl-, C₂-C₅-Alkenyl- oder C₂-C₅-Alkinygruppel und R" ausgewählt aus der Gruppe bestehend aus C₁-C₅ -Alkyl-, C₂-C₅-Alkenyl-, C₂-C₅-Alkinyl-, C₆-C₁₂-Aryl-, Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H- Indolyl-, Isoindolyl-, Indolinyl-, Indolizinyl-, Indazolyl-, Dihydroindolyl-, tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppe ; und ihre teilweise reduzierten Formen; oder eine Heteroarylgruppe mit 6 oder weniger Mitglieder mit einer Arylgruppe von 6 oder weniger Mitgliedern fusioniert oder separat zwei Heteroaryle von 6 oder weniger Mitglieder miteinander verschmolzen; oder ein Radikal der Formel-OR¹² oder ein Radikal der Formel -C (O)-R¹², wobei R¹² ausgewählt ist aus der Gruppe bestehend aus C₁-C₅-Alkyl-, C₂-C₅-Alkenyl-, Cyclopropyl-, Cyclobutyl-, Cyclopentyl-, Cyclohexyl-, Norbornan, diamantene, und C₅-C₆-Cycloalkenylgruppen ; C₁-C₅ -Alkyl mit einem Kohlenstoffatom mit einem Sauerstoffatom, C₁-C₅-Alkylgruppe mit einem Kohlenstoffatom, das durch ein Sauerstoffatom ersetzt wird, C₁-C₅-Alkylgruppe mit einer Carbonylamino gruppe oder Aminocarbonyl gruppe, die zwei Kohlenstoffatome in der verzweigten oder unverzweigten Kette ersetzt und Thioalkyl ersetzt.

3. Verbindung nach Anspruch 1, worin die Verbindung der folgenden chemischen Struktur entspricht: 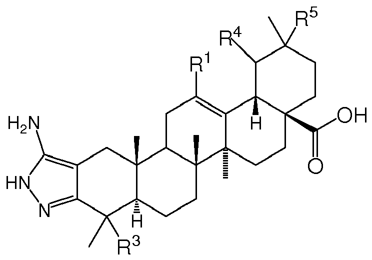 R¹ ist ausgewählt aus der Gruppe bestehend aus Wasserstoff, Halogen, substituierten oder nicht substituierten C₁-C₅-Alkylgruppe, substituiertes oder nicht C₂-C₅-Alkenylgruppe, Cyclopropyl-, Cyclobutyl-, Cyclopentyl-, Cyclohexyl-, Norbornan, diamantene, C₅-C₆-Cycloalkenyl-, substituierten oder nicht substituierten C₆ C₁₂-Aryl und substituiertem oder nicht Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl-, Indolizinyl-, Indazolyl-, Dihydroindolyl-, tetrahydroindolyl-, Purinyl-, Pyrazinyl-, Chinolinyl-, Isochinolinyl-, Tetrahydroisochinolinyl-, Chinoxalinyl-, Chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen; und ihre teilweise reduzierten Formen; oder oder eine Heteroarylgruppe mit 6 oder weniger Mitglieder mit einer Arylgruppe von 6 oder weniger Mitgliedern fusioniert oder separat zwei Heteroaryle von 6 oder weniger Mitglieder miteinander verschmolzen, vorzugsweise R¹ Wasserstoff ist.

4. Verbindung nach Anspruch 3, R¹ ist ausgewählt aus der Gruppe bestehend aus Halogene, substituiertem oder nicht substituiertem C₁-C₅- Alkylgruppe, substituiertem oder nicht substituiertem C₂-C₅- Alkenylgruppe, Cyclopropyl-, Cyclobutyl-, Cyclopentyl-, Cyclohexyl-, Norbornan, diamantene, C₅ - C₆ Cycloalkenyl-, substituierten oder nicht substituierten C₆-C₁₂ Aryl- und substituierten oder nicht substituierten, Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H- Indolyl-, Isoindolyl-, Indolinyl Indolizinyl-, Indazolyl-, Dihydroindolyl-, tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen ; und deren partiell reduzierten Formen; oder oder eine Heteroarylgruppe mit 6 oder weniger Mitglieder mit einer Arylgruppe von 6 oder weniger Mitgliedern fusioniert oder separat zwei Heteroaryle von 6 oder weniger Mitglieder miteinander verschmolzen, vorzugsweise R¹ ist ausgewählt aus der Gruppe bestehend aus Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H- Indolyl-, Isoindolyl-, Indolinyl-, Indolizinyl-, Indazolyl-, Dihydroindolyl-, tetrahydroindolyl-, Purinyl-, Pyrazinyl-, Chinolinyl-, Isochinolinyl-, Tetrahydroisochinolinyl-, Chinoxalinyl-, Chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinyl-, Pyrrolopyridinyl und tetrahydropyrrolopyridinylgruppen, optional von Einheiten, ausgewählt aus der Gruppe bestehend aus C₁-C₅ -Alkyl-, Halogen, C₁-C₅- Alkyl-, worin ein oder mehrere Wasserstoff substituiert Atome an ein Mitglied Atom in der C₁-C₅- Alkylgruppe gebunden ist mit 1, 2, 3 oder 4 Halogenatome, Hydroxyl-, Amino-, C₁-C₅ -Alkyl oder C₂-C₅- Alkenyl mit einem Stickstoffatom ersetzt einem endständigen Kohlenstoffatom in der Fassung unverzweigte oder verzweigte C₁-C₅ -Alkylgruppe oder C₂-C₅-Alkenylgruppe, worin ein oder mehrere Wasserstoffatome an ein Mitglied Atom in der C₁-C₅ -Alkyl oder C₂-C₅-Alkenylrest verbunden ist mit einer oder mehreren Hydroxylgruppen, Nitril-, C₁-C₅ ersetzt Alkyl oder C₂-C₅-Alkenyl mit einer Nitrilgruppe ersetzen einer endständigen Kohlenstoffatom in der unverzweigten oder verzweigten Kette, einen Rest der Formel R'OR ", worin R' ist C₁-C₅ -Alkyl-, C₂-C₅-Alkenyl- oder C₂-C₅-Alkinygruppel und R" ausgewählt aus der Gruppe bestehend aus C₁-C₅ -Alkyl-, C₂-C₅- Alkenyl-, C₂-C₅-Alkinyl-, C₆-C₁₂-Aryl-, Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H- Indolyl-, Isoindolyl-, Indolinyl-, Indolizinyl-, Indazolyl-, Dihydroindolyl-, tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppe ; und ihre teilweise reduzierten Formen; oder eine Heteroarylgruppe mit 6 oder weniger Mitglieder mit einer Arylgruppe von 6 oder weniger Mitgliedern fusioniert oder separat zwei Heteroaryle von 6 oder weniger Mitglieder miteinander verschmolzen; oder ein Radikal der Formel-OR¹² oder ein Radikal der Formel -C(O)-R¹², wobei R¹² ausgewählt ist aus der Gruppe bestehend aus C₁-C₅-Alkyl-, C₂-C₅-Alkenyl-, Cyclopropyl-, Cyclobutyl-, Cyclopentyl-, Cyclohexyl-, Norbornan, diamantene, und C₅-C₆-Cycloalkenylgruppen; C₁-C₅ -Alkyl mit einem Kohlenstoffatom mit einem Sauerstoffatom, C₁-C₅-Alkylgruppe mit einem Kohlenstoffatom, das durch ein Sauerstoffatom ersetzt wird, C₁-C₅-Alkylgruppe mit einer Carbonylamino gruppe oder Aminocarbonyl gruppe, die zwei Kohlenstoffatome in der verzweigten oder unverzweigten Kette ersetztund Thioalkyl ersetzt.

5. Verbindung nach Anspruch 1, wobei die Verbindung der folgenden chemischen
Struktur entspricht 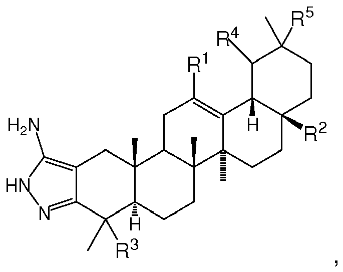 R¹ ist ausgewählt aus der Gruppe bestehend aus Wasserstoff, Halogen, substituiertem oder nicht substituiertem C₁-C₅-Alkylgruppe, substituiertem oder nicht substituiertem C₂-C₅-Alkenylgruppe, C₅-C₆-Cycloalkenyl-, substituierten oder nicht substituierten C₆-C₁₂Arylgruppe und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H- Indolyl-, Isoindolyl-, Indolinyl-, Indolizinyl-, Indazolyl-, Dihydroindolyl-, tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen, und deren partiell reduzierten Formen ; oder eine Heteroarylgruppe von 6 oder weniger Mitgliedern, die mit einer Arylgruppe von 6 oder weniger Mitgliedern fusioniert ist, oder zwei separate Heteroarylgruppen von 6 oder weniger Mitglieder, die fusioniert sind ; wobei R² ausgewählt ist aus der Gruppe bestehend aus Carboxyl-, Amid-, Hydroxyamid,-methylamid, CH₂N(CH₃)₂, -CH₂NR⁶R⁷,

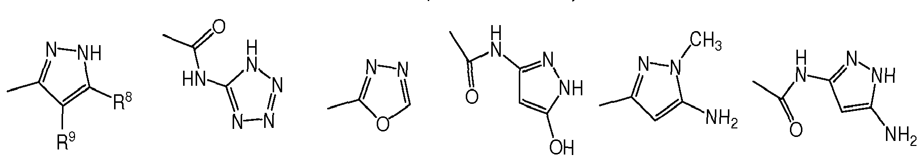

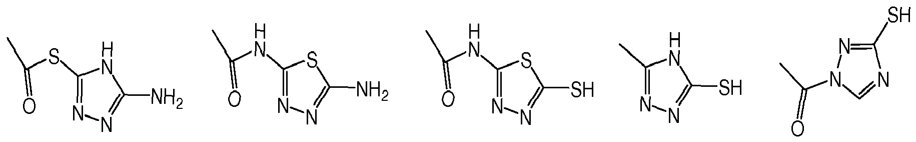

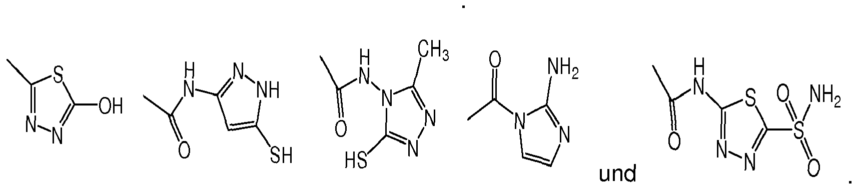

6. Verbindung nach Anspruch 5, wobei R¹ ist ausgewählt aus der Gruppe bestehend aus Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H -Indolyl-, Isoindolyl-, Indolinyl-, Indolizinyl-, Indazolyl-, Dihydroindolyl-, und tetrahydroindolylgruppen, optional von Einheiten, ausgewählt aus der Gruppe bestehend aus C₁-C₅ -Alkyl-, Halogen, C₁-C₅- Alkyl-, worin ein oder mehrere Wasserstoff substituiert Atome an ein Mitglied Atom in der C₁-C₅- Alkylgruppe gebunden ist mit 1, 2, 3 oder 4 Halogenatome, Hydroxyl-, Amino-, C₁-C₅ -Alkyl oder C₂-C₅- Alkenyl mit einem Stickstoffatom ersetzt einem endständigen Kohlenstoffatom in der Fassung unverzweigte oder verzweigte C₁-C₅ -Alkylgruppe oder C₂-C₅- Alkenylgruppe, worin ein oder mehrere Wasserstoffatome an ein Mitglied Atom in der C₁-C₅ -Alkyl oder C₂-C₅-Alkenylrest verbunden ist mit einer oder mehreren Hydroxylgruppen, Nitril-, C₁-C₅ ersetzt Alkyl oder C₂-C₅-Alkenyl mit einer Nitrilgruppe ersetzen einer endständigen Kohlenstoffatom in der unverzweigten oder verzweigten Kette, einen Rest der Formel R'OR ", worin R' ist C₁-C₅ -Alkyl-, C₂-C₅-Alkenyl- oder C₂-C₅-Alkinygruppel und R" ausgewählt aus der Gruppe bestehend aus C₁-C₅ -Alkyl-, C₂-C₅- Alkenyl-, C₂-C₅- Alkinyl-, C₆-C₁₂-Aryl-, Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H- Indolyl-, Isoindolyl-, Indolinyl-, Indolizinyl-, Indazolyl-, Dihydroindolyl-, tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppe ; und ihre teilweise reduzierten Formen; oder eine Heteroarylgruppe mit 6 oder weniger Mitglieder mit einer Arylgruppe von 6 oder weniger Mitgliedern fusioniert oder separat zwei Heteroaryle von 6 oder weniger Mitglieder miteinander verschmolzen; oder ein Radikal der Formel-OR¹² oder ein Radikal der Formel -C (O)-R¹², wobei R¹² ausgewählt ist aus der Gruppe bestehend aus C₁-C₅-Alkyl-, C₂-C₅-Alkenyl-, Cyclopropyl-, Cyclobutyl-, Cyclopentyl-, Cyclohexyl-, Norbornan, diamantene, und C₅-C₆-Cycloalkenylgruppen ; C₁-C₅ -Alkyl mit einem Kohlenstoffatom mit einem Sauerstoffatom, C₁-C₅-Alkylgruppe mit einem Kohlenstoffatom, das durch ein Sauerstoffatom ersetzt wird, C₁-C₅-Alkylgruppe mit einer Carbonylamino gruppe oder Aminocarbonyl gruppe, die zwei Kohlenstoffatome in der verzweigten oder unverzweigten Kette ersetztund Thioalkyl ersetzt.

7. Verbindung nach Anspruch 1, wobei die Verbindung der folgenden chemischen Struktur entspricht 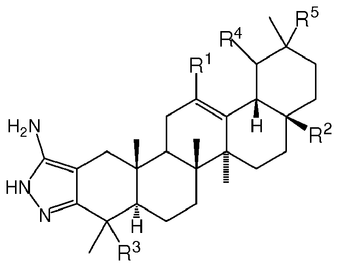
worin R² ist 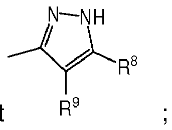
worin R⁸ ist ausgewählt aus der Gruppe bestehend aus Hydroxyl- und Aminogruppen ; und worin R⁹ ausgewählt ist ausgewählt aus der Gruppe bestehend aus Wasserstoff, Halogen, C₁-C₅ -Alkyl-, C₂ -C₅- Alkenyl-, C₂-C₅- Alkinyl-, Morpholinyl -, Piperazinyl-, C₁-C₅- Alkyl-piperazinyl -, Heterocycloalkyl-, Cyclopropyl-, Cyclobutyl-, Cyclopentyl-, Cyclohexyl-, Norbornan, diamantene, C₅-C₆- Cycloalkenylgruppen ; und C₁-C₅ -Alkyl-, C₂-C₅-Alkenyl und C₂-C₅-Alkinylgruppen, C₁-C₅-Alkyl-, C₂-C₅-Alkenyl- und C₂-C₅-Alkinylgruppen, gegebenenfalls substituiert durch Gruppen, die aus ausgewählt sind aus der Gruppe bestehend aus Hydroxyl, Amino, C₁-C₅-Alkyl- oder C₂-C₅-Alkenylgruppen mit einem Stickstoffatom, das ein endständiges Kohlenstoffatom in der substituierten unverzweigte oder verzweigte Kette ersetzt, Halogen, Radikal der Formel-OR¹² oder ein Radikal der Formel -C (O)-R¹², wobei R¹² ausgewählt ist aus der Gruppe bestehend aus C₁-C₅ -Alkyl-, C₂ -C₅- Alkenyl-, Cyclopropyl-, Cyclobutyl-, Cyclopentyl-, Cyclohexyl-, norbornan, diamantene und C₅-C₆-Cycloalkenylgruppen ; C₁-C₅ -Alkyl mit einem Kohlenstoffatom mit einem Sauerstoffatom, C₁-C₅-Alkylgruppe mit einem Kohlenstoffatom, das durch ein Sauerstoffatom ersetzt wird, C₁-C₅-Alkylgruppe mit einer Carbonylamino gruppe oder Aminocarbonyl gruppe, die zwei Kohlenstoffatome in der verzweigten oder unverzweigten Kette ersetzt, Carboxyl-, Amid-, Hydroxyamidgruppen, -CONHCH₃, - NHCONH₂, -SO₂NH₂, -SO₂CH₃, -NHCOCH₃, -NHCSNH₂, und -NHSO₂CH₃, vorzugsweise R⁹ ist Wasserstoff ; und pharmazeutisch verträgliche Salze davon.

8. Verbindung nach Anspruch 7, worin R¹ ist ausgewählt aus der Gruppe bestehend aus substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl Indolizinyl-, Indazolyl-, Dihydroindolyl-, tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl- , Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen; und ihre teilweise reduzierten Formen; oder eine Heteroarylgruppe von 6 oder weniger Mitgliedern, die mit einer Arylgruppe von 6 oder weniger Mitgliedern fusioniert ist, oder zwei separate Heteroarylgruppen von 6 oder weniger Mitglieder, die fusioniert sind,
wobei R² ist 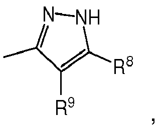
worin R⁸ ausgewählt aus der Gruppe bestehend aus Hydroxyl- und Aminogruppen ist; und wobei R⁹ Wasserstoff ist.

9. Verbindung nach Anspruch 1, wobei die Verbindung der folgenden chemischen Struktur entspricht: 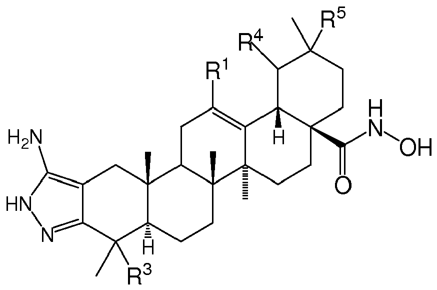 R¹ ist ausgewählt aus der Gruppe bestehend aus Wasserstoff, Methyl-, Halogen, substituiertem oder nicht substituiertem C₁-C₅-Alkylgruppe, substituiertem oder nicht substituiertem C₂-C₅-Alkenylgruppe substituiertem oder nicht substituiertem C₆-C₁₂-Arylgruppe und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl-, Indolizinyl-, Indazolyl-, Dihydroindolyl-, tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen; und ihre teilweise reduzierten Formen; oder eine Heteroarylgruppe von 6 oder weniger Mitgliedern, die mit einer Arylgruppe von 6 oder weniger Mitgliedern fusioniert ist, oder zwei separate Heteroarylgruppen von 6 oder weniger Mitglieder, die fusioniert sind.

10. Ein Verfahren die Bildung und das Wachstum von Biofilmen zu inhibieren oder zu reduzieren, wobei das Verfahren aufweist: Das in Kontakt bringen des besagten Biofilms oder einer Zelle, die einen Biofilm bilden kann, mit einer wirksamen Menge einer Verbindung oder einer Komposition, die die Verbindungen aufweist, mit folgender Struktur 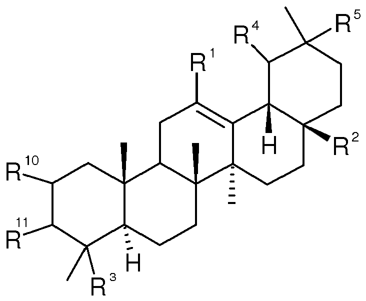
R¹ ist ausgewählt aus der Gruppe bestehend aus Wasserstoff, Methyl-, Halogen, C₁-C₅-Alkyl wobei ein oder mehrere Wasserstoffatome, die an einem Atom innerhalb der C₁-C₅-Alkylgruppe gebunden sind, durch 1, 2, 3 oder 4 Halogenatome, Nitril, C₁-C₅-Alkyl oder C₂-C₅-Alkenyl mit einer Nitrilgruppe, ein endständiges Kohlenstoffatom in der unverzweigten oder verzweigten Kette ersetzt, substituierten oder nicht substituierten C₁-C₅-Alkylgruppe, substituierten oder nicht substituierten C₂-C₅-Alkenylgruppe, substituierten oder nicht substituierten C₂-C₅-Alkinylgruppe, Cyclopropyl-, Cyclobutyl-, Cyclopentyl-, Cyclohexyl-, Norbornan-, Diamantene, C₅-C₆-Cycloalkenyl-, substituierten oder nicht substituierten C₆-C₁₂Arylgruppe und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl-, Indolizinyl-, Indazolyl-, Dihydroindolyl-, Tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen ersetzt ist; und deren partiell reduzierten Formen; oder eine Heteroarylgruppe von 6 oder weniger Mitgliedern, die mit einer Arylgruppe von 6 oder weniger Mitgliedern fusioniert ist, oder zwei separate Heteroarylgruppen von 6 oder weniger Mitglieder, die fusioniert sind; wobei R² ausgewählt ist aus der Gruppe bestehend aus Carboxyl-, Amid-, Hydroxyamid,-methylamid, CH₂N(CH₃)₂, -CH₂NR⁶R⁷, 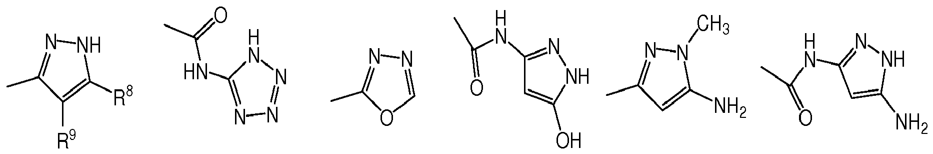 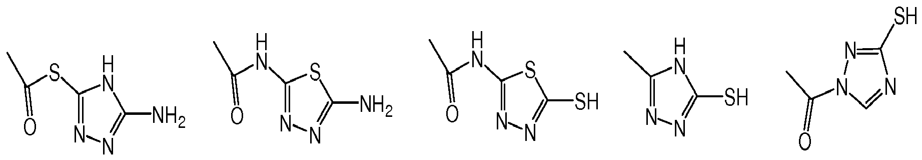 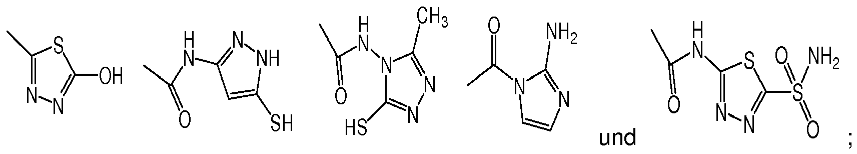
R³ ist ausgewählt aus der Gruppe bestehend aus Wasserstoff und Methyl;wobei die eine der Gruppen R⁴ und R⁵ Wasserstoff ist und die andere Methyl; R⁶ und R⁷ sind unabhängig voneinander ausgewählt aus der Gruppe bestehend aus Wasserstoff, Methyl-, Ethyl-, Propyl-, Isopropyl und Butyl; wobei R⁸ ausgewählt ist aus der Gruppe bestehend aus Hydroxyl-, Amin, -N(CH₃)₂, and -NHCH₃; und wobei R⁹ ausgewählt ist aus der Gruppe bestehend aus Wasserstoff, Halogen, C₁-C₅-Alkyl-, C₂-C₅-Alkenyl-, C₂-C₅-Alkinyl-, Morpholinyl-, Piperazinyl-, C₁-C₅-Alkyl-piperazinyl-, Heterocycloalkyl-, Cyclopropyl-, Cyclobutyl-, Cyclopentyl-, Cyclohexyl- , Norbornan-, Adamantan-, C₅-C₆-Cycloalkenylgruppen ; und C₁-C₅-Alkyl-, C₂-C₅-Alkenyl- und C₂-C₅-Alkinylgruppen, gegebenenfalls substituiert durch Gruppen, die aus ausgewählt sind aus der Gruppe bestehend aus Hydroxyl, Amino, C₁-C₅-Alkyl- oder C₂-C₅-Alkenylgruppen mit einem Stickstoffatom, das ein endständiges Kohlenstoffatom in der substituierten unverzweigte oder verzweigte Kette ersetzt, Halogen, C₁-C₅-Alkylgruppe mit einem Kohlenstoffatom, das durch ein Sauerstoffatom ersetzt wird, C₁-C₅-Alkylgruppe mit einer Carbonylamino gruppe oder Aminocarbonyl gruppe, die zwei Kohlenstoffatome in der verzweigten oder unverzweigten Kette ersetzt, Carboxyl-, Amid-, Hydroxyamid, -CONHCH₃, - NHCONH₂, -SO₂NH₂, -SO₂CH₃, -NHCOCH₃, -NHCSNH₂, -NHSO₂CH₃, und ein Radikal der Formel-OR¹² oder ein Radikal der Formel -C (O)-R¹², wobei R¹² ausgewählt ist aus der Gruppe bestehend aus C₁-C₅-Alkyl-, C₂-C₅-Alkenyl-, Cyclopropyl-, Cyclobutyl-, Cyclopentyl-, Cyclohexyl-, Norbornan, diamantene, und C₅-C₆-Cycloalkenylgruppen; und pharmazeutisch akzeptable Salze von diesen, ; und wobei R¹⁰ und R¹¹ zusammen mit den Kohlenstoffatomen, an die sie gebunden sind, einen aromatischen N- Heterocyclus, der substituiert oder nicht substituiert ist, ausgewählt aus der Gruppe bestehend aus Indol, Imidazol, Chinolin, Isochinolin, Pyridin, Triazin, Pyrazin, Pyrimidin, Pyridazin, Isoindols, Pyrrol, Benzimidazol, Purin, Pyrazol, Benzopyrazol, Indazol, Chinoxalin, Acridin, Chinazolin, Indolizin, Carbazol und Cinnolin ; und pharmazeutisch akzeptable Salze davon,
mit der Beschränkung, dass das Verfahren nicht am menschlichen oder tierischen Körper durchgeführt wird.

11. Verfahren nach Anspruch 10, wobei die Verbindung der folgenden chemischen Struktur entspricht: 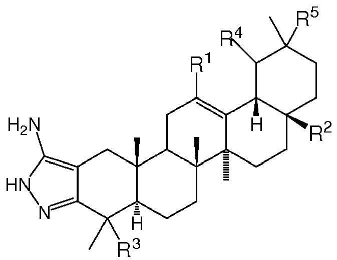
R¹ ist ausgewählt aus der Gruppe bestehend aus Wasserstoff, Methyl-, Halogen, C₁-C₅-Alkyl wobei ein oder mehrere Wasserstoffatome, die an einem Atom innerhalb der C₁-C₅-Alkylgruppe gebunden sind, durch 1, 2, 3 oder 4 Halogenatome, Nitril, C₁-C₅-Alkyl oder C₂-C₅-Alkenyl mit einer Nitrilgruppe, ein endständiges Kohlenstoffatom in der unverzweigten oder verzweigten Kette ersetzt, substituierten oder nicht substituierten C₁-C₅-Alkylgruppe, substituierten oder nicht substituierten C₂-C₅-Alkenylgruppe, substituierten oder nicht substituierten C₂-C₅-Alkinylgruppe, Cyclopropyl-, Cyclobutyl-, Cyclopentyl-, Cyclohexyl-, Norbornan-, Diamantene, C₅-C₆-Cycloalkenyl-, substituierten oder nicht substituierten C₆-C₁₂Arylgruppe und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl-, Indolizinyl-, Indazolyl-, Dihydroindolyl-, Tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen ersetzt ist; und deren partiell reduzierten Formen; oder eine Heteroarylgruppe von 6 oder weniger Mitgliedern, die mit einer Arylgruppe von 6 oder weniger Mitgliedern fusioniert ist, oder zwei separate Heteroarylgruppen von 6 oder weniger Mitglieder, die fusioniert sind; wobei R² ausgewählt ist aus der Gruppe bestehend aus Carboxyl-, Amid-, Hydroxyamid,-methylamid, CH₂N(CH₃)₂, -CH₂NR⁶R⁷, 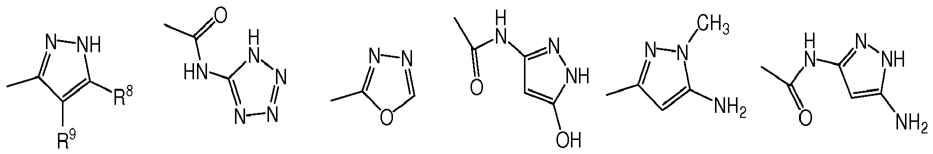 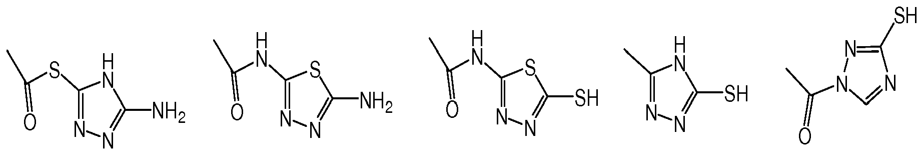 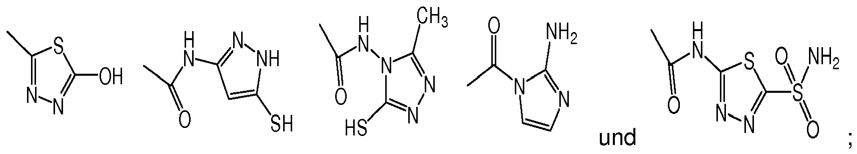
R¹ ist ausgewählt aus der Gruppe bestehend aus Wasserstoff und Methyl ; wobei die eine der Gruppen R⁴ und R⁵ Wasserstoff ist und die andere Methyl; R⁶ und R⁷ sind unabhängig voneinander ausgewählt aus der Gruppe bestehend aus Wasserstoff, Methyl-, Ethyl-, Propyl-, Isopropyl- und Butylgruppen ; wobei R⁸ ausgewählt ist aus der Gruppe bestehend aus Hydroxyl-, Amin, -N(CH₃)₂, and -NHCH₃; und wobei R⁹ ausgewählt ist aus der Gruppe bestehend aus Wasserstoff, Halogen, C₁-C₅-Alkyl-, C₂-C₅-Alkenyl-, C₂-C₅-Alkinyl-, Morpholinyl-, Piperazinyl-, C₁-C₅-Alkyl-piperazinyl-, Heterocycloalkyl-, Cyclopropyl-, Cyclobutyl-, Cyclopentyl-, Cyclohexyl-, Norbornan-, Adamantan-, C₅-C₆-Cycloalkenylgruppen ; und C₁-C₅-Alkyl-, C₂-C₅-Alkenyl- und C₂-C₅-Alkinylgruppen, gegebenenfalls substituiert durch Gruppen, die aus ausgewählt sind aus der Gruppe bestehend aus Hydroxyl, Amino, C₁-C₅-Alkyl- oder C₂-C₅-Alkenylgruppen mit einem Stickstoffatom, das ein endständiges Kohlenstoffatom in der substituierten unverzweigte oder verzweigte Kette ersetzt, Halogen, C₁-C₅-Alkylgruppe mit einem Kohlenstoffatom, das durch ein Sauerstoffatom ersetzt wird, C₁-C₅-Alkylgruppe mit einer Carbonylamino gruppe oder Aminocarbonyl gruppe, die zwei Kohlenstoffatome in der verzweigten oder unverzweigten Kette ersetzt, Carboxyl-, Amid-, Hydroxyamid, -CONHCH₃, -NHCONH₂, -SO₂NH₂, -SO₂CH₃, -NHCOCH₃, - NHCSNH₂, -NHSO₂CH₃, und ein Radikal der Formel-OR¹² oder ein Radikal der Formel -C (O)-R¹², wobei R¹² ausgewählt ist aus der Gruppe bestehend aus C₁-C₅-Alkyl-, C₂-C₅-Alkenyl-, Cyclopropyl-, Cyclobutyl-, Cyclopentyl-, Cyclohexyl-, Norbornan, diamantene, und C₅-C₆-Cycloalkenylgruppen; und pharmazeutisch akzeptable Salze von diesen.

12. Verfahren nach Anspruch 10, wobei die Verbindung der folgenden chemischen Struktur entspricht: 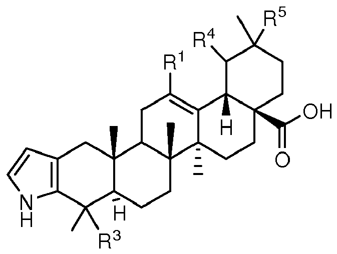 R¹ ist ausgewählt aus der Gruppe bestehend aus Wasserstoff, Methyl-, Halogen, C₁-C₅-Alkyl wobei ein oder mehrere Wasserstoffatome, die an einem Atom innerhalb der C₁-C₅-Alkylgruppe gebunden sind, durch 1, 2, 3 oder 4 Halogenatome, Nitril, C₁-C₅ -Alkyl-NitrilGruppe, substituierten oder nicht substituierten C₁-C₅-Alkylgruppe, substituierten oder nicht substituierten C₂-C₅-Alkenylgruppe, substituierten oder nicht substituierten C₂-C₅-Alkinylgruppe, Cyclopropyl-, Cyclobutyl-, Cyclopentyl-, Cyclohexyl-, Norbornan-, Diamantene, C₅-C₆-Cycloalkenyl-, substituierten oder nicht substituierten C₆-C₁₂Arylgruppe und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl-, Indolizinyl-, Indazolyl-, Dihydroindolyl-, Tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl- , Benzothienyl-, furopyridinyl-, Phthalazinyl-, napthyridinyl-, pyrazolopyridyl-, Pyrazolopyrimidinylgruppen, und ihre teilweise reduzierten Formen ; eine Heteroarylgruppe von 6 oder weniger Mitgliedern, die mit einer Arylgruppe von 6 oder weniger Mitgliedern fusioniert ist, oder zwei separate Heteroarylgruppen von 6 oder weniger Mitglieder, die fusioniert sind, R³ ausgewählt ist aus der Gruppe, bestehend aus Wasserstoff und Methyl; und einer von R⁴ und R⁵ Wasserstoff und das andere Methyl ; und Salze davon.

13. Verfahren nach Anspruch 10, wobei die Verbindung an das Anhänger chemischen Struktur entspricht:

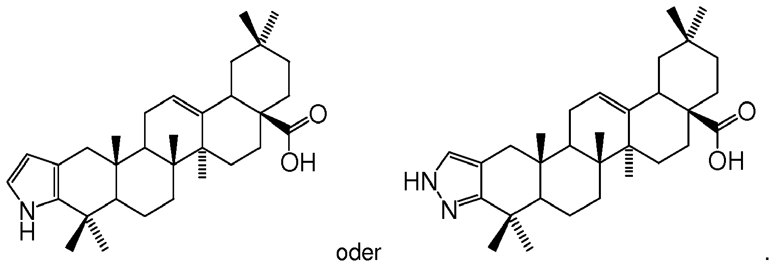

14. Eine Verbindung zur Verwendung in der Reduktion der Virulenz von gram-negativen Bakterien, die Verbindung entsprechend der folgenden chemischen Struktur : 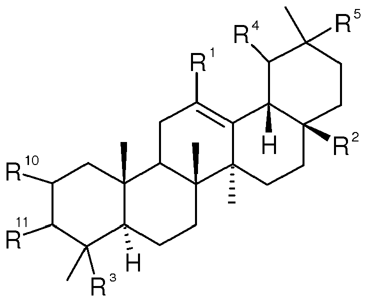
R¹ ist ausgewählt aus der Gruppe bestehend aus Wasserstoff, Methyl-, Halogen, C₁-C₅-Alkyl wobei ein oder mehrere Wasserstoffatome, die an einem Atom innerhalb der C₁-C₅-Alkylgruppe gebunden sind, durch 1, 2, 3 oder 4 Halogenatome, Nitril, C₁-C₅-Alkyl oder C₂-C₅-Alkenyl mit einer Nitrilgruppe, ein endständiges Kohlenstoffatom in der unverzweigten oder verzweigten Kette ersetzt, substituierten oder nicht substituierten C₁-C₅-Alkylgruppe, substituierten oder nicht substituierten C₂-C₅-Alkenylgruppe, substituierten oder nicht substituierten C₂-C₅-Alkinylgruppe, Cyclopropyl-, Cyclobutyl-, Cyclopentyl-, Cyclohexyl-, Norbornan-, Diamantene, C₅-C₆-Cycloalkenyl-, substituierten oder nicht substituierten C₆-C₁₂Arylgruppe und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl-, Indolizinyl-, Indazolyl-, Dihydroindolyl-, Tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen ersetzt ist; und deren partiell reduzierten Formen; oder eine Heteroarylgruppe von 6 oder weniger Mitgliedern, die mit einer Arylgruppe von 6 oder weniger Mitgliedern fusioniert ist, oder zwei separate Heteroarylgruppen von 6 oder weniger Mitglieder, die fusioniert sind; wobei R² ausgewählt ist aus der Gruppe bestehend aus Carboxyl-, Amid-, Hydroxyamid,-methylamid, CH₂N(CH₃)₂, -CH₂NR⁶R⁷, 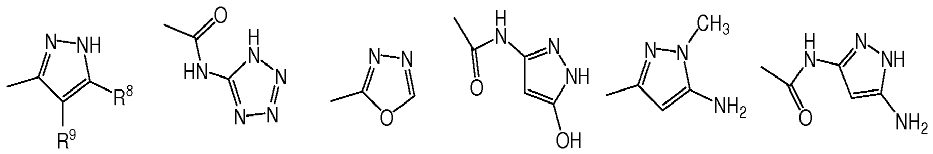 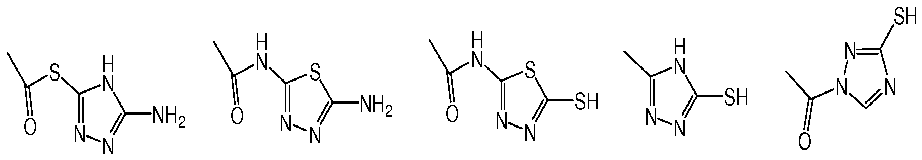 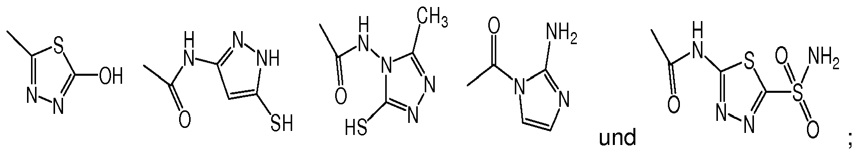
R³ ist ausgewählt aus der Gruppe bestehend aus Wasserstoff und Methyl;wobei die eine der Gruppen R⁴ und R⁵ Wasserstoff ist und die andere Methyl; R⁶ und R⁷ sind unabhängig voneinander ausgewählt aus der Gruppe bestehend aus Wasserstoff, Methyl-, Ethyl-, Propyl-, Isopropyl und Butyl; wobei R⁸ ausgewählt ist aus der Gruppe bestehend aus Hydroxyl-, Amin, -N(CH₃)₂, and -NHCH₃; und wobei R⁹ ausgewählt ist aus der Gruppe bestehend aus Wasserstoff, Halogen, C₁-C₅-Alkyl-, C₂-C₅-Alkenyl-, C₂-C₅-Alkinyl-, Morpholinyl-, Piperazinyl-, C₁-C₅-Alkyl-piperazinyl-, Heterocycloalkyl-, Cyclopropyl-, Cyclobutyl-, Cyclopentyl-, Cyclohexyl- , Norbornan-, Adamantan-, C₅-C₆-Cycloalkenylgruppen ; und C₁-C₅-Alkyl-, C₂-C₅-Alkenyl- und C₂-C₅-Alkinylgruppen, gegebenenfalls substituiert durch Gruppen, die aus ausgewählt sind aus der Gruppe bestehend aus Hydroxyl, Amino, C₁-C₅-Alkyl- oder C₂-C₅-Alkenylgruppen mit einem Stickstoffatom, das ein endständiges Kohlenstoffatom in der substituierten unverzweigte oder verzweigte Kette ersetzt, Halogen, C₁-C₅-Alkylgruppe mit einem Kohlenstoffatom, das durch ein Sauerstoffatom ersetzt wird, C₁-C₅-Alkylgruppe mit einer Carbonylamino gruppe oder Aminocarbonyl gruppe, die zwei Kohlenstoffatome in der verzweigten oder unverzweigten Kette ersetzt, Carboxyl-, Amid-, Hydroxyamid, -CONHCH₃, - NHCONH₂, -SO₂NH₂, -SO₂CH₃, -NHCOCH₃, -NHCSNH₂, -NHSO₂CH₃, und ein Radikal der Formel-OR¹² oder ein Radikal der Formel -C (O)-R¹², wobei R¹² ausgewählt ist aus der Gruppe bestehend aus C₁-C₅-Alkyl-, C₂-C₅-Alkenyl-, Cyclopropyl-, Cyclobutyl-, Cyclopentyl-, Cyclohexyl-, Norbornan, diamantene, und C₅-C₆-Cycloalkenylgruppen; und pharmazeutisch akzeptable Salze von diesen, ; und wobei R¹⁰ und R¹¹ zusammen mit den Kohlenstoffatomen, an die sie gebunden sind, einen aromatischen N- Heterocyclus, der substituiert oder nicht substituiert ist, ausgewählt aus der Gruppe bestehend aus Indol, Imidazol, Chinolin, Isochinolin, Pyridin, Triazin, Pyrazin, Pyrimidin, Pyridazin, Isoindols, Pyrrol, Benzimidazol, Purin, Pyrazol, Benzopyrazol, Indazol, Chinoxalin, Acridin, Chinazolin, Indolizin, Carbazol und Cinnolin ; und pharmazeutisch akzeptable Salze davon.

15. Verbindung nach Anspruch 14 zur Verwendung in der Reduktion der Virulenz von gram-negativen Bakterien, die Verbindung entsprechend der folgenden chemischen Struktur : 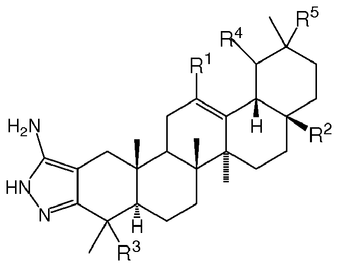
R¹ ist ausgewählt aus der Gruppe bestehend aus Wasserstoff, Methyl , Halogen, C₁-C₅-Alkyl wobei ein oder mehrere Wasserstoffatome, die an einem Atom innerhalb der C₁-C₅-Alkylgruppe gebunden sind, durch 1, 2, 3 oder 4 Halogenatome, Nitril, C₁-C₅-Alkyl oder C₂-C₅-Alkenyl mit einer Nitrilgruppe, ein endständiges Kohlenstoffatom in der unverzweigten oder verzweigten Kette ersetzt, substituierten oder nicht substituierten C₁-C₅-Alkylgruppe, substituierten oder nicht substituierten C₂-C₅-Alkenylgruppe, substituierten oder nicht substituierten C₂-C₅-Alkinylgruppe, Cyclopropyl-, Cyclobutyl-, Cyclopentyl-, Cyclohexyl-, Norbornan-, Diamantene, C₅-C₆-Cycloalkenyl-, substituierten oder nicht substituierten C₆-C₁₂Arylgruppe und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl-, Indolizinyl-, Indazolyl-, Dihydroindolyl-, Tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl-, Benzothienyl-, furopyridinyl-, Phthalazinyl-, napthyridinyl-, pyrazolopyridyl-, Pyrazolopyrimidinylgruppen ersetzt ist; und deren partiell reduzierten Formen; oder eine Heteroarylgruppe von 6 oder weniger Mitgliedern, die mit einer Arylgruppe von 6 oder weniger Mitgliedern fusioniert ist, oder zwei separate Heteroarylgruppen von 6 oder weniger Mitglieder, die fusioniert sind; wobei R² ausgewählt ist aus der Gruppe bestehend aus Carboxyl-, Amid-, Hydroxyamid,-methylamid, CH₂N(CH₃)₂, -CH₂NR⁶R⁷, 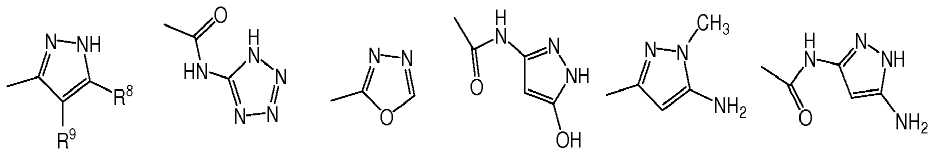 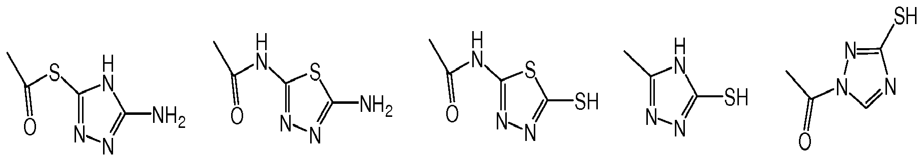 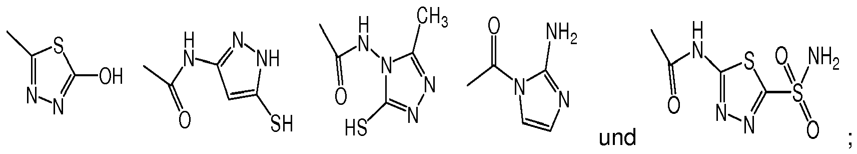
R¹ ist ausgewählt aus der Gruppe bestehend aus Wasserstoff und Methyl ; wobei die eine der Gruppen R⁴ und R⁵ Wasserstoff ist und die andere Methyl; R⁶ und R⁷ sind unabhängig voneinander ausgewählt aus der Gruppe bestehend aus Wasserstoff, Methyl-, Ethyl-, Propyl-, Isopropyl- und Butylgruppen ; wobei R⁸ ausgewählt ist aus der Gruppe bestehend aus Hydroxyl-, Amin, -N(CH₃)₂, and -NHCH₃; und wobei R⁹ ausgewählt ist aus der Gruppe bestehend aus Wasserstoff, Halogen, C₁-C₅-Alkyl-, C₂-C₅-Alkenyl-, C₂-C₅-Alkinyl-, Morpholinyl-, Piperazinyl-, C₁-C₅-Alkyl-piperazinyl-, Heterocycloalkyl-, Cyclopropyl-, Cyclobutyl-, Cyclopentyl-, Cyclohexyl-, Norbornan-, Adamantan-, C₅-C₆-Cycloalkenylgruppen ; und C₁-C₅-Alkyl-, C₂-C₅-Alkenyl- und C₂-C₅-Alkinylgruppen, gegebenenfalls substituiert durch Gruppen, die aus ausgewählt sind aus der Gruppe bestehend aus Hydroxyl, Amino, C₁-C₅-Alkyl- oder C₂-C₅-Alkenylgruppen mit einem Stickstoffatom, das ein endständiges Kohlenstoffatom in der substituierten unverzweigte oder verzweigte Kette ersetzt, Halogen, C₁-C₅-Alkylgruppe mit einem Kohlenstoffatom, das durch ein Sauerstoffatom ersetzt wird, C₁-C₅-Alkylgruppe mit einer Carbonylamino gruppe oder Aminocarbonyl gruppe, die zwei Kohlenstoffatome in der verzweigten oder unverzweigten Kette ersetzt, Carboxyl-, Amid-, Hydroxyamid, -CONHCH₃, -NHCONH₂, -SO₂NH₂, -SO₂CH₃, -NHCOCH₃, - NHCSNH₂, -NHSO₂CH₃, und ein Radikal der Formel-OR¹² oder ein Radikal der Formel -C (O)-R¹², wobei R¹² ausgewählt ist aus der Gruppe bestehend aus C₁-C₅-Alkyl-, C₂-C₅-Alkenyl-, Cyclopropyl-, Cyclobutyl-, Cyclopentyl-, Cyclohexyl-, Norbornan, diamantene, und C₅-C₆-Cycloalkenylgruppen; und pharmazeutisch akzeptable Salze von diesen.

16. Verbindung nach Anspruch 14 zur Verwendung in der Reduktion der Virulenz von gram-negativen Bakterien, die Verbindung entsprechend der folgenden chemischen Struktur : 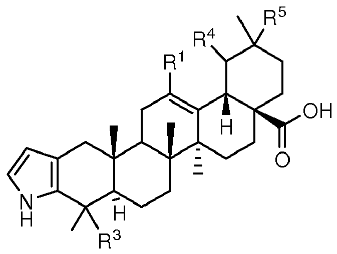 R¹ ist ausgewählt aus der Gruppe bestehend aus Wasserstoff, Methyl-, Halogen, C₁-C₅-Alkyl wobei ein oder mehrere Wasserstoffatome, die an einem Atom innerhalb der C₁-C₅-Alkylgruppe gebunden sind, durch 1, 2, 3 oder 4 Halogenatome, Nitril, C₁-C₅ -Alkyl-NitrilGruppe, substituierten oder nicht substituierten C₁-C₅-Alkylgruppe, substituierten oder nicht substituierten C₂-C₅-Alkenylgruppe, substituierten oder nicht substituierten C₂-C₅-Alkinylgruppe, Cyclopropyl-, Cyclobutyl-, Cyclopentyl-, Cyclohexyl-, Norbornan-, Diamantene, C₅-C₆-Cycloalkenyl-, substituierten oder nicht substituierten C₆-C₁₂Arylgruppe und substituierten oder nicht substituierten Pyrrolyl-, Pyrazolyl-, Imidazolyl-, Oxazolyl-, Isoxazolyl-, Oxadiazolyl-, Thiazolyl-, Isothiazolyl-, Thienyl-, Furanyl-, Furazanyl-, Pyridinyl-, Pyrimidinyl-, Pyridazinyl-, Indolyl-, 3H-Indolyl-, Isoindolyl-, Indolinyl-, Indolizinyl-, Indazolyl-, Dihydroindolyl-, Tetrahydroindolyl-, Purinyl-, Pyrazinyl-, Chinolinyl-, Isochinolinyl-, Tetrahydroisochinolinyl-, Chinoxalinyl-, chinazolinyl-, Cinnolinyl-, Pteridinyl-, benzimidazolyl-, Benzopyranyl-, benzoxazolyl-, Benzisoxazolyl-, benzofuranyl-, Isobenzofuranyl-, benzothiazolyl-, Benzisothiazolyl- , Benzothienyl-, furopyridinyl-, Phthalazinyl-, napthyridinyl-, pyrazolopyridyl-, Pyrazolopyrimidinylgruppen, und ihre teilweise reduzierten Formen ; eine Heteroarylgruppe von 6 oder weniger Mitgliedern, die mit einer Arylgruppe von 6 oder weniger Mitgliedern fusioniert ist, oder zwei separate Heteroarylgruppen von 6 oder weniger Mitglieder, die fusioniert sind, R³ ausgewählt ist aus der Gruppe, bestehend aus Wasserstoff und Methyl; und einer von R⁴ und R⁵ Wasserstoff und das andere Methyl ; und Salze davon.

## Revendications

1. Composé selon la structure chimique suivante : 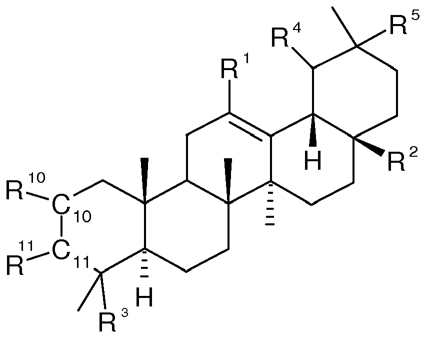
dans laquelle R¹⁰ et R¹¹ forment avec les atomes de carbone auxquels ils sont liés 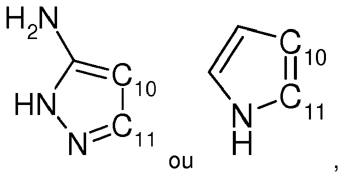
et lorsque R¹⁰ et R¹¹ forment avec les atomes de carbone auxquels ils sont liés 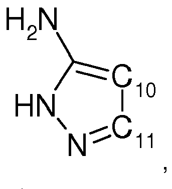
R¹ est choisi dans le groupe constitué par un hydrogène, un méthyle, un halogénure, un C₁-C₅-alkyle dans lequel un ou plusieurs atomes d'hydrogène liés à un atome du C₁-C₅-alkyle sont remplacés par 1, 2, 3 ou 4 atomes d'halogénure, un nitrile, un C₁-C₅-alkyle ou C₂-C₅-alcényle avec un groupe nitrile en remplacement d'un atome terminal de carbone dans la chaîne principale ou ramifiée, un C₁-C₅ alkyle substitué ou non, un C₂-C₅ alcényle substitué ou non, un C₂-C₅ alcynyle substitué ou non, un cyclopropyle, un cyclobutyle, un cyclopentyle, un cyclohexyle, un norbornane, un adamantine, un C₅-C₆ cycloalcényle substitué ou non, un C₆-C₁₂ aryle substitué ou non ; un groupe pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H-indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinoléinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leur formes substituées ou non et leur formes partiellement réduites ; un hétéroaryle de 6 membres ou moins fusionné avec un groupe aryle de 6 membres ou moins, deux groupes distincts d'hétéroaryle de 6 membres ou moins fusionnés; R² est choisi dans un groupe constitué par un carboxyle, un amide, un hydroxyamide, un méthylamide, - CH₂N(CH₃)₂, -CH₂NR⁶R⁷, 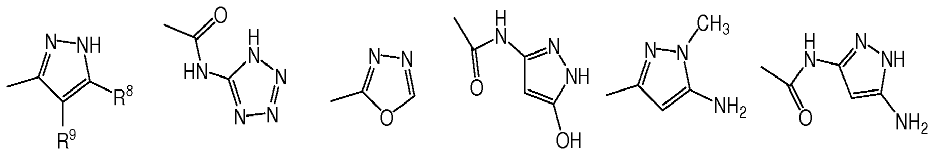 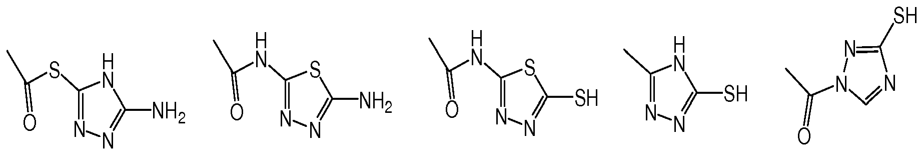 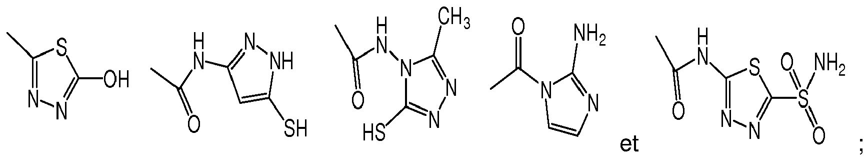
R³ est choisi dans le groupe constitué par un hydrogène et un groupe méthyle; l'un quelconque de R⁴ et R⁵ est un atome d'hydrogène et l'autre représente un groupe méthyle; R⁶ et R⁷ sont indépendamment choisis dans le groupe constitué par un hydrogène, un méthyle, un éthyle, un propyle, un isopropyle, et un butyle; R⁸ est choisi dans le groupe consistant en un hydroxyle, un amino, -N(CH₃)₂, et NHCH₃ ; et R⁹ est choisi dans le groupe constitué par un hydrogène, un halogénure, un C₁-C₅-alkyle, C₂-C₅-alcényle, C₂-C₅-alcynyle, un morpholinyle, un pipérazinyle, un C₁-C₅ alkyle pipérazinyle, un hétérocycloalkyle, un cyclopropyle, un cyclobutyle, un cyclopentyle, un cyclohexyle, un norbornane, un adamantine, un C₅-C₆-cycloalcényle ; un C₁-C₅-alkyle, un C₂-C₅-alcényle ou un C₂-C₅-alcynyle, éventuellement substitués par des groupements choisis parmi un hydroxyle, un amino, un C₁-C₅-alkyle ou un C₂-C₅-alcényle avec un atome d'azote remplaçant un atome de carbone terminal dans la chaîne principale ou ramifiée, un halogénure, un C₁-C₅-alkyle avec un atome de carbone remplacé par un atome d'oxygène, un C₁-C₅-alkyle avec un carbonylamino ou un aminocarbonyle remplaçant deux atomes de carbone dans la chaîne principale ou ramifiée, un carboxyle, un amide, un hydroxyamide, CONHCH₃, - NHCONH₂, - SO₂NH₂, - SO₂CH₃, - NHCOCH₃, - NHCSNH₂, -NHSO₂CH₃, un radical de formule -OR¹² ou un radical de formule -C(O)-R¹², dans lequel R¹² est choisi parmi un C₁-C₅-alkyle, un C₂-C₅-alcényle, un cyclopropyle, un cyclobutyle, un cyclopentyle, un cyclohexyle, un norbornane, un adamantine, et un C₅-C₆-cycloalcényle; et leurs sels pharmaceutiquement acceptables,
ou
lorsque R¹⁰ et R¹¹ forment avec les atomes de carbone auxquels ils sont liés 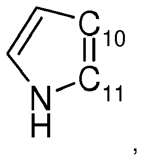
R¹ est choisi dans le groupe constitué par un hydrogène, un méthyle, un halogénure, un nitrile, un C₁-C₅-alkyle substitué ou non, un C₂-C₅-alcényle substitué ou non, un C₂-C₅-alcynyle substitué ou non, un C₂-C₅-aryle substitué ou non ; un pyrrolyle, un pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyle, phtalazinyle, napthyridinyle, pyrazolopyridyle, pyrazolopyrimidinyle, leurs formes substituées ou non, et leur formes partiellement réduites ; ou un hétéroaryle de 6 membres ou moins fusionné avec un groupe aryle de 6 membres ou moins, ou deux groupes distincts d'hétéroaryle de 6 membres ou moins fusionnés ; R² est un groupe carboxyle ; R³ est choisi dans le groupe constitué par un hydrogène et un groupe méthyle; et l'un quelconque de R⁴ et R⁵ est un atome d'hydrogène et l'autre représente un groupement méthyle; et leurs sels pharmaceutiquement acceptables.

2. Composé selon la revendication 1, le composé correspondant à la structure chimique suivante : 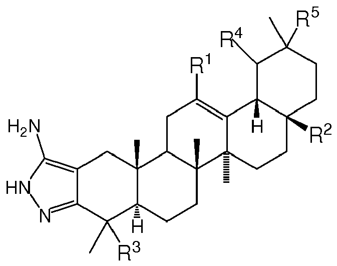 dans laquelle R¹ est choisi dans le groupe constitué par un méthyle, un halogénure, un groupe C₁-C₅-alkyle dans lequel un ou plusieurs atomes d'hydrogène liés à un atome du groupe C₁-C₅-alkyle sont remplacés par 1, 2, 3 ou 4 atomes d'halogénure, un nitrile, un groupe C₁-C₅-alkyle ou C₂-C₅-alcényle avec un groupe nitrile remplaçant un atome terminal de carbone dans la chaîne principale ou ramifiée, un C₁-C₅-alkyle substitué ou non, C₂-C₅-alcényle substitué ou non, C₂-C₅-alcynyle substitué ou non, cyclopropyle, cyclobutyle, cyclopentyle, cyclohexyle, norbornane, adamantine, C₅-C₆-cycloalcényle, C₆-C₁₂-aryle substitué ou non ; pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, pyrrolopyridinyle, tetrahydropyrrolopyridinyl, éventuellement substitués par des groupements choisis parmi un C₁-C₅-alkyle, un halogénure, un C₁-C₅-alkyle dans lequel un ou plusieurs hydrogènes attachés à un atome du groupe C₁-C₅-alkyle sont remplacés par 1, 2, 3 ou 4 atomes halogénure, un hydroxyle, un amino, un C₁-C₅-alkyle ou un C₂-C₅-alcényle avec un atome d'azote remplaçant un atome de carbone terminal dans la chaîne principale ou ramifiée, un groupe C₁-C₅-alkyle ou C₂-C₅-alcényle dans lequel un ou plusieurs atomes d'hydrogène liés à un atome du C₁-C₅-alkyle ou du C₂-C₅-alcényle sont remplacés par un ou plusieurs groupes hydroxyle, nitrile, C₁-C₅ alkyle ou C₂-C₅-alcényle avec un groupement nitrile remplaçant un atome de carbone terminal dans la chaîne principale ou ramifiée, un radical de formule R'OR", dans lequel R' est un groupe C₁-C₅-alkyle, C₂-C₅-alcényle ou C₂-C₅-alcynyle, et R" est un groupe C₁-C₅-alkyle, C₂-C₅-alcényle, C₂-C₅-alcynyle, C₆-C₁₂-aryle, pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, et leur formes partiellement réduites ; ou un hétéroaryle de 6 membres ou moins fusionné avec un groupe aryle de 6 membres ou moins, ou deux groupes distincts d'hétéroaryle de 6 membres ou moins fusionnés ou un hétérocycloalkyle; un radical de formule -OR¹² ou un radical de formule -C(O)-R¹², dans lequel R¹² est choisi dans le groupe constitué d'un C₁-C₅-alkyle, C₂-C₅-alcényle, cyclopropyle, cyclobutyle, cyclopentyle, cyclohexyle, norbornane, adamantine, un C₅-C₆-cycloalcényle, un groupe C₁-C₅-alkyle avec un atome de carbone remplacé par un atome d'oxygène, un groupe C₁-C₅-alkyle avec un carbonylamino ou aminocarbonyle remplaçant deux atomes de carbone dans la chaîne principale ou ramifiée, et un thioalkyle.

3. Composé selon la revendication 1, le composé correspondant à la structure chimique suivante : 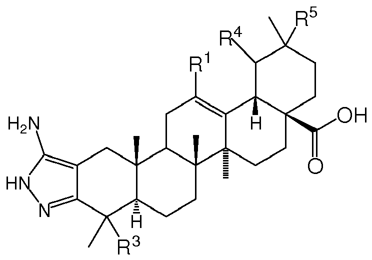 dans laquelle R¹ est choisi dans le groupe constitué par un atome d'hydrogène, un halogénure, un groupe C₁-C₅-alkyle substitué ou non, C₂-C₅-alcényle substitué ou non, cyclopropyle, cyclobutyle, cyclopentyle, cyclohexyle, norbornane, adamantine, C₅-C₆-cycloalcényle, C₆-C₁₂-aryle substitué ou non ; pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinoléinyle, quinoxalinyle, quinazolinyle, cinnolinyle, ptéridinyle, benzimidazolyle, benzopyranyle, benzoxazolyl, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées ou non, et leur formes partiellement réduites ; ou un hétéroaryle de 6 membres ou moins fusionné avec un groupe aryle de 6 membres ou moins, ou deux groupes distincts d'hétéroaryle de 6 membres ou moins fusionnés, et de préférence R¹ est un atome d'hydrogène.

4. Composé selon la revendication 3, R¹ étant choisi dans le groupe constitué par un halogénure, un groupe C₁-C₅-alkyle substitué ou non, C₂-C₅-alcényle substitué ou non, cyclopropyle, cyclobutyle, cyclopentyle, cyclohexyle, norbornane, adamantine, C₅-C₆-cycloalkényle, C₆-C₁₂-aryle substitué ou non ; pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées ou non, et leur formes partiellement réduites ; ou un hétéroaryle de 6 membres ou moins condensé avec un groupe aryle de 6 membres ou moins, ou deux groupes distincts d'hétéroaryle de six membres ou moins fusionnés ensemble, de préférence R¹ est choisi dans le groupe consistant en pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, pyrrolopyridinyle, tetrahydropyrrolopyridinyle, éventuellement substitués de groupements choisis parmi un C₁-C₅-alkyle, un halogénure, un C₁-C₅-alkyle dans lequel un ou plusieurs atomes d'hydrogène liés à un atome du C₁-C₅-alkyle sont remplacés par 1, 2, 3 ou 4 atomes halogénure, un hydroxyle, un amino, C₁-C₅-alkyle ou C₂-C₅-alcényle avec un atome d'azote remplaçant un atome de carbone terminal dans la chaîne principale ou ramifiée, un groupe C₁-C₅-alkyle ou C₂-C₅-alcényle dans lequel un ou plusieurs atomes d'hydrogène liés à un atome du C₁-C₅-alkyle en ou du C₂-C₅-alcényle est remplacée par un ou plusieurs groupes hydroxyle, un nitrile, un C₁-C₅-alkyle ou un C₂-C₅-alcényle avec un groupement nitrile remplaçant un atome de carbone terminal dans la chaîne principale ou ramifiée, un radical de formule R'OR", dans lequel R' est un groupe C₁-C₅-alkyle, C₂-C₅-alcényle ou C₂-C₅-alcynyle, et R" représente un groupe C₁-C₅-alkyle, C₂-C₅-alcényle, C₂-C₅-alcynyle, C₆-C₁₂-aryle, pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, et leur formes partiellement réduites ; ou un hétéroaryle de 6 membres ou moins fusionné avec un groupe aryle de 6 membres ou moins, ou deux groupes distincts d'hétéroaryle de six membres ou moins fusionnés ou un hétérocycloalkyle, un groupe C₁-C₅-alkyle avec un atome de carbone remplacé par un atome d'oxygène, un C₁-C₅-alkyle avec un carbonylamino ou un aminocarbonyle remplaçant deux atomes de carbone dans la chaîne principale ou ramifiée, un radical de formule -OR¹² ou un radical de formule -C(O)-R¹², dans lequel R¹² est choisi dans le groupe constitué par C₁-C₅-alkyle, C₂-C₅-alcényle, cyclopropyle, cyclobutyle, cyclopentyle, cyclohexyle, norbornane, adamantine, C₅-C₆-cycloalcényle, et thioalkyle.

5. Composé selon la revendication 1, le composé correspondant à la structure chimique suivante : 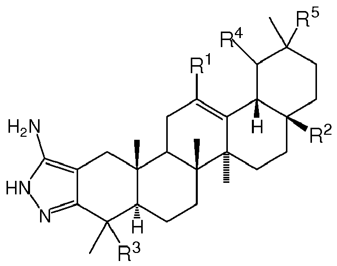 dans laquelle R¹ est choisi dans le groupe constitué par un atome d'hydrogène, un halogénure, un C1-C5-alkyle substitué ou non, un C2-C5-alcényle substitué ou non, un C5-C6-cycloalcényle, un C6-C12-aryle substitué ou non ; un groupe pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinoléinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées ou non, et leurs formes partiellement réduites ; ou un hétéroaryle de 6 membres ou moins fusionné avec un groupe aryle de 6 membres ou moins, ou deux groupes distincts d'hétéroaryle de 6 membres ou moins fusionnés ; et dans laquelle R² est choisi dans le groupe constitué par un amide, un hydroxyamide, méthylamide, -CH₂N(CH₃)₂, -CH₂NR⁶R⁷,

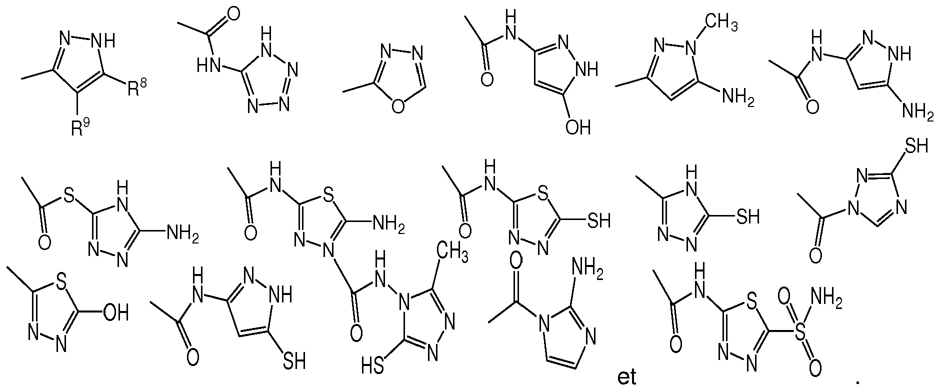

6. Composé selon la revendication 5, R¹ étant choisi parmi le groupe constitué de pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, et tetrahydroindolyl, éventuellement substitués avec un groupement choisi parmi un C₁-C₅-alkyle, un halogénure, un C₁-C₅-alkyle dans lequel un ou plusieurs atomes d'hydrogène liés à un atome du C₁-C₅-alkyle est remplacé par 1, 2, 3 ou 4 atomes d'halogénure, un hydroxyle, un amino, C₁-C₅-alkyle ou C₂-C₅-alcényle avec un atome d'azote remplaçant un atome de carbone terminal dans la chaîne principale ou ramifiée, un groupe C₁-C₅-alkyle ou C₂-C₅-alcényle dans lequel un ou plusieurs atomes d'hydrogène liés à un atome du C₁-C₅-alkyle ou du C₂-C₅-alcényle sont remplacés par un ou plusieurs groupes hydroxyle, nitrile, C₁-C₅-alkyle ou C₂-C₅-alcényle avec un groupe nitrile remplaçant un carbone terminal dans la chaîne principale ou ramifiée, un radical de formule R'OR ", dans lequel R' est un groupe C₁-C₅-alkyle, C₂-C₅-alcényle ou C₂- C₅-alcynyle et R" est un groupe C₁-C₅-alkyle, C₂-C₅-alcényle, C₂-C₅-alcynyle, aryle, pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, et leur formes partiellement réduites ; ou un hétéroaryle de 6 membres ou moins fusionné avec un groupe aryle de 6 membres ou moins, deux groupes distincts d'hétéroaryle de six membres ou moins fusionnés ou un hétérocycloalkyle, un radical de formule -OR¹² ou un radical de formule -C(O)- R¹², dans lequel R¹² est choisi dans le groupe constitué d'un C₁-C₅-alkyle, C₂-C₅-alcényle, cyclopropyle, cyclobutyle, cyclopentyle, cyclohexyle, norbornane, adamantine, ou C₅-C₆-cycloalcényle; un groupe C₁-C₅-alkyle avec un atome de carbone remplacé par un atome d'oxygène, un groupe C₁-C₅-alkyle avec un carbonylamino ou un aminocarbonyle remplaçant deux atomes de carbone dans la chaîne principale ou ramifiée, et un thioalkyle.

7. Composé selon la revendication 1, le composé correspondant à la structure chimique suivante : 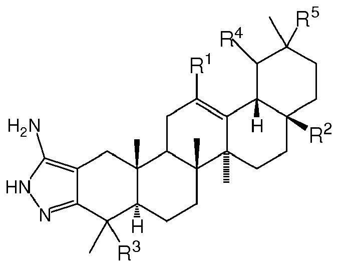
dans laquelle R2 est 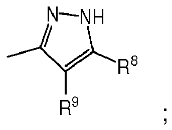
Et dans laquelle R⁸ est choisi dans le groupe constitué des groupes hydroxyle et amino; et dans laquelle R⁹ est choisi dans le groupe constitué par un atome d'hydrogène, un halogénure, un C₁-C₅-alkyle, C₂-C₅-alcényle, C₂-C₅-alcynyle, un morpholinyle, un pipérazinyle, un C1-C5-alkyle pipérazinyle, hétérocycloalkyle, cyclopropyle, cyclobutyle, cyclopentyle, cyclohexyle, norbornane, adamantine, C₅-C₆-cycloalcényle ; un groupe C₁-C₅-alkyle, C₂-C₅-alcényle, ou C₂-C₅-alcynyle éventuellement substitués par des groupements choisis parmi un hydroxyle, un amino, C₁-C₅-alkyle ou C₂-C₅-alcényle avec un atome d'azote remplaçant un atome de carbone terminal dans la chaîne principale ou ramifiée, un halogénure, un radical de formule - OR¹² ou un radical de formule - C(O)-R¹², dans laquelle R¹² est choisi dans le groupe constitué d'un C₁-C₅-alkyle, C₂-C₅-alcényle, cyclopropyle, cyclobutyle, cyclopentyle, cyclohexyle, norbornane, adamantin, et C₅-C₆-cycloalcényle ; un C₁-C₅-alkyle avec un atome de carbone remplacé par un atome d'oxygène, un C₁-C₅-alkyle avec un carbonylamino ou un aminocarbonyle remplaçant deux atomes de carbone dans la chaîne principale ou ramifiée, carboxyle, amide, hydroxyamide, -CONHCH₃, -NHCONH₂, -SO₂NH₂, - SO₂CH₃, -NHCOCH₃, -NHCSNH₂, et -NHSO₂CH₃, de préférence R⁹ représente un atome d'hydrogène; et leurs sels pharmaceutiquement acceptables.

8. Composé selon la revendication 7, R¹ étant choisi dans le groupe constitué par un pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H-indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées et leur formes partiellement réduites ; ou un hétéroaryle de 6 membres ou moins fusionné avec un groupe aryle de 6 membres ou moins, ou deux groupes distincts d'hétéroaryle de six membres ou moins fusionnés, R² étant 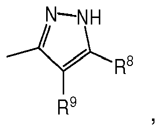 R⁸ étant choisi dans le groupe constitué des groupes hydroxyle et amino; et R⁹ étant un atome d'hydrogène.

9. Composé selon la revendication 1, le composé correspondant à la structure chimique suivante : 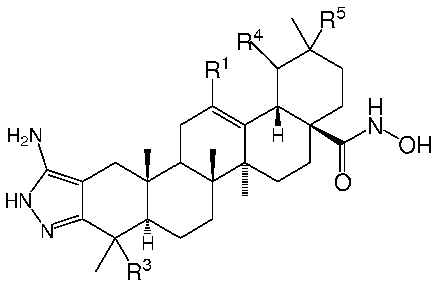 dans laquelle R¹ est choisi dans le groupe constitué par un atome d'hydrogène, un méthyle, un halogénure, un C₁-C₅-alkyle substitué ou non, un C₂-C₅-alcényle substitué ou non, un C₆-C₁₂-aryle substitué ou non ; pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H-indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées ou non, et leur formes partiellement réduites ; ou un hétéroaryle de 6 membres ou moins fusionné avec un groupe aryle de 6 membres ou moins, ou deux groupes distincts d'hétéroaryle de 6 membres ou moins fusionnés.

10. Procédé pour inhiber ou réduire la formation ou la croissance d'un biofilm, comprenant la mise en contact avec ledit biofilm ou avec une cellule capable de former ou développer ledit biofilm d'une quantité efficace d'un composé ou d'une composition comprenant le composé correspondant à la structure chimique suivante : 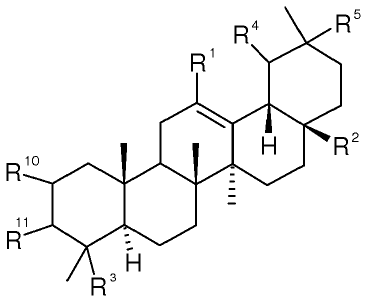
dans laquelle R¹ est choisi dans le groupe constitué par un atome d'hydrogène, un méthyle, un halogénure, un C₁-C₅-alkyle dans lequel un ou plusieurs atomes d'hydrogène liés à un atome du groupe C₁-C₅-alkyle sont remplacés par 1, 2, 3 ou 4 atomes d'halogénure, un nitrile, un C₁-C₅-alkyle ou C₂-C₅-alcényle avec un groupe nitrile remplaçant un atome de carbone teminal dans la chaîne principale ou ramifiée, un C₁-C₅-alkyle substitué ou non, un C₂-C₅-alcényle substitué ou non, un C₂-C₅-alcynyle substitué ou non, un cyclopropyle, un cyclobutyle, un cyclopentyle, un cyclohexyle, un norbornane, un adamantine, un C₅-C₆-cycloalcényle, un C₆-C₁₂-aryle substitué ou non ; un pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées ou non, et leur formes partiellement réduites ; ou un hétéroaryle de 6 membres ou moins fusionné avec un groupe aryle de 6 membres ou moins, ou deux groupes distincts d'hétéroaryle de 6 membres ou moins fusionnés,
dans laquelle R² est choisi dans le groupe constitué par un groupe carboxyle, amide, hydroxyamide, méthylamide, -CH₂N(CH₃)₂, -CH₂NR⁶R⁷, 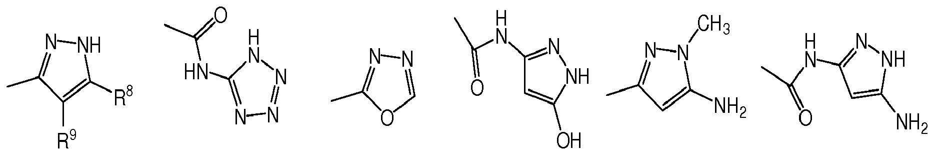 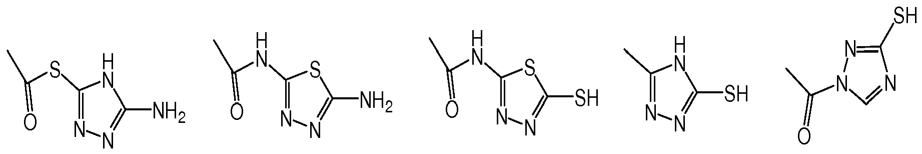 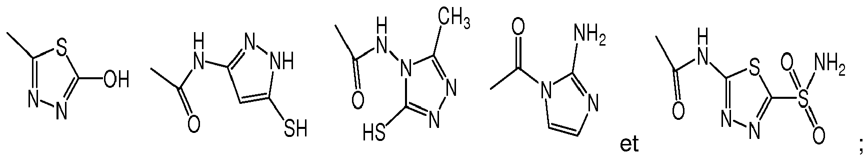
dans laquelle R³ est choisi dans le groupe constitué par un hydrogène et un méthyle ; dans laquelle l'un quelconque de R⁴ et R⁵ est un atome d'hydrogène et l'autre représente un méthyle; dans laquelle R⁶ et R⁷ sont indépendamment choisis parmi un atome d'hydrogène, un méthyle, un éthyle, un propyle, un isopropyle, et un butyle ; dans laquelle R⁸ est choisi dans le groupe consistant en un hydroxyle, un amino, -N(CH₃)₂, et NHCH₃ ; dans laquelle R⁹ est choisi dans le groupe constitué par un atome d'hydrogène, un halogénure, un C₁-C₅-alkyle, un C₂-C₅-alcényle, un C₂-C₅-alcynyle, un morpholinyle, un pipérazinyle, un C₁-C₅-alkyle pipérazinyle, hétérocycloalkyle, cyclopropyle, cyclobutyle, cyclopentyle, cyclohexyle, norbornane, adamantine, C₅-C₆-cycloalcényle, un C₁-C₅-alkyle, un C₂-C₅-alcényle et un C₂-C₅-alcynyle éventuellement substitués par des groupements choisis parmi un hydroxyle, un amino, un C₁-C₅-alkyle ou un C₂-C₅-alcényle avec un atome d'azote remplaçant un atome de carbone terminal dans la chaîne principale ou ramifiée, un halogénure, un C₁-C₅-alkyle avec un atome de carbone remplacé par un atome d'oxygène, un C₁-C₅-alkyle avec un carbonylamino ou un aminocarbonyle remplaçant deux atomes de carbone dans la chaîne principale ou ramifiée, un carboxyle, un amide, un hydroxyamide, -CONHCH₃, -NHCONH₂, - SO₂NH₂, -SO₂CH₃, -NHCOCH₃, -NHCSNH₂, NHSO₂CH₃, un radical de formule - OR¹² ou un radical de formule -C(O)-R¹², dans lequel R¹² est choisi dans le groupe constitué d'un C₁-C₅-alkyle, C₂-C₅-alcényle, cyclopropyle, cyclobutyle, cyclopentyle, cyclohexyle, norbornane, adamantine, un C₅-C₆-cycloalcényle ; et dans lequel R¹⁰ et R¹¹ avec les atomes de carbone auxquels ils sont liés forment un N- hétérocycle aromatique substitué ou non et choisi dans le groupe constitué par un indole, imidazole, quinoléine, isoquinoléine, pyridine, triazine, pyrazine, pyrimidine, pyridazine attachés, isoindole, pyrrole, benzimidazole, purine, pyrazole, benzopyrazole, indazole, quinoxaline, acridine, quinazoline, indolizine, carbazole et cinnoline ; et les sels pharmaceutiquement acceptables de ceux-ci,
à la condition que le procédé ne soit pas employé sur un corps humain ou animal.

11. Procédé selon la revendication 10, dans lequel le composé correspond à la structure chimique suivante: 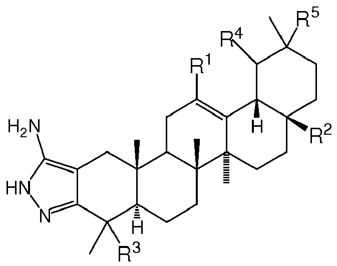
dans laquelle R¹ est choisi dans le groupe constitué par un atome d'hydrogène, un méthyle, un halogénure, un C₁-C₅-alkyle dans lequel un ou plusieurs atomes d'hydrogène liés à un atome du C₁-C₅-alkyle sont remplacés par 1, 2, 3, ou 4 atomes d'halogénure, un nitrile, un C₁-C₅-alkyle nitrile, un C1-C5 alkyle substitué ou non, un C₂-C₅-alcényle substitué ou non, un C₂-C₅-alcynyle substitué ou non, un cyclopropyle, un cyclobutyle, un cyclopentyle, un cyclohexyle, un norbornane, un adamantine, un C₅-C₆-cycloalkényle, un C₆-C₁₂-aryle substitué ou non ; pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées ou non, et leur formes partiellement réduites ; ou un hétéroaryle de 6 membres ou moins fusionné avec un groupe aryle de 6 membres ou moins, ou deux groupes distincts d'hétéroaryle de 6 membres ou moins fusionnés ; dans laquelle R² est choisi dans le groupe constitué par un groupe carboxyle, amide, hydroxyamide, méthylamide, -CH₂N(CH₃)₂, -CH₂NR⁶R⁷, 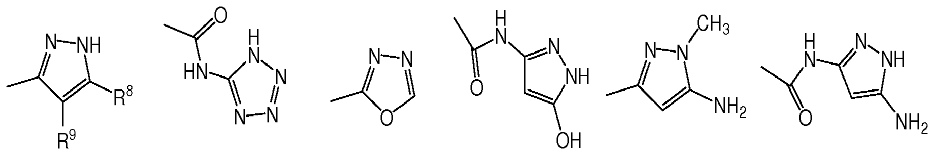 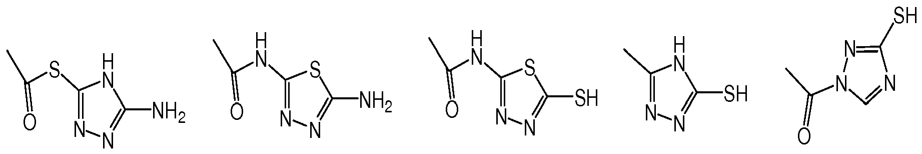 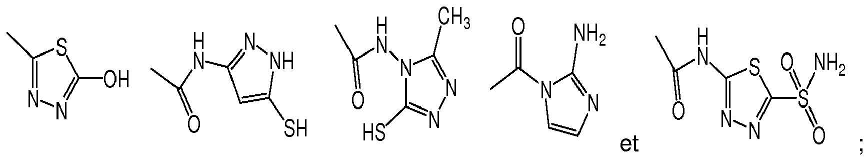
dans laquelle R³ est choisi dans le groupe constitué par l'hydrogène et un groupe méthyle ; dans laquelle l'un quelconque de R⁴ et R⁵ est un atome d'hydrogène et l'autre représente un groupe méthyle ; dans laquelle R⁶ et R⁷ sont indépendamment choisis dans le groupe constitué par un atome d'hydrogène, un méthyle, un éthyle, un propyle, un isopropyle, et un butyle ; dans laquelle R⁸ est choisi dans le groupe consistant en hydroxyle, amino, -N(CH₃)₂ et NHCH₃ ; et dans laquelle R⁹ est choisi dans le groupe constitué par un atome d'hydrogène, un halogénure, un C₁-C₅-alkyle, un C₂-C₅-alcényle, un C₂-C₅-alcynyle, un morpholinyle, un pipérazinyle, un C₁-C₅-alkyle pipérazinyle, un hétérocycloalkyle, un cyclopropyle, cyclobutyle, cyclopentyle, cyclohexyle, norbornane, adamantin, un C₅-C₆-cycloalcényle ; un groupe C₁-C₅-alkyle, C₂-C₅-alcényle, ou C₂-C₅-alcynyle éventuellement substitué par des groupements choisis parmi un hydroxyle, un amino, un C₁-C₅-alkyle ou C₂-C₅-alcényle avec un atome d'azote remplaçant un atome de carbone terminal dans la chaîne principale ou ramifiée, un halogénure, un groupe C₁-C₅-alkyle avec un atome de carbone remplacé par un atome d'oxygène, un groupe C₁-C₅-alkyle avec un carbonylamino ou un aminocarbonyle remplaçant deux atomes de carbone dans la chaîne principale ou ramifiée, carboxyle, amide, hydroxyamide, -CONHCH₃, -NHCONH₂, -SO₂NH₂, -SO₂CH₃, - NHCOCH₃, -NHCSNH₂, -NHSO₂CH₃, un radical de formule -OR¹² ou un radical de formule -C(O)-R¹², dans lequel R¹² est choisi dans le groupe constitué d'un C₁-C₅-alkyle, C₂-C₅-alcényle, un cyclopropyle, cyclobutyle, cyclopentyle, cyclohexyle, norbornane, adamantine, et C₅-C₆-cycloalcényle ; et leurs sels pharmaceutiquement acceptables.

12. Procédé selon la revendication 10, dans lequel le composé correspond à la structure chimique suivante: 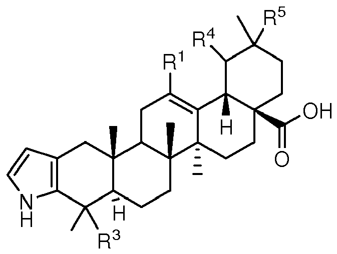 dans laquelle R¹ est choisi dans le groupe constitué par un atome d'hydrogène, un méthyle, un halogénure, un C₁-C₅-alkyle dans lequel un ou plusieurs atomes d'hydrogène liés à un atome du groupe C₁-C₅-alkyle sont remplacés par 1, 2, 3, ou 4 halogénure, un nitrile, un C₁-C₅-alkyle nitrile, un C1-C5-alkyle substitué ou non, un C₂-C₅-alcényle substitué ou non, un C₂-C₅-alcynyle substitué ou non, un cyclopropyle, cyclobutyle, cyclopentyle, cyclohexyle, norbornane, adamantine, un C₅-C₆-cycloalkényle, un C₆-C₁₂-aryle substitué ou non ; un pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyle, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées ou non, et leur formes partiellement réduites ; ou un hétéroaryle de 6 membres ou moins fusionné avec un groupe aryle de 6 membres ou moins, ou deux groupes distincts d'hétéroaryle de 6 membres ou moins fusionnés ; dans laquelle R³ est choisi dans le groupe constitué par l'hydrogène et un groupe méthyle ; et dans laquelle l'un quelconque de R⁴ et R⁵ est un atome d'hydrogène et l'autre représente un méthyle ; et leurs sels.

13. Procédé selon la revendication 10, dans lequel le composé correspond à la structure chimique suivante :

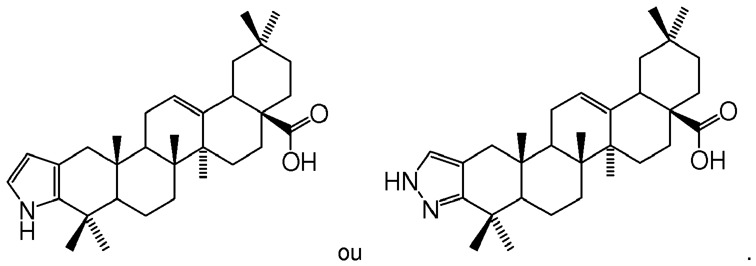

14. Composé pour une utilisation dans la réduction de la virulence des bactéries Gram-négatives, le composé correspondant à la structure chimique suivante: 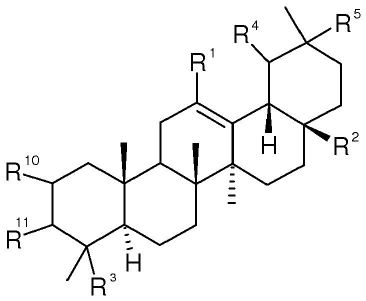
dans laquelle R¹ est choisi dans le groupe constitué par un atome d'hydrogène, un méthyle, un halogénure, un C₁-C₅-alkyle dans lequel un ou plusieurs atomes d'hydrogène liés à un atome du groupe C₁-C₅-alkyle en sont remplacés par 1, 2, 3 ou 4 atomes d'halogénure, un nitrile, un C₁-C₅-alkyle ou un C₂-C₅-alcényle avec un groupe nitrile remplaçant un atome de carbone terminal dans la chaîne principale ou ramifiée, un C₁-C₅-alkyle substitué ou non, un C₂-C₅-alcényle substitués ou non, un C₂-C₅-alcynyle substitué ou non, un cyclopropyle, un cyclobutyle, un cyclopentyle, un cyclohexyle, un norbornane, un adamantine, un C₅-C₆-cycloalcényle, un C₆-C₁₂-aryle substitué ; ou pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées ou non, et leur formes partiellement réduites ; ou un hétéroaryle de 6 membres ou moins fusionné avec un groupe aryle de 6 membres ou moins, ou deux groupes disctincts d'hétéroaryle de 6 membres ou moins fusionnés ; dans laquelle R² est choisi dans le groupe constitué par un carboxyle, un amide, un hydroxyamide, un méthylamide, -CH₂N(CH₃)₂, -CH₂NR⁶R⁷, 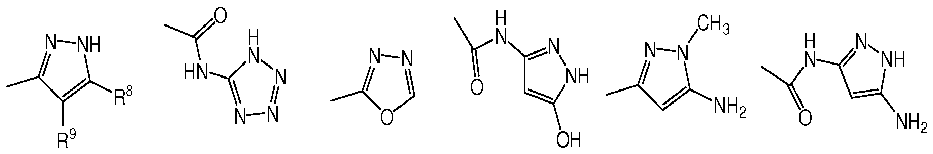 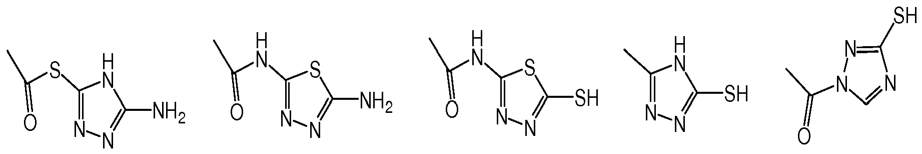 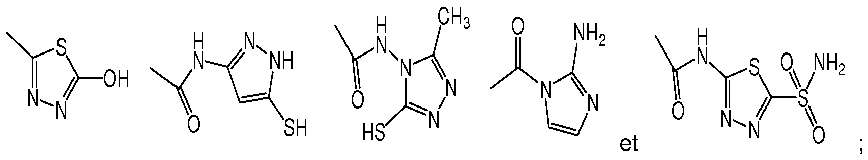
dans laquelle R³ est choisi dans le groupe constitué par un hydrogène et un méthyle ; dans laquelle l'un quelconque de R⁴ et R⁵ est un atome d'hydrogène et l'autre représente un méthyle ; dans laquelle R⁶ et R⁷ sont indépendamment choisis dans le groupe constitué par un hydrogène, un méthyle, un éthyle, un propyle, un isopropyle, et un butyle; dans laquelle R⁸ est choisi dans le groupe consistant en un hydroxyle, un amino, -N(CH₃)₂, et NHCH₃ ; dans laquelle R⁹ est choisi dans le groupe constitué par un hydrogène, un halogénure, un C₁-C₅-alkyle, un C₂-C₅-alcényle, un C₂-C₅-alcynyle, un morpholinyle, un pipérazinyle, un C₁-C₅-alkyle pipérazinyle, un hétérocycloalkyle, un cyclopropyle, un cyclobutyle, un cyclopentyle, un cyclohexyle, un norbornane, un adamantine, un C₅-C₆-cycloalcényle ; un C₁-C₅-alkyle, un C₂-C₅-alcényle, et un C₂-C₅-alcynyle éventuellement substitués par des groupements choisis parmi un hydroxyle, un amino, un C₁-C₅-alkyle ou un C₂-C₅-alcényle avec un atome d'azote remplaçant un atome de carbone terminal dans la chaîne principale ou ramifiée, un halogénure, un C₁-C₅-alkyle avec un atome de carbone remplacé par un atome d'oxygène, un C₁-C₅-alkyle avec un aminocarbonyle ou un carbonylamino remplaçant deux atomes de carbone dans la chaîne principale ou ramifiée, un carboxyle, un amide, un hydroxyamide, -CONHCH₃, -NHCONH₂, -SO₂NH₂, -SO₂CH₃, -NHCOCH₃, - NHCSNH₂, -NHSO₂CH₃, un radical de formule -OR¹² ou un radical de formule - C(O)-R¹², dans lequel R¹² est choisi dans le groupe constitué d'un C₁-C₅-alkyle, un C₂-C₅-alcényle, un cyclopropyle, un cyclobutyle, un cyclopentyle, un cyclohexyle, un norbornane, un adamantine, et un C₅-C₆-cycloalcényle; et dans laquelle R¹⁰ et R¹¹ avec les atomes de carbone auxquels ils sont liés forment un N-hétérocycle aromatique substitué ou non choisi dans le groupe constitué par l'indole, l'imidazole, la quinoléine, l'isoquinoléine, la pyridine, la triazine, la pyrazine, la pyrimidine, la pyridazine, l'isoindole, le pyrrole, le benzimidazole, la purine, le pyrazole, le benzopyrazole, l'indazole, la quinoxaline, l'acridine, la quinazoline, l'indolizine, le carbazole et la cinnoline ; et les sels pharmaceutiquement acceptables de ceux-ci.

15. Composé selon la revendication 14 pour une utilisation dans la réduction de la virulence des bactéries gram-négatives, dans lequel le composé correspond à la structure chimique suivante: 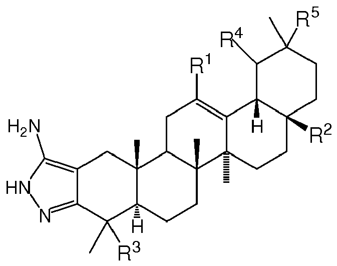
dans laquelle R¹ est choisi dans le groupe constitué par un hydrogène, un méthyle, un halogénure, un C₁-C₅-alkyle dans lequel un ou plusieurs atomes d'hydrogène liés à un atome du C₁-C₅-alkyle sont remplacés par 1, 2, 3, ou 4 halogénures, un nitrile, un C₁-C₅-nitrile d'alkyle, un C₁-C₅-alkyle substitué ou non, un C₂-C₅-alcényle substitués ou non, un C₂-C₅-alcynyle substitué ou non, un cyclopropyle, un cyclobutyle, un cyclopentyle, un cyclohexyle, un norbornane, un adamantine, un C₅-C₆-cycloalkényle, un C₆-C₁₂-aryle substitué ou non ; un pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées ou non, et leur formes partiellement réduites ; un hétéroaryle de 6 membres ou moins fusionné avec un groupe aryle de 6 membres ou moins, ou deux groupes disctincts d'hétéroaryle de 6 membres ou moins fusionnés ; dans laquelle R² est choisi dans le groupe constitué par un carboxyle, un amide, un hydroxyamide, un méthylamide, -CH₂N(CH₃)₂, -CH₂NR⁶R⁷, 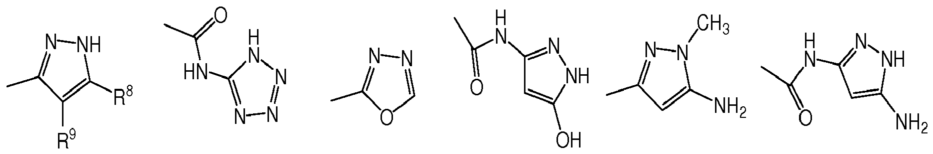 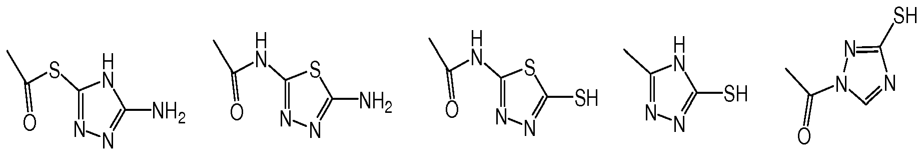 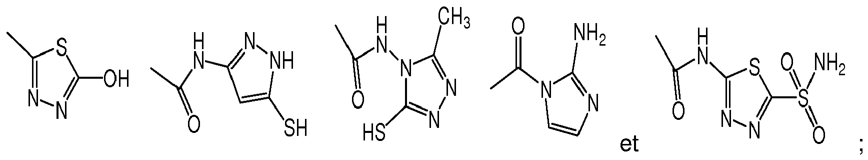
dans laquelle R³ est choisi dans le groupe constitué par un hydrogène et un méthyle ; dans laquelle l'un quelconque de R⁴ et R⁵ est un atome d'hydrogène et l'autre représente un méthyle ; dans laquelle R⁶ et R⁷ sont indépendamment choisis dans le groupe constitué par un hydrogène, un méthyle, un éthyle, un propyle, un isopropyle, et un butyle ; dans laquelle R⁸ est choisi dans le groupe consistant en un hydroxyle, un amino, -N(CH₃)₂, et -NHCH₃ ; et dans laquelle R⁹ est choisi dans le groupe constitué par un hydrogène, un halogénure, un C₁-C₅-alkyle, un C₂-C₅-alcényle, un C₂-C₅-alcynyle, un morpholinyle, un pipérazinyle, un C₁-C₅-alkyle pipérazinyle, un hétérocycloalkyle, un cyclopropyle, un cyclobutyle, un cyclopentyle, un cyclohexyle, un norbornane, un adamantine, un C₅-C₆-cycloalcényle ; un C₁-C₅-alkyle, un C₂-C₅-alcényle ou un C₂-C₅-alcynyle éventuellement substitués par des groupements choisis parmi un hydroxyle, un amino, un C₁-C₅-alkyle ou un C₂-C₅-alcényle avec un atome d'azote remplaçant un atome de carbone terminal dans la chaîne principale ou ramifiée, un halogénure, un C₁-C₅-alkyle avec un atome de carbone remplacé par un atome d'oxygène, un C₁-C₅-alkyle avec un aminocarbonyle ou un carbonylamino remplaçant deux atomes de carbone dans la chaîne principale ou ramifiée, un carboxyle, un amide, un hydroxyamide, -CONHCH₃, -NHCONH₂, -SO₂NH₂, -SO₂CH₃, -NHCOCH₃, - NHCSNH₂, -NHSO₂CH₃, un radical de formule -OR¹² ou un radical de formule - C(O)-R¹², dans lequel R¹² est choisi dans le groupe constitué d'un C₁-C₅-alkyle, un C₂-C₅-alcényle, un cyclopropyle, un cyclobutyle, un cyclopentyle, un cyclohexyle, un norbornane, un adamantine, et un C₅-C₆-cycloalcényle ; et leurs sels pharmaceutiquement acceptables.

16. Composé selon la revendication 14 pour son utilisation dans la réduction de la virulence des bactéries gram-négatives, dans lequel le composé correspond à la structure chimique suivante: 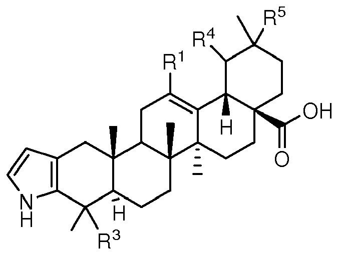 dans laquelle R¹ est choisi dans le groupe constitué par un atome d'hydrogène, un méthyle, un halogénure, un nitrile, un C₁-C₅-alkyle substitué ou non, un C₁-C₅-alcényle substitués ou non, un C₁-C₅-alcynyle substitués ou non, un C₆-C₁₂-aryle substitué ou non ; un pyrrolyle, pyrazolyle, imidazolyle, oxazolyle, isoxazolyle, oxadiazolyle, thiazolyle, isothiazolyle, thiényle, furanyle, furazanyle, pyridinyle, pyrimidinyle, pyridazinyle, indolyle, 3H -indolyle, isoindolyle, indolinyle, indolizinyle, indazolyle, dihydroindolyle, tetrahydroindolyl, purinyle, pyrazinyle, quinolinyle, isoquinolinyle, tétrahydroisoquinolinyle, quinoxalinyle, quinazolinyle, cinnolinyle, ptéridinyle, benzimidazolyle, benzopyranyle, benzoxazolyle, benzisoxazolyle, benzofuranyle, isobenzofuranyle, benzothiazolyle, benzisothiazolyle, benzothiényle, furopyridinyl, phtalazinyle, napthyridinyl, pyrazolopyridyl, pyrazolopyrimidinyle, leurs formes substituées ou non, et leur formes partiellement réduites ; ou un hétéroaryle de 6 membres ou moins fusionné avec un groupe aryle de 6 membres ou moins, ou deux groupes disctincts d'hétéroaryle de 6 membres ou moins fusionnés ; dans laquelle R³ est choisi dans le groupe constitué par un hydrogène et un méthyle; et dans laquelle l'un quelconque de R⁴ et R⁵ est un atome d'hydrogène et l'autre représente un méthyle; et leurs sels pharmaceutiquement acceptables.
